# EUROPEAN PATENT SPECIFICATION

(11) **EP 3 380 474 B1**
(45) Date of publication and mention of the grant of the patent: **21.08.2019**
(21) Application number: 16798788.2
(22) Date of filing: 23.11.2016
(51) Int. Cl.: C07D 487/04, A61K 31/519, A61P 11/06, A61P 29/00

(54) **JANUS KINASES INHIBITORS, COMPOSITIONS THEREOF AND USE THEREOF**
JANUS KINASE INHIBITOREN, DEREN ZUSAMMENSETZUNG UND IHRE ANWENDUNG
INHIBITEURS DE JANUS KINASES, COMPOSITIONS LES COMPRENANT ET LEURS UTILISATIONS

(30) Priority: 23.11.2015 WO PCT/CN2015/095310; 24.11.2015 WO PCT/CN2015/095423
(43) Date of publication of application: 03.10.2018
(73) Proprietor: F. Hoffmann-La Roche AG, 4070 Basel (CH)
(72) Inventor: CHENG, Yun-Xing, Beijing 100176 (CN); GIBBONS, Paul, South San Francisco, California 94080 (US); KELLAR, Terry, South San Francisco, California 94080 (US); LI, Wei, Beijing 100176 (CN); MENDONCA, Rohan, South San Francisco, California 94080 (US); YUEN, Po-wai, Beijing 100176 (CN); ZAK, Mark Edward, South San Francisco, California 94080 (US); ZHANG, Lei, Beijing 100176 (CN)
(74) Representative: Schirlin, Julien
(86) International application number: PCT/EP2016/078544
(87) International publication number: WO 2017/089390

(56) References cited:
- WO-A1-2010/051549
- WO-A2-2011/003065

## Description

### CROSS-REFERENCE TO RELATED APPLICATIONS

This application claims the benefit of International Application No. PCT/CN2015/095310, filed November 23, 2015, and International Application No. PCT/CN/2015/095423, filed November 24, 2015.

### FIELD OF THE INVENTION

The invention relates to compounds of Formula (00A), which are inhibitors of a Janus kinase, such as JAK1, as well as compositions containing these compounds, and methods of use including, but not limited to, diagnosis or treatment of patients suffering from a condition responsive to the inhibition of a JAK kinase.

### BACKGROUND OF INVENTION

Cytokine pathways mediate a broad range of biological functions, including many aspects of inflammation and immunity. Janus kinases (JAK), including JAK1, JAK2, JAK3 and TYK2, are cytoplasmic protein kinases that associate with type I and type II cytokine receptors and regulate cytokine signal transduction. Cytokine engagement with cognate receptors triggers activation of receptor associated JAKs and this leads to JAK-mediated tyrosine phosphorylation of signal transducer and activator of transcription (STAT) proteins and ultimately transcriptional activation of specific gene sets (Schindler et al., 2007, J. Biol. Chem. 282: 20059-63). JAK1, JAK2 and TYK2 exhibit broad patterns of gene expression, while JAK3 expression is limited to leukocytes. Cytokine receptors are typically functional as heterodimers, and as a result, more than one type of JAK kinase is usually associated with cytokine receptor complexes. The specific JAKs associated with different cytokine receptor complexes have been determined in many cases through genetic studies and corroborated by other experimental evidence. Exemplary therapeutic benefits of the inhibition of JAK enzymes are discussed, for example, in International Application No. WO 2013/014567.

JAK1 was initially identified in a screen for novel kinases (Wilks A.F., 1989, Proc. Natl. Acad. Sci. U.S.A. 86:1603-1607). Genetic and biochemical studies have shown that JAK1 is functionally and physically associated with the type I interferon (e.g., IFNalpha), type II interferon (e.g., IFNgamma), and IL-2 and IL-6 cytokine receptor complexes (Kisseleva et al., 2002, Gene 285:1-24; Levy et al., 2005, Nat. Rev. Mol. Cell Biol. 3:651-662; O'Shea et al., 2002, Cell, 109 (suppl.): S121-S131). JAK1 knockout mice die perinatally due to defects in LIF receptor signaling (Kisseleva et al., 2002, Gene 285:1-24; O'Shea et al., 2002, Cell, 109 (suppl.): S121-S131). Characterization of tissues derived from JAK1 knockout mice demonstrated critical roles for this kinase in the IFN, IL-10, IL-2/IL-4 and IL-6 pathways. A humanized monoclonal antibody targeting the IL-6 pathway (Tocilizumab) was approved by the European Commission for the treatment of moderate-to-severe rheumatoid arthritis (Scheinecker et al., 2009, Nat. Rev. Drug Discov. 8:273-274).

CD4 T cells play an important role in asthma pathogenesis through the production of TH2 cytokines within the lung, including IL-4, IL-9 and IL-13 (Cohn et al., 2004, Annu. Rev. Immunol. 22:789-815). IL-4 and IL-13 induce increased mucus production, recruitment of eosinophils to the lung, and increased production of IgE (Kasaian et al., 2008, Biochem. Pharmacol. 76(2): 147-155). IL-9 leads to mast cell activation, which exacerbates the asthma symptoms (Kearley et al., 2011, Am. J. Resp. Crit. Care Med., 183(7): 865-875). The IL-4Rα chain activates JAK1 and binds to either IL-4 or IL-13 when combined with the common gamma chain or the IL-13Rα1 chain respectively (Pernis et al., 2002, J. Clin. Invest. 109(10):1279-1283). The common gamma chain can also combine with IL-9Rα, to bind to IL-9, and IL-9Rα activates JAK1 as well (Demoulin et al., 1996, Mol. Cell Biol. 16(9):4710-4716). While the common gamma chain activates JAK3, it has been shown that JAK1 is dominant over JAK3, and inhibition of JAK1 is sufficient to inactivate signaling through the common gamma chain despite JAK3 activity (Haan et al., 2011, Chem. Biol. 18(3):314-323). Inhibition of IL-4, IL-13 and IL-9 signaling by blocking the JAK/STAT signaling pathway can alleviate asthmatic symptoms in pre-clinical lung inflammation models (Mathew et al., 2001, J. Exp. Med. 193(9): 1087-1096; Kudlacz et. al., 2008, Eur. J. Pharmacol. 582(1-3): 154-161).

Biochemical and genetic studies have shown an association between JAK2 and single-chain (e.g., EPO), IL-3 and interferon gamma cytokine receptor families (Kisseleva et al., 2002, Gene 285:1-24; Levy et al., 2005, Nat. Rev. Mol. Cell Biol. 3:651-662; O'Shea et al., 2002, Cell, 109 (suppl.): S121-S131). Consistent with this, JAK2 knockout mice die of anemia (O'Shea et al., 2002, Cell, 109 (suppl.): S121-S131). Kinase activating mutations in JAK2 (e.g., JAK2 V617F) are associated with myeloproliferative disorders in humans.

JAK3 associates exclusively with the gamma common cytokine receptor chain, which is present in the IL-2, IL-4, IL-7, IL-9, IL-15 and IL-21 cytokine receptor complexes. JAK3 is critical for lymphoid cell development and proliferation and mutations in JAK3 result in severe combined immunodeficiency (SCID) (O'Shea et al., 2002, Cell, 109 (suppl.): S121-S131). Based on its role in regulating lymphocytes, JAK3 and JAK3-mediated pathways have been targeted for immunosuppressive indications (e.g., transplantation rejection and rheumatoid arthritis) (Baslund et al., 2005, Arthritis & Rheumatism 52:2686-2692; Changelian et al., 2003, Science 302: 875-878).

TYK2 associates with the type I interferon (e.g., IFNalpha), IL-6, IL-10, IL-12 and IL-23 cytokine receptor complexes (Kisseleva et al., 2002, Gene 285:1-24; Watford, W.T. & O'Shea, J.J., 2006, Immunity 25:695-697). Consistent with this, primary cells derived from a TYK2 deficient human are defective in type I interferon, IL-6, IL-10, IL-12 and IL-23 signaling. A fully human monoclonal antibody targeting the shared p40 subunit of the IL-12 and IL-23 cytokines (Ustekinumab) was recently approved by the European Commission for the treatment of moderate-to-severe plaque psoriasis (Krueger et al., 2007, N. Engl. J. Med. 356:580-92; Reich et al., 2009, Nat. Rev. Drug Discov. 8:355-356). In addition, an antibody targeting the IL-12 and IL-23 pathways underwent clinical trials for treating Crohn's Disease (Mannon et al., 2004, N. Engl. J. Med. 351:2069-79).

International Patent Application Publication Number WO 2011/003065 discusses certain pyrazolopyrimidine compounds that are reported to useful as inhibitors of one or more Janus kinases. Data for certain specific compounds showing inhibition of JAK1, JAK2, JAK3, and TYK2 kinases is presented therein.

Currently there remains a need for additional compounds that are inhibitors of Janus kinases. For example, there is a need for compounds that possess useful potency as inhibitors of one or more Janus kinases (e.g., JAK1) in combination with other pharmacological properties that are necessary to achieve a useful therapeutic benefit. For example, there is a need for potent compounds that demonstrate selectivity for one Janus kinase over other kinases in general (e.g., selectivity for JAK1 over other kinases such as leucine-rich repeat kinase 2 (LRRK2)). There is also a need for potent compounds that demonstrate selectivity for one Janus kinase over other Janus kinases (e.g., selectivity for JAK1 over other Janus kinases). Kinases demonstrating selectivity for JAK1 could provide a therapeutic benefit, with fewer side effects, in conditions responsive to the inhibition of JAK1. Additionally there is currently a need for potent JAK1 inhibitors that possess other properties (e.g., melting point, pK, solubility, etc.) necessary for formulation and administration by inhalation. Such compounds would be particularly useful for treating conditions such as, for example, asthma.

There exists a need in the art for additional or alternative treatments of conditions mediated by JAK kinases, such as those described above.

### SUMMARY OF INVENTION

Provided herein are pyrazolopyrimidines that inhibit JAK1 kinase.

Accordingly, one embodiment provides a compound of Formula (00A): 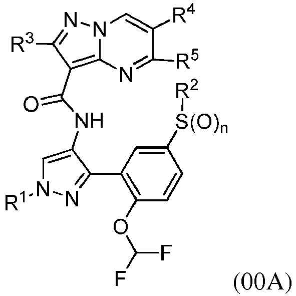 or a salt thereof, wherein:
R¹ is H, C₁-C₆alkyl, C₂-C₆alkenyl, C₂-C₆alkynyl, -(C₀-C₃alkyl)CN, -(C₀-C₃alkyl)OR^{a}, - (C₀-C₃alkyl)R^{a}, -(C₀-C₃alkyl)SR^{a}, -(C₀-C₃alkyl)NR^{a}R^{b}, -(C₀-C₃alkyl)OCF₃, -(C₀-C₃alkyl)CF₃, -(C₀-C₃alkyl)NO₂, -(C₀-C₃alkyl)C(O)R^{a}, -(C₀-C₃alkyl)C(O)OR^{a}, -(C₀-C₃alkyl)C(O)NR^{a}R^{b}, - (C₀-C₃alkyl)NR^{a}C(O)R^{b}, -(C₀-C₃alkyl)S(O)₁₋₂R^{a}, -(C₀-C₃alkyl)NR^{a}S(O)₁₋₂R^{b}, -(C₀-C₃alkyl)S(O)₁₋₂NR^{a}R^{b}, -(C₀-C₃alkyl)(5-6-membered heteroaryl) or -(C₀-C₃alkyl)phenyl, wherein R¹ is optionally substituted by one or more groups independently selected from the group consisting of halogen, C₁-C₃alkyl, oxo, -CF₃, -(C₀-C₃alkyl)OR^{c} and -(C₀-C₃alkyl)NR^{c}R^{d};
R^{a} is independently hydrogen, C₁-C₆alkyl, C₃-C₆ cycloalkyl, 3-10 membered heterocyclyl, 5-6 membered heteroaryl, -C(O)R^{c}, -C(O)OR^{c}, -C(O)NR^{c}R^{d}, -NR^{c}C(O)R^{d}, - S(O)₁₋₂R^{c}, -NR^{c}S(O)₁₋₂R^{d} or -S(O)₁₋₂NR^{c}R^{d}, wherein any C₃-C₆ cycloalkyl, 3-10 membered heterocyclyl, and 5-6 membered heteroaryl of R^{a} is optionally substituted with one or more groups R^{e};
R^{b} is independently hydrogen or C₁-C₃alkyl, wherein said alkyl is optionally substituted by one or more groups independently selected from the group consisting of halogen and oxo; or
R^{c} and R^{d} are independently selected from the group consisting of hydrogen, 3-6 membered heterocyclyl, C₃-C₆ cycloalkyl, and C₁-C₃alkyl, wherein any 3-6 membered heterocyclyl, C₃-C₆ cycloalkyl, and C₁-C₃alkyl of R^{c} and R^{d} is optionally substituted by one or more groups independently selected from the group consisting of halogen and oxo; or R^{c} and R^{d} are taken together with the atom to which they are attached to form a 3-6-membered heterocyclyl, optionally substituted by one or more groups independently selected from the group consisting of halogen, oxo, -CF₃ and C₁-C₃alkyl;
each R^{e} is independently selected from the group consisting of oxo, OR^{f}, NR^{f}R^{g}, halogen, 3-10 membered heterocyclyl, C₃-C₆ cycloalkyl, and C₁-C₆alkyl, wherein any C₃-C₆ cycloalkyl and C₁-C₆alkyl of R^{e} is optionally substituted by one or more groups independently selected from the group consisting of OR^{f}, NR^{f}R^{g}, halogen, 3-10 membered heterocyclyl, oxo, and cyano, and wherein any 3-10 membered heterocyclyl of R^{e} is optionally substituted by one or more groups independently selected from the group consisting of halogen, oxo, cyano, -CF₃, NR^{h}R^{k}, 3-6 membered heterocyclyl, and C₁-C₃alkyl that is optionally substituted by one or more groups independently selected from the group consisting of halogen, oxo, OR^{f}, and NR^{h}R^{k};
R^{f} and R^{g} are each independently selected from the group consisting of hydrogen, C₁-C₆alkyl, 3-6 membered heterocyclyl, and C₃-C₆ cycloalkyl, wherein any C₁-C₆alkyl, 3-6 membered heterocyclyl, and C₃-C₆ cycloalkyl of R^{f} and R^{g} is optionally substituted by one or more R^{m};
each R^{m} is independently selected from the group consisting of halogen, cyano, oxo, C₃-C₆cycloalkyl, 3-6 membered heterocyclyl, hydroxy, and NR^{h}R^{k}, wherein any C₃-C₆cycloalkyl and 3-6 membered heterocyclyl of R^{m} is optionally substituted with one or more groups independently selected from the group consisting of halogen, oxo, cyano, and C₁-C₃alkyl;
R^{h} and R^{k} are each independently selected from the group consisting of hydrogen and C₁-C₆alkyl that is optionally substituted by one or more groups independently selected from the group consisting of halogen, cyano, 3-6 membered heterocyclyl, and oxo; or R^{h} and R^{k} are taken together with the atom to which they are attached to form a 3-6-membered heterocyclyl that is optionally substituted by one or more groups independently selected from the group consisting of halogen, cyano, oxo, -CF₃ and C₁-C₃alkyl that is optionally substituted by one or more groups independently selected from the group consisting of halogen and oxo;
R² is C₁-C₆alkyl, C₂-C₆ alkenyl, C₂-C₆ alkynyl, C₃-C₆ cycloalkyl, 3-6-membered heterocyclyl, (C₃-C₆ cycloalkyl)C₁-C₆alkyl, (3-6-membered heterocyclyl)C₁-C₆alkyl, -C(O)(C₃-C₆ cycloalkyl), or -C(O)(3-6-membered heterocyclyl), wherein R² is optionally substituted with one or more halo;
n is 0, 1, or 2;
R³ is H or NH₂;
R⁴ is H or CH₃; and
R⁵ is H or NH₂.

Also provided is a pharmaceutical composition comprising, a compound of Formula (00A) or a pharmaceutically acceptable salt thereof, and a pharmaceutically acceptable carrier, dilient or excipient.

Also provided is a compound of Formula (00A) or a pharmaceutically acceptable salt thereof for use in therapy, such as in the treatment of an inflammatory disease (e.g., asthma). Also provided is the use of a compound of Formula (00A) or a pharmaceutically acceptable salt thereof for the preparation of a medicament for the treatment of an inflammatory disease. Also disclosed is a method of preventing, treating or lessening the severity of a disease or condition responsive to the inhibition of a Janus kinase activity in a patient, comprising administering to the patient a therapeutically effective amount of a compound of Formula (00A) or a pharmaceutically acceptable salt thereof.

Certain compounds of Formula (00A) possess beneficial potency as inhibitors of one or more Janus kinases (e.g., JAK1). Certain compounds are also a) selective for one Janus kinase over other kinases, b) selective for JAK1 over other Janus kinases, and/or c) possess other properties (e.g., melting point, pK, solubility, etc.) necessary for formulation and administration by inhalation. Certain compounds of Formula (00A) may be particularly useful for treating conditions such as asthma.

### DESCRIPTION OF THE DRAWINGS

The foregoing aspects and many of the attendant advantages will become more readily appreciated as the same become better understood by reference to the following detailed description, when taken in conjunction with the accompanying drawings, where:

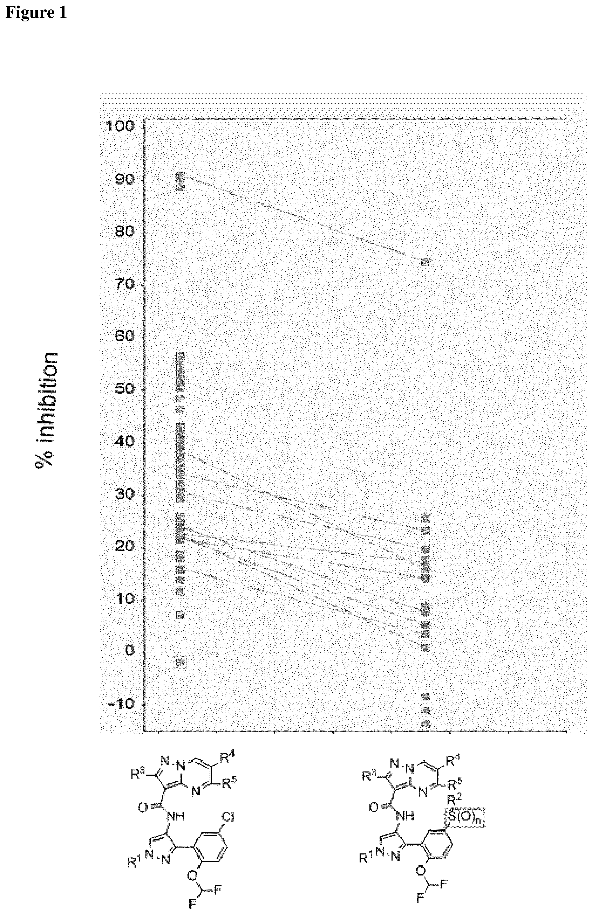

Figure 1 illustrates a matched pair analysis of certain compounds of the present invention (points on the right) and corresponding compounds wherein the group -S(O)ₙ-R² is replaced with Cl (points on the left).

### DETAILED DESCRIPTION OF THE INVENTION

### DEFINITIONS

"Halogen" or "halo" refers to F, Cl, Br or I. Additionally, terms such as "haloalkyl," are meant to include monohaloalkyl and polyhaloalkyl.

The term "alkyl" refers to a saturated linear or branched-chain monovalent hydrocarbon radical, wherein the alkyl radical may be optionally substituted. In one example, the alkyl radical is one to eighteen carbon atoms (C₁-C₁₈). In other examples, the alkyl radical is C₀-C₆, C₀-C₅, C₀-C₃, C₁-C₁₂, C₁-C₁₀, C₁-C₈, C₁-C₆, C₁-C₅, C₁-C₄, or C₁-C₃. C₀ alkyl refers to a bond. Examples of alkyl groups include methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, - CH₂CH₂CH₂CH₃), 2-methyl-1-propyl (i-Bu, i-butyl, -CH₂CH(CH₃)₂), 2-butyl (s-Bu, s-butyl, - CH(CH₃)CH₂CH₃), 2-methyl-2-propyl (t-Bu, t-butyl, -C(CH₃)₃), 1-pentyl (n-pentyl, - CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, 1-heptyl and 1-octyl. In some embodiments, substituents for "optionally substituted alkyls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list.

The term "alkenyl" refers to linear or branched-chain monovalent hydrocarbon radical with at least one site of unsaturation, i.e., a carbon-carbon double bond, wherein the alkenyl radical may be optionally substituted, and includes radicals having "cis" and "trans" orientations, or alternatively, "E" and "Z" orientations. In one example, the alkenyl radical is two to eighteen carbon atoms (C₂-C₁₈). In other examples, the alkenyl radical is C₂-C₁₂, C₂-C₁₀, C₂-C₈, C₂-C₆ or C₂-C₃. Examples include, but are not limited to, ethenyl or vinyl (-CH=CH₂), prop-1-enyl (-CH=CHCH₃), prop-2-enyl (-CH₂CH=CH₂), 2-methylprop-1-enyl, but-1-enyl, but-2-enyl, but-3-enyl, buta-1,3-dienyl, 2-methylbuta-1,3-diene, hex-1-enyl, hex-2-enyl, hex-3-enyl, hex-4-enyl and hexa-1,3-dienyl. In some embodiments, substituents for "optionally substituted alkenyls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list.

The term "alkynyl" refers to a linear or branched monovalent hydrocarbon radical with at least one site of unsaturation, i.e., a carbon-carbon, triple bond, wherein the alkynyl radical may be optionally substituted. In one example, the alkynyl radical is two to eighteen carbon atoms (C₂-C₁₈). In other examples, the alkynyl radical is C₂-C₁₂, C₂-C₁₀, C₂-C₈, C₂-C₆ or C₂-C₃. Examples include, but are not limited to, ethynyl (-C≡CH), prop-1-ynyl (-C≡CCH₃), prop-2-ynyl (propargyl, -CH₂C≡CH), but-1-ynyl, but-2-ynyl and but-3-ynyl. In some embodiments, substituents for "optionally substituted alkynyls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list.

"Alkylene" refers to a saturated, branched or straight chain hydrocarbon group having two monovalent radical centers derived by the removal of two hydrogen atoms from the same or two different carbon atoms of a parent alkane. In one example, the divalent alkylene group is one to eighteen carbon atoms (C₁-C₁₈). In other examples, the divalent alkylene group is C₀-C₆, C₀-C₅, C₀-C₃, C₁-C₁₂, C₁-C₁₀, C₁-C₈, C₁-C₆, C₁-C₅, C₁-C₄, or C₁-C₃. The group C₀ alkylene refers to a bond. Example alkylene groups include methylene (-CH₂-), 1,1-ethyl (-CH(CH₃)-), (1,2-ethyl (-CH₂CH₂-), 1,1-propyl (-CH(CH₂CH₃)-), 2,2-propyl (-C(CH₃)₂-), 1,2-propyl (-CH(CH₃)CH₂-), 1,3-propyl (-CH₂CH₂CH₂-), 1,1-dimethyleth-1,2-yl (-C(CH₃)₂CH₂-), 1,4-butyl (-CH₂CH₂CH₂CH₂-), and the like.

The term "heteroalkyl" refers to a straight or branched chain monovalent hydrocarbon radical, consisting of the stated number of carbon atoms, or, if none are stated, up to 18 carbon atoms, and from one to five heteroatoms selected from the group consisting of O, N, Si and S, and wherein the nitrogen and sulfur atoms can optionally be oxidized and the nitrogen heteroatom can optionally be quaternized. In some embodiments, the heteroatom is selected from O, N and S, wherein the nitrogen and sulfur atoms can optionally be oxidized and the nitrogen heteroatom can optionally be quaternized. The heteroatom(s) can be placed at any interior position of the heteroalkyl group, including the position at which the alkyl group is attached to the remainder of the molecule (e.g., -O-CH₂-CH₃). Examples include -CH₂-CH₂-O-CH₃, -CH₂-CH₂-NH-CH₃, -CH₂-CH₂-N(CH₃)-CH₃, -CH₂-S-CH₂-CH₃, -S(O)-CH₃, -CH₂-CH₂-S(O)₂-CH₃, - Si(CH₃)₃ and -CH₂-CH=N-OCH₃. Up to two heteroatoms can be consecutive, such as, for example, -CH₂-NH-OCH₃ and -CH₂-O-Si(CH₃)₃. Heteroalkyl groups can be optionally substituted. In some embodiments, substituents for "optionally substituted heteroalkyls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list.

"Amino" means primary (i.e., -NH₂), secondary (i.e., -NRH), tertiary (i.e., -NRR) and quaternary (i.e., -N(+)RRR) amines, that are optionally substituted, in which each R is the same or different and selected from alkyl, cycloalkyl, aryl, and heterocyclyl, wherein the alkyl, cycloalkyl, aryl and heterocyclyl groups are as defined herein. Particular secondary and tertiary amines are alkylamine, dialkylamine, arylamine, diarylamine, aralkylamine and diaralkylamine, wherein the alkyl and aryl portions can be optionally substituted. Particular secondary and tertiary amines are methylamine, ethylamine, propylamine, isopropylamine, phenylamine, benzylamine, dimethylamine, diethylamine, dipropylamine and diisopropylamine. In some embodiments, R groups of a quarternary amine are each independently optionally substituted alkyl groups.

"Aryl" refers to a carbocyclic aromatic group, whether or not fused to one or more groups, having the number of carbon atoms designated, or if no number is designated, up to 14 carbon atoms. One example includes aryl groups having 6-14 carbon atoms. Another example includes aryl groups having 6-10 carbon atoms. Examples of aryl groups include phenyl, naphthyl, biphenyl, phenanthrenyl, naphthacenyl, 1,2,3,4-tetrahydronaphthalenyl, 1H-indenyl, 2,3-dihydro-1H-indenyl, and the like (see, e.g., Lang's Handbook of Chemistry (Dean, J. A., ed.) 13th ed. Table 7-2 [1985]). A particular aryl is phenyl. Substituted phenyl or substituted aryl means a phenyl group or aryl group substituted with one, two, three, four or five substituents, for example, 1-2, 1-3 or 1-4 substituents, such as chosen from groups specified herein (see "optionally substituted" definition), such as F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list. Examples of the term "substituted phenyl" include a mono- or di(halo)phenyl group such as 2-chlorophenyl, 2-bromophenyl, 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl, 2,4-difluorophenyl and the like; a mono- or di(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(alkyl)phenyl group such as 4-methylphenyl, 2,4-dimethylphenyl, 2-methylphenyl, 4-(isopropyl)phenyl, 4-ethylphenyl, 3-(n-propyl)phenyl and the like; a mono or di(alkoxy)phenyl group, for example, 3,4-dimethoxyphenyl, 3-methoxy-4-benzyloxyphenyl, 3-ethoxyphenyl, 4-(isopropoxy)phenyl, 4-(t-butoxy)phenyl, 3-ethoxy-4-methoxyphenyl and the like; 3- or 4- trifluoromethylphenyl; a mono- or dicarboxyphenyl or (protected carboxy)phenyl group such 4-carboxyphenyl, a mono- or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or (protected aminomethyl)phenyl such as 2-(aminomethyl)phenyl or 2,4-(protected aminomethyl)phenyl; or a mono- or di(N-(methylsulfonylamino))phenyl such as 3-(N-methylsulfonylamino))phenyl. Also, the term "substituted phenyl" represents disubstituted phenyl groups where the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl, 2-chloro-5-difluoromethoxy and the like, as well as trisubstituted phenyl groups where the substituents are different, for example 3-methoxy-4-benzyloxy-6-methyl sulfonylamino, 3-methoxy-4-benzyloxy-6-phenyl sulfonylamino, and tetrasubstituted phenyl groups where the substituents are different such as 3-methoxy-4-benzyloxy-5-methyl-6-phenyl sulfonylamino. In some embodiments, a substituent of an aryl, such as phenyl, comprises an amide. For example, an aryl (e.g., phenyl) substituent may be -(CH₂)₀₋₄CONR'R", wherein R' and R" each independently refer to groups including, for example, hydrogen; unsubstituted C₁-C₆alkyl; C₁-C₆alkyl substituted by halogen, OH, CN, unsubstituted C₁-C₆alkyl, unsubstituted C₁-C₆ alkoxy, oxo or NR'R"; unsubstituted C₁-C₆ heteroalkyl; C₁-C₆ heteroalkyl substituted by halogen, OH, CN, unsubstituted C₁-C₆alkyl, unsubstituted C₁-C₆ alkoxy, oxo or NR'R"; unsubstituted C₆-C₁₀ aryl; C₆-C₁₀ aryl substituted by halogen, OH, CN, unsubstituted C₁-C₆alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆alkyl, unsubstituted C₁-C₆ alkoxy, oxo or NR'R"; or R' and R" can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆alkyl, unsubstituted C₁-C₆ alkoxy, oxo or NR'R".

"Cycloalkyl" refers to a non-aromatic, saturated or partially unsaturated hydrocarbon ring group wherein the cycloalkyl group may be optionally substituted independently with one or more substituents described herein. In one example, the cycloalkyl group is 3 to 12 carbon atoms (C₃-C₁₂). In other examples, cycloalkyl is C₃-C₈, C₃-C₁₀ or C₅-C₁₀. In other examples, the cycloalkyl group, as a monocycle, is C₃-C₈, C₃-C₆ or C₅-C₆. In another example, the cycloalkyl group, as a bicycle, is C₇-C₁₂. In another example, the cycloalkyl group, as a spiro system, is C₅-C₁₂. Examples of monocyclic cycloalkyl include cyclopropyl, cyclobutyl, cyclopentyl, 1-cyclopent-1-enyl, 1-cyclopent-2-enyl, 1-cyclopent-3-enyl, cyclohexyl, perdeuteriocyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl and cyclododecyl. Exemplary arrangements of bicyclic cycloalkyls having 7 to 12 ring atoms include, but are not limited to, [4,4], [4,5], [5,5], [5,6] or [6,6] ring systems. Exemplary bridged bicyclic cycloalkyls include, but are not limited to, bicyclo[2.2.1]heptane, bicyclo[2.2.2]octane and bicyclo[3.2.2]nonane. Examples of spiro cycloalkyl include, spiro[2.2]pentane, spiro[2.3]hexane, spiro[2.4]heptane, spiro[2.5]octane and spiro[4.5]decane. In some embodiments, substituents for "optionally substituted cycloalkyls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, aryl and heterocyclic portions thereof may be optionally substituted, such as by one to four instances of substituents selected from this same list. In some embodiments, a substituent of a cycloalkyl comprises an amide. For example, a cycloalkyl substituent may be -(CH₂)₀₋₄CONR'R", wherein R' and R" each independently refer to groups including, for example, hydrogen; unsubstituted C₁₋C₆alkyl; C₁-C₆alkyl substituted by halogen, OH, CN, unsubstituted C₁-C₆alkyl, unsubstituted C₁-C₆ alkoxy, oxo or NR'R"; unsubstituted C₁-C₆ heteroalkyl; C₁-C₆ heteroalkyl substituted by halogen, OH, CN, unsubstituted C₁-C₆alkyl, unsubstituted C₁-C₆ alkoxy, oxo or NR'R"; unsubstituted C₆-C₁₀ aryl; C₆-C₁₀ aryl substituted by halogen, OH, CN, unsubstituted C₁-C₆alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆alkyl, unsubstituted C₁-C₆ alkoxy, oxo or NR'R"; or R' and R" can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆alkyl, unsubstituted C₁-C₆ alkoxy, oxo or NR'R".

The terms "compound(s) of the invention," and "compound(s) of the present invention" and the like, unless otherwise indicated, include compounds of Formulae (00A), (00B), (00C), (00D), (00E), (00F), and (00G), and the compounds of the Examples herein, including stereoisomers (including atropisomers), geometric isomers, tautomers, solvates, metabolites, isotopes, salts (e.g., pharmaceutically acceptable salts), and prodrugs thereof. In some embodiments, solvates, metabolites, isotopes or prodrugs are excluded, or any combination thereof.

"Guanidine" or "guanidinyl" means the group -NH-C(NH)-NHR in which R is hydrogen, alkyl, cycloalkyl, aryl or heterocyclyl, wherein the alkyl, cycloalkyl, aryl and heterocyclyl groups are as defined herein. A particular guanidine is the group -NH-C(NH)-NH₂.

"Heterocyclic group", "heterocyclic", "heterocycle", "heterocyclyl", or "heterocyclo" are used interchangeably and refer to any mono-, bi-, tricyclic or spiro, saturated or unsaturated, aromatic (heteroaryl) or non-aromatic (e.g., heterocycloalkyl), ring system, having 3 to 20 ring atoms (e.g., 3-10 ring atoms), where the ring atoms are carbon, and at least one atom in the ring or ring system is a heteroatom selected from nitrogen, sulfur or oxygen. If any ring atom of a cyclic system is a heteroatom, that system is a heterocycle, regardless of the point of attachment of the cyclic system to the rest of the molecule. In one example, heterocyclyl includes 3-11 ring atoms ("members") and includes monocycles, bicycles, tricycles and spiro ring systems, wherein the ring atoms are carbon, where at least one atom in the ring or ring system is a heteroatom selected from nitrogen, sulfur or oxygen. In one example, heterocyclyl includes 1 to 4 heteroatoms. In one example, heterocyclyl includes 1 to 3 heteroatoms. In another example, heterocyclyl includes 3- to 7-membered monocycles having 1-2, 1-3 or 1-4 heteroatoms selected from nitrogen, sulfur or oxygen. In another example, heterocyclyl includes 4- to 6-membered monocycles having 1-2, 1-3 or 1-4 heteroatoms selected from nitrogen, sulfur or oxygen. In another example, heterocyclyl includes 3-membered monocycles. In another example, heterocyclyl includes 4-membered monocycles. In another example, heterocyclyl includes 5-6 membered monocycles, e.g., 5-6 membered heteroaryl. In another example, heterocyclyl includes 3-11 membered heterocycloyalkyls, such as 4-11 membered heterocycloalkyls. In some embodiments, a heterocycloalkyl includes at least one nitrogen. In one example, the heterocyclyl group includes 0 to 3 double bonds. Any nitrogen or sulfur heteroatom may optionally be oxidized (e.g., NO, SO, SO₂), and any nitrogen heteroatom may optionally be quaternized (e.g., [NR₄]⁺Cl⁻, [NR₄]⁺OH⁻). Example heterocycles are oxiranyl, aziridinyl, thiiranyl, azetidinyl, oxetanyl, thietanyl, 1,2-dithietanyl, 1,3-dithietanyl, pyrrolidinyl, dihydro-1H-pyrrolyl, dihydrofuranyl, tetrahydrofuranyl, dihydrothienyl, tetrahydrothienyl, imidazolidinyl, piperidinyl, piperazinyl, isoquinolinyl, tetrahydroisoquinolinyl, morpholinyl, thiomorpholinyl, 1,1-dioxo-thiomorpholinyl, dihydropyranyl, tetrahydropyranyl, hexahydrothiopyranyl, hexahydropyrimidinyl, oxazinanyl, thiazinanyl, thioxanyl, homopiperazinyl, homopiperidinyl, azepanyl, oxepanyl, thiepanyl, oxazepinyl, oxazepanyl, diazepanyl, 1,4-diazepanyl, diazepinyl, thiazepinyl, thiazepanyl, tetrahydrothiopyranyl, oxazolidinyl, thiazolidinyl, isothiazolidinyl, 1,1-dioxoisothiazolidinonyl, oxazolidinonyl, imidazolidinonyl, 4,5,6,7-tetrahydro[2H]indazolyl, tetrahydrobenzoimidazolyl, 4,5,6,7-tetrahydrobenzo[d]imidazolyl, 1,6-dihydroimidazol[4,5-d]pyrrolo[2,3-b]pyridinyl, thiazinyl, oxazinyl, thiadiazinyl, oxadiazinyl, dithiazinyl, dioxazinyl, oxathiazinyl, thiatriazinyl, oxatriazinyl, dithiadiazinyl, imidazolinyl, dihydropyrimidyl, tetrahydropyrimidyl, 1-pyrrolinyl, 2-pyrrolinyl, 3-pyrrolinyl, indolinyl, thiapyranyl, 2H-pyranyl, 4H-pyranyl, dioxanyl, 1,3-dioxolanyl, pyrazolinyl, pyrazolidinyl, dithianyl, dithiolanyl, pyrimidinonyl, pyrimidindionyl, pyrimidin-2,4-dionyl, piperazinonyl, piperazindionyl, pyrazolidinylimidazolinyl, 3-azabicyclo[3.1.0]hexanyl, 3,6-diazabicyclo[3.1.1]heptanyl, 6-azabicyclo[3.1.1]heptanyl, 3-azabicyclo[3.1.1]heptanyl, 3-azabicyclo[4.1.0]heptanyl, azabicyclo[2.2.2]hexanyl, 2-azabicyclo[3.2.1]octanyl, 8-azabicyclo[3.2.1]octanyl, 2-azabicyclo[2.2.2]octanyl, 8-azabicyclo[2.2.2]octanyl, 7-oxabicyclo[2.2.1]heptane, azaspiro[3.5]nonanyl, azaspiro[2.5]octanyl, azaspiro[4.5]decanyl, 1-azaspiro[4.5]decan-2-only, azaspiro[5.5]undecanyl, tetrahydroindolyl, octahydroindolyl, tetrahydroisoindolyl, tetrahydroindazolyl, 1,1-dioxohexahydrothiopyranyl. Examples of 5-membered heterocycles containing a sulfur or oxygen atom and one to three nitrogen atoms are thiazolyl, including thiazol-2-yl and thiazol-2-yl N-oxide, thiadiazolyl, including 1,3,4-thiadiazol-5-yl and 1,2,4-thiadiazol-5-yl, oxazolyl, for example oxazol-2-yl, and oxadiazolyl, such as 1,3,4-oxadiazol-5-yl, and 1,2,4-oxadiazol-5-yl. Example 5-membered ring heterocycles containing 2 to 4 nitrogen atoms include imidazolyl, such as imidazol-2-yl; triazolyl, such as 1,3,4-triazol-5-yl; 1,2,3-triazol-5-yl, 1,2,4-triazol-5-yl, and tetrazolyl, such as 1H-tetrazol-5-yl. Example benzo-fused 5-membered heterocycles are benzoxazol-2-yl, benzthiazol-2-yl and benzimidazol-2-yl. Example 6-membered heterocycles contain one to three nitrogen atoms and optionally a sulfur or oxygen atom, for example pyridyl, such as pyrid-2-yl, pyrid-3-yl, and pyrid-4-yl; pyrimidyl, such as pyrimid-2-yl and pyrimid-4-yl; triazinyl, such as 1,3,4-triazin-2-yl and 1,3,5-triazin-4-yl; pyridazinyl, in particular pyridazin-3-yl, and pyrazinyl. The pyridine N-oxides and pyridazine N-oxides and the pyridyl, pyrimid-2-yl, pyrimid-4-yl, pyridazinyl and the 1,3,4-triazin-2-yl groups, are other example heterocycle groups. Heterocycles may be optionally substituted. For example, substituents for "optionally substituted heterocycles" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, oxo, trifluoromethyl, difluoromethyl, sulfonylamino, methanesulfonylamino, SO, SO₂, phenyl, piperidinyl, piperizinyl, and pyrimidinyl, wherein the alkyl, aryl and heterocyclic portions thereof may be optionally substituted, such as by one to four instances of substituents selected from this same list. In some embodiments, a substituent of a heterocyclic group, such as a heteroaryl or heterocycloalkyl, comprises an amide. For example, a heterocyclic (e.g., heteroaryl or heterocycloalkyl) substituent may be -(CH₂)₀₋₄CONR'R", wherein R' and R" each independently refer to groups including, for example, hydrogen; unsubstituted C₁₋C₆alkyl; C₁₋C₆alkyl substituted by halogen, OH, CN, unsubstituted C₁-C₆alkyl, unsubstituted C₁-C₆ alkoxy, oxo or NR'R"; unsubstituted C₁-C₆ heteroalkyl; C₁-C₆ heteroalkyl substituted by halogen, OH, CN, unsubstituted C₁-C₆alkyl, unsubstituted C₁-C₆ alkoxy, oxo or NR'R"; unsubstituted C₆-C₁₀ aryl; C₆-C₁₀ aryl substituted by halogen, OH, CN, unsubstituted C₁-C₆alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆alkyl, unsubstituted C₁-C₆ alkoxy, oxo or NR'R"; or R' and R" can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆alkyl, unsubstituted C₁-C₆ alkoxy, oxo or NR'R".

"Heteroaryl" refers to any mono-, bi-, or tricyclic ring system where at least one ring is a 5- or 6-membered aromatic ring containing from 1 to 4 heteroatoms selected from nitrogen, oxygen, and sulfur, and in an example embodiment, at least one heteroatom is nitrogen. See, for example, Lang's Handbook of Chemistry (Dean, J. A., ed.) 13^{th} ed. Table 7-2 [1985]. Included in the definition are any bicyclic groups where any of the above heteroaryl rings are fused to an aryl ring, wherein the aryl ring or the heteroaryl ring is joined to the remainder of the molecule. In one embodiment, heteroaryl includes 5-6 membered monocyclic aromatic groups where one or more ring atoms is nitrogen, sulfur or oxygen. Example heteroaryl groups include thienyl, furyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, triazinyl, tetrazinyl, tetrazolo[1,5-b]pyridazinyl, imidazol[1,2-a]pyrimidinyl and purinyl, as well as benzo-fused derivatives, for example benzoxazolyl, benzofuryl, benzothiazolyl, benzothiadiazolyl, benzotriazolyl, benzoimidazolyl and indolyl. Heteroaryl groups can be optionally substituted. In some embodiments, substituents for "optionally substituted heteroaryls" include one to four instances of F, Cl, Br, I, OH, SH, CN, NH₂, NHCH₃, N(CH₃)₂, NO₂, N₃, C(O)CH₃, COOH, CO₂CH₃, methyl, ethyl, propyl, iso-propyl, butyl, isobutyl, cyclopropyl, methoxy, ethoxy, propoxy, trifluoromethyl, difluoromethyl, sulfonylamino, methanesulfonylamino, SO, SO₂, phenyl, piperidinyl, piperizinyl, and pyrimidinyl, wherein the alkyl, phenyl and heterocyclic portions thereof may be optionally substituted, such as by one to four instances of substituents selected from this same list. In some embodiments, a substituent of a heteroaryl comprises an amide. For example, a heteroaryl substituent may be -(CH₂)₀₋₄CONR'R", wherein R' and R" each independently refer to groups including, for example, hydrogen; unsubstituted C₁₋C₆alkyl; C₁-C₆alkyl substituted by halogen, OH, CN, unsubstituted C₁-C₆alkyl, unsubstituted C₁-C₆ alkoxy, oxo or NR'R"; unsubstituted C₁-C₆ heteroalkyl; C₁-C₆ heteroalkyl substituted by halogen, OH, CN, unsubstituted C₁-C₆alkyl, unsubstituted C₁-C₆ alkoxy, oxo or NR'R"; unsubstituted C₆-C₁₀ aryl; C₆-C₁₀ aryl substituted by halogen, OH, CN, unsubstituted C₁-C₆alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆alkyl, unsubstituted C₁-C₆ alkoxy, oxo or NR'R"; or R' and R" can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆alkyl, unsubstituted C₁-C₆ alkoxy, oxo or NR'R".

In particular embodiments, a heterocyclyl group is attached at a carbon atom of the heterocyclyl group. By way of example, carbon bonded heterocyclyl groups include bonding arrangements at position 2, 3, 4, 5, or 6 of a pyridine ring, position 3, 4, 5, or 6 of a pyridazine ring, position 2, 4, 5, or 6 of a pyrimidine ring, position 2, 3, 5, or 6 of a pyrazine ring, position 2, 3, 4, or 5 of a furan, tetrahydrofuran, thiofuran, thiophene, pyrrole or tetrahydropyrrole ring, position 2, 4, or 5 of an oxazole, imidazole or thiazole ring, position 3, 4, or 5 of an isoxazole, pyrazole, or isothiazole ring, position 2 or 3 of an aziridine ring, position 2, 3, or 4 of an azetidine ring, position 2, 3, 4, 5, 6, 7, or 8 of a quinoline ring or position 1, 3, 4, 5, 6, 7, or 8 of an isoquinoline ring.

In certain embodiments, the heterocyclyl group is N-attached. By way of example, nitrogen bonded heterocyclyl or heteroaryl groups include bonding arrangements at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline.

The term "alkoxy" refers to a linear or branched monovalent radical represented by the formula -OR in which R is alkyl, as defined herein. Alkoxy groups include methoxy, ethoxy, propoxy, isopropoxy, mono-, di- and tri-fluoromethoxy and cyclopropoxy.

"Acyl" means a carbonyl containing substituent represented by the formula -C(O)-R in which R is hydrogen, alkyl, cycloalkyl, aryl or heterocyclyl, wherein the alkyl, cycloalkyl, aryl and heterocyclyl are as defined herein. Acyl groups include alkanoyl (e.g., acetyl), aroyl (e.g., benzoyl), and heteroaroyl (e.g., pyridinoyl).

"Optionally substituted" unless otherwise specified means that a group may be unsubstituted or substituted by one or more (e.g., 0, 1, 2, 3, 4, or 5 or more, or any range derivable therein) of the substituents listed for that group in which said substituents may be the same or different. In an embodiment, an optionally substituted group has 1 substituent. In another embodiment an optionally substituted group has 2 substituents. In another embodiment an optionally substituted group has 3 substituents. In another embodiment an optionally substituted group has 4 substituents. In another embodiment an optionally substituted group has 5 substituents.

Optional substituents for alkyl radicals, alone or as part of another substituent (e.g., alkoxy), as well as alkylenyl, alkenyl, alkynyl, heteroalkyl, heterocycloalkyl, and cycloalkyl, also each alone or as part of another substituent, can be a variety of groups, such as those described herein, as well as selected from the group consisting of halogen; oxo; CN; NO; N₃; -OR'; perfluoro-C₁-C₄ alkoxy; unsubstituted C₃-C₇ cycloalkyl; C₃-C₇ cycloalkyl substituted by halogen, OH, CN, unsubstituted C₁-C₆alkyl, unsubstituted C₁-C₆ alkoxy, oxo or NR'R"; unsubstituted C₆-C₁₀ aryl (e.g., phenyl); C₆-C₁₀ aryl substituted by halogen, OH, CN, unsubstituted C₁-C₆alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆alkyl, unsubstituted C₁-C₆ alkoxy, oxo or NR'R"; -NR'R"; -SR'; -SiR'R"R"'; -OC(O)R'; -C(O)R'; -CO₂R'; -CONR'R"; -OC(O)NR'R"; -NR"C(O)R'; -NR"'C(O)NR'R"; -NR"C(O)₂R'; -S(O)₂R'; -S(O)₂NR'R"; -NR'S(O)₂R"; -NR"'S(O)₂NR'R"; amidinyl; guanidinyl; -(CH₂)₁₋₄-OR'; -(CH₂)₁₋₄-NR'R"; -(CH2)₁₋₄-SR'; -(CH₂)₁₋₄-SiR'R"R"'; -(CH₂)₁₋₄-OC(O)R'; -(CH₂)₁₋₄-C(O)R'; -(CH₂)_{1- 4}-CO₂R'; and O-(CH₂)₁₋₄CONR'R", or combinations thereof, in a number ranging from zero to (2m'+1), where m' is the total number of carbon atoms in such radical. R', R" and R'" each independently refer to groups including, for example, hydrogen; unsubstituted C₁₋C₆alkyl; C₁₋C₆alkyl substituted by halogen, OH, CN, unsubstituted C₁-C₆alkyl, unsubstituted C₁-C₆ alkoxy, oxo or NR'R"; unsubstituted C₁-C₆ heteroalkyl; C₁-C₆ heteroalkyl substituted by halogen, OH, CN, unsubstituted C₁-C₆alkyl, unsubstituted C₁-C₆ alkoxy, oxo or NR'R"; unsubstituted C₆-C₁₀ aryl; C₆-C₁₀ aryl substituted by halogen, OH, CN, unsubstituted C₁-C₆alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆alkyl, unsubstituted C₁-C₆ alkoxy, oxo or NR'R". When R' and R" are attached to the same nitrogen atom, they can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆alkyl, unsubstituted C₁-C₆ alkoxy, oxo or NR'R". For example, -NR'R" is meant to include 1-pyrrolidinyl and 4-morpholinyl.

Similarly, optional substituents for the aryl and heteroaryl groups are varied. In some embodiments, substituents for aryl and heteroaryl groups are selected from the group consisting of halogen; CN; NO; N₃; -OR'; perfluoro-C₁-C₄ alkoxy; unsubstituted C₃-C₇ cycloalkyl; C₃-C₇ cycloalkyl substituted by halogen, OH, CN, unsubstituted C₁-C₆alkyl, unsubstituted C₁-C₆ alkoxy, oxo or NR'R"; unsubstituted C₆-C₁₀ aryl (e.g., phenyl); C₆-C₁₀ aryl substituted by halogen, OH, CN, unsubstituted C₁-C₆alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆alkyl, unsubstituted C₁-C₆ alkoxy, oxo or NR'R"; -NR'R"; -SR'; -SiR'R"R"'; -OC(O)R'; -C(O)R'; -CO₂R'; -CONR'R"; -OC(O)NR'R"; -NR"C(O)R'; -NR"'C(O)NR'R"; -NR"C(O)₂R'; -S(O)₂R'; -S(O)₂NR'R"; -NR'S(O)₂R"; -NR"'S(O)₂NR'R"; amidinyl; guanidinyl; -(CH₂)₁₋₄-OR'; -(CH₂)₁₋₄-NR'R"; -(CH₂)₁₋₄-SR'; -(CH₂)₁₋₄-SiR'R"R"'; -(CH₂)₁₋₄-OC(O)R'; -(CH₂)₁₋₄-C(O)R'; -(CH₂)₁₋₄-CO₂R'; and -(CH₂)₁₋₄CONR'R", or combinations thereof, in a number ranging from zero to (2m'+1), where m' is the total number of carbon atoms in such radical. R', R" and R'" each independently refer to groups including, for example, hydrogen; unsubstituted C₁₋C₆alkyl; C₁-C₆alkyl substituted by halogen, OH, CN, unsubstituted C₁-C₆alkyl, unsubstituted C₁-C₆ alkoxy, oxo or NR'R"; unsubstituted C₁₋C₆ heteroalkyl; C₁-C₆ heteroalkyl substituted by halogen, OH, CN, unsubstituted C₁-C₆alkyl, unsubstituted C₁-C₆ alkoxy, oxo or NR'R"; unsubstituted C₆-C₁₀ aryl; C₆-C₁₀ aryl substituted by halogen, OH, CN, unsubstituted C₁-C₆alkyl, unsubstituted C₁-C₆ alkoxy, or NR'R"; unsubstituted 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S); and 3-11 membered heterocyclyl (e.g., 5-6 membered heteroaryl containing 1 to 4 heteroatoms selected from O, N and S or 4-11 membered heterocycloalkyl containing 1 to 4 heteroatoms selected from O, N and S) substituted by halogen, OH, CN, unsubstituted C₁-C₆alkyl, unsubstituted C₁-C₆ alkoxy, oxo or NR'R". When R' and R" are attached to the same nitrogen atom, they can be combined with the nitrogen atom to form a 3-, 4-, 5-, 6-, or 7-membered ring wherein a ring atom is optionally substituted with N, O or S and wherein the ring is optionally substituted with halogen, OH, CN, unsubstituted C₁-C₆alkyl, unsubstituted C₁-C₆ alkoxy, oxo or NR'R". For example, -NR'R" is meant to include 1-pyrrolidinyl and 4-morpholinyl.

The term "oxo" refers to =O or (=O)₂.

As used herein a wavy line " 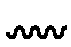 " that intersects a bond in a chemical structure indicate the point of attachment of the atom to which the wavy bond is connected in the chemical structure to the remainder of a molecule, or to the remainder of a fragment of a molecule. In some embodiments, an arrow together with an asterisk is used in the manner of a wavy line to indicate a point of attachment.

In certain embodiments, divalent groups are described generically without specific bonding configurations. It is understood that the generic description is meant to include both bonding configurations, unless specified otherwise. For example, in the group R¹-R²-R³, if the group R² is described as -CH₂C(O)-, then it is understood that this group can be bonded both as R¹-CH₂C(O)-R³, and as R¹-C(O)CH₂-R ³, unless specified otherwise.

The phrase "pharmaceutically acceptable" refers to molecular entities and compositions that do not produce an adverse, allergic or other untoward reaction when administered to an animal, such as, for example, a human, as appropriate.

Compounds of the present invention may be in the form of a salt, such as a pharmaceutically acceptable salt. "Pharmaceutically acceptable salts" include both acid and base addition salts. "Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, carbonic acid, phosphoric acid and the like, and organic acids may be selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, benzenesulfonic acid, p-toluenesulfonic acid, salicyclic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Particular base addition salts are the ammonium, potassium, sodium, calcium and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particular organic non-toxic bases include isopropylamine, diethylamine, ethanolamine, tromethamine, dicyclohexylamine, choline, and caffeine.

In some embodiments, a salt is selected from a hydrochloride, hydrobromide, trifluoroacetate, sulphate, phosphate, acetate, fumarate, maleate, tartrate, lactate, citrate, pyruvate, succinate, oxalate, methanesulphonate, p-toluenesulphonate, bisulphate, benzenesulphonate, ethanesulphonate, malonate, xinafoate, ascorbate, oleate, nicotinate, saccharinate, adipate, formate, glycolate, palmitate, L-lactate, D-lactate, aspartate, malate, L-tartrate, D-tartrate, stearate, furoate (e.g., 2-furoate or 3-furoate), napadisylate (naphthalene-1,5-disulfonate or naphthalene-1-(sulfonic acid)-5-sulfonate), edisylate (ethane-1,2-disulfonate or ethane-1-(sulfonic acid)-2-sulfonate), isethionate (2-hydroxyethylsulfonate), 2-mesitylenesulphonate, 2-naphthalenesulphonate, 2,5-dichlorobenzenesulphonate, D-mandelate, L-mandelate, cinnamate, benzoate, adipate, esylate, malonate, mesitylate (2-mesitylenesulphonate), napsylate (2-naphthalenesulfonate), camsylate (camphor- 10-sulphonate, for example (1S)-(+)-10-camphorsulfonic acid salt), glutamate, glutarate, hippurate (2-(benzoylamino)acetate), orotate, xylate (p-xylene-2-sulphonate), and pamoic (2,2'-dihydroxy-1,1'-dinaphthylmethane-3,3'-dicarboxylate).

A "sterile" formulation is aseptic or free from all living microorganisms and their spores.

"Stereoisomers" refer to compounds that have identical chemical constitution, but differ with regard to the arrangement of the atoms or groups in space. Stereoisomers include diastereomers, enantiomers, conformers and the like.

"Chiral" refers to molecules which have the property of non-superimposability of the mirror image partner, while the term "achiral" refers to molecules which are superimposable on their mirror image partner.

"Diastereomer" refers to a stereoisomer with two or more centers of chirality and whose molecules are not mirror images of one another. Diastereomers have different physical properties, e.g., melting points, boiling points, spectral properties or biological activities. Mixtures of diastereomers may separate under high resolution analytical procedures such as electrophoresis and chromatography such as HPLC.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

Certain compounds of the present invention can exist in unsolvated forms as well as solvated forms, including hydrated forms. A "solvate" refers to an association or complex of one or more solvent molecules and a compound of the present invention. Examples of solvents that form solvates include water, isopropanol, ethanol, methanol, DMSO, ethyl acetate, acetic acid, and ethanolamine. Certain compounds of the present invention can exist in multiple crystalline or amorphous forms. In general, all physical forms are intended to be within the scope of the present invention. The term "hydrate" refers to the complex where the solvent molecule is water.

A "metabolite" refers to a product produced through metabolism in the body of a specified compound or salt thereof. Such products can result, for example, from the oxidation, reduction, hydrolysis, amidation, deamidation, esterification, deesterification, enzymatic cleavage, and the like, of the administered compound.

Metabolite products typically are identified by preparing a radiolabelled (e.g., ¹⁴C or ³H) isotope of a compound of the invention, administering it in a detectable dose (e.g., greater than about 0.5 mg/kg) to an animal such as rat, mouse, guinea pig, monkey, or to a human, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, e.g., by MS, LC/MS or NMR analysis. In general, analysis of metabolites is done in the same way as conventional drug metabolism studies well known to those skilled in the art. The metabolite products, so long as they are not otherwise found in vivo, are useful in diagnostic assays for therapeutic dosing of the compounds of the invention.

"Amino-protecting group" as used herein refers to a derivative of the groups commonly employed to block or protect an amino group while reactions are carried out on other functional groups on the compound. Examples of such protecting groups include carbamates, amides, alkyl and aryl groups, and imines, as well as many N-heteroatom derivatives which can be removed to regenerate the desired amine group. Particular amino protecting groups are Pmb (p-Methoxybenzyl), Boc (tert-Butyloxycarbonyl), Fmoc (9-Fluorenylmethyloxycarbonyl) and Cbz (Carbobenzyloxy). Further examples of these groups are found in T. W. Greene and P. G. M. Wuts, "Protecting Groups in Organic Synthesis, 3rd ed., John Wiley & Sons, Inc., 1999. The term "protected amino" refers to an amino group substituted with one of the above amino-protecting groups.

"Carboxy-protecting group" as used herein refers to those groups that are stable to the conditions of subsequent reaction(s) at other positions of the molecule, which may be removed at the appropriate point without disrupting the remainder of the molecule, to give the unprotected carboxy-group. Examples of carboxy protecting groups include, ester groups and heterocyclyl groups. Ester derivatives of the carboxylic acid group may be employed to block or protect the carboxylic acid group while reactions are carried out on other functional groups on the compound. Examples of such ester groups include substituted arylalkyl, including substituted benzyls, such as 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, alkyl or substituted alkyl esters such as methyl, ethyl, t-butyl allyl or t-amyl, triphenylmethyl (trityl), 4-methoxytrityl, 4,4'-dimethoxytrityl, 4,4',4"-trimethoxytrityl, 2-phenylprop-2-yl, thioesters such as t-butyl thioester, silyl esters such as trimethylsilyl, t-butyldimethylsilyl esters, phenacyl, 2,2,2-trichloroethyl, beta-(trimethylsilyl)ethyl, beta-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. Another example of carboxy-protecting groups are heterocyclyl groups such as 1,3-oxazolinyl. Further examples of these groups are found in T. W. Greene and P. G. M. Wuts, "Protecting Groups in Organic Synthesis, 3rd ed., John Wiley & Sons, Inc., 1999. The term "protected carboxy" refers to a carboxy group substituted with one of the above carboxy-protecting groups.

"Hydroxy-protecting group" as used herein refers to a derivative of the hydroxy group commonly employed to block or protect the hydroxy group while reactions are carried out on other functional groups on the compound. Examples of such protecting groups include tetrahydropyranyloxy, benzoyl, acetoxy, carbamoyloxy, benzyl, and silylethers (e.g., TBS, TBDPS) groups. Further examples of these groups are found in T. W. Greene and P. G. M. Wuts, "Protecting Groups in Organic Synthesis, 3rd ed., John Wiley & Sons, Inc., 1999. The term "protected hydroxy" refers to a hydroxy group substituted with one of the above hydroxy-protecting groups.

A "subject," "individual," or "patient" is a vertebrate. In certain embodiments, the vertebrate is a mammal. Mammals include, but are not limited to, farm animals (such as cows), sport animals, pets (such as guinea pigs, cats, dogs, rabbits and horses), primates, mice and rats. In certain embodiments, a mammal is a human. In embodiments comprising administration of a compound of Formula (00A) or a pharmaceutically acceptable salt thereof to a patient, the patient may be in need thereof.

The term "Janus kinase" refers to JAK1, JAK2, JAK3 and TYK2 protein kinases. In some embodiments, a Janus kinase may be further defined as one of JAK1, JAK2, JAK3 or TYK2. In any embodiment, any one of JAK1, JAK2, JAK3 and TYK2 may be specifically excluded as a Janus kinase. In some embodiments, a Janus kinase is JAK1. In some embodiments, a Janus kinase is a combination of JAK1 and JAK2.

The terms "inhibiting" and "reducing," or any variation of these terms, includes any measurable decrease or complete inhibition to achieve a desired result. For example, there may be a decrease of about, at most about, or at least about 5%, 10%, 15%, 20%, 25%, 30%, 35%, 40%, 45%, 50%, 55%, 60%, 65%, 70%, 75%, 80%, 85%, 90%, 95%, 99%, or more, or any range derivable therein, reduction of activity (e.g., JAK1 activity) compared to normal.

In some embodiments, a compound of Formula (00A) is selective for inhibition of JAK1 over JAK3 and TYK2. In some embodiments, a compound of Formula (00A) is selective for inhibition of JAK1 over JAK2, JAK3, or TYK2, or any combination of JAK2, JAK3, or TYK2. In some embodiments, a compound of Formula (00A) is selective for inhibition of JAK1 and JAK2 over JAK3 and TYK2. In some embodiments, a compound of Formula (00A) is selective for inhibition of JAK1 over JAK3. By "selective for inhibition" it is meant that the compound is at least a 5%, 10%, 15%, 20%, 25%, 30%, 35%, 40%, 45%, 50%, 55%, 60%, 65%, 70%, 75%, 80%, 85%, 90%, 95%, 99%, or more, or any range derivable therein, better inhibitor of a particular Janus kinase (e.g., JAK1) activity compared to another particular Janus kinase (e.g., JAK3) activity, or is at least a 2-, 3-, 4-, 5-, 10-, 25-, 50-, 100-, 250-, or 500-fold better inhibitor of a particular Janus kinase (e.g., JAK1) activity compared to another particular Janus kinase (e.g., JAK3) activity.

"Therapeutically effective amount" means an amount of a compound of the present invention, such as a compound of Formula (00A) that (i) treats or prevents the particular disease, condition or disorder, or (ii) attenuates, ameliorates or eliminates one or more symptoms of the particular disease, condition, or disorder, and optionally (iii) prevents or delays the onset of one or more symptoms of the particular disease, condition or disorder described herein. In some embodiments, the therapeutically effective amount is an amount sufficient to decrease or alleviate the symptoms of an autoimmune or inflammatory disease (e.g., asthma). In some embodiments, a therapeutically effective amount is an amount of a chemical entity described herein sufficient to significantly decrease the activity or number of B-cells. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; or relieve to some extent one or more of the symptoms associated with the cancer. To the extent the drug may prevent growth or kill existing cancer cells, it may be cytostatic or cytotoxic. For cancer therapy, efficacy can, for example, be measured by assessing the time to disease progression (TTP) or determining the response rate (RR).

"Treatment" (and variations such as "treat" or "treating") refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, stabilized (i.e., not worsening) state of disease, decreasing the rate of disease progression, amelioration or palliation of the disease state, prolonging survival as compared to expected survival if not receiving treatment and remission or improved prognosis. In some embodiments, compounds of the invention, such as a compound of Formula (00A) are used to delay development of a disease or disorder or to slow the progression of a disease or disorder. Those in need of treatment include those already with the condition or disorder as well as those prone to have the condition or disorder, (for example, through a genetic mutation) or those in which the condition or disorder is to be prevented.

"Inflammatory disorder" refers to any disease, disorder or syndrome in which an excessive or unregulated inflammatory response leads to excessive inflammatory symptoms, host tissue damage, or loss of tissue function. "Inflammatory disorder" also refers to a pathological state mediated by influx of leukocytes or neutrophil chemotaxis.

"Inflammation" refers to a localized, protective response elicited by injury or destruction of tissues, which serves to destroy, dilute, or wall off (sequester) both the injurious agent and the injured tissue. Inflammation is notably associated with influx of leukocytes or neutrophil chemotaxis. Inflammation can result from infection with pathogenic organisms and viruses and from noninfectious means such as trauma or reperfusion following myocardial infarction or stroke, immune responses to foreign antigens, and autoimmune responses. Accordingly, inflammatory disorders amenable to treatment with a compound of the present invention, such as a compound of Formula (00A) encompass disorders associated with reactions of the specific defense system as well as with reactions of the nonspecific defense system.

"Specific defense system" refers to the component of the immune system that reacts to the presence of specific antigens. Examples of inflammation resulting from a response of the specific defense system include the classical response to foreign antigens, autoimmune diseases, and delayed type hypersensitivity responses mediated by T-cells. Chronic inflammatory diseases, the rejection of solid transplanted tissue and organs, e.g., kidney and bone marrow transplants, and graft versus host disease (GVHD), are further examples of inflammatory reactions of the specific defense system.

The term "nonspecific defense system" refers to inflammatory disorders that are mediated by leukocytes that are incapable of immunological memory (e.g., granulocytes, and macrophages). Examples of inflammation that result, at least in part, from a reaction of the nonspecific defense system include inflammation associated with conditions such as adult (acute) respiratory distress syndrome (ARDS) or multiple organ injury syndromes; reperfusion injury; acute glomerulonephritis; reactive arthritis; dermatoses with acute inflammatory components; acute purulent meningitis or other central nervous system inflammatory disorders such as stroke; thermal injury; inflammatory bowel disease; granulocyte transfusion associated syndromes; and cytokine-induced toxicity.

"Autoimmune disease" refers to any group of disorders in which tissue injury is associated with humoral or cell-mediated responses to the body's own constituents. Non-limiting examples of autoimmune diseases include rheumatoid arthritis, lupus and multiple sclerosis.

"Allergic disease" as used herein refers to any symptoms, tissue damage, or loss of tissue function resulting from allergy. "Arthritic disease" as used herein refers to any disease that is characterized by inflammatory lesions of the joints attributable to a variety of etiologies. "Dermatitis" as used herein refers to any of a large family of diseases of the skin that are characterized by inflammation of the skin attributable to a variety of etiologies. "Transplant rejection" as used herein refers to any immune reaction directed against grafted tissue, such as organs or cells (e.g., bone marrow), characterized by a loss of function of the grafted and surrounding tissues, pain, swelling, leukocytosis, and thrombocytopenia. The therapeutic methods of the present invention include methods for the treatment of disorders associated with inflammatory cell activation.

"Inflammatory cell activation" refers to the induction by a stimulus (including, but not limited to, cytokines, antigens or auto-antibodies) of a proliferative cellular response, the production of soluble mediators (including but not limited to cytokines, oxygen radicals, enzymes, prostanoids, or vasoactive amines), or cell surface expression of new or increased numbers of mediators (including, but not limited to, major histocompatability antigens or cell adhesion molecules) in inflammatory cells (including but not limited to monocytes, macrophages, T lymphocytes, B lymphocytes, granulocytes (i.e., polymorphonuclear leukocytes such as neutrophils, basophils, and eosinophils), mast cells, dendritic cells, Langerhans cells, and endothelial cells). It will be appreciated by persons skilled in the art that the activation of one or a combination of these phenotypes in these cells can contribute to the initiation, perpetuation, or exacerbation of an inflammatory disorder.

In some embodiments, inflammatory disorders which can be treated according to the methods of this invention include, but are not limited to, asthma, rhinitis (e.g., allergic rhinitis), allergic airway syndrome, atopic dermatitis, bronchitis, rheumatoid arthritis, psoriasis, contact dermatitis, chronic obstructive pulmonary disease and delayed hypersensitivity reactions.

The terms "cancer" and "cancerous", "neoplasm", and "tumor" and related terms refer to or describe the physiological condition in mammals that is typically characterized by unregulated cell growth. A "tumor" comprises one or more cancerous cells. Examples of cancer include carcinoma, blastoma, sarcoma, seminoma, glioblastoma, melanoma, leukemia, and myeloid or lymphoid malignancies. More particular examples of such cancers include squamous cell cancer (e.g., epithelial squamous cell cancer) and lung cancer including small-cell lung cancer, non-small cell lung cancer ("NSCLC"), adenocarcinoma of the lung and squamous carcinoma of the lung. Other cancers include skin, keratoacanthoma, follicular carcinoma, hairy cell leukemia, buccal cavity, pharynx (oral), lip, tongue, mouth, salivary gland, esophageal, larynx, hepatocellular, gastric, stomach, gastrointestinal, small intestine, large intestine, pancreatic, cervical, ovarian, liver, bladder, hepatoma, breast, colon, rectal, colorectal, genitourinary, biliary passage, thyroid, papillary, hepatic, endometrial, uterine, salivary gland, kidney or renal, prostate, testis, vulval, peritoneum, anal, penile, bone, multiple myeloma, B-cell lymphoma, central nervous system, brain, head and neck, Hodgkin's, and associated metastases. Examples of neoplastic disorders include myeloproliferative disorders, such as polycythemia vera, essential thrombocytosis, myelofibrosis, such as primary myelofibrosis, and chronic myelogenous leukemia (CML).

A "chemotherapeutic agent" is an agent useful in the treatment of a given disorder, for example, cancer or inflammatory disorders. Examples of chemotherapeutic agents are well-known in the art and include examples such as those disclosed in U.S. Publ. Appl. No. 2010/0048557, incorporated herein by reference. Additionally, chemotherapeutic agents include pharmaceutically acceptable salts, acids or derivatives of any of chemotherapeutic agents, as well as combinations of two or more of them.

"Package insert" is used to refer to instructions customarily included in commercial packages of therapeutic products that contain information about the indications, usage, dosage, administration, contraindications or warnings concerning the use of such therapeutic products.

Unless otherwise stated, structures depicted herein include compounds that differ only in the presence of one or more isotopically enriched atoms. Exemplary isotopes that can be incorporated into compounds of the present invention, such as a compound of Formula (00A) include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I, and ¹²⁵I, respectively. Isotopically-labeled compounds (e.g., those labeled with ³H and ¹⁴C) can be useful in compound or substrate tissue distribution assays. Tritiated (i.e., ³H) and carbon-14 (i.e., ¹⁴C) isotopes can be useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements). In some embodiments, in compounds of Formula (00A) one or more hydrogen atoms are replaced by ²H or ³H, or one or more carbon atoms are replaced by ¹³C- or ¹⁴C-enriched carbon. Positron emitting isotopes such as ¹⁵O, ¹³N, ¹¹C, and ¹⁸F are useful for positron emission tomography (PET) studies to examine substrate receptor occupancy. Isotopically labeled compounds can generally be prepared by procedures analogous to those disclosed in the Schemes or in the Examples herein, by substituting an isotopically labeled reagent for a non-isotopically labeled reagent.

It is specifically contemplated that any limitation discussed with respect to one embodiment of the invention may apply to any other embodiment of the invention. Furthermore, any compound or composition of the invention may be used in any method of the invention, and any method of the invention may be used to produce or to utilize any compound or composition of the invention.

The use of the term "or" is used to mean "and/or" unless explicitly indicated to refer to alternatives only or the alternative are mutually exclusive, although the disclosure supports a definition that refers to only alternatives and "and/or."

Throughout this application, the term "about" is used to indicate that a value includes the standard deviation of error for the device or method being employed to determine the value.

As used herein, "a" or "an" means one or more, unless clearly indicated otherwise. As used herein, "another" means at least a second or more.

Headings used herein are intended only for organizational purposes.

### INHIBITORS OF JANUS KINASES

One embodiment provides a compound of Formula (00A) or a salt thereof.

In one embodiment R¹ is H, C₁-C₆alkyl, -(C₀-C₃alkyl)R^{a}, -(C₀-C₃alkyl)NR^{a}R^{b}, -(C₀-C₃alkyl)C(O)R^{a}, or -(C₀-C₃alkyl)C(O)OR^{a}.

In one embodiment R¹ is H.

In one embodiment R¹ is C₁-C₆alkyl.

In one embodiment R¹ is -(C₀-C₃alkyl)R^{a}.

In one embodiment R¹ is -(C₀-C₃alkyl)NR^{a}R^{b}.

In one embodiment R¹ is -(C₀-C₃alkyl)C(O)R^{a}.

In one embodiment R¹ is -(C₀-C₃alkyl)C(O)OR^{a}.

In one embodiment R¹ is selected from the group consisting of H, methyl,

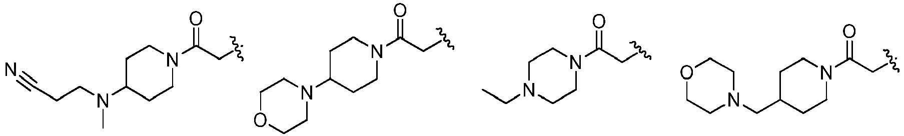

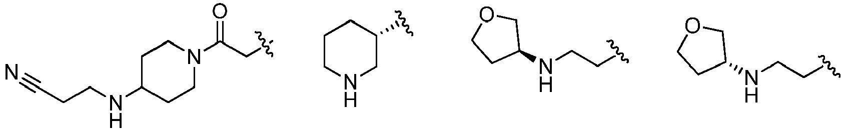

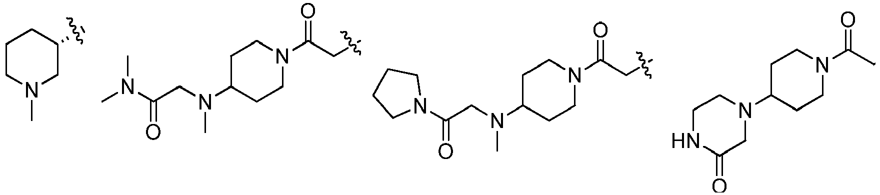

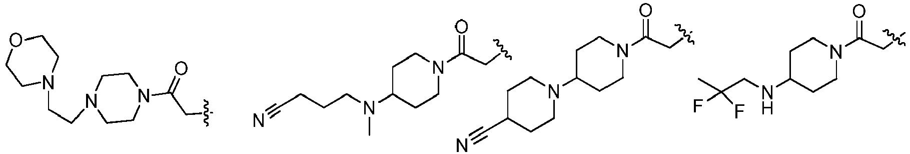

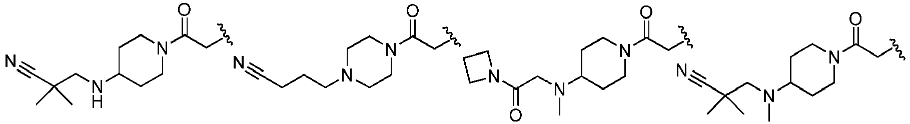

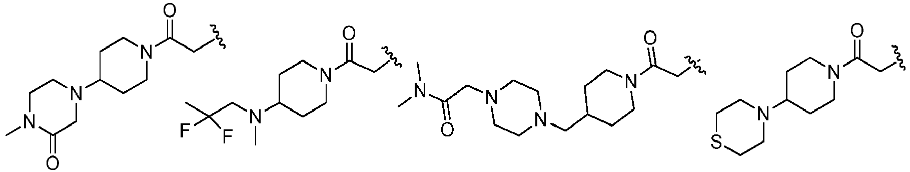

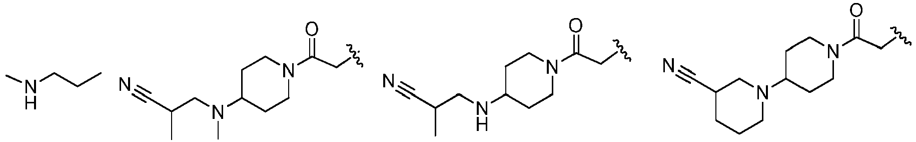

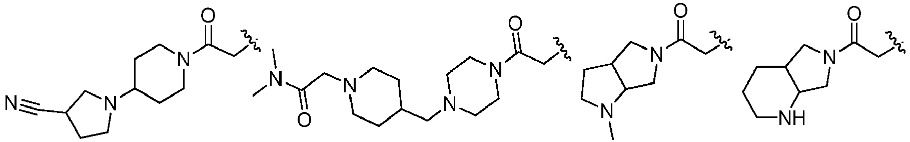

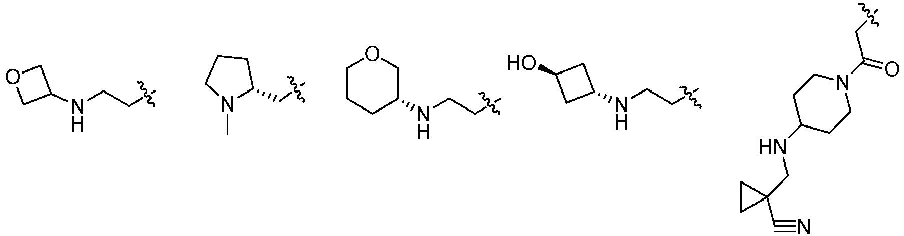

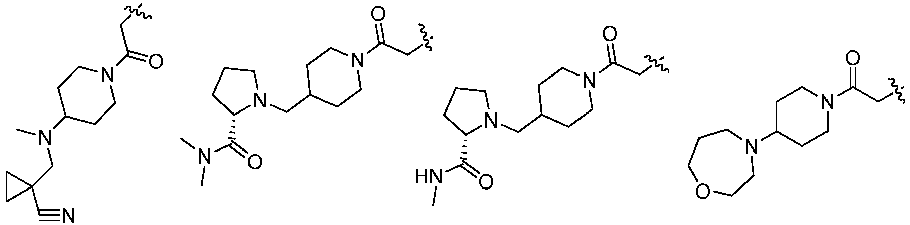

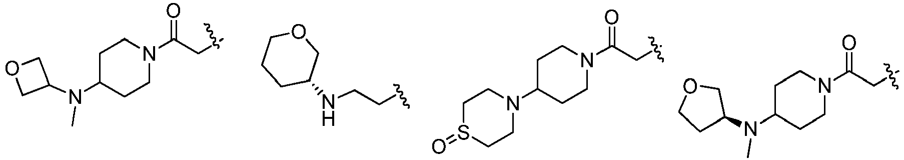

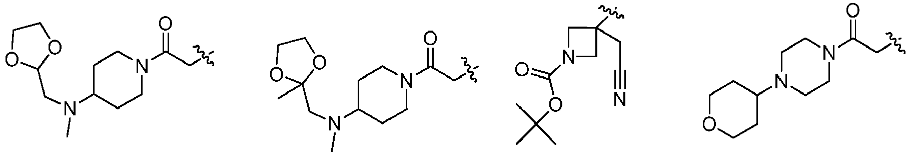

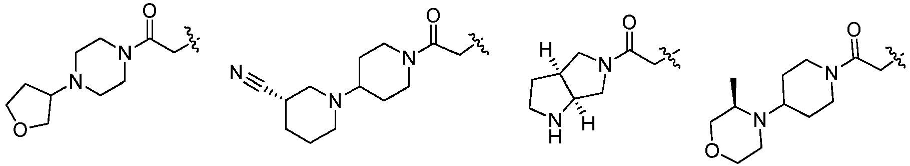

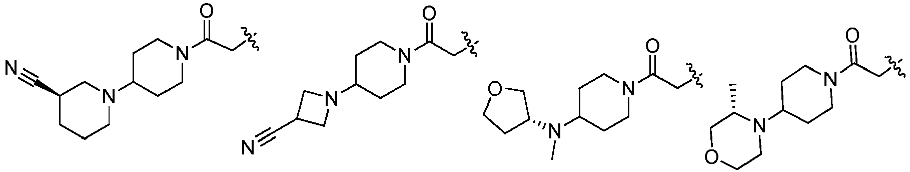

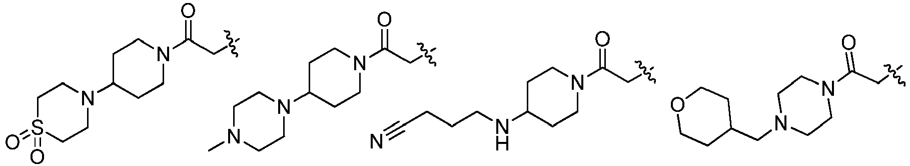

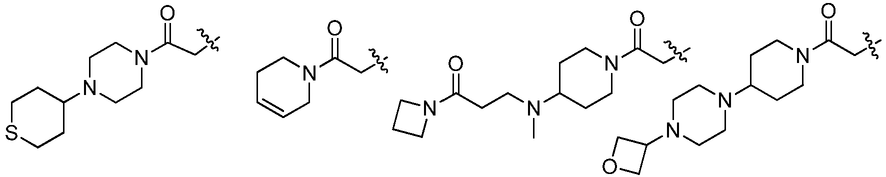

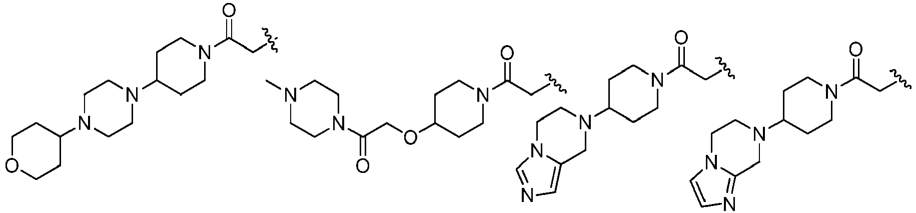

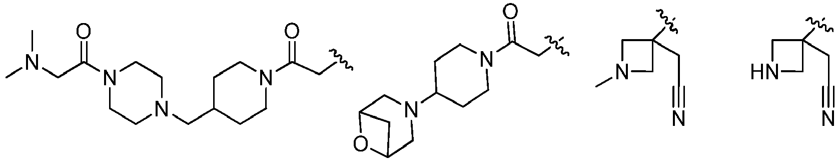

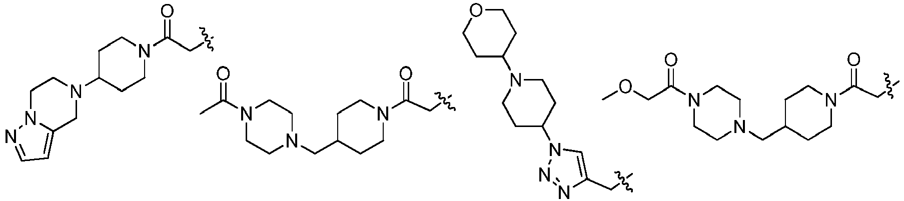

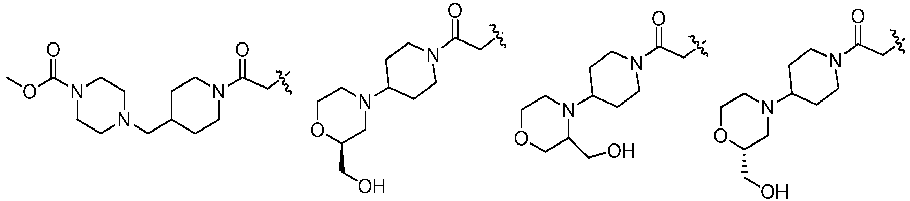

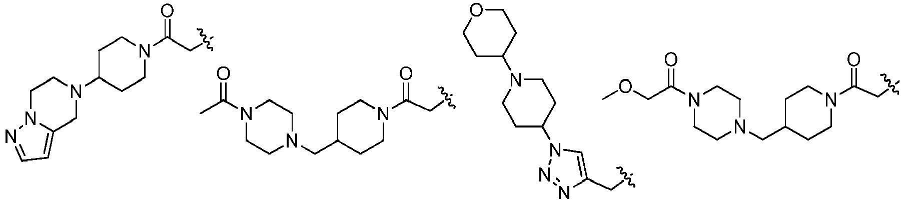

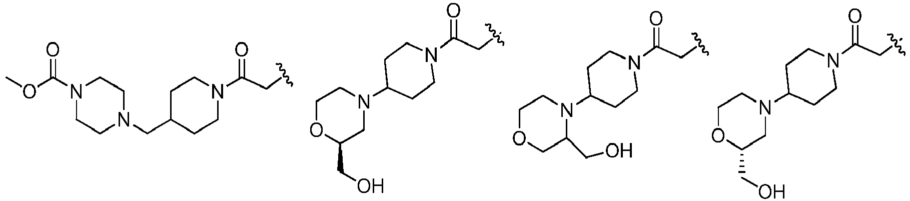

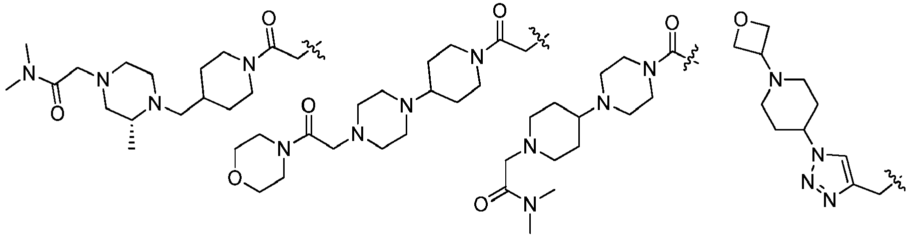

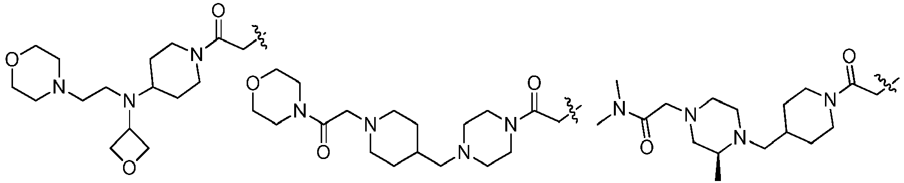

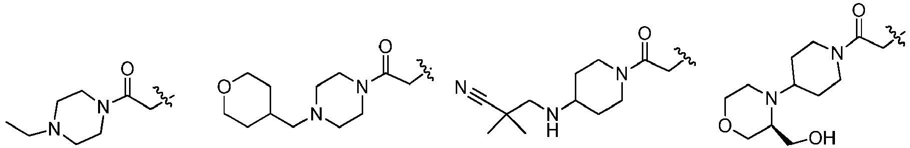

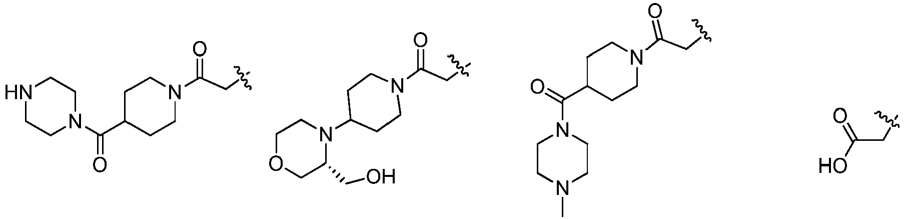

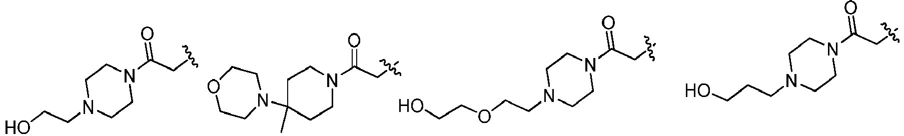

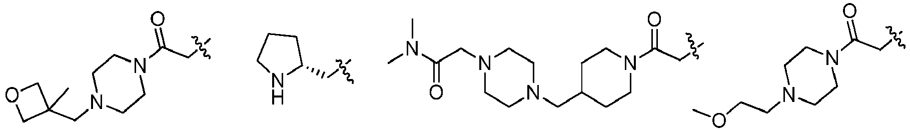

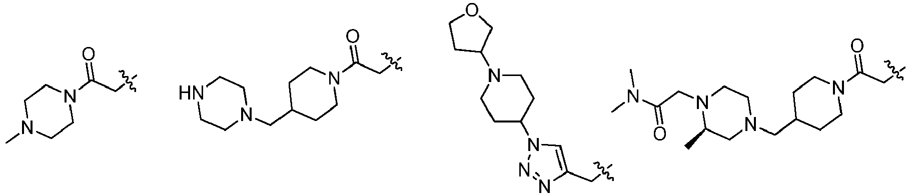

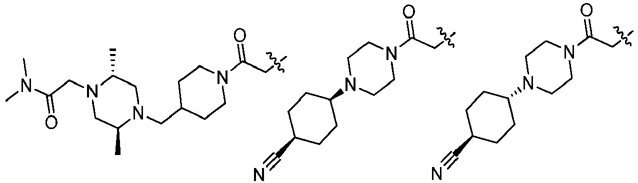

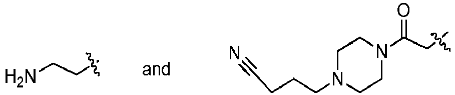

In one embodiment R² is C₁-C₆alkyl, C₃-C₆ cycloalkyl, or 3-6-membered heterocyclyl.

In one embodiment R² is methyl, ethyl, isopropyl, cyclopropyl, or oxetanyl.

In one embodiment n is 0.

In one embodiment n is 1.

In one embodiment n is 2.

One embodiment provides a compound of Formula (00B): 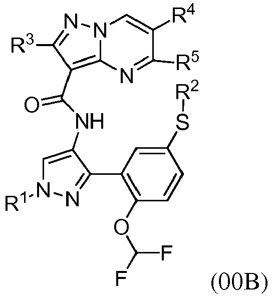 or a salt thereof.

One embodiment provides a compound of Formula (00C): 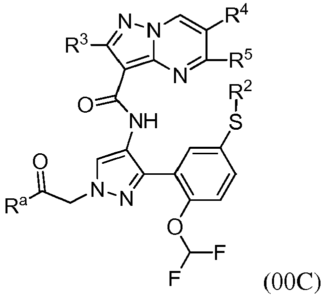 or a salt thereof.

One embodiment provides a compound of Formula (00D): 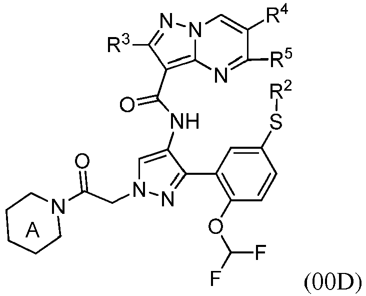 wherein the ring A is optionally substituted with one or more groups R^{e}; or a salt thereof.

One embodiment provides a compound of Formula (00E): 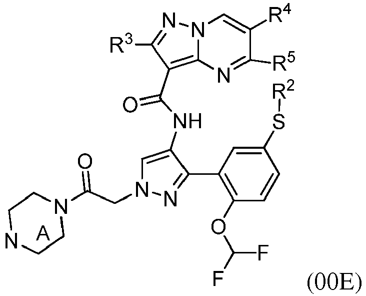 wherein the ring A is optionally substituted with one or more groups R^{e}; or a salt thereof.

One embodiment provides a compound of Formula (00F): 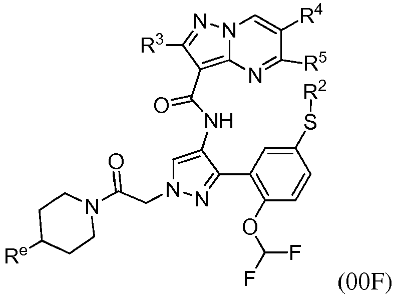 or a salt thereof.

One embodiment provides a compound of Formula (00G): 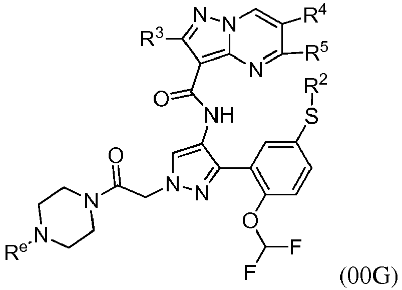 or a salt thereof.

In one embodiment R^{e} is selected from the group consisting of methyl, ethyl,

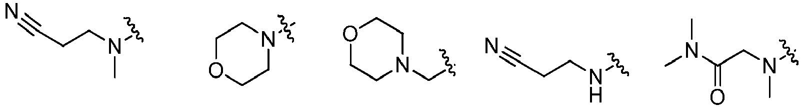

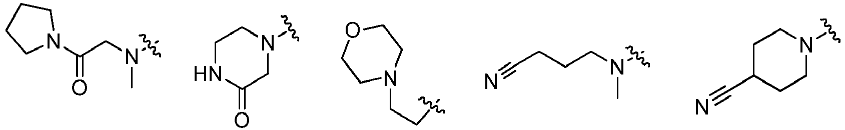

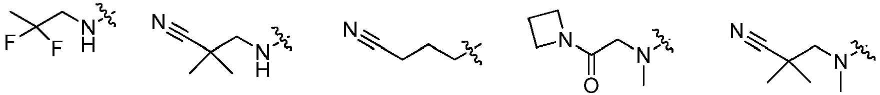

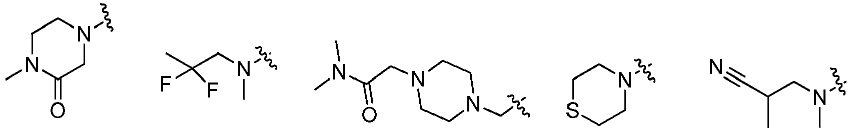

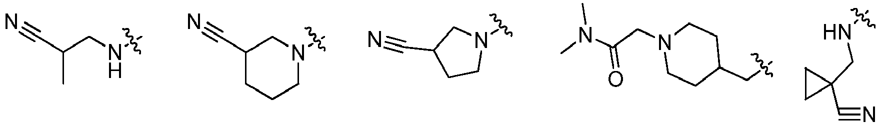

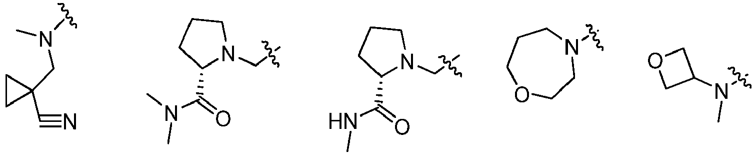

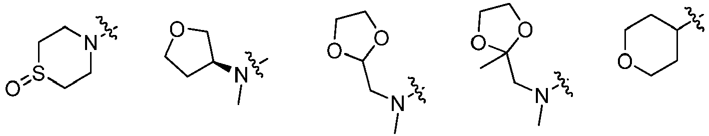

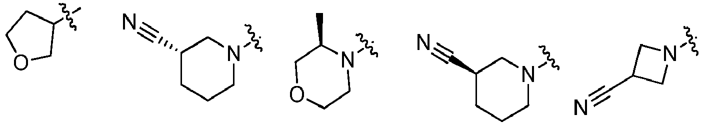

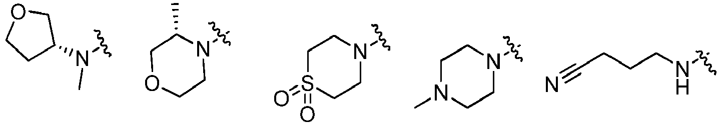

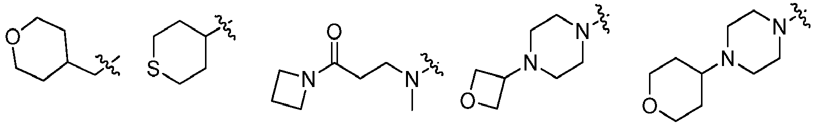

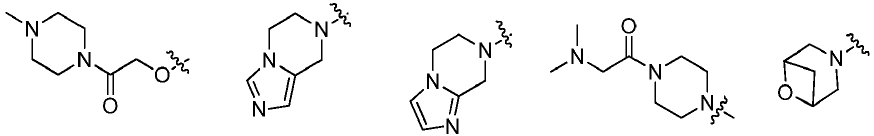

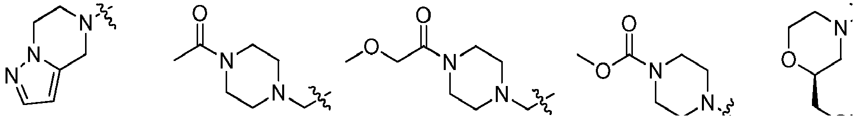

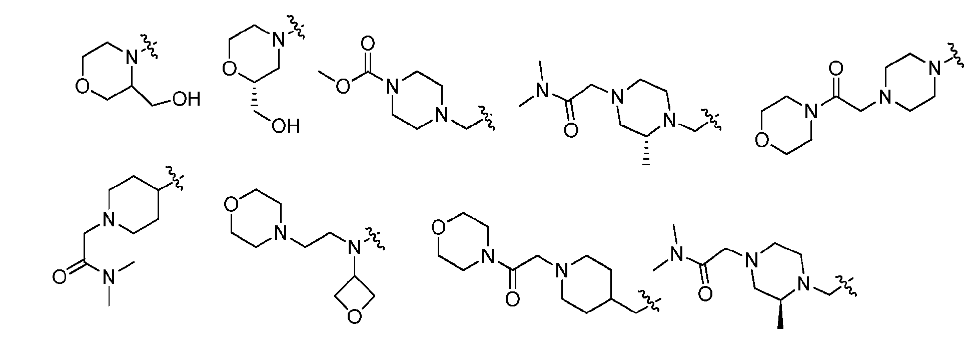

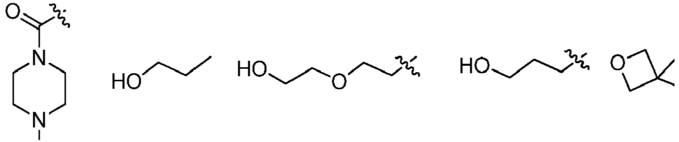

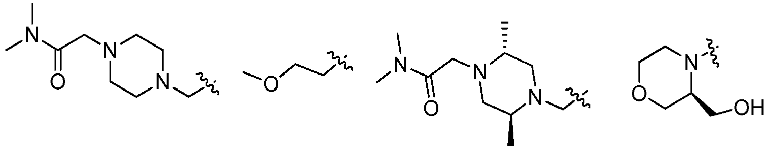

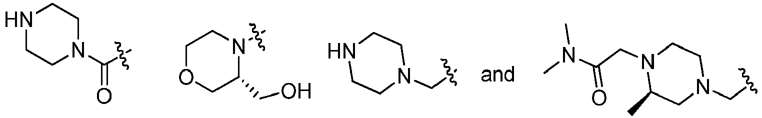

One embodiment provides compounds as described in Examples 1-194 and salts and free bases thereof.

In one embodiment R¹ is C₁-C₆alkyl, C₂-C₆alkenyl, C₂-C₆alkynyl, -(C₀-C₃alkyl)CN, -(C₀-C₃alkyl)OR^{a}, -(C₀-C₃alkyl)R^{a}, -(C₀-C₃alkyl)SR^{a}, -(C₀-C₃alkyl)NR^{a}R^{b}, -(C₀-C₃alkyl)OCF₃, - (C₀-C₃alkyl)CF₃, -(C₀-C₃alkyl)NO₂, -(C₀-C₃alkyl)C(O)R^{a}, -(C₀-C₃alkyl)C(O)OR^{a}, -(C₀-C₃alkyl)C(O)NR^{a}R^{b}, -(C₀-C₃alkyl)NR^{a}C(O)R^{b}, -(C₀-C₃alkyl)S(O)₁₋₂R^{a}, -(C₀-C₃alkyl)NR^{a}S(O)₁₋₂R^{b}, -(C₀-C₃alkyl)S(O)₁₋₂NR^{a}R^{b}, -(C₀-C₃alkyl)(5-6-membered heteroaryl) or -(C₀-C₃alkyl)phenyl, wherein R¹ is optionally substituted by one or more groups independently selected from the group consisting of halogen, C₁-C₃alkyl, oxo, -CHF₂, CH₂F, -CF₃, -(C₀-C₃alkyl)OR^{c} and -(C₀-C₃alkyl)NR^{c}R^{d}.

In one embodiment R³ is H.

In one embodiment R⁴ is H.

In one embodiment, R⁵ is H.

In one embodiment, each of R³, R⁴ and R⁵ is H.

In one embodiment the compound is: 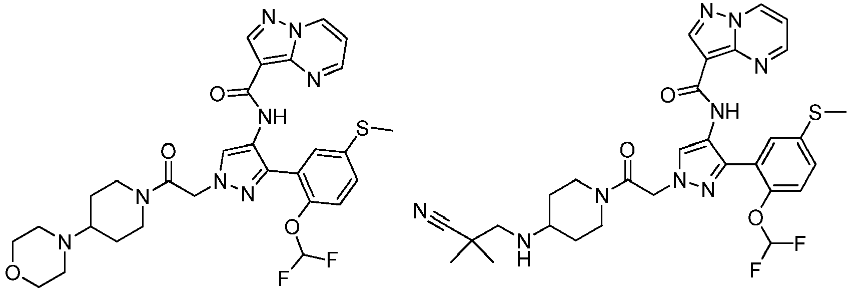 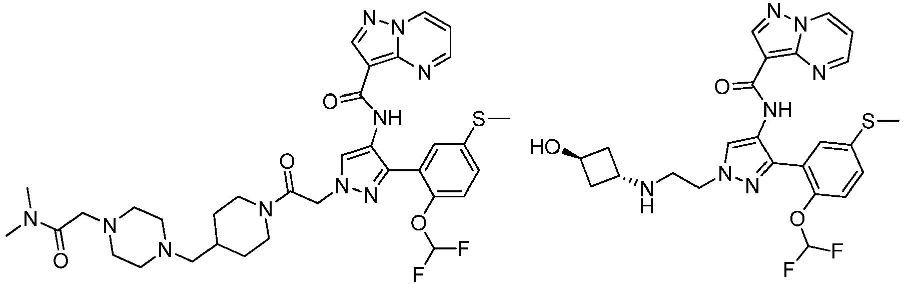 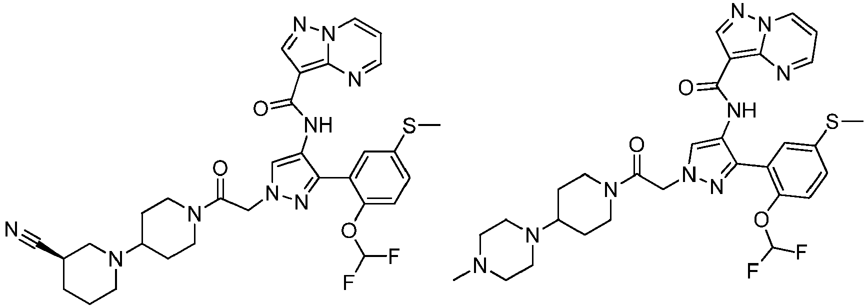 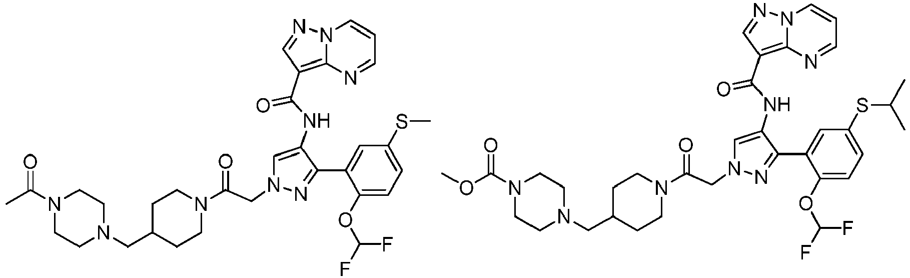 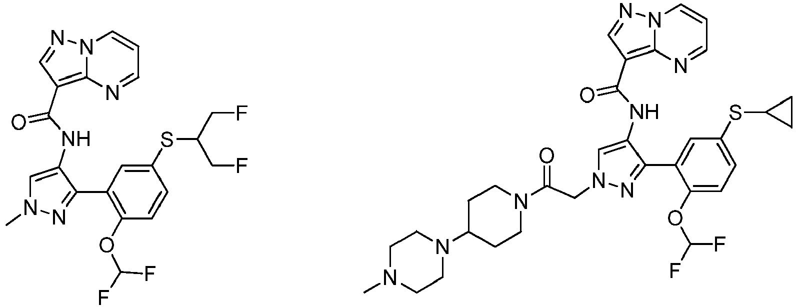 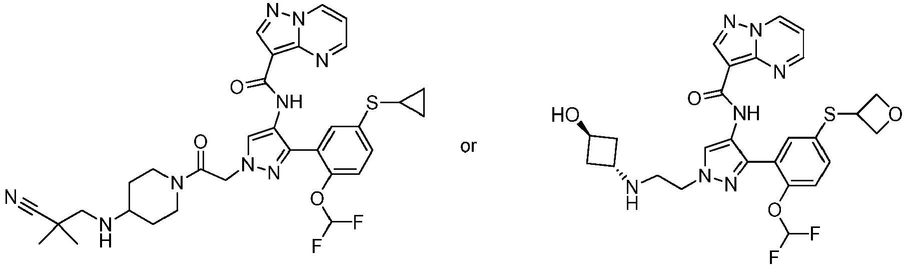 or a salt thereof.

In one embodiment the compound is: 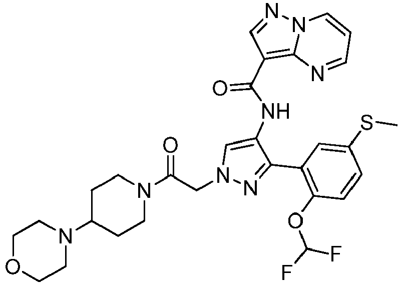 or a salt thereof.

In one embodiment the compound is: 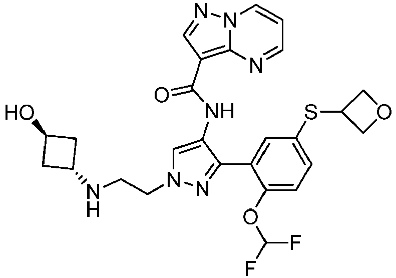 or a salt thereof.

In one embodiment the compound is: 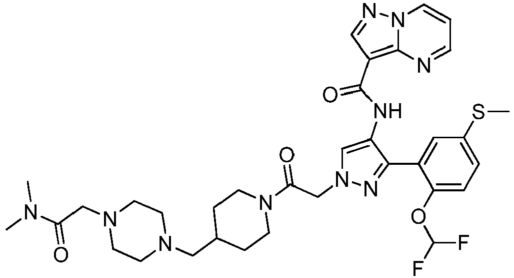 or a salt thereof.

In one embodiment the compound is: 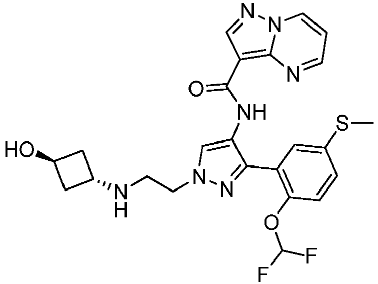 or a salt thereof.

In one embodiment the compound is: 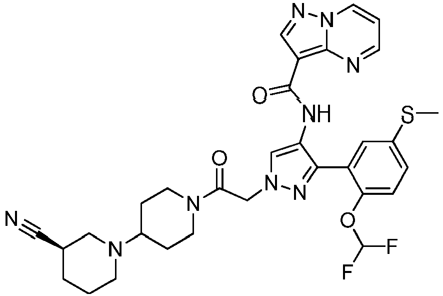 or a salt thereof.

In one embodiment the disease or condition is cancer, polycythemia vera, essential thrombocytosis, myelofibrosis, chronic myelogenous leukemia (CML), rheumatoid arthritis, inflammatory bowel syndrome, Chron's disease, psoriasis, contact dermatitis or delayed hypersensitivity reactions.

In one embodiment a compound of Formula (00A) or a pharmaceutically acceptable salt thereof, for use in the treatment of cancer, polycythemia vera, essential thrombocytosis, myelofibrosis, chronic myelogenous leukemia (CML), rheumatoid arthritis, inflammatory bowel syndrome, Chron's disease, psoriasis, contact dermatitis or delayed hypersensitivity reactions is provided.

In one embodiment a composition that is formulated for administration by inhalation is provided.

In one embodiment a metered dose inhaler that comprises a compound of Formula (00A) or a pharmaceutically acceptable salt thereof is provided.

In one embodiment the compound of Formula (00A) or the pharmaceutically acceptable salt thereof is at least five-times more potent as an inhibitor of JAK1 than as an inhibitor of LRRK2.

In one embodiment the compound of Formula (00A) or the pharmaceutically acceptable salt thereof is at least ten-times more potent as an inhibitor of JAK1 than as an inhibitor of LRRK2.

In one embodiment the compound of Formula (00A) or the pharmaceutically acceptable salt thereof is at least five-times more potent as an inhibitor of JAK1 than as an inhibitor of JAK2.

In one embodiment the compound of Formula (00A) or the pharmaceutically acceptable salt thereof is at least ten-times more potent as an inhibitor of JAK1 than as an inhibitor of JAK2.

In one embodiment the compound of Formula (00A) or the pharmaceutically acceptable salt thereof is at least five-times more potent as an inhibitor of JAK1 than as an inhibitor of JAK3.

In one embodiment the compound of Formula (00A) or the pharmaceutically acceptable salt thereof is at least ten-times more potent as an inhibitor of JAK1 than as an inhibitor of JAK3.

Also disclosed is a method for treating hair loss in a mammal comprising administering a compound of Formula (00A) or a pharmaceutically acceptable salt thereof to the mammal is provided.

In one embodiment a compound of Formula (00A) or a pharmaceutically acceptable salt thereof for use in the treatment of hair loss is provided.

In one embodiment the use of a compound of Formula (00A) or a pharmaceutically acceptable salt thereof to prepare a medicament for treating hair loss in a mammal is provided.

Also provided are methods of synthesizing compounds of the present invention. For example, provided is a method of preparing a compound of Formula (i) 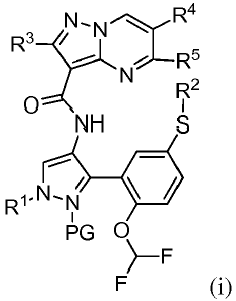 wherein:
R¹ is H, C₁-C₆alkyl, C₂-C₆alkenyl, C₂-C₆alkynyl, -(C₀-C₃alkyl)CN, -(C₀-C₃alkyl)OR^{a}, - (C₀-C₃alkyl)R^{a}, -(C₀-C₃alkyl)SR^{a}, -(C₀-C₃alkyl)NR^{a}R^{b}, -(C₀-C₃alkyl)OCF₃, -(C₀-C₃alkyl)CF₃, -(C₀-C₃alkyl)NO₂, -(C₀-C₃alkyl)C(O)R^{a}, -(C₀-C₃alkyl)C(O)OR^{a}, -(C₀-C₃alkyl)C(O)NR^{a}R^{b}, - (C₀-C₃alkyl)NR^{a}C(O)R^{b}, -(C₀-C₃alkyl)S(O)₁₋₂R^{a}, -(C₀-C₃alkyl)NR^{a}S(O)₁₋₂R^{b}, -(C₀-C₃alkyl)S(O)₁₋₂NR^{a}R^{b}, -(C₀-C₃alkyl)(5-6-membered heteroaryl) or -(C₀-C₃alkyl)phenyl, wherein R¹ is optionally substituted by one or more groups independently selected from the group consisting of halogen, C₁-C₃alkyl, oxo, -CF₃, -(C₀-C₃alkyl)OR^{c} and -(C₀-C₃alkyl)NR^{c}R^{d};
R^{a} is independently hydrogen, C₁-C₆alkyl, C₃-C₆ cycloalkyl, 3-10 membered heterocyclyl, 5-6 membered heteroaryl, -C(O)R^{c}, -C(O)OR^{c}, -C(O)NR^{c}R^{d}, -NR^{c}C(O)R^{d}, - S(O)₁₋₂R^{c}, -NR^{c}S(O)₁₋₂R^{d} or -S(O)₁₋₂NR^{c}R^{d}, wherein any C₃-C₆ cycloalkyl, 3-10 membered heterocyclyl, and 5-6 membered heteroaryl of R^{a} is optionally substituted with one or more groups R^{e};
R^{b} is independently hydrogen or C₁-C₃alkyl, wherein said alkyl is optionally substituted by one or more groups independently selected from the group consisting of halogen and oxo; or
R^{c} and R^{d} are independently selected from the group consisting of hydrogen, 3-6 membered heterocyclyl, C₃-C₆ cycloalkyl, and C₁-C₃alkyl, wherein any 3-6 membered heterocyclyl, C₃-C₆ cycloalkyl, and C₁-C₃alkyl of R^{c} and R^{d} is optionally substituted by one or more groups independently selected from the group consisting of halogen and oxo; or R^{c} and R^{d} are taken together with the atom to which they are attached to form a 3-6-membered heterocyclyl, optionally substituted by one or more groups independently selected from the group consisting of halogen, oxo, -CF₃ and C₁-C₃alkyl;
each R^{e} is independently selected from the group consisting of oxo, OR^{f}, NR^{f}R^{g}, halogen, 3-10 membered heterocyclyl, C₃-C₆ cycloalkyl, and C₁-C₆alkyl, wherein any C₃-C₆ cycloalkyl and C₁-C₆alkyl of R^{e} is optionally substituted by one or more groups independently selected from the group consisting of OR^{f}, NR^{f}R^{g}, halogen, 3-10 membered heterocyclyl, oxo, and cyano, and wherein any 3-10 membered heterocyclyl of R^{e} is optionally substituted by one or more groups independently selected from the group consisting of halogen, oxo, cyano, -CF₃, NR^{h}R^{k}, 3-6 membered heterocyclyl, and C₁-C₃alkyl that is optionally substituted by one or more groups independently selected from the group consisting of halogen, oxo, OR^{f}, and NR^{h}R^{k};
R^{f} and R^{g} are each independently selected from the group consisting of hydrogen, C₁-C₆alkyl, 3-6 membered heterocyclyl, and C₃-C₆ cycloalkyl, wherein any C₁-C₆alkyl, 3-6 membered heterocyclyl, and C₃-C₆ cycloalkyl of R^{f} and R^{g} is optionally substituted by one or more R^{m};
each R^{m} is independently selected from the group consisting of halogen, cyano, oxo, C₃-C₆cycloalkyl, 3-6 membered heterocyclyl, hydroxy, and NR^{h}R^{k}, wherein any C₃-C₆cycloalkyl and 3-6 membered heterocyclyl of R^{m} is optionally substituted with one or more groups independently selected from the group consisting of halogen, oxo, cyano, and C₁-C₃alkyl;
R^{h} and R^{k} are each independently selected from the group consisting of hydrogen and C₁-C₆alkyl that is optionally substituted by one or more groups independently selected from the group consisting of halogen, cyano, 3-6 membered heterocyclyl, and oxo; or R^{h} and R^{k} are taken together with the atom to which they are attached to form a 3-6-membered heterocyclyl that is optionally substituted by one or more groups independently selected from the group consisting of halogen, cyano, oxo, -CF₃ and C₁-C₃alkyl that is optionally substituted by one or more groups independently selected from the group consisting of halogen and oxo;
R² is C₁-C₆alkyl, C₂-C₆alkenyl, C₂-C₆alkynyl, C₃-C₆ cycloalkyl, 3-6-membered heterocyclyl, (C₃-C₆ cycloalkyl)C₁-C₆alkyl, (3-6-membered heterocyclyl)C₁-C₆alkyl, -C(O)(C₃-C₆ cycloalkyl), or -C(O)(3-6-membered heterocyclyl), wherein R² is optionally substituted with one or more halo;
R³ is H or NH₂;
R⁴ is H or CH₃;
R⁵ is H or NH₂; and
PG is a protecting group, such as an acid labile protecting group (e.g., [2-(trimethylsilyl)ethoxy]methyl acetal (SEM), p-methoxybenzyl (PMB), 2,4- or 3,4-dimethoxybenzyl (DMB)),
wherein any substituent (e.g., R¹, R², R³, R⁵) is protected by one or more appropriate protecting group that may be the same or different than PG,
comprising reacting a compound of Formula (ii) 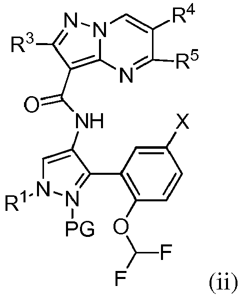
wherein X is a leaving group, such as halogen, tosylate (OTs) or triflate (OTf),
with a sodium salt, R¹SNa,
a palladium catalyst (e.g., Pd(OAc)₂, Pd₂(dba)₃),
and a phosphine ligand (e.g., Xantphos),
in the presence of solvent (e.g., a nonpolar, aprotic solvent such as toluene or dioxane) and under an inert atmosphere. See, e.g., Mispelaere-Canivet et al., Tetrahedron 61:5253-5259 (2005) and Fernandez-Rodriguez et al., J. Amer. Chem. Soc. 126:2180-2181 (2006). Reaction conditions may include heat.

Compounds of the invention may contain one or more asymmetric carbon atoms. Accordingly, the compounds may exist as diastereomers, enantiomers or mixtures thereof. The syntheses of the compounds may employ racemates, diastereomers or enantiomers as starting materials or as intermediates. Mixtures of particular diastereomeric compounds may be separated, or enriched in one or more particular diastereomers, by chromatographic or crystallization methods. Similarly, enantiomeric mixtures may be separated, or enantiomerically enriched, using the same techniques or others known in the art. Each of the asymmetric carbon or nitrogen atoms may be in the R or S configuration and both of these configurations are within the scope of the invention.

In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined. Unless otherwise specified, if solid wedges or dashed lines are used, relative stereochemistry is intended.

Disclosed are prodrugs of the compounds of Formula (00A), including known amino-protecting and carboxy-protecting groups which are released, for example hydrolyzed, to yield the compound of the present invention under physiologic conditions.

The term "prodrug" refers to a precursor or derivative form of a pharmaceutically active substance that is less efficacious to the patient compared to the parent drug and is capable of being enzymatically or hydrolytically activated or converted into the more active parent form. See, e.g., Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). Prodrugs include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, and 5-fluorocytosine and 5-fluorouridine prodrugs.

A particular class of prodrugs are compounds in which a nitrogen atom in an amino, amidino, aminoalkyleneamino, iminoalkyleneamino or guanidino group is substituted with a hydroxy group, an alkylcarbonyl (-CO-R) group, an alkoxycarbonyl (-CO-OR), or an acyloxyalkyl-alkoxycarbonyl (-CO-O-R-O-CO-R) group where R is a monovalent or divalent group, for example alkyl, alkylene or aryl, or a group having the Formula -C(O)-O-CP1P2-haloalkyl, where P1 and P2 are the same or different and are hydrogen, alkyl, alkoxy, cyano, halogen, alkyl or aryl. In a particular embodiment, the nitrogen atom is one of the nitrogen atoms of the amidino group of the compounds of Formula (00A). Prodrugs may be prepared by reacting a compound of Formula (00A) with an activated group, such as acyl groups, to bond, for example, a nitrogen atom in the compound to the exemplary carbonyl of the activated acyl group. Examples of activated carbonyl compounds are those containing a leaving group bonded to the carbonyl group, and include, for example, acyl halides, acyl amines, acyl pyridinium salts, acyl alkoxides, acyl phenoxides such as p-nitrophenoxy acyl, dinitrophenoxy acyl, fluorophenoxy acyl, and difluorophenoxy acyl. The reactions are generally carried out in inert solvents at reduced temperatures such as -78 to about 50°C. The reactions may also be carried out in the presence of an inorganic base, for example potassium carbonate or sodium bicarbonate, or an organic base such as an amine, including pyridine, trimethylamine, triethylamine, triethanolamine, or the like.

Additional types of prodrugs are also disclosed. For instance, a free carboxyl group of a compound of Formula (00A) can be derivatized as an amide or alkyl ester. As another example, compounds of the present invention comprising free hydroxy groups can be derivatized as prodrugs by converting the hydroxy group into a group such as, but not limited to, a phosphate ester, hemisuccinate, dimethylaminoacetate, or phosphoryloxymethyloxycarbonyl group, as outlined in Fleisher, D. et al., (1996) Improved oral drug delivery: solubility limitations overcome by the use of prodrugs Advanced Drug Delivery Reviews, 19:115. Carbamate prodrugs of hydroxy and amino groups are also included, as are carbonate prodrugs, sulfonate esters and sulfate esters of hydroxy groups. Derivatization of hydroxy groups as (acyloxy)methyl and (acyloxy)ethyl ethers, wherein the acyl group can be an alkyl ester optionally substituted with groups including, but not limited to, ether, amine and carboxylic acid functionalities, or where the acyl group is an amino acid ester as described above, are also encompassed. Prodrugs of this type are described in J. Med. Chem., (1996), 39:10. More specific examples include replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, alpha-amino(C₁-C₄)alkanoyl, arylacyl and alpha-aminoacyl, or alpha-aminoacyl-alpha-aminoacyl, where each alpha-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

"Leaving group" refers to a portion of a first reactant in a chemical reaction that is displaced from the first reactant in the chemical reaction. Examples of leaving groups include, but are not limited to, halogen atoms, alkoxy and sulfonyloxy groups. Example sulfonyloxy groups include, but are not limited to, alkylsulfonyloxy groups (for example methyl sulfonyloxy (mesylate group) and trifluoromethylsulfonyloxy (triflate group)) and arylsulfonyloxy groups (for example p-toluenesulfonyloxy (tosylate group) and p-nitrosulfonyloxy (nosylate group)).

### SYNTHESIS OF JANUS KINASE INHIBITOR COMPOUNDS

Compounds may be synthesized by synthetic routes described herein. In certain embodiments, processes well-known in the chemical arts can be used, in addition to, or in light of, the description contained herein. The starting materials are generally available from commercial sources such as Aldrich Chemicals (Milwaukee, Wis.) or are readily prepared using methods well known to those skilled in the art (e.g., prepared by methods generally described in Louis F. Fieser and Mary Fieser, Reagents for Organic Synthesis, v. 1-19, Wiley, N.Y. (1967-1999 ed.), Beilsteins Handbuch der organischen Chemie, 4, Aufl. ed. Springer-Verlag, Berlin, including supplements (also available via the Beilstein online database)), or Comprehensive Heterocyclic Chemistry, Editors Katrizky and Rees, Pergamon Press, 1984.

Compounds may be prepared singly or as compound libraries comprising at least 2, for example 5 to 1,000 compounds, or 10 to 100 compounds. Libraries of compounds may be prepared by a combinatorial 'split and mix' approach or by multiple parallel syntheses using either solution phase or solid phase chemistry, by procedures known to those skilled in the art. Thus according to a further aspect of the invention there is provided a compound library comprising at least 2 compounds of the present invention, such as compounds of Formula (00A).

For illustrative purposes, reaction Schemes depicted below provide routes for synthesizing the compounds of the present invention as well as key intermediates. For a more detailed description of the individual reaction steps, see the Examples section below. Those skilled in the art will appreciate that other synthetic routes may be used. Although some specific starting materials and reagents are depicted in the Schemes and discussed below, other starting materials and reagents can be substituted to provide a variety of derivatives or reaction conditions. In addition, many of the compounds prepared by the methods described below can be further modified in light of this disclosure using conventional chemistry well known to those skilled in the art.

In the preparation of compounds of the present invention, protection of remote functionality (e.g., primary or secondary amine) of intermediates may be necessary. The need for such protection will vary depending on the nature of the remote functionality and the conditions of the preparation methods. Suitable amino-protecting groups include acetyl, trifluoroacetyl, benzyl, phenylsulfonyl, t-butoxycarbonyl (BOC), benzyloxycarbonyl (CBz) and 9-fluorenylmethyleneoxycarbonyl (Fmoc). The need for such protection is readily determined by one skilled in the art. For a general description of protecting groups and their use, see T. W. Greene, Protective Groups in Organic Synthesis, John Wiley & Sons, New York, 1991.

Other conversions commonly used in the synthesis of compounds of the present invention, and which can be carried out using a variety of reagents and conditions, include the following:
(1) Reaction of a carboxylic acid with an amine to form an amide. Such a transformation can be achieved using various reagents known to those skilled in the art but a comprehensive review can be found in Tetrahedron, 2005, 61, 10827-10852.
(2) Reaction of a primary or secondary amine with an aryl halide or pseudo halide, e.g., a triflate, commonly known as a "Buchwald-Hartwig cross-coupling," can be achieved using a variety of catalysts, ligands and bases. A review of these methods is provided in Comprehensive Organic Name Reactions and Reagents, 2010, 575-581.
(3) A palladium cross-coupling reaction between an aryl halide and a vinyl boronic acid or boronate ester. This transformation is a type of "Suzuki-Miyaura cross-coupling," a class of reaction that has been thoroughly reviewed in Chemical Reviews, 1995, 95(7), 2457-2483.
(4) The hydrolysis of an ester to give the corresponding carboxylic acid is well known to those skilled in the art and conditions include: for methyl and ethyl esters, the use of a strong aqueous base such as lithium, sodium or potassium hydroxide or a strong aqueous mineral acid such as HCl; for a tert-butyl ester, hydrolysis would be carried out using acid, for example, HCl in dioxane or trifluoroacetic acid (TFA) in dichloromethane (DCM).

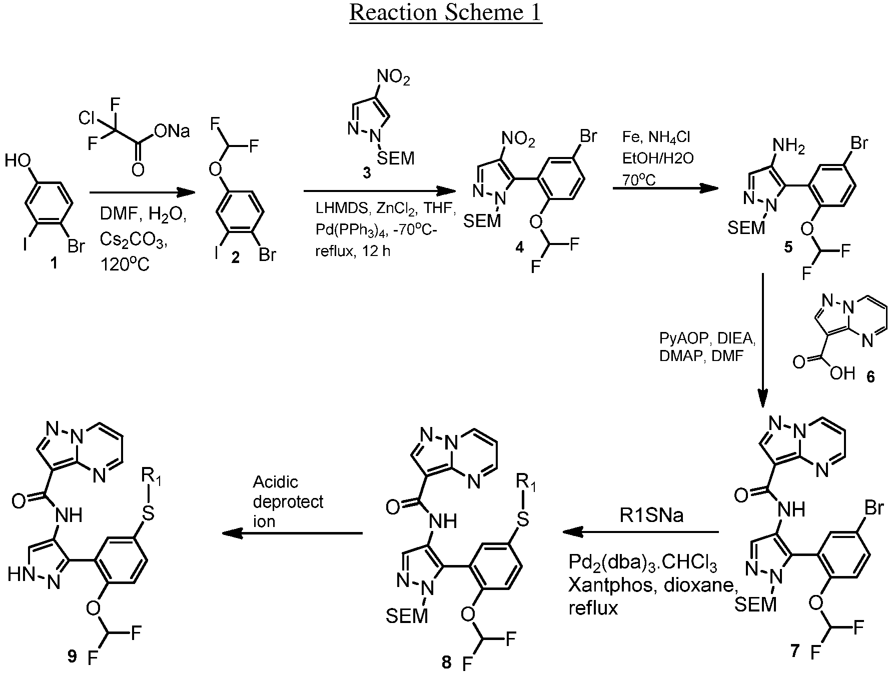

As shown in Scheme 1, compounds of type 2 may be prepared by the reaction of an appropriate phenol such as compound 1 with 2-chloro-2,2-difluoroacetic acid sodium salt at elevated temperature in the presence of a base such as Cs₂CO₃ and in an appropriate solvent. Reaction of compound 3 with a base such as LHMDS then ZnCl₂, followed by exposure to a palladium catalyst such as Pd(PPh₃)₄ and compounds of type 2 produces compounds of type 4. Nitro reduction produces compounds of type 5, which may then be coupled to appropriate carboxylic acids such as 6 to produce amides of type 7. Exposure of compounds such as 7 to thiolates in the presence of an appropriate palladium catalyst, phosphine ligand, and solvent then produces compounds of type 8. Exposure of 8 to acidic deprotection conditions removes the SEM protecting group to produce compounds of type 9.

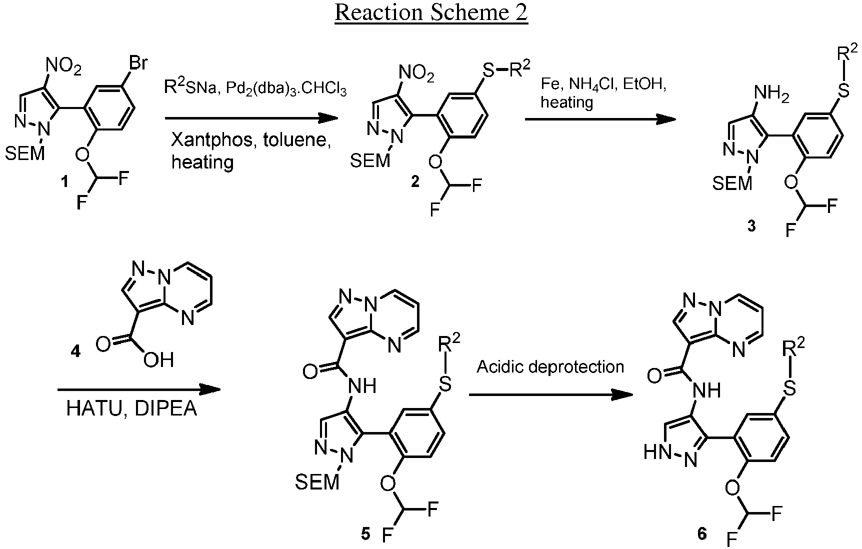

As shown in Scheme 2, compounds of type 2 may be prepared by reacting compounds of type 1 with thiolates in the presence of a palladium catalyst, phosphine ligand, and solvent. Nitro reduction, followed by amide bond formation and SEM deprotection then produces compounds of type 6.

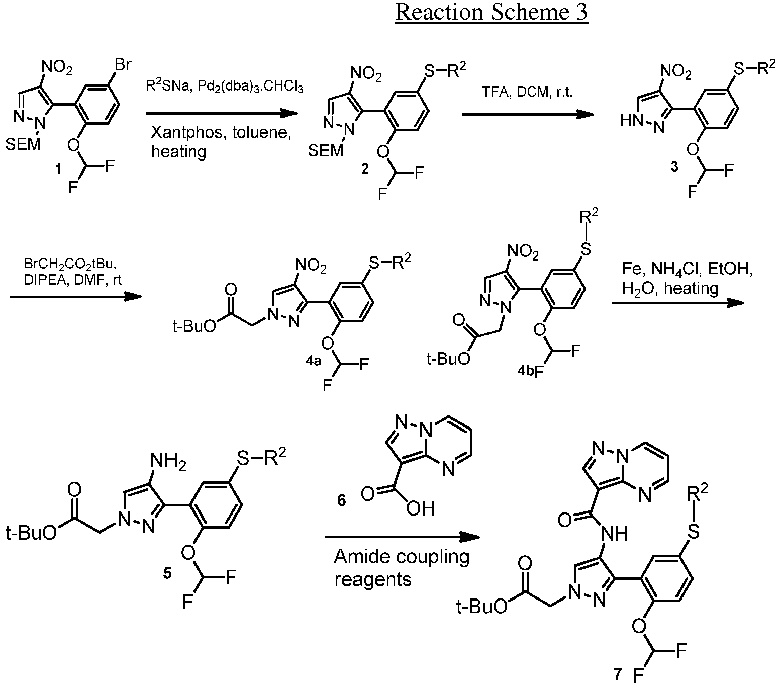

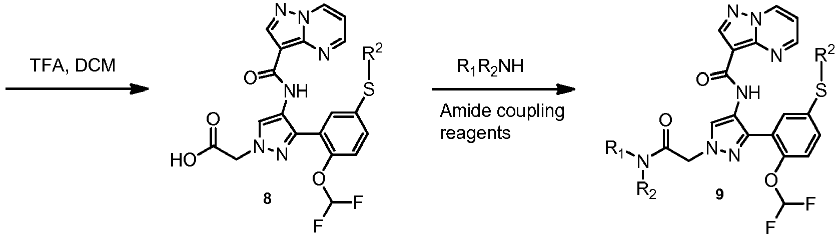

As shown in Scheme 3, compounds of type 2 may be prepared by reacting compounds of type 1 with thiolates in the presence of a palladium catalyst, phosphine ligand, and solvent. SEM deprotection produces compounds of type 3. Reaction of compounds of type 3 with an appropriate electrophile such as an alkyl bromide in the presence of base such as DIPEA then produces compounds of type 4a and 4b in various ratios depending on reaction conditions such as the nature of the electrophile, base, solvent, and reaction temperature. In some instances regioisomers 4a and 4b may be separated by methods such as chromatography or crystallization, or the mixture may be carried forward to subsequent steps, with a possible separation at a later stage of the synthetic sequence. Nitro reduction of 4a, followed by coupling to an appropriate carboxylic acid such as 6 produces compounds of type 7. *t*-Butyl ester deprotection, followed by coupling to appropriate amines produces compounds of type 9.

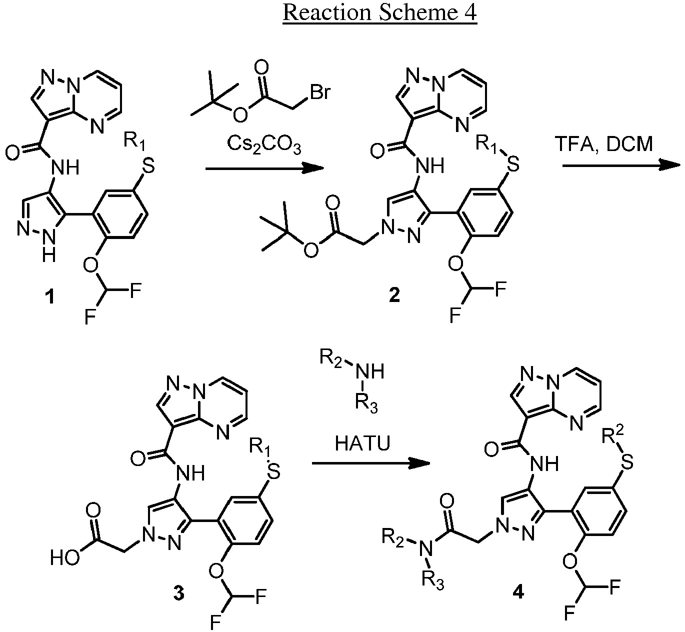

As shown in Scheme 4, compounds of type 2 may be obtained by reaction compounds of type 1 with an appropriate electrophile such as an alkyl bromide in the presence of base. *t*-Butyl ester deprotection, followed by coupling to appropriate amines produces compounds of type 4.

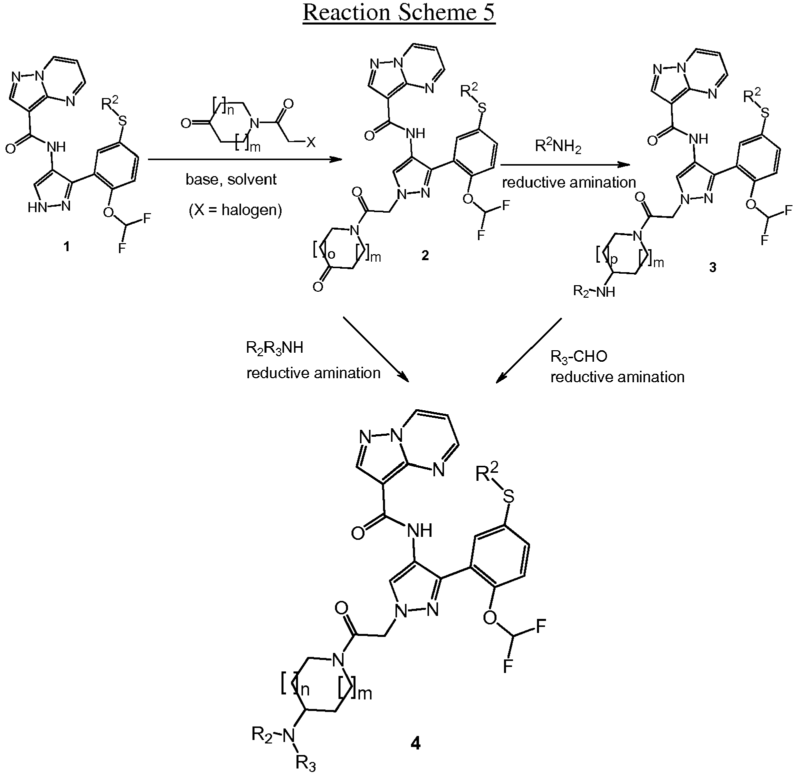

As shown in Scheme 5, compounds of type 2 may be obtained by the reaction of compounds of type 1 with an appropriate electrophile such as an alkyl halide in the presence of base. Compounds of type 4 may be obtained by either a single reductive amination with an appropriate amine, or by an initial reductive amination with an appropriate amine to produce compounds of type 3, followed by a second reductive amination with an appropriate aldehyde .

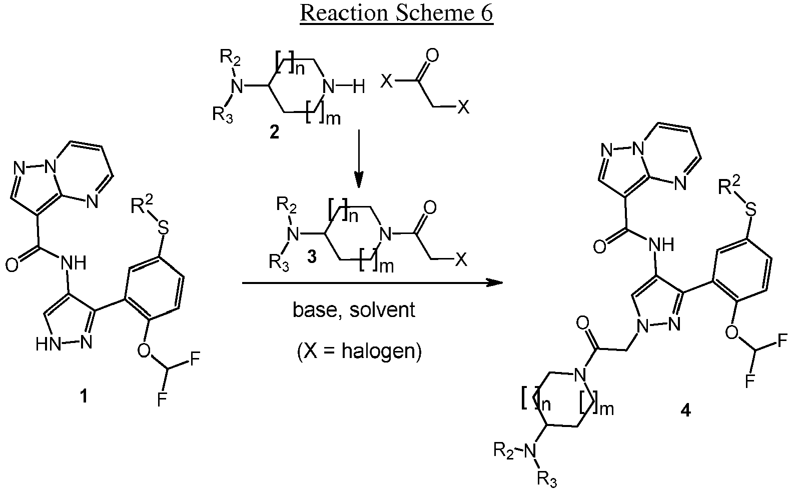

As shown in Scheme 6, compounds of type 3 may be obtained by the reaction of a haloacetyl halide with an appropriate amine such as 2. Alkylation of compounds of type 1 with compounds of type 3 in the presence of a base produces compounds of type 4.

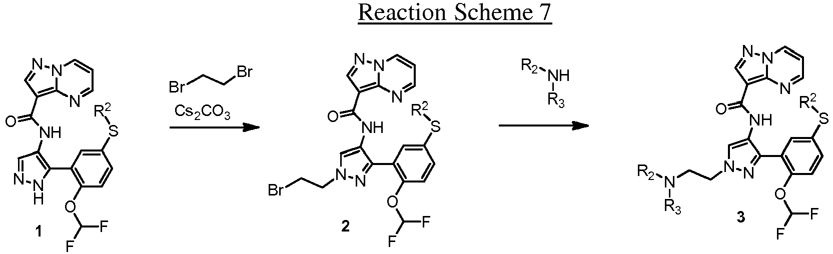

As shown in Scheme 7, reaction of compounds of type 1 with 1,2-dibromoethane in the presence of base produces compounds of type 2. Reaction of compounds of type 2 with an appropriate amine produces compounds of type 3.

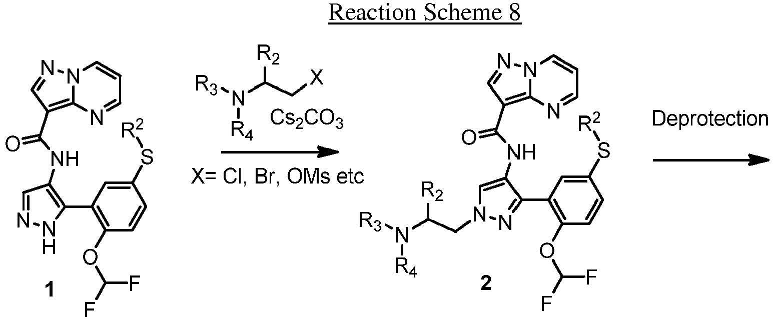

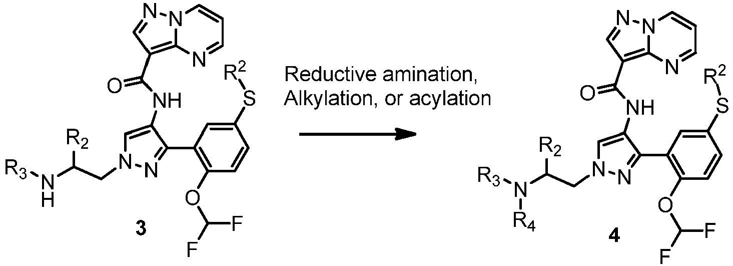

As shown in Scheme 8, alkylation of compounds of type 1 with an appropriate electrophile in the presence of base produces compounds of type 2. In certain instances R⁴ is a protecting group, which may be removed to liberate a reactive amine of type 3. Compounds of type 3 may be reacted with appropriate electrophiles under reductive amination, alkylation, or acylation conditions to produce compounds of type 4.

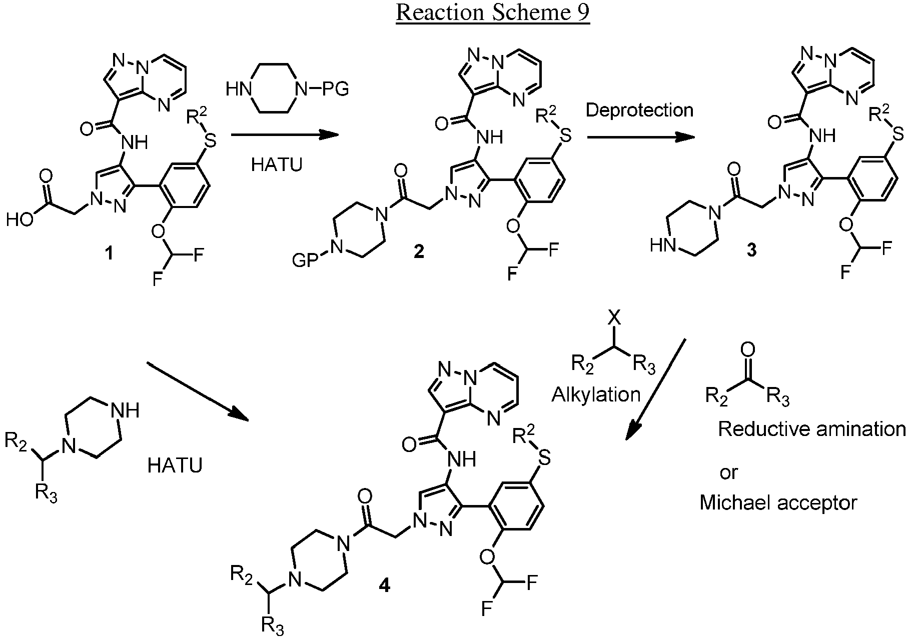

Compounds of type 2 may be reacted with substituted piperazines under amide bond forming conditions to produce compounds of types 2 and 4. Compounds of type 2 containing protected piperazines may be deprotected to liberate compounds of type 3. The reactive amine present in compounds of type 3 may be reacted with a variety of electrophiles (in the case of reductive amination, a reducing agent is also added) to produce compounds of type 4.

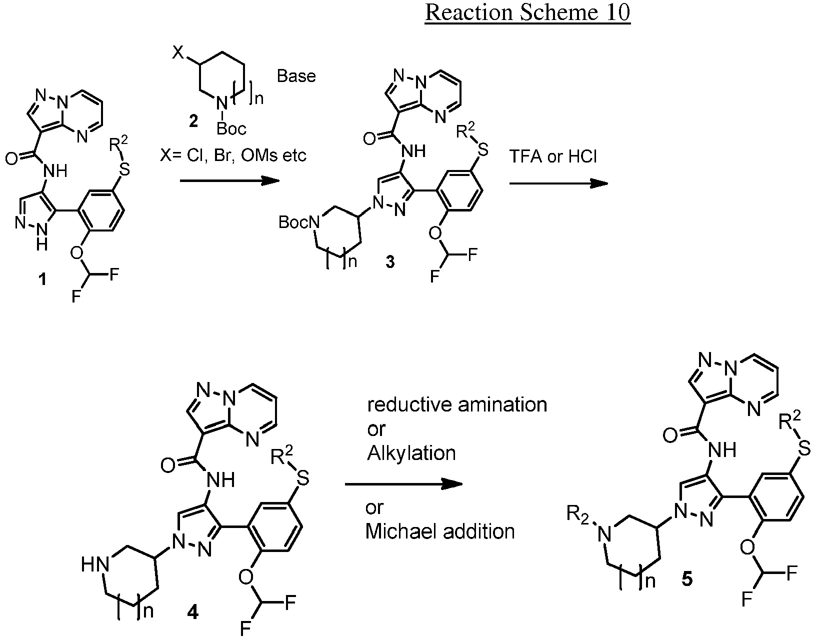

Compounds of type 3 may be obtained by reacting compounds of type 1 with an appropriate electrophile such as 2 in the presence of base. Removal of a protecting group such as a tert-butyl carbamate produces compounds of type 4 possessing a reactive amine. Reaction of compounds of type 4 with appropriate electrophiles (in the case of reductive amination, a reducing agent is also added) produces compounds of type 5.

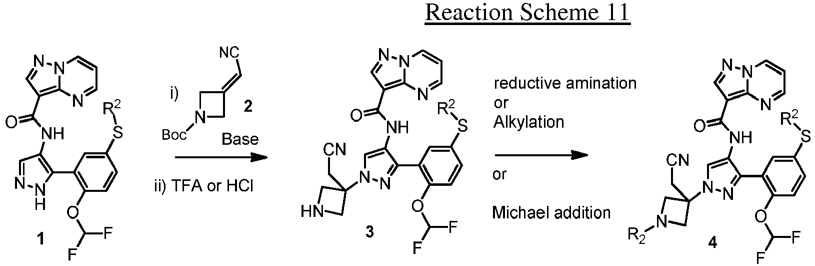

Compounds of type 3 may be obtained by reacting compounds of type 1 with a Michael acceptor such as compound 2 in the presence of base, followed by protecting group removal under acidic conditions. Reaction of compounds of type 3 with appropriate electrophiles (in the case of reductive amination, a reducing agent is also added) produces compounds of type 4.

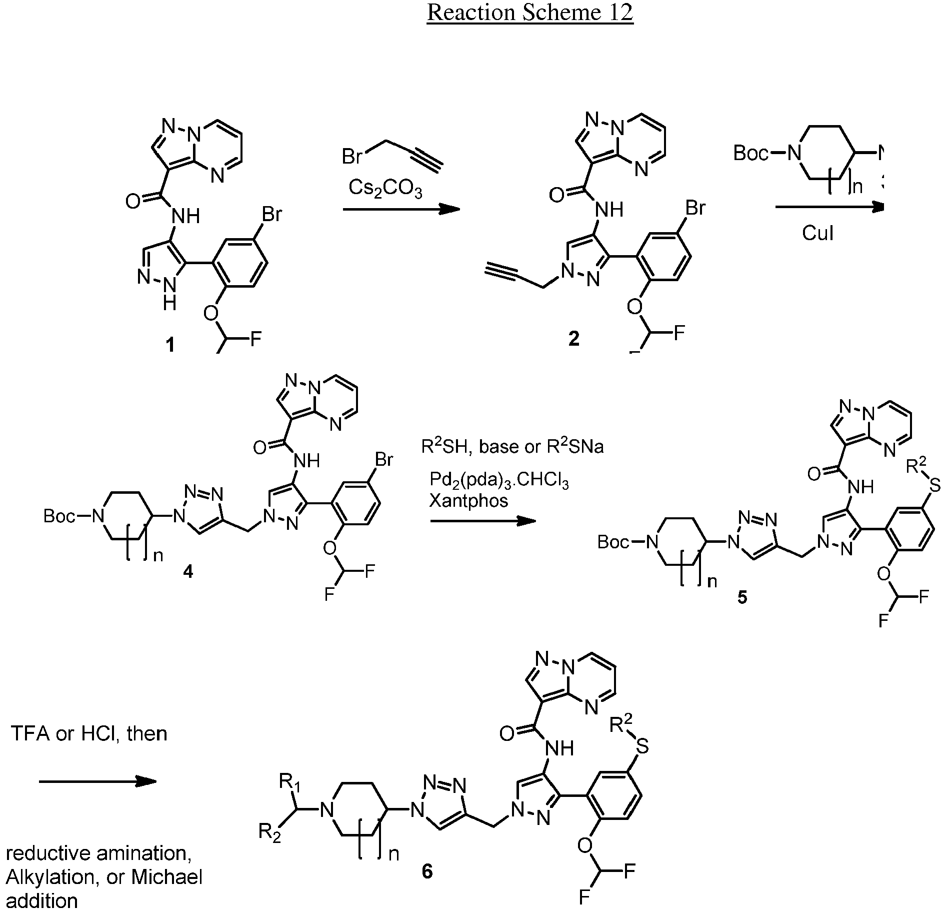

As shown in Scheme 12, compounds of type 2 may be obtained by the reaction of compounds of type 1 with a propargylic halide in the presence of base. Reaction of 2 with an azide such as 3 in the presence of an additive such as copper iodide produces triazoles such as 4. Reaction of 4 with a thiolate in the presence of a palladium catalyst and phosphine ligand produces compounds of type 5. Removal of the protecting group under acidic conditions, followed by reaction of the exposed amine with an appropriate electrophile (in the case of reductive amination, a reducing agent is also added) produces compounds of type 6.

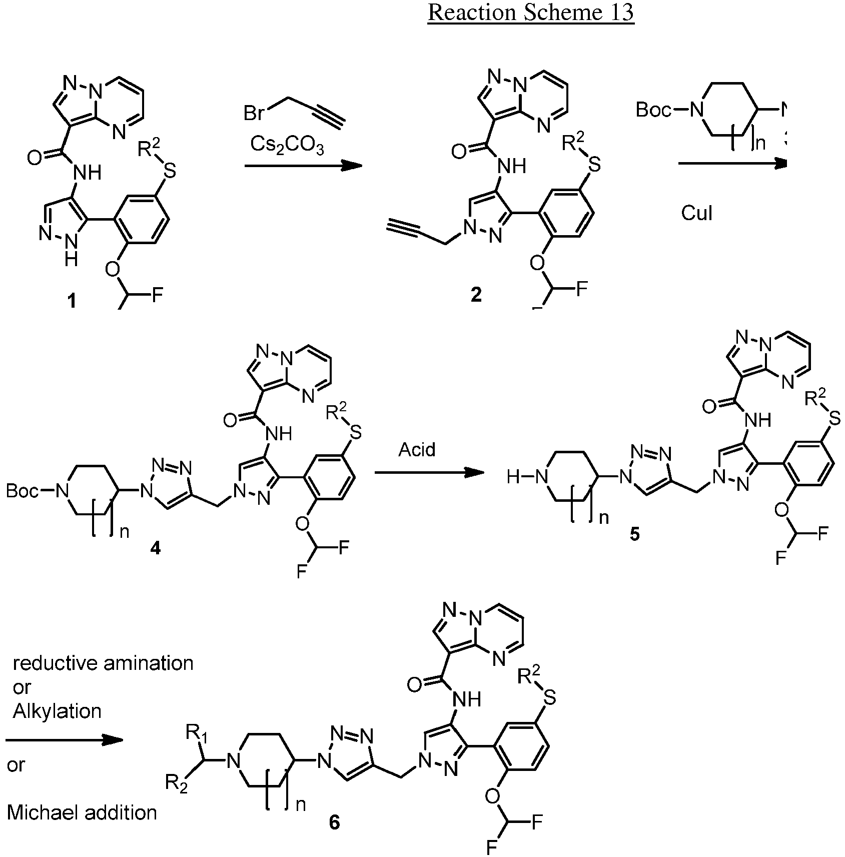

As shown in Scheme 13, compounds of type 2 may be obtained by the reaction of compounds of type 1 with a propargylic halide in the presence of base. Reaction of 2 with an azide such as 3 in the presence of an additive such as copper iodide produces triazoles such as 4. Removal of the protecting group present in 4 under acidic conditions, followed by reaction of the exposed amine with an appropriate electrophile (in the case of reductive amination, a reducing agent is also added) produces compounds of type 6.

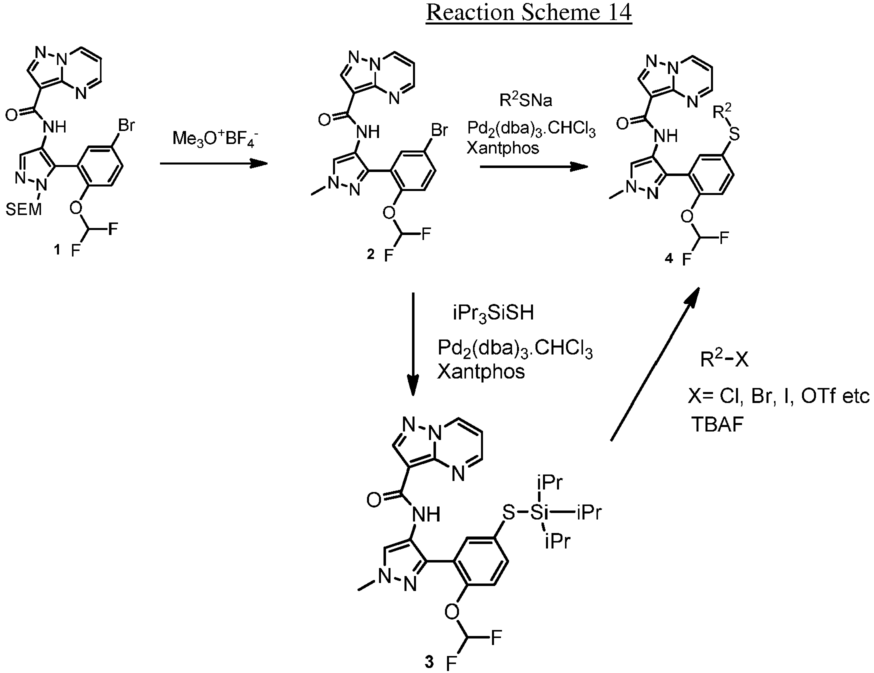

As shown in Scheme 14, compounds of type 2 may be obtained by reacting compounds of type 1 with trimethyloxonium tetrafluoroborate. Compounds of type 4 may be obtained by exposure of compounds such as 2 to thiolates in the presence of an appropriate palladium catalyst and phosphine ligand. Alternatively, compound 2 may be treated with a silane-protected thiol in the presense of an appropriate palladium catalyst and phosphine ligand to produce compounds of type 3. Treatment of 3 with an alkyl halide or alkyl halide equivalent in the presence of a fluoride source (TBAF) may produce compounds of type 4.

It will be appreciated that where appropriate functional groups exist, compounds of various formulae or any intermediates used in their preparation may be further derivatised by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

In a further example, primary amine or secondary amine groups may be converted into amide groups (-NHCOR' or -NRCOR') by acylation. Acylation may be achieved by reaction with an appropriate acid chloride in the presence of a base, such as triethylamine, in a suitable solvent, such as dichloromethane, or by reaction with an appropriate carboxylic acid in the presence of a suitable coupling agent such HATU (O-(7-azabenzotriazol-1-yl)-N,N,N',N'-tetramethyluronium hexafluorophosphate) in a suitable solvent such as dichloromethane. Similarly, amine groups may be converted into sulphonamide groups (-NHSO₂R' or-NR"SO₂R') groups by reaction with an appropriate sulphonyl chloride in the presence of a suitable base, such as triethylamine, in a suitable solvent such as dichloromethane. Primary or secondary amine groups can be converted into urea groups (-NHCONR'R" or -NRCONR'R") by reaction with an appropriate isocyanate in the presence of a suitable base such as triethylamine, in a suitable solvent, such as dichloromethane.

An amine (-NH₂) may be obtained by reduction of a nitro (-NO₂) group, for example by catalytic hydrogenation, using for example hydrogen in the presence of a metal catalyst, for example palladium on a support such as carbon in a solvent such as ethyl acetate or an alcohol e.g., methanol. Alternatively, the transformation may be carried out by chemical reduction using for example a metal, e.g., tin or iron, in the presence of an acid such as hydrochloric acid.

In a further example, amine (-CH₂NH₂) groups may be obtained by reduction of nitriles (-CN), for example by catalytic hydrogenation using for example hydrogen in the presence of a metal catalyst, for example palladium on a support such as carbon, or Raney nickel, in a solvent such as an ether e.g., a cyclic ether such as tetrahydrofuran, at an appropriate temperature, for example from about -78 °C to the reflux temperature of the solvent.

In a further example, amine (-NH₂) groups may be obtained from carboxylic acid groups (-CO₂H) by conversion to the corresponding acyl azide (-CON₃), Curtius rearrangement and hydrolysis of the resultant isocyanate (-N=C=O).

Aldehyde groups (-CHO) may be converted to amine groups (-CH₂NR'R")) by reductive amination employing an amine and a borohydride, for example sodium triacetoxyborohydride or sodium cyanoborohydride, in a solvent such as a halogenated hydrocarbon, for example dichloromethane, or an alcohol such as ethanol, where necessary in the presence of an acid such as acetic acid at around ambient temperature.

In a further example, aldehyde groups may be converted into alkenyl groups (-CH=CHR') by the use of a Wittig or Wadsworth-Emmons reaction using an appropriate phosphorane or phosphonate under standard conditions known to those skilled in the art.

Aldehyde groups may be obtained by reduction of ester groups (such as -CO₂Et) or nitriles (-CN) using diisobutylaluminium hydride in a suitable solvent such as toluene. Alternatively, aldehyde groups may be obtained by the oxidation of alcohol groups using any suitable oxidising agent known to those skilled in the art.

Ester groups (-CO₂R') may be converted into the corresponding acid group (-CO₂H) by acid- or base-catalused hydrolysis, depending on the nature of R. If R is *t*-butyl, acid-catalysed hydrolysis can be achieved for example by treatment with an organic acid such as trifluoroacetic acid in an aqueous solvent, or by treatment with an inorganic acid such as hydrochloric acid in an aqueous solvent.

Carboxylic acid groups (-CO₂H) may be converted into amides (CONHR' or - CONR'R") by reaction with an appropriate amine in the presence of a suitable coupling agent, such as HATU, in a suitable solvent such as dichloromethane.

In a further example, carboxylic acids may be homologated by one carbon (i.e -CO₂H to -CH₂CO₂H) by conversion to the corresponding acid chloride (-COCl) followed by Arndt-Eistert synthesis.

In a further example, -OH groups may be generated from the corresponding ester (e.g., - CO₂R'), or aldehyde (-CHO) by reduction, using for example a complex metal hydride such as lithium aluminium hydride in diethyl ether or tetrahydrofuran, or sodium borohydride in a solvent such as methanol. Alternatively, an alcohol may be prepared by reduction of the corresponding acid (-CO₂H), using for example lithium aluminium hydride in a solvent such as tetrahydrofuran, or by using borane in a solvent such as tetrahydrofuran.

Alcohol groups may be converted into leaving groups, such as halogen atoms or sulfonyloxy groups such as an alkylsulfonyloxy, e.g., trifluoromethylsulfonyloxy or arylsulfonyloxy, e.g., *p*-toluenesulfonyloxy group using conditions known to those skilled in the art. For example, an alcohol may be reacted with thioyl chloride in a halogenated hydrocarbon (e.g., dichloromethane) to yield the corresponding chloride. A base (e.g., triethylamine) may also be used in the reaction.

In another example, alcohol, phenol or amide groups may be alkylated by coupling a phenol or amide with an alcohol in a solvent such as tetrahydrofuran in the presence of a phosphine, e.g., triphenylphosphine and an activator such as diethyl-, diisopropyl, or dimethylazodicarboxylate. Alternatively alkylation may be achieved by deprotonation using a suitable base e.g., sodium hydride followed by subsequent addition of an alkylating agent, such as an alkyl halide.

Aromatic halogen substituents in the compounds may be subjected to halogen-metal exchange by treatment with a base, for example a lithium base such as *n*-butyl or *t*-butyl lithium, optionally at a low temperature, e.g., around -78 °C, in a solvent such as tetrahydrofuran, and then quenched with an electrophile to introduce a desired substituent. Thus, for example, a formyl group may be introduced by using *N,N*-dimethylformamide as the electrophile. Aromatic halogen substituents may alternatively be subjected to metal (e.g., palladium or copper) catalysed reactions, to introduce, for example, acid, ester, cyano, amide, aryl, heteraryl, alkenyl, alkynyl, thio- or amino substituents. Suitable procedures which may be employed include those described by Heck, Suzuki, Stille, Buchwald or Hartwig.

Aromatic halogen substituents may also undergo nucleophilic displacement following reaction with an appropriate nucleophile such as an amine or an alcohol. Advantageously, such a reaction may be carried out at elevated temperature in the presence of microwave irradiation.

### METHODS OF SEPARATION

In each of the exemplary Schemes it may be advantageous to separate reaction products from one another or from starting materials. The desired products of each step or series of steps is separated or purified (hereinafter separated) to the desired degree of homogeneity by the techniques common in the art. Typically such separations involve multiphase extraction, crystallization or trituration from a solvent or solvent mixture, distillation, sublimation, or chromatography. Chromatography can involve any number of methods including, for example: reverse-phase and normal phase; size exclusion; ion exchange; supercritical fluid; high, medium, and low pressure liquid chromatography methods and apparatus; small scale analytical; simulated moving bed (SMB) and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like.

Selection of appropriate methods of separation depends on the nature of the materials involved. Example separation methods include boiling point, and molecular weight in distillation and sublimation, presence or absence of polar functional groups in chromatography, stability of materials in acidic and basic media in multiphase extraction, and the like. One skilled in the art will apply techniques most likely to achieve the desired separation.

Diastereomeric mixtures can be separated into their individual diastereoisomers on the basis of their physical chemical differences by methods well known to those skilled in the art, such as by chromatography or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereoisomers and converting (e.g., hydrolyzing) the individual diastereoisomers to the corresponding pure enantiomers. Also, some of the compounds of the present invention may be atropisomers (e.g., substituted biaryls) and are considered as part of this invention. Enantiomers can also be separated by use of a chiral HPLC column or supercritical fluid chromatography.

A single stereoisomer, e.g., an enantiomer, substantially free of its stereoisomer may be obtained by resolution of the racemic mixture using a method such as formation of diastereomers using optically active resolving agents (Eliel, E. and Wilen, S., Stereochemistry of Organic Compounds, John Wiley & Sons, Inc., New York, 1994; Lochmuller, C. H., J. Chromatogr., 113(3):283-302 (1975)). Racemic mixtures of chiral compounds of the invention can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions. See: Drug Stereochemistry, Analytical Methods and Pharmacology, Irving W. Wainer, Ed., Marcel Dekker, Inc., New York (1993).

Diastereomeric salts can be formed by reaction of enantiomerically pure chiral bases such as brucine, quinine, ephedrine, strychnine, α,-methyl-β-phenylethylamine (amphetamine), and the like with asymmetric compounds bearing acidic functionality, such as carboxylic acid and sulfonic acid. The diastereomeric salts may be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts.

Alternatively, the substrate to be resolved is reacted with one enantiomer of a chiral compound to form a diastereomeric pair (Eliel, E. and Wilen, S., Stereochemistry of Organic Compounds, John Wiley & Sons, Inc., New York, 1994, p. 322). Diastereomeric compounds can be formed by reacting asymmetric compounds with enantiomerically pure chiral derivatizing reagents, such as menthyl derivatives, followed by separation of the diastereomers and hydrolysis to yield the pure or enriched enantiomer. A method of determining optical purity involves making chiral esters, such as a menthyl ester, e.g., (-) menthyl chloroformate in the presence of base, or Mosher ester, α,-methoxy-α,-(trifluoromethyl)phenyl acetate (Jacob, J. Org. Chem. 47:4165 (1982)), of the racemic mixture, and analyzing the NMR spectrum for the presence of the two atropisomeric enantiomers or diastereomers. Stable diastereomers of atropisomeric compounds can be separated and isolated by normal- and reverse-phase chromatography following methods for separation of atropisomeric naphthyl-isoquinolines (WO 96/15111, incorporated herein by reference). By method (3), a racemic mixture of two enantiomers can be separated by chromatography using a chiral stationary phase (Chiral Liquid Chromatography W. J. Lough, Ed., Chapman and Hall, New York, (1989); Okamoto, J. of Chromatogr. 513:375-378 (1990)). Enriched or purified enantiomers can be distinguished by methods used to distinguish other chiral molecules with asymmetric carbon atoms, such as optical rotation and circular dichroism. The absolute stereochemistry of chiral centers and enatiomers can be determined by x-ray crystallography.

Positional isomers and intermediates for their synthesis, may be observed by characterization methods such as NMR and analytical HPLC. For certain compounds where the energy barrier for interconversion is sufficiently high, the E and Z isomers may be separated, for example by preparatory HPLC.

### PHARMACEUTICAL COMPOSITIONS AND ADMINISTRATION

The compounds with which the invention is concerned are JAK kinase inhibitors, such as JAK1 inhibitors, and are useful in the treatment of several diseases, for example, inflammatory diseases, such as asthma.

Accordingly, another embodiment provides pharmaceutical compositions or medicaments containing a compound of the invention, such as a compound of Formula (00A) or a pharmaceutically acceptable salt thereof, and a pharmaceutically acceptable carrier, diluent or excipient, as well as methods of using the compounds of the invention to prepare such compositions and medicaments.

In one example, a compound of Formula (00A) or a pharmaceutically acceptable salt thereof may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but typically ranges anywhere from about 3 to about 8. In one example, a compound of Formula (00A or a pharmaceutically acceptable salt thereof is formulated in an acetate buffer, at pH 5. In another embodiment, the compounds of the present invention, such as a compound of Formula (00A) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

It will be understood that the specific dose level for any particular patient will depend upon a variety of factors including the activity of the specific compound employed, the age, body weight, general health, sex, diet, time of administration, route of administration, rate of excretion, drug combination and the severity of the particular disease undergoing treatment. Optimum dose levels and frequency of dosing will be determined by clinical trial, as is required in the pharmaceutical art. In general, the daily dose range for oral administration will lie within the range of from about 0.001 mg to about 100 mg per kg body weight of a human, often 0.01 mg to about 50 mg per kg, for example 0.1 to 10 mg per kg, in single or divided doses. In general, the daily dose range for inhaled administration will lie within the range of from about 0.1 µg to about 1 mg per kg body weight of a human, preferably 0.1 µg to 50 µg per kg, in single or divided doses. On the other hand, it may be necessary to use dosages outside these limits in some cases.

The compounds of the invention, such as a compound of Formula (00A) or a pharmaceutically acceptable salt thereof, may be administered by any suitable means, including oral, topical (including buccal and sublingual), rectal, vaginal, transdermal, parenteral, subcutaneous, intraperitoneal, intrapulmonary, intradermal, intrathecal, inhaled and epidural and intranasal, and, if desired for local treatment, intralesional administration. Parenteral infusions include intramuscular, intravenous, intraarterial, intraperitoneal, or subcutaneous administration. In some embodiments, inhaled administration is employed.

The compounds of the present invention, such as a compound of Formula (00A), or a pharmaceutically acceptable salt thereof, may be administered in any convenient administrative form, e.g., tablets, powders, capsules, lozenges, granules, solutions, dispersions, suspensions, syrups, sprays, vapors, suppositories, gels, emulsions, patches, etc. Such compositions may contain components conventional in pharmaceutical preparations, e.g., diluents (e.g., glucose, lactose or mannitol), carriers, pH modifiers, buffers, sweeteners, bulking agents, stabilizing agents, surfactants, wetting agents, lubricating agents, emulsifiers, suspending agents, preservatives, antioxidants, opaquing agents, glidants, processing aids, colorants, perfuming agents, flavoring agents, other known additives as well as further active agents.

Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. For example, carriers include solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, pp 1289-1329, 1990). Except insofar as any conventional carrier is incompatible with the active ingredient, its use in the therapeutic or pharmaceutical compositions is contemplated. Exemplary excipients include dicalcium phosphate, mannitol, lactose, starch, magnesium stearate, sodium saccharine, cellulose, magnesium carbonate or combinations thereof. A pharmaceutical composition may comprise different types of carriers or excipients depending on whether it is to be administered in solid, liquid or aerosol form, and whether it need to be sterile for such routes of administration.

For example, tablets and capsules for oral administration may be in unit dose presentation form, and may contain conventional excipients such as binding agents, for example syrup, acacia, gelatin, sorbitol, tragacanth, or polyvinyl-pyrrolidone; fillers, for example, lactose, sugar, maize-starch, calcium phosphate, sorbitol or glycine; tabletting lubricant, for example, magnesium stearate, talc, polyethylene glycol or silica; disintegrants, for example, potato starch, or acceptable wetting agents such as sodium lauryl sulfate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example, sorbitol, syrup, methyl cellulose, glucose syrup, gelatin hydrogenated edible fats; emulsifying agents, for example, lecithin, sorbitan monooleate, or acacia; non-aqueous vehicles (which may include edible oils), for example, almond oil, fractionated coconut oil, oily esters such as glycerine, propylene glycol, or ethyl alcohol; preservatives, for example, methyl or propyl p-hydroxybenzoate or sorbic acid, and if desired conventional flavoring or coloring agents.

For topical application to the skin, a compound may be made up into a cream, lotion or ointment. Cream or ointment formulations which may be used for the drug are conventional formulations well known in the art, for example as described in standard textbooks of pharmaceutics such as the British Pharmacopoeia.

Compounds of the invention, such as a compound of Formula (00A or a pharmaceutically acceptable salt thereof, may also be formulated for inhalation, for example, as a nasal spray, or dry powder or aerosol inhalers. For delivery by inhalation, the compound is typically in the form of microparticles, which can be prepared by a variety of techniques, including spray-drying, freeze-drying and micronisation. Aerosol generation can be carried out using, for example, pressure-driven jet atomizers or ultrasonic atomizers, such as by using propellant-driven metered aerosols or propellant-free administration of micronized compounds from, for example, inhalation capsules or other "dry powder" delivery systems.

By way of example, a composition of the invention may be prepared as a suspension for delivery from a nebulizer or as an aerosol in a liquid propellant, for example, for use in a pressurized metered dose inhaler (PMDI). Propellants suitable for use in a PMDI are known to the skilled person, and include CFC-12, HFA-134a, HFA-227, HCFC-22 (CCl₂F₂) and HFA-152 (CH₄F₂ and isobutane).

In some embodiments, a composition of the invention is in dry powder form, for delivery using a dry powder inhaler (DPI). Many types of DPI are known.

Microparticles for delivery by administration may be formulated with excipients that aid delivery and release. For example, in a dry powder formulation, microparticles may be formulated with large carrier particles that aid flow from the DPI into the lung. Suitable carrier particles are known, and include lactose particles; they may have a mass median aerodynamic diameter of, for example, greater than 90 µm.

In the case of an aerosol-based formulation, an example is:

| | |
|---|---|
| Compound of the invention* | 24 mg / canister |
| Lecithin, NF Liq. Conc. | 1.2 mg / canister |
| Trichlorofluoromethane, NF | 4.025 g / canister |
| Dichlorodifluoromethane, NF | 12.15 g / canister. |

| | |
|---|---|
| * Such as a compound of Formula (00A) or a pharmaceutically acceptable salt thereof. | |

A compound, such as a compound of Formula (00A) or a pharmaceutically acceptable salt thereof, may be dosed as described depending on the inhaler system used. In addition to the compound, the administration forms may additionally contain excipients as described above, or, for example, propellants (e.g., Frigen in the case of metered aerosols), surface-active substances, emulsifiers, stabilizers, preservatives, flavorings, fillers (e.g., lactose in the case of powder inhalers) or, if appropriate, further active compounds.

For the purposes of inhalation, a large number of systems are available with which aerosols of optimum particle size can be generated and administered, using an inhalation technique which is appropriate for the patient. In addition to the use of adaptors (spacers, expanders) and pear-shaped containers (e.g., Nebulator®, Volumatic®), and automatic devices emitting a puffer spray (Autohaler®), for metered aerosols, in the case of powder inhalers in particular, a number of technical solutions are available (e.g., Diskhaler®, Rotadisk®, Turbohaler® or the inhalers, for example, as described in U.S. Patent No. 5,263,475, incorporated herein by reference). Additionally, compounds of the invention, such as a compound of Formula (00A) or a pharmaceutically acceptable salt thereof, may be delivered in multi-chamber devices thus allowing for delivery of combination agents.

The compound, such as a compound of Formula (00A) or a pharmaceutically acceptable salt thereof, may also be administered parenterally in a sterile medium. Depending on the vehicle and concentration used, the compound can either be suspended or dissolved in the vehicle. Advantageously, adjuvants such as a local anaesthetic, preservative or buffering agents can be dissolved in the vehicle.

### TARGETED INHALED DRUG DELIVERY

Optimisation of drugs for delivery to the lung by topical (inhaled) administration has been recently reviewed (Cooper, A. E. et al. Curr. Drug Metab. 2012, 13, 457-473). Due to limitations in the delivery device, the dose of an inhaled drug is likely to be low (approximately <1mg/day) in humans which necessitates highly potent molecules. For compounds destined to be delivered via dry powder inhalation there is also a requirement to be able to generate crystalline forms of the compound that can be micronized to 1-5 µm in size. Additionally, the compound needs to maintain a sufficient concentration in the lung over a given time period so as to be able to exert a pharmacological effect of the desired duration, and for pharmacological targets where systemic inhibition of said target is undesired, to have a low systemic exposure. The lung has an inherently high permeability to both large molecules (proteins, peptides) as well as small molecules with concomitant short lung half-lives, thus it is necessary to attenuate the lung absorption rate through modification of one or more features of the compounds: minimizing membrane permeability, reducing dissolution rate, or introducing a degree of basicity into the compound to enhance binding to the phospholipid-rich lung tissue or through trapping in acidic sub-cellular compartments such as lysosomes (pH 5). Accordingly, in some embodiments, compounds of the present invention exhibit one or more of these features.

### METHODS OF TREATMENT WITH AND USES OF JANUS KINASE INHIBITORS

The compounds of the present invention, such as a compound of Formula (00A) or a pharmaceutically acceptable salt thereof, inhibit the activity of a Janus kinase, such as JAK1 kinase. For example, a compound of Formula (00A) or a pharmaceutically acceptable salt thereof, inhibits the phosphorylation of signal transducers and activators of transcription (STATs) by JAK1 kinase as well as STAT mediated cytokine production. Compounds of the present invention are useful for inhibiting JAK1 kinase activity in cells through cytokine pathways, such as IL-6, IL-15, IL-7, IL-2, IL-4, IL-9, IL-10, IL-13, IL-21, G-CSF, IFNalpha, IFNbeta or IFNgamma pathways. Accordingly, in one embodiment is provided a method of contacting a cell with a compound of Formula (00A) or a pharmaceutically acceptable salt thereof, to inhibit a Janus kinase activity in the cell (e.g., JAK1 activity).

The compounds can be used for the treatment of immunological disorders driven by aberrant IL-6, IL-15, IL-7, IL-2, IL-4, IL9, IL-10, IL-13, IL-21, G-CSF, IFNalpha, IFNbeta or IFNgamma cytokine signaling.

Accordingly, one embodiment includes a compound of Formula (00A) or a pharmaceutically acceptable salt thereof, for use in therapy.

In some embodiments, there is provided use a compound of Formula (00A) or a pharmaceutically acceptable salt thereof, in the treatment of an inflammatory disease. Further provided is use of a compound of Formula (00A) or a pharmaceutically acceptable salt thereof for the preparation of a medicament for the treatment of an inflammatory disease, such as asthma. Also provided is a compound of Formula (00A) or a pharmaceutically acceptable salt thereof for use in the treatment of an inflammatory disease, such as asthma.

Another embodiment includes a method of preventing, treating or lessening the severity of a disease or condition, such as asthma, responsive to the inhibition of a Janus kinase activity, such as JAK1 kinase activity, in a patient. The method can include the step of administering to a patient a therapeutically effective amount of a compound of Formula (00A) or a pharmaceutically acceptable salt thereof. In one embodiment, the disease or condition responsive to the inhibition of a Janus kinase, such as JAK1 kinase, is asthma.

In one embodiment, the disease or condition is cancer, stroke, diabetes, hepatomegaly, cardiovascular disease, multiple sclerosis, Alzheimer's disease, cystic fibrosis, viral disease, autoimmune diseases, atherosclerosis, restenosis, psoriasis, rheumatoid arthritis, inflammatory bowel disease, asthma, allergic disorders, inflammation, neurological disorders, a hormone-related disease, conditions associated with organ transplantation (e.g., transplant rejection), immunodeficiency disorders, destructive bone disorders, proliferative disorders, infectious diseases, conditions associated with cell death, thrombin-induced platelet aggregation, liver disease, pathologic immune conditions involving T cell activation, CNS disorders or a myeloproliferative disorder.

In one embodiment, the inflammatory disease is rheumatoid arthritis, psoriasis, asthma, inflammatory bowel disease, contact dermatitis or delayed hypersensitivity reactions. In one embodiment, the autoimmune disease is rheumatoid arthritis, lupus or multiple sclerosis.

In one embodiment, the cancer is breast, ovary, cervix, prostate, testis, penile, genitourinary tract, seminoma, esophagus, larynx, gastric, stomach, gastrointestinal, skin, keratoacanthoma, follicular carcinoma, melanoma, lung, small cell lung carcinoma, non-small cell lung carcinoma (NSCLC), lung adenocarcinoma, squamous carcinoma of the lung, colon, pancreas, thyroid, papillary, bladder, liver, biliary passage, kidney, bone, myeloid disorders, lymphoid disorders, hairy cells, buccal cavity and pharynx (oral), lip, tongue, mouth, salivary gland, pharynx, small intestine, colon, rectum, anal, renal, prostate, vulval, thyroid, large intestine, endometrial, uterine, brain, central nervous system, cancer of the peritoneum, hepatocellular cancer, head cancer, neck cancer, Hodgkin's or leukemia.

In one embodiment, the disease is a myeloproliferative disorder. In one embodiment, the myeloproliferative disorder is polycythemia vera, essential thrombocytosis, myelofibrosis or chronic myelogenous leukemia (CML).

Another embodiment includes the use of a compound of Formula (00A) or a pharmaceutically acceptable salt thereof, for the manufacture of a medicament for the treatment of a disease described herein (e.g., an inflammatory disorder, an immunological disorder or cancer). In one embodiment, the invention provides a method of treating a disease or condition as described herein e.g., an inflammatory disorder, an immunological disorder or cancer) by targeting inhibition of a JAK kinase, such as JAK1.

### COMBINATION THERAPY

The compounds may be employed alone or in combination with other agents for treatment. The second compound of a pharmaceutical composition or dosing regimen typically has complementary activities to the compound of this invention such that they do not adversely affect each other. Such agents are suitably present in combination in amounts that are effective for the purpose intended. The compounds may be administered together in a unitary pharmaceutical composition or separately and, when administered separately this may occur simultaneously or sequentially. Such sequential administration may be close or remote in time.

For example, other compounds may be combined with a compound of Formula (00A) or a pharmaceutically acceptable salt thereof for the prevention or treatment of inflammatory diseases, such as asthma. Suitable therapeutic agents for a combination therapy include, but are not limited to: an adenosine A2A receptor antagonist; an anti-infective; a non-steroidal Glucocorticoid Receptor (GR Receptor) agonist; an antioxidant; a □2 adrenoceptor agonist; a CCR1 antagonist; a chemokine antagonist (not CCR1); a corticosteroid; a CRTh2 antagonist; a DP1 antagonist; a formyl peptide receptor antagonist; a histone deacetylase activator; a chloride channel hCLCA1 blocker; an epithelial sodium channel blocker (ENAC blocker; an inter-cellular adhesion molecule 1 blocker (ICAM blocker); an IKK2 inhibitor; a JNK inhibitor; a cyclooxygenase inhibitor (COX inhibitor); a lipoxygenase inhibitor; a leukotriene receptor antagonist; a dual □2 adrenoceptor agonist/M3 receptor antagonist (MABA compound); a MEK-1 inhibitor; a myeloperoxidase inhibitor (MPO inhibitor); a muscarinic antagonist; a p38 MAPK inhibitor; a phosphodiesterase PDE4 inhibitor; a phosphatidylinositol 3-kinase δ inhibitor (PI3-kinase δ inhibitor); a phosphatidylinositol 3-kinase γ inhibitor (PI3-kinase γ inhibitor); a peroxisome proliferator activated receptor agonist (PPARγ agonist); a protease inhibitor; a retinoic acid receptor modulator (RAR γ modulator); a statin; a thromboxane antagonist; a TLR7 receptor agonist; or a vasodilator.

In addition, a compound of Formula (00A) or a pharmaceutically acceptable salt thereof, may be combined with: (1) corticosteroids, such as alclometasone dipropionate, amelometasone, beclomethasone dipropionate, budesonide, butixocort propionate, biclesonide, blobetasol propionate, desisobutyrylciclesonide, dexamethasone, dtiprednol dicloacetate, fluocinolone acetonide, fluticasone furoate, fluticasone propionate, loteprednol etabonate (topical) or mometasone furoate; (2) β2-adrenoreceptor agonists such as salbutamol, albuterol, terbutaline, fenoterol, bitolterol, carbuterol, clenbuterol, pirbuterol, rimoterol, terbutaline, tretoquinol, tulobuterol and long acting β2-adrenoreceptor agonists such as metaproterenol, isoproterenol, isoprenaline, salmeterol, indacaterol, formoterol (including formoterol fumarate), arformoterol, carmoterol, abediterol, vilanterol trifenate, olodaterol; (3) corticosteroid/long acting β2 agonist combination products such as salmeterol/fluticasone propionate (Advair®, also sold as Seretide®), formoterol/budesonide (Symbicort®), formoterol/fluticasone propionate (Flutiform®), formoterol/ciclesonide, formoterol/mometasone furoate, indacaterol/mometasone furoate, vilanterol trifenate/fluticasone furoate, or arformoterol/ciclesonide; (4) anticholinergic agents, for example, muscarinic-3 (M3) receptor antagonists such as ipratropium bromide, tiotropium bromide, aclidinium (LAS-34273), glycopyrronium bromide, umeclidinium bromide; (5) M3-anticholinergic/β2-adrenoreceptor agonist combination products such as vilanterol /umeclidinium (Anoro® Ellipta®), olodaterol/tiotropium bromide, glycopyrronium bromide/indacaterol (Ultibro®, also sold as Xoterna®), fenoterol hydrobromide/ipratropium bromide (Berodual®), albuterol sulfate/ipratropium bromide (Combivent®), formoterol fumarate/glycopyrrolate, or aclidinium bromide/formoterol (6) dual pharmacology M3-anticholinergic/β2-adrenoreceptor agonists such as batefenterol succinate, AZD-2115 or LAS-190792; (7) leukotriene modulators, for example, leukotriene antagonists such as montelukast, zafirulast or pranlukast or leukotriene biosynthesis inhibitors such as zileuton, or LTB4 antagonists such as amelubant, or FLAP inhibitors such as fiboflapon, GSK-2190915; (8) phosphodiesterase-IV (PDE-IV) inhibitors (oral or inhaled), such as roflumilast, cilomilast, oglemilast, rolipram, tetomilast, AVE-8112, revamilast, CHF 6001; (9) antihistamines, for example, selective histamine-1 (H1) receptor antagonists such as fexofenadine, citirizine, loratidine or astemizole or dual H1/H3 receptor antagonists such as GSK 835726, or GSK 1004723; (10) antitussive agents, such as codeine or dextramorphan; (11) a mucolytic, for example, N-acetyl cysteine or fudostein; (12) a expectorant/mucokinetic modulator, for example, ambroxol, hypertonic solutions (e.g., saline or mannitol) or surfactant; (13) a peptide mucolytic, for example, recombinant human deoxyribonoclease I (dornase-alpha and rhDNase) or helicidin; (14) antibiotics, for example azithromycin, tobramycin or aztreonam; (15) non-selective COX-1/COX-2 inhibitors, such as ibuprofen or ketoprofen; (16) COX-2 inhibitors, such as celecoxib and rofecoxib; (17) VLA-4 antagonists, such as those described in WO97/03094 and WO97/02289, each incorporated herein by reference; (18) TACE inhibitors and TNF-α inhibitors, for example anti-TNF monoclonal antibodies, such as Remicade® and CDP-870 and TNF receptor immunoglobulin molecules, such as Enbrel®; (19) inhibitors of matrix metalloprotease, for example MMP-12; (20) human neutrophil elastase inhibitors, such as BAY-85-8501 or those described in WO2005/026124, WO2003/053930 and WO06/082412, each incorporated herein by reference; (21) A2b antagonists such as those described in WO2002/42298, incorporated herein by reference; (22) modulators of chemokine receptor function, for example antagonists of CCR3 and CCR8; (23) compounds which modulate the action of other prostanoid receptors, for example, a thromboxane A₂ antagonist; DP1 antagonists such as laropiprant or asapiprant CRTH2 antagonists such as OC000459, fevipiprant, ADC 3680 or ARRY 502; (24) PPAR agonists including PPAR alpha agonists (such as fenofibrate), PPAR delta agonists, PPAR gamma agonists such as pioglitazone, rosiglitazone and balaglitazone; (25) methylxanthines such as theophylline or aminophylline and methylxanthine/corticosteroid combinations such as theophylline/budesonide, theophylline/fluticasone propionate, theophylline/ciclesonide, theophylline/mometasone furoate and theophylline/beclometasone dipropionate; (26) A2a agonists such as those described in EP1052264 and EP1241176; (27) CXCR2 or IL-8 antagonists such as AZD-5069, AZD-4721, danirixin; (28) IL-R signalling modulators such as kineret and ACZ 885; (29) MCP-1 antagonists such as ABN-912; (30) a p38 MAPK inhibitor such as BCT197, JNJ49095397, losmapimod or PH-797804; (31) TLR7 receptor agonists such as AZD 8848; (32) PI3-kinase inhibitors such as RV1729 or GSK2269557.

In some embodiments a compound of Formula (00A) or a pharmaceutically acceptable salt thereof, can be used in combination with one or more additional drugs, for example anti-hyperproliferative, anti-cancer, cytostatic, cytotoxic, anti-inflammatory or chemotherapeutic agents, such as those agents disclosed in U.S. Publ. Appl. No. 2010/0048557, incorporated herein by reference. A compound of Formula (00A) or a pharmaceutically acceptable salt thereof, can be also used in combination with radiation therapy or surgery, as is known in the art.

### ARTICLES OF MANUFACTURE

Another embodiment includes an article of manufacture (e.g., a kit) for treating a disease or disorder responsive to the inhibition of a Janus kinase, such as a JAK1 kinase. The kit can comprise:
(a) a first pharmaceutical composition comprising a compound of Formula (00A) or a pharmaceutically acceptable salt thereof; and
(b) instructions for use.

In another embodiment, the kit further comprises:
(c) a second pharmaceutical composition, such as a pharmaceutical composition comprising an agent for treatment as described above, such as an agent for treatment of an inflammatory disorder, or a chemotherapeutic agent.

In one embodiment, the instructions describe the simultaneous, sequential or separate administration of said first and second pharmaceutical compositions to a patient in need thereof.

In one embodiment, the first and second compositions are contained in separate containers. In another embodiment, the first and second compositions are contained in the same container.

Containers for use include, for example, bottles, vials, syringes, blister pack, etc. The containers may be formed from a variety of materials such as glass or plastic. The container includes a compound of Formula (00A) or a pharmaceutically acceptable salt thereof, which is effective for treating the condition and may have a sterile access port (for example the container may be an intravenous solution bag or a vial having a stopper pierceable by a hypodermic injection needle). The label or package insert indicates that the compound is used for treating the condition of choice, such as asthma or cancer. In one embodiment, the label or package inserts indicates that the compound can be used to treat a disorder. In addition, the label or package insert may indicate that the patient to be treated is one having a disorder characterized by overactive or irregular Janus kinase activity, such as overactive or irregular JAK1 activity. The label or package insert may also indicate that the compound can be used to treat other disorders.

Alternatively, or additionally, the kit may further comprise a second (or third) container comprising a pharmaceutically acceptable buffer, such as bacteriostatic water for injection (BWFI), phosphate-buffered saline, Ringer's solution or dextrose solution. It may further include other materials desirable from a commercial and user standpoint, including other buffers, diluents, filters, needles, and syringes.

In order to illustrate the invention, the following examples are included. However, it is to be understood that these examples do not limit the invention and are only meant to suggest a method of practicing the invention. Persons skilled in the art will recognize that the chemical reactions described may be readily adapted to prepare other compounds of the present invention, and alternative methods for preparing the compounds are within the scope of this invention. For example, the synthesis of non-exemplified compounds according to the invention may be successfully performed by modifications apparent to those skilled in the art, e.g., by appropriately protecting interfering groups, by utilizing other suitable reagents known in the art other than those described, or by making routine modifications of reaction conditions. Alternatively, other reactions disclosed herein or known in the art will be recognized as having applicability for preparing other compounds of the invention.

### EXAMPLES

### General Experimental Details

All solvents and commercial reagents were used as received unless otherwise stated. Where products were purified by chromatography on silica this was carried out using either a glass column manually packed with silica gel (Kieselgel 60, 220-440 mesh, 35-75 µm) or an Isolute® SPE Si II cartridge. 'Isolute SPE Si cartridge' refers to a pre-packed polypropylene column containing unbonded activated silica with irregular particles with average size of 50 µµm and nominal 60Å porosity. Where an Isolute® SCX-2 cartridge was used, 'Isolute® SCX-2 cartridge' refers to a pre-packed polypropylene column containing a non-end-capped propylsulphonic acid functionalised silica strong cation exchange sorbent.

### Procedures and LCMS Conditions

### Method A (LCMS15)

Experiments were performed on a SHIMADZU 20A HPLC with a C18-reverse-phase column (50 x 3 mm XtimateTM -C18, 2.2 µm particle size), elution with solvent A: water + 0.05% trifluoroacetic acid; solvent B: acetonitrile + 0.05% trifluoroacetic acid. Gradient:

| Gradient - Time | flow ml/min | %A | %B |
|---|---|---|---|
| 0.00 | 1.0 | 95 | 5 |
| 2.00 | 1.0 | 0 | 100 |
| 3.20 | 1.0 | 0 | 100 |
| 3.30 | 1.0 | 95 | 5 |

Detection - UV (220 and 254 nm) and ELSD

### Method B (LCMS30)

Experiments were performed on a SHIMADZU 20A HPLC with a C18-reverse-phase column (50 x 2.1 mm XtimateTM -C18, 2.6 µm particle size), elution with solvent A: Water / 0.05%TFA; solvent B: Acetonitrile/0.05%TFA:

| Gradient - Time | flow ml/min | %A | %B |
|---|---|---|---|
| 0.00 | 1.0 | 95 | 5 |
| 1.10 | 1.0 | 0 | 100 |
| 1.60 | 1.0 | 0 | 100 |
| 1.7 | 1.0 | 95 | 5 |

Detection - UV (220 and 254 nm) and ELSD

### Method C (LCMS33)

Experiments were performed on a SHIMADZU 20A HPLC with a C18-reverse-phase column (50 x 3 mm XtimateTM -C18, 2.2 µm particle size), elution with solvent A: water + 0.1% formic acid; solvent B: acetonitrile + 0.05% formic acid. Gradient:

| Gradient - Time | flow ml/min | %A | %B |
|---|---|---|---|
| 0.00 | 1.0 | 95 | 5 |
| 2.00 | 1.0 | 0 | 100 |
| 3.10 | 1.0 | 0 | 100 |
| 3.20 | 1.0 | 95 | 5 |

Detection - UV (220 and 254 nm) and ELSD

### Method D (LCMS34)

Experiments were performed on a SHIMADZU 20A HPLC with a C18-reverse-phase column (50 x 3 mm, Gemini-NX 3µ-C18 110A, 3.0 µm particle size), elution with solvent A: water/5 mM NH₄HCO₃; solvent B: acetonitrile. Gradient:

| Gradient - Time | flow ml/min | %A | %B |
|---|---|---|---|
| 0.00 | 1.2 | 90 | 10 |
| 2.20 | 1.2 | 5 | 95 |
| 3.20 | 1.2 | 5 | 95 |
| 3.30 | 1.2 | 90 | 10 |

Detection - UV (220 and 254 nm) and ELSD

### Method E (LCMS39)

Experiments were performed on a SHIMADZU 20A HPLC with a C18-reverse-phase column (50 x 2.1 mm XtimateTM -C18, 2.7 µm particle size), elution with solvent A: Water / 0.1%FA; solvent B: Acetonitrile/0.1%FA:

| Gradient - Time | flow ml/min | %A | %B |
|---|---|---|---|
| 0.00 | 1.0 | 90 | 10 |
| 1.10 | 1.0 | 0 | 100 |
| 1.60 | 1.0 | 0 | 100 |
| 1.70 | 1.0 | 90 | 10 |

Detection - UV (220 and 254 nm) and ELSD

### Method F (LCMS45)

Experiments were performed on a SHIMADZU 20A HPLC with a C18-reverse-phase column (30 x 2.1 mm Ascentis Express C18, 2.7 µm particle size), elution with solvent A: water + 0.05% trifluoroacetic acid; solvent B: acetonitrile + 0.05% trifluoroacetic acid. Gradient:

| Gradient - Time | flow ml/min | %A | %B |
|---|---|---|---|
| 0.00 | 1.0 | 95 | 5 |
| 4.00 | 1.0 | 40 | 60 |
| 4.50 | 1.0 | 5 | 95 |
| 5.00 | 1.0 | 5 | 95 |
| 5.10 | 1.0 | 95 | 5 |

Detection - UV (220 and 254 nm) and ELSD

### Method G (LCMS45)

Experiments were performed on a SHIMADZU 20A HPLC with a C18-reverse-phase column (50 x 2.1 mm XtimateTM -C18, 2.7 µm particle size), elution with solvent A: water + 0.05% trifluoroacetic acid; solvent B: acetonitrile + 0.05% trifluoroacetic acid. Gradient:

| Gradient - Time | flow ml/min | %A | %B |
|---|---|---|---|
| 0.00 | 1.0 | 95 | 5 |
| 1.10 | 1.0 | 0 | 100 |
| 1.60 | 1.0 | 0 | 100 |
| 1.70 | 1.0 | 95 | 5 |

Detection - UV (220 and 254 nm) and ELSD

### Method H (LCMS45)

Experiments were performed on a SHIMADZU 20A HPLC with a C18-reverse-phase column (50 x 2.1 mm XtimateTM -C18, 2.7 µm particle size), elution with solvent A: water + 0.05% trifluoroacetic acid; solvent B: acetonitrile + 0.05% trifluoroacetic acid. Gradient:

| Gradient - Time | flow ml/min | %A | %B |
|---|---|---|---|
| 0.00 | 1.0 | 95 | 5 |
| 2.00 | 1.0 | 5 | 95 |
| 2.60 | 1.0 | 5 | 95 |
| 2.70 | 1.0 | 95 | 5 |

Detection - UV (220 and 254 nm) and ELSD

### Method I (LCMS53)

Experiments were performed on a SHIMADZU 20A HPLC with a C18-reverse-phase column (50 x 3.0 mm XtimateTM XB -C18, 2.6 µm particle size), elution with solvent A: Water / 0.05%TFA; solvent B: Acetonitrile/0.05%TFA:

| Gradient - Time | flow ml/min | %A | %B |
|---|---|---|---|
| 0.00 | 1.5 | 95 | 5 |
| 1.20 | 1.5 | 0 | 100 |
| 1.70 | 1.5 | 0 | 100 |
| 1.80 | 1.5 | 95 | 5 |

Detection - UV (220 and 254 nm) and ELSD

### Method J (LCMS34)

Experiments were performed on a SHIMADZU 20A HPLC with a C18-reverse-phase column (50 x 3 mm, Gemini-NX 3µ-C18 110A, 3.0 µm particle size), elution with solvent A: water/5 mM NH₄HCO₃; solvent B: acetonitrile. Gradient:

| Gradient - Time | flow ml/min | %A | %B |
|---|---|---|---|
| 0.00 | 1.2 | 90 | 10 |
| 3.60 | 1.2 | 30 | 70 |
| 5.20 | 1.2 | 30 | 70 |
| 5.30 | 1.2 | 90 | 10 |

Detection - UV (220 and 254 nm) and ELSD

### Method K (LCMS15)

Experiments were performed on a SHIMADZU 20A HPLC with a C18-reverse-phase column (50 x 3 mm Shim-Pack XR-ODS, 2.2 µm particle size), elution with solvent A: water + 0.05% trifluoroacetic acid; solvent B: acetonitrile + 0.05% trifluoroacetic acid. Gradient:

| Gradient - Time | flow ml/min | %A | %B |
|---|---|---|---|
| 0.00 | 1.0 | 95 | 5 |
| 3.20 | 1.0 | 40 | 60 |
| 3.80 | 1.0 | 0 | 100 |
| 4.60 | 1.0 | 0 | 100 |
| 4.75 | 1.0 | 95 | 5 |

Detection - UV (220 and 254 nm) and ELSD

### Method L (LCMS34)

Experiments were performed on a SHIMADZU 20A HPLC with a C18-reverse-phase column (50 x 3 mm, Gemini-NX 3µ-C18 110A, 3.0 µm particle size), elution with solvent A: water/5 mM NH₄HCO₃; solvent B: acetonitrile. Gradient:

| Gradient - Time | flow ml/min | %A | %B |
|---|---|---|---|
| 0.00 | 1.2 | 90 | 10 |
| 4.00 | 1.2 | 40 | 60 |
| 4.50 | 1.2 | 10 | 90 |
| 5.50 | 1.2 | 10 | 90 |
| 5.70 | 1.2 | 90 | 10 |

Detection - UV (220 and 254 nm) and ELSD

### Method M (LCMS34)

Experiments were performed on a SHIMADZU 20A HPLC with a C18-reverse-phase column (50 x 3 mm, Gemini-NX 3µ-C18 110A, 3.0 µm particle size), elution with solvent A: water/5 mM NH₄HCO₃; solvent B: acetonitrile. Gradient:

| Gradient - Time | flow ml/min | %A | %B |
|---|---|---|---|
| 0.00 | 1.2 | 90 | 10 |
| 3.20 | 1.2 | 30 | 70 |
| 4.30 | 1.2 | 30 | 70 |
| 4.40 | 1.2 | 90 | 10 |

Detection - UV (220 and 254 nm) and ELSD

### Method N (LCMS40)

Experiments were performed on a SHIMADZU 20A HPLC with a C18-reverse-phase column (50 x 2.1 mm Asentis Express C18, 2.7 µm particle size), elution with solvent A: Water / 0.1%FA; solvent B: Acetonitrile/0.05%FA:

| Gradient - Time | flow ml/min | %A | %B |
|---|---|---|---|
| 0.00 | 1.0 | 95 | 5 |
| 3.70 | 1.0 | 55 | 45 |
| 4.30 | 1.0 | 5 | 95 |
| 4.80 | 1.0 | 5 | 95 |
| 4.90 | 1.0 | 95 | 5 |

Detection - UV (220 and 254 nm) and ELSD

### Method O (LCMS53)

Experiments were performed on a SHIMADZU 20A HPLC with a C18-reverse-phase column (50 x 2.1 mm Ascentis Express C18, 2.7 µm particle size), elution with solvent A: water + 0.05% trifluoroacetic acid; solvent B: acetonitrile + 0.05% trifluoroacetic acid. Gradient:

| Gradient - Time | flow ml/min | %A | %B |
|---|---|---|---|
| 0.00 | 1.0 | 95 | 5 |
| 2.00 | 1.0 | 0 | 100 |
| 2.70 | 1.0 | 0 | 100 |
| 2.80 | 1.0 | 95 | 5 |

Detection - UV (220 and 254 nm) and ELSD

### Method P

Experiments were performed on an Agilent 1290 UHPLC coupled with Agilent MSD (6140) mass spectrometer using ESI as ionization source. The LC separation used a Phenomenex XB-C18, 1.7mm, 50 × 2.1 mm column with a 0.4 ml / minute flow rate. Solvent A is water with 0.1% FA and solvent B is acetonitrile with 0.1% FA. The gradient is 2 - 98% solvent B over 7 min and holds at 98%B for 1.5 min following equilibration for 1.5 min. LC column temperature is 40 °C. UV absorbance was collected at 220nm and 254nm.

### List of Common Abbreviations

- ACN: Acetonitrile
- Brine: Saturated aqueous sodium chloride solution
- CH₃OD: Deuterated Methanol
- CDCl₃: Deuterated Chloroform
- DCM: Dichloromethane
- DIEA or DIPEA: Diisopropylethylamine
- DMA: Dimethylacetamide
- DMAP: 4-Dimethylaminopyridine
- DMF: Dimethylformamide
- DMSO: Dimethylsulfoxide
- DMSO-*d6*: Deuterated dimethylsulfoxide
- EDC or EDCI: 1-Ethyl-3-(3-dimethylaminopropyl)carbodiimide
- EtOAc: Ethyl acetate
- EtOH: Ethanol
- FA: Formic Acid
- HOAc: Acetic acid
- g: Gram
- h: hour
- HATU: (O-(7-azabenzotriazol-1-yl)-N,N,N',N'-tetramethyluronium hexafluorophosphate)
- HCl: Hydrochloric acid
- HOBt: Hydroxybenzotriazole
- HPLC: High performance liquid chromatography
- IMS: Industrial methylated spirits
- L: Liter
- LCMS: Liquid chromatography-mass spectrometry
- LiHMDS or LHMDS: Lithium hexamethydisylazide
- MDAP: Mass directed automated purification
- MeCN: Acetonitrile
- MeOH: Methanol
- min: minute
- mg: Milligram
- mL: Millilitre
- NMR: Nuclear magnetic resonance spectroscopy
- Pd₂(dba)₃.CHCl₃: Tris(dibenzylideneacetone)dipalladium(0)-chloroform adduct
- PE: Petroleum ether
- Prep-HPLC: Preparative high perfomance liquid chromatography
- SCX-2: Strong cation exchange
- TBAF: Tetra-n-butylammonium fluoride
- THF: Tetrahydrofuran
- TFA: Trifluoroacetic acid
- Xantphos: 4,5-Bis(diphenylphosphino)-9,9-dimethylxanthene

### Example 1

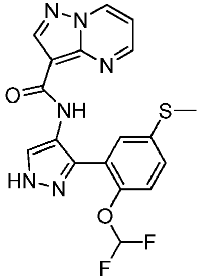

N-(3-(2-(difluoromethoxy)-5-(methylthio)phenyl)-1H-pyrazol-4-yl)pyrazolo[1,5-a]pyrimidine-3-carboxamide

To a solution of 4-bromo-2-iodophenol (282 g, 943.447 mmol) in N,N-dimethylformamide (2000 mL) and water (500 mL) was added sodium 2-chloro-2,2-difluoroacetate (216 g, 1.417 mol), Cs₂CO₃ (617 g, 1.894 mol). The resulting mixture was stirred overnight at 120°C, allowed to cool to room temperature, poured into ice water (3000 mL). The resulting solution was extracted with ethyl acetate (3x1500 mL) and the organic layers combined. The ethyl acetate extracts were washed with brine (1000 mL), dried over anhydrous sodium sulfate and concentrated under vacuum. The residue was purified by flash chromatography on silica gel eluting with ethyl acetate/petroleum ether (1:10) to afford 300 g (91%) of 4-bromo-1-(difluoromethoxy)-2-iodobenzene as a yellow oil. ¹H NMR (300 MHz, CDCl₃) □: (ppm) 7.96 (dd, *J* = 5.7 Hz, 2.4 Hz, 1H), 7.45 (dd, *J* = 8.7 *Hz*, 2.4 Hz, 1H), 7.03 (d, *J* = 8.7 Hz, 1H), 6.39 (t, *J* = 72.9 Hz, 1H).

To a solution of 4-nitro-1-[[2-(trimethylsilyl)ethoxy]methyl]-1H-pyrazole (100 g, 410.95 mmol) in anhydrous THF (1000 mL) was added dropwise a solution of LiHMDS (490 mL, 1.0 mol/L in THF) with stirring at -70°C under nitrogen. The resulting solution was stirred for 1 h at -50°C and then cooled to -70°C. ZnCl₂ (500 mL, 0.7 mol/L in THF) was added dropwise at - 70°C. The resulting solution was allowed to warm to room temperature and stirred at room temperature for 1 h. To the mixture was added 4-bromo-1-(difluoromethoxy)-2-iodobenzene (150 g, 859.818 mmol), Pd(PPh₃)₄ (24 g, 20.769 mmol). The resulting solution was heated at reflux overnight. The resulting mixture was concentrated under vacuum. This reaction was repeated twice. The residue was applied onto a silica gel column eluting with ethyl acetate/petroleum ether (1:20). This resulted in 300 g (79%) of 5-[5-bromo-2-(difluoromethoxy)phenyl]-4-nitro-1-[[2-(trimethylsilyl)ethoxy]methyl]-1H-pyrazole as a light yellow solid in all. ¹H NMR (300 MHz, CDCl₃) δ: (ppm) 8.27 (s, 1H), 7.68 (dd, *J* = 8.7, 2.4 Hz, 1H), 7.62 (d, *J* = 2.4 Hz, 1H), 7.19 (d, *J* = 8.4 Hz, 1H), 6.39 (t, *J* = 72.5 Hz, 1H), 5.44-5.19 (m, 2H), 3.72-3.54 (m, 2H), 0.94-0.89 (m, 2H),
0.02 (s, 9H).

Into a 1000-mL round-bottom flask purged and maintained with an inert atmosphere of nitrogen, was placed toluene (500 mL), 5-[5-bromo-2-(difluoromethoxy)phenyl]-4-nitro-1-[[2-(trimethylsilyl)ethoxy]methyl]-1H-pyrazole (60 g, 129.21 mmol), NaSMe (26 g, 371 mmol), Pd₂(dba)₃.CHCl₃ (6.7 g, 6.47 mmol), XantPhos (7.5 g, 12.96 mmol). The resulting mixture was stirred overnight at 85°C. The resulting mixture was concentrated under vacuum. This reaction was repeated three times. The residue was applied onto a silica gel column eluting with ethyl acetate/petroleum ether (1:20). The appropriate fractions were combined and concentrated under vacuum. This resulted in 171 g of 5-[2-(difluoromethoxy)-5-(methylsulfanyl)phenyl]-4-nitro-1-[[2-(trimethylsilyl)ethoxy]methyl]-1H-pyrazole as a yellow solid in all. LC/MS (Method F, ESI) : [M+H]⁺ = 432.1, R_{T} = 1.23 min; ¹H NMR (300 MHz, CDCl₃) δ: (ppm) 8.25 (s, 1H), 7.42 (dd, *J* = 8.7, 2.4 Hz, 1H), 7.34 (d, *J* = 2.1 Hz, 1H), 7.23 (d, *J* = 8.7 Hz, 1H), 6.39 (t, *J* = 72.9 Hz, 1H), 5.36-5.22 (m, 2H), 3.74-3.55 (m, 2H), 2.51 (s, 3H), 0.94-0.90 (m, 2H), 0.02 (s, 9H).

To a mixture of 5-[2-(difluoromethoxy)-5-(methylsulfanyl)phenyl]-4-nitro-1-[[2-(trimethylsilyl)ethoxy]methyl]-1H-pyrazole (171 g, 407.851 mmol), ethanol (2000 mL), water (200 mL) was added iron powder (228 g, 4.083 mol), NH₄Cl (120 g, 2.243 mol). The reaction mixture was stirred at reflux for 3 h under nitrogen, and cooled to room temperature. The solids were filtered out. The filtrate was concentrated under vacuum. The residue was dissolved in 3000 mL of ethyl acetate and washed with 1x500 mL of brine. The organic phase was dried over anhydrous sodium sulfate and concentrated under vacuum. This resulted in 148 g of 5-[2-(difluoromethoxy)-5-(methylsulfanyl)phenyl]-1-[[2-(trimethylsilyl)ethoxy]methyl]-1H-pyrazol-4-amine as yellow oil. LC/MS (Method F, ESI): [M+H]⁺ = 402.1, R_{T}= 0.93 min.

Into a 3000-mL 3-necked round-bottom flask, was placed DMA (1500 mL), 5-[2-(difluoromethoxy)-5-(methylsulfanyl)phenyl]-1-[[2-(trimethylsilyl)ethoxy]methyl]-1H-pyrazol-4-amine (148 g), pyrazolo[1,5-a]pyrimidine-3-carboxylic acid (102 g), HATU (325 g), 4-dimethylaminopyridine (4.5 g), DIPEA (142 g). The resulting solution was stirred for 3 h at 60°C, poured into ice water (2000 mL), extracted with 3x2000 mL of ethyl acetate and the organic layers combined. The resulting mixture was washed with 1x1000 mL of brine. The mixture was dried over anhydrous sodium sulfate and concentrated under vacuum. The residue was applied onto a silica gel column eluting with ethyl acetate/petroleum ether (4:1) to give 200 g of N-[5-[2-(difluoromethoxy)-5-(methylsulfanyl)phenyl]-1-[[2-(trimethylsilyl)ethoxy]methyl]-1H-pyrazol-4-yl]pyrazolo[1,5-a]pyrimidine-3-carboxamide as a light yellow solid. LC/MS (Method A, ESI) : [M+H]⁺ = 547.2, R_{T} = 1.10 min; ¹H NMR (300 MHz, CDCl₃) δ: (ppm) 9.63 (s, 1H), 8.77 (dd, *J* = 7.0, 1.7 Hz, 1H), 8.73 (s, 1H), 8.51 (dd, *J* = 4.2, 1.8 Hz, 1H), 8.38 (s, 1H), 7.50 (d, *J* = 2.4 Hz, 1H), 7.39 (dd, *J* = 8.7, 2.4 Hz, 1H), 7.30 (d, *J* = 8.7 Hz, 1H), 6.98 (dd, *J =* 6.9, 4.2 Hz, 1H), 6.39 (t, *J* = 73.2 Hz, 1H), 5.46-5.38 (m, 2H), 3.70-3.59 (m, 2H), 2.52 (s, 3H), 0.92-0.85 (m, 2H), 0.03 (s, 9H).

To a solution of N-[5-[2-(difluoromethoxy)-5-(methylsulfanyl)phenyl]-1-[[2-(trimethylsilyl)ethoxy]methyl]-1H-pyrazol-4-yl]pyrazolo[1,5-a]pyrimidine-3-carboxamide (60 g) in methanol (600 mL) was added concentrated HCl solution (300 mL). The resulting solution was stirred overnight at 35°C. The resulting mixture was concentrated under vacuum. The solids were collected by filtration. The solid was suspended in 200 mL of water. The pH value of the solution was adjusted to 8 with saturated sodium bicarbonate. The product was collected by filtration, dried to give 30 g (66%) of N-[3-[2-(difluoromethoxy)-5-(methylsulfanyl)phenyl]-1H-pyrazol-4-yl]pyrazolo[1,5-a]pyrimidine-3-carboxamide as a light yellow solid. LC/MS (Method G, ESI): [M+H]⁺ = 417.0, R_{T} = 0.80 min; ¹H NMR (300 MHz, DMSO-*d₆*) δ: (ppm) 13.02 (s, 1H), 9.71 (s, 1H), 9.33 (dd, *J* = 6.9, 1.5 Hz, 1H), 8.68 (dd, *J* = 4.1, 1.4 Hz, 1H), 8.66 (s, 1H), 8.24 (s, 1H), 7.47-7.36 (m, 3H), 7.27 (dd, *J* = 6.9, 4.2 Hz, 1H), 7.17 (t, *J* = 73.8 Hz, 1H), 2.51 (s, 3H).

### Intermediate 1

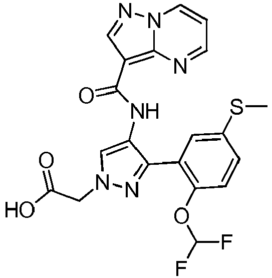

2-(3-(2-(difluoromethoxy)-5-(methylthio)phenyl)-4-(pyrazolo[1,5-a]pyrimidine-3-carboxamido)-1H-pyrazol-1-yl)acetic acid

Into a 250-mL round-bottom flask purged and maintained with an inert atmosphere of nitrogen, was placed hydrogen chloride saturated solution in dioxane (150 mL), 5-[2-(difluoromethoxy)-5-(methylsulfanyl)phenyl]-4-nitro-1-[[2-(trimethylsilyl)ethoxy]methyl]-1H-pyrazole (10.0 g, 23.2 mmol). The resulting solution was stirred overnight at room temperature. The resulting mixture was concentrated under vacuum. The reaction was then quenched by the addition of 300 mL of sodium bicarbonate. The resulting solution was extracted with 5x500 mL of ethyl acetate and the organic layers combined and dried over anhydrous sodium sulfate and concentrated under vacuum. The residue was applied onto a silica gel column eluting with ethyl acetate/petroleum ether (30%). This resulted in 4.01 g (57%) of 3-[2-(difluoromethoxy)-5-(methylsulfanyl)phenyl]-4-nitro-1H-pyrazole as a yellow solid.LC/MS (Method G, ESI): [M+H]⁺ = 302.3, R_{T} = 0.89 min.

Into a 100-mL round-bottom flask, was placed N,N-dimethylformamide (30 mL), 3-[2-(difluoromethoxy)-5-(methylsulfanyl)phenyl]-4-nitro-1H-pyrazole (3.50 g, 11.6 mmol), tert-butyl 2-bromoacetate (4.50 g, 23.1 mmol). This was followed by the addition of DIPEA (4.50 g, 34.8 mmol). 30 The resulting solution was stirred for 3 h at room temperature. The reaction was then quenched by the addition of 100 mL of water/ice. The resulting solution was extracted with 3x200 mL of ethyl acetate and the organic layers combined and dried over anhydrous sodium sulfate and concentrated under vacuum. The residue was applied onto a silica gel column eluting with ethyl acetate/petroleum ether (20%). The collected fractions were combined and concentrated under vacuum. This resulted in 4.01 g (83%) of tert-butyl 2-[3-[2-(difluoromethoxy)-5-(methylsulfanyl)phenyl]-4-nitro-1H-pyrazol-1-yl]acetate as yellow oil. LC/MS (Method G, ESI): [M+H]⁺ = 416.4, R_{T} = 1.10 min.

Into a 100-mL round-bottom flask, was placed ethanol (30 mL), water (5 mL), tert-butyl 2-[3-[2-(difluoromethoxy)-5-(methylsulfanyl)phenyl]-4-nitro-1H-pyrazol-1-yl]acetate (1.00 g, 2.41 mmol), Fe (1.35 g, 24.2 mmol), NH₄Cl (640 mg, 12.0 mmol). The resulting solution was stirred for 2 h at 95°C in an oil bath. The solids were filtered out. The resulting mixture was concentrated under vacuum. The residue was dissolved in 200 mL of EtOAc. The resulting mixture was washed with 1x100 mL of brine. The mixture was dried over anhydrous sodium sulfate and concentrated under vacuum. This resulted in 800 mg (86%) of tert-butyl 2-[4-amino-3-[2-(difluoromethoxy)-5-(methylsulfanyl)phenyl]-1H-pyrazol-1-yl]acetate as yellow oil. LC/MS (Method G, ESI): [M+H]⁺ = 386.1, R_{T} = 0.82 min.

Into a 100-mL 3-necked round-bottom flask, was placed DMA (30 mL), tert-butyl 2-(4-amino-3-(2-(difluoromethoxy)-5-(methylthio)phenyl)-1H-pyrazol-1-yl)acetate (2.00 g, 5.19 mmol), pyrazolo[1,5-a]pyrimidine-3-carboxylic acid (1.44 g, 8.83 mmol), HATU (4.59 g, 8.83 mmol), 4-dimethylaminopyridine (0.06 g, 0.52 mmol), DIPEA (2.01 g, 15.6 mmol). The resulting solution was stirred for 3h at 60°C in an oil bath. The reaction was then quenched by the addition of 200 mL of water/ice. The resulting solution was extracted with 3x200 mL of ethyl acetate and the organic layers combined. The resulting mixture was washed with 1x100 mL of brine. The mixture was dried over anhydrous sodium sulfate and concentrated under vacuum. The residue was applied onto a silica gel column eluting with ethyl acetate/petroleum ether (80%). The collected fractions were combined and concentrated under vacuum. This resulted in 2.51 g (92.6%) of N-[5-[2-(difluoromethoxy)-5-(methylsulfanyl)phenyl]-1-[[2-(trimethylsilyl)ethoxy]methyl]-1H-pyrazol-4-yl]pyrazolo[1,5-a]pyrimidine-3-carboxamide as a light yellow solid. LC/MS (Method G, ESI): [M+H]⁺ = 531.2, R_{T} = 1.06 min.

Into a 100-mL round-bottom flask, was placed dichloromethane (40 mL), tert-butyl 2-[3-[2-(difluoromethoxy)-5-(methylsulfanyl)phenyl]-4-[pyrazolo[1,5-a]pyrimidine-3-amido]-1H-pyrazol-1-yl]acetate (1.80 g, 3.39 mmol), trifluoroacetic acid (20 mL). The resulting solution was stirred for 5 h at room temperature. The resulting mixture was concentrated under vacuum. The reaction was then quenched by the addition of 50 mL of water. The solids were collected by filtration. This resulted in 1.11 g (68%) of 2-[3-[2-(difluoromethoxy)-5-(methylsulfanyl)phenyl]-4-[pyrazolo[1,5-a]pyrimidine-3-amido]-1H-pyrazol-1-yl]acetic acid as a yellow solid. LC/MS (Method A, ESI): [M+H]⁺ = 475.1, R_{T} = 0.81 min; ¹H NMR (300 MHz, DMSO-*d₆*) δ (ppm) 9.77 (s, 1H), 9.36 (dd, *J* = 7.0, 1.6 Hz, 1H), 8.73-8.65 (m, 2H), 8.39 (s, 1H), 7.49-7.38 (m, 3H), 7.31 (dd, *J* = 7.0, 4.2 Hz, 1H), 7.23 (t, *J* = 73.8Hz , 1H), 5.07 (s, 2H), 2.51 (s, 3H).

### Intermediate 2

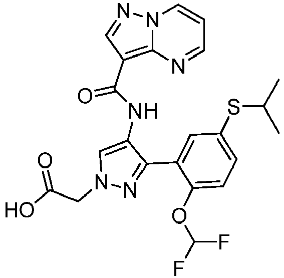

2-[3-[2-(difluoromethoxy)-5-(propan-2-ylsulfanyl)phenyl]-4-[pyrazolo[1,5-a]pyrimidine-3-amido]-1H-pyrazol-1-yl] acetic acid

To a suspension of 5-[5-bromo-2-(difluoromethoxy)phenyl]-4-nitro-1-[[2-(trimethylsilyl)ethoxy]methyl]-1H-pyrazole (5.01 g, 10.8 mmol), (propan-2-ylsulfanyl)sodium (3.17 g, 32.3 mmol) in dioxane (10 mL) was added Pd₂(dba)₃.CHCl₃ (557 mg, 0.538 mmol) and XantPhos (623 mg, 1.08 mmol) under nitrogen atmosphere. The resulting solution was stirred for 14 h at 85°C in an oil bath under N₂ atmosphere. The resulting mixture was concentrated under vacuum. The residue was applied onto a silica gel column eluting with dichloromethane/petroleum ether (3/1) to give 3.79 g (77%) of 5-[2-(difluoromethoxy)-5-(propan-2-ylsulfanyl)phenyl]-4-nitro-1-[[2-(trimethylsilyl)ethoxy]methyl]-1H-pyrazole as a light yellow crude solid. ¹H-NMR (300 MHz, CDCl₃): δ (ppm) 8.26 (s, 1H), 7.62-7.58 (m, 1H), 7.49 (d, *J* = 2.1 Hz, 1H), 7.23 (dd, *J* = 6.6, 1.2 Hz, 1H), 6.38 (t, *J* = 73.8 Hz, 1H), 5.37-5.23 (m, 2H), 3.71-3.54 (m, 2H), 3.42-3.34 (m, 1H), 1.32 (d, J= 6.6 Hz, 6H), 0.95-0.86 (m, 2H), 0.01 (s, 9H).

To a solution of 5-[2-(difluoromethoxy)-5-(propan-2-ylsulfanyl)phenyl]-4-nitro-1-[[2-(trimethylsilyl)ethoxy]methyl]-1H-pyrazole (3.81 g, 8.27 mmol) in dichloromethane (24 mL) was added TFA (6.0 mL). The resulting solution was stirred for 2 h at room temperature. The resulting mixture was concentrated under vacuum. This resulted in 2.98 g (crude) of 3-[2-(difluoromethoxy)-5-(propan-2-ylsulfanyl)phenyl]-4-nitro-1H-pyrazole as a light yellow solid. TLC: PE/ EtOAc = 2/1, R_{f} = 0.1.

To a solution of 3-[2-(difluoromethoxy)-5-(propan-2-ylsulfanyl)phenyl]-4-nitro-1H-pyrazole (2.98 g, 9.11 mmol) and DIPEA (5.71 g, 44.2 mmol) in N,N-dimethylformamide (30 mL) was added tert-butyl 2-bromoacetate (5.17 g, 26.5 mmol) at room temperature. The resulting solution was stirred for 14 h at this temperature. The resulting mixture was concentrated under vacuum. The residue was dissolved in EtOAc (100 ml) and the mixture was washed with water and brine. The resulting mixture was dried over anhydrous sodium sulfate and concentrated under vacuum. The residue was applied onto a silica gel column eluting with dichloromethane/petroleum ether (3/1) to obtain 2.64 g (64%) of tert-butyl 2-[3-[2-(difluoromethoxy)-5-(propan-2-ylsulfanyl)phenyl]-4-nitro-1H-pyrazol-1-yl]acetate as a light yellow solid. TLC: PE/EtOAc = 4/1, R_{f} = 0.3.

To a solution of tert-butyl 2-[3-[2-(difluoromethoxy)-5-(propan-2-ylsulfanyl)phenyl]-4-nitro-1H-pyrazol-1-yl]acetate (2.60 g, 5.86 mmol) in ethanol (30 mL) was added NH₄Cl (940 mg, 17.6 mmol), Fe (1.64 g, 29.4 mmol) and water (3.0 mL). The resulting solution was stirred for 1 h at 90°C in an oil bath. The resulting mixture was concentrated under vacuum. The residue was suspended in 100 mL of EtOAc. The solids were filtrated out. The filtrate was washed with water and brine further dried over anhydrous sodium sulfate and concentrated under vacuum. The residue was applied onto a silica gel column eluting with ethyl acetate/petroleum ether (1/2) to give 1.72 g (70%) of tert-butyl 2-[4-amino-3-[2-(difluoromethoxy)-5-(propan-2-ylsulfanyl)phenyl]-1H-pyrazol-1-yl]acetate as a light yellow solid. TLC: PE/EtOAc= 1/1, R_{f} = 0.1.

To a solution of tert-butyl 2-[4-amino-3-[2-(difluoromethoxy)-5-(propan-2-ylsulfanyl)phenyl]-1H-pyrazol-1-yl]acetate (1.70 g, 4.11 mmol) and pyrazolo[1,5-a]pyrimidine-3-carboxylic acid (1.01 g, 6.19 mmol) in DMA (30 mL) was added 4-dimethylaminopyridine (100 mg, 0.822 mmol), DIPEA (1.59 g, 12.3 mmol) and PyAOP (3.21 g, 6.17 mmol). The resulting solution was stirred for 14 h at 45°C in an oil bath. The resulting mixture was concentrated under vacuum. The residue was dissolved in 300 mL of EtOAc. The resulting mixture was washed with 2x100 mL of water and 1x100 mL of brine. The mixture was dried over anhydrous sodium sulfate and concentrated under vacuum. The residue was applied onto a silica gel column eluting with ethyl acetate/petroleum ether (2/1) to obtain 2.05 g (87%) of tert-butyl 2-[3-[2-(difluoromethoxy)-5-(propan-2-ylsulfanyl)phenyl]-4-[pyrazolo[1,5-a]pyrimidine-3-amido]-1H-pyrazol-1-yl]acetate as a light yellow solid. LC/MS (Method G, ESI): [M+H]⁺ = 575.3, R_{T}= 1.18 min.

To a solution of tert-butyl 2-[3-[2-(difluoromethoxy)-5-(propan-2-ylsulfanyl)phenyl]-4-[pyrazolo[1,5-a]pyrimidine-3-amido]-1H-pyrazol-1-yl]acetate (2.01 g, 3.58 mmol) in dioxane (20 ml) was added a solution of hydrogen chloride in dioxane (20 mL, 15% w/w). The resulting solution was stirred for 3 h at room temperature. The resulting mixture was concentrated under vacuum. This resulted in 1.68 g (crude) of 2-[3-[2-(difluoromethoxy)-5-(propan-2-ylsulfanyl)phenyl]-4-[pyrazolo[1,5-a]pyrimidine-3-amido]-1H-pyrazol-1-yl]acetic acid as a light yellow solid. LC/MS (Method D, ESI): [M+H]⁺ = 503.2, R_{T}= 1.20min; ¹H-NMR (400 MHz, DMSO-*d₆*): δ (ppm) 9.70 (s, 1H), 9.34 (dd, *J* = 7.2, 1.6 Hz, 1H), 8.69-8.67 (m, 2H), 8.25 (s, 1H), 7.53 (dd, *J* = 8.4, 2.4 Hz, 1H), 7.50 (d, *J* = 2.4Hz, 1H), 7.40 (d, *J* = 8.0 Hz, 1H), 7.28 (dd, *J* = 7.0, 4.2 Hz, 1H), 7.25 (t, *J* = 73.6 Hz, 1H), 4.62 (s, 2H), 3.52-3.42 (m, 1H), 1.22 (d, *J* = 6.8 Hz, 6H).

### Intermediate 3

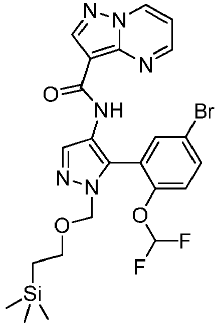

N-(5-(5-bromo-2-(difluoromethoxy)phenyl)-1-((2-(trimethylsilyl)ethoxy)methyl)-1H-pyrazol-4-yl)pyrazolo[1,5-a]pyrimidine-3-carboxamide

To a solution of 5-(5-bromo-2-(difluoromethoxy)phenyl)-4-nitro-1-((2-(trimethylsilyl)ethoxy)methyl)-1H-pyrazole (50.1 g, 108 mmol) in ethanol (2000 mL) and water (200 mL) was added Fe (60.1 g, 1.07 mol) and NH₄Cl (28.0 g, 0.523 mol). The resulting solution was stirred for 3 h at 100°C in an oil bath. The solids were filtered out. The filtrate was concentrated under vacuum. The residue was dissolved in 3000 mL of ethyl acetate. The resulting mixture was washed with 1x500 mL of brine. The mixture was dried over anhydrous sodium sulfate and concentrated under vacuum to give 50.1 g of crude product of 5-(5-bromo-2-(difluoromethoxy)phenyl)-1-((2-(trimethylsilyl)ethoxy)methyl)-1H-pyrazol-4-amine as yellow oil. LC/MS (Method G, ESI): [M+H]⁺ = 434.2, R_{T} = 0.93 min.

To a solution of 5-(5-bromo-2-(difluoromethoxy)phenyl)-1-((2-(trimethylsilyl)ethoxy)methyl)-1H-pyrazol-4-amine (50.1 g, 115 mmol) in DMA (1500 mL) was added pyrazolo[1,5-a]pyrimidine-3-carboxylic acid (32.1 g, 196.0 mmol), PyAOP (102 g, 196 mmol), DMAP (1.41 g, 11.0 mmol) and DIPEA (44.1 g, 0.341 mol). The resulting solution was stirred for 3h at 60°C in an oil bath. The reaction was then quenched by the addition of 2000 mL of water/ice. The resulting solution was extracted with 3x2000 mL of ethyl acetate and the organic layers combined. The resulting mixture was washed with 1x1000 mL of brine. The mixture was dried over anhydrous sodium sulfate and concentrated under vacuum. The residue was applied onto a silica gel column eluting with ethyl acetate/petroleum ether (80%). The collected fractions were combined and concentrated under vacuum. The solid was stirred with 150 mL of H₂O at rt, and the solid were collected by filtration. Dried in air. This resulted in 60.1 g (91%) of N-(5-(5-bromo-2-(difluoromethoxy)phenyl)-1-((2-(trimethylsilyl)ethoxy)methyl)-1H-pyrazol-4-yl)pyrazolo[1,5-a]pyrimidine-3-carboxamide as a light yellow solid. LCMS (Method G, ESI): [M+H]⁺ = 579.1 & 581.1, R_{T} = 1.10 min. ¹H NMR (300 MHz, CDCl₃): δ (ppm) 9.62 (s, 1H), 8.80 (dd, *J* = *6.9,* 1.7 Hz, 1H), 8.73 (s, 1H), 8.53 (dd, *J* = 4.2, 1.7 Hz, 1H), 8.38 (s, 1H), 7.79 (d, *J* = 2.4 Hz, 1H), 7.67 (dd, *J* = 8.8, 2.5 Hz, 1H), 7.29 (d, *J* = 1.4 Hz, 1H), 7.00 (dd, *J* = 6.9, 4.2 Hz, 1H), 6.43 (t, *J* = 72.6 Hz, 1H), 5.53 - 5.27 (m, 2H), 3.73 - 3.50 (m, 2H), 0.88 (ddd, *J* = 9.5, 6.4, 4.4 Hz, 2H), 0.00 (s, 9H).

### Intermediate 4

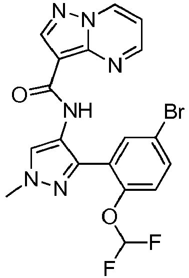

N-[3-[5-bromo-2-(difluoromethoxy)phenyl]-1-methyl-1H-pyrazol-4-yl]pyrazolo[1,5-a]pyrimidine-3-carboxamide

To a solution of N-[5-[5-bromo-2-(difluoromethoxy)phenyl]-1-[[2-(trimethylsilyl)ethoxy]methyl]-1H-pyrazol-4-yl]pyrazolo[1,5-a]pyrimidine-3-carboxamide (10.1 g, 17.3 mmol) in dichloromethane (200 mL) was added Me₃OBF₄ (2.81 g, 18.9 mmol) at room temperature. The resulting solution was stirred for 2 h at room temperature. Then EtOH was added 10 mL to the reaction mixture, and the reaction mixture was stirred for 1h, To this solution was added 5.0 mL of HCl (con.) and stirring for 1h, The resulting mixture was concentrated under vacuum. The pH value of the solution was adjusted to 8 with sodium bicarbonate (20%). The resulting solution was extracted with 3x300 mL of ethyl acetate and the organic layers combined and dried over anhydrous sodium sulfate and concentrated under vacuum. The residue was applied onto a silica gel column eluting with ethyl acetate/petroleum ether (80%) to give 5.5 g (69%) of N-[3-[5-bromo-2-(difluoromethoxy)phenyl]-1-methyl-1H-pyrazol-4- yl]pyrazolo[1,5-a]pyrimidine-3-carboxamide as a light yellow solid. ¹H NMR (400 MHz, CDCl₃): δ (ppm) 9.86 (s, 1H), 8.80 (dd, *J* = 7.0, 1.6 Hz, 1H), 8.74 (s, 1H), 8.60 (dd, *J* = 4.2, 1.6 Hz, 1H), 8.32 (s, 1H), 7.85 (d, *J* = 2.4 Hz, 1H), 7.58 (dd, *J* = 8.4, 2.4 Hz, 1H), 7.24 (d, *J* = 8.8 Hz, 1H), 7.02 (dd, *J* = 7.0, 4.2 Hz, 1H), 6.49 (t, *J* = 74.0 Hz, 1H), 4.01 (s, 3H).

### Example 5

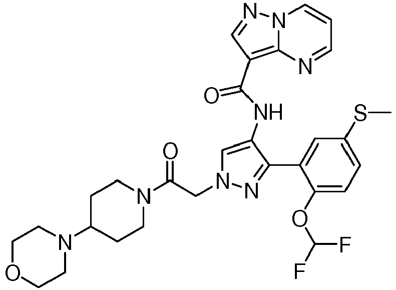

N-(3-(2-(difluoromethoxy)-5-(methylthio)phenyl)-1-(2-(4-morpholinopiperidin-1-yl)-2-oxoethyl)- 1H-pyrazol-4-yl)pyrazolo[1,5-a]pyrimidine-3-carboxamide

To a solution of 2-[3-[2-(difluoromethoxy)-5-(methylsulfanyl)phenyl]-4-[pyrazolo[1,5-a]pyrimidine-3-amido]-1H-pyrazol-1-yl]acetic acid (700 mg, 1.48 mmol), N,N-dimethylformamide (20 mL) was added EDC.HCl (564 mg, 2.94 mmol), HOBt (400 mg, 2.96 mmol), DIPEA (762 mg, 5.90 mmol) and 4-(piperidin-4-yl)morpholine (502 mg, 2.95 mmol) under nitrogen. The resulting solution was stirred overnight at room temperature. Additional amounts of EDC.HCl (564 mg, 2.94 mmol,), HOBt (400 mg, 2.96 mmol) and DIPEA (762 mg, 5.90 mmol) were added. The resulting solution was stirred overnight at room temperature and concentrated under vacuum. The residue was applied onto a silica gel column eluting with dichloromethane/methanol (20/1∼5/1). The appropriate fractions were combined and concentrated under vacuum. The residue was further purified by Preparative HPLC: Column, XBridge Prep C₁₈ OBD Column, 19* 150mm, 5um; mobile phase, Water with 10 mM NH₄HCO₃ and MeCN (20.0% MeCN up to 50.0% in 7 min); Detector, UV 254nm, and the appropriate fractions were pooled and concentrated under vacuum. The residue was recrystallized from a mixture of ethyl acetate and hexane to give 361.5 mg (39%) of N-[3-[2-(difluoromethoxy)-5-(methylsulfanyl)phenyl]-1-[2-[4-(morpholin-4-yl)pipendin-1-yl]-2-oxoethyl]-1H-pyrazol-4-yl]pyrazolo[1,5-a]pyrimidine-3-carboxamide as an off-white solid. LC/MS (Method A, ESI): [M+H]⁺ = 627.3, R_{T}= 1.47 min; ¹H NMR (400 MHz, CD₃OD-*d₄*): δ (ppm) 9.08 (dd, *J* = 7.1, 1.7 Hz, 1H), 8.69-8.53 (m, 2H), 8.36 (s, 1H), 7.54 (d, *J* = 2.4 Hz, 1H), 7.46 (dd, *J* = 8.7, 2.5 Hz, 1H), 7.36 (d, *J* = 8.7 Hz, 1H), 7.20 (dd, *J* = 7.0, 4.2 Hz, 1H), 6.75 (t, *J* = 74.1 Hz, 1H), 5.34-5.18 (m, 2H), 4.57-4.54 (m, 1H), 4.11-4.07 (m, 1H), 3.75-3.65 (m, 4H), 3.25-3.08 (m, 1H), 2.89-2.69 (m, 1H), 2.62-2.59 (m, 4H), 2.56 (s, 3 H), 2.55-2.45 (m, 1H), 2.03-1.96 (m, 2H), 1.54-1.45 (m, 2H).

### Example 11

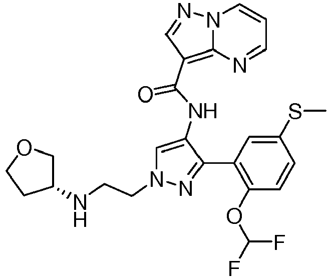

(R)-N-(3-(2-(difluoromethoxy)-5-(methylthio)phenyl)-1-(2-((tetrahydrofuran-3-yl)amino)ethyl)-1H-pyrazol-4-yl)pyrazolo[1,5-a]pyrimidine-3-carboxamide

Into a 500-mL round-bottom flask purged and maintained with an inert atmosphere of nitrogen, was placed N-[3-[2-(difluoromethoxy)-5-(methylsulfanyl)phenyl]-1H-pyrazol-4-yl]pyrazolo[1,5-a]pyrimidine-3-carboxamide (5.00 g, 12.0 mmol), tetrahydrofuran (150 mL), Cs₂CO₃ (15.7 g, 48.2 mmol,), 1,2-dibromoethane (45.0 g, 240 mmol). The reaction mixture was stirred for 2 h at 80°C and allowed to cool to room temperature, concentrated under vacuum. The crude product was re-crystallized from mixed solvents of hexane/ethyl acetate in the ratio of 3/1. The solids were collected by filtration. This resulted in 5.80 g (92%) of N-[1-(2-bromoethyl)-3-[2-(difluoromethoxy)-5-(methylsulfanyl)phenyl]-1H-pyrazol-4-yl]pyrazolo[1,5-a]pyrimidine-3-carboxamide as a light yellow solid. LCMS (Method A, ESI): [M+H]⁺ = 525.1, R_{T}= 1.48 min.

Into a 100-mL round-bottom flask purged and maintained with an inert atmosphere of nitrogen, was placed CH₃CN (25 mL), N-[1-(2-bromoethyl)-3-[2-(difluoromethoxy)-5-(methylsulfanyl)phenyl]-1H-pyrazol-4-yl]pyrazolo[1,5-a]pyrimidine-3-carboxamide (200 mg, 0.382 mmol), DIPEA (247 mg, 1.91 mmol), (3R)-oxolan-3-amine (100 mg, 1.15 mmol). The resulting solution was stirred overnight at 80°C. The resulting mixture was concentrated under vacuum. The residue was applied onto a silica gel column eluting with dichloromethane/methanol (10/1). The crude product (200 mg) was purified by Prep-HPLC with the following conditions: Column, XBridge RP18 OBD column, 19^{∗}150mm, 5um; mobile phase A: Water with 10 mM NH₄HCO₃, mobile phase B: ACN; (32% B increasing to B: 64% B in 8 min); Detector, UV 254 nm. This resulted in 27.2 mg (13%) of N-[3-[2-(difluoromethoxy)-5-(methylsulfanyl)phenyl]-1-(2-[[(3R)-oxolan-3-yl]amino]ethyl)-1H-pyrazol-4-yl]pyrazolo[1,5-a]pyrimidine-3-carboxamide as a light yellow solid. LC/MS (Method A, ESI): [M+H]⁺ = 530.2, R_{T} = 1.46 min; ¹H NMR (300 MHz, DMSO-*d₆*): δ (ppm) 9.73 (s, 1H), 9.34 (dd, *J* = 6.9, 1.8 Hz, 1H), 8.68 (d, *J* = 1.5 Hz, 1H), 8.66 (s, 1H), 8.35 (s, 1H), 7.46-7.36 (m, 3H), 7.27 (dd, J = 6.9 Hz, 4.2 Hz, 1H), 7.17 (t, *J* = 73.8 Hz, 1H), 4.24 (s, 2H), 3.74-3.31 (m, 3H), 3.10-3.04 (m, 2H), 3.16-2.96 (m, 2H), 2.51 (s, 3H), 2.01-1.92 (m, 1H), 1.68-1.62 (m, 1H).

### Example 12

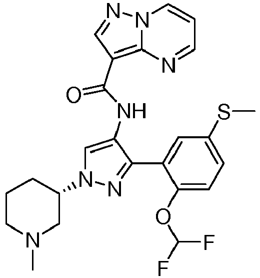

(S)-N-(3-(2-(difluoromethoxy)-5-(methylthio)phenyl)-1-(1-methylpiperidin-3-yl)-1H-pyrazol-4-yl)pyrazolo[1,5-a]pyrimidine-3-carboxamide

To a mixture of Cs₂CO₃ (1.93 g, 5.92 mmol), N-[3-[2-(difluoromethoxy)-5-(methylsulfanyl)phenyl]-1H-pyrazol-4-yl]pyrazolo[1,5-a]pyrimidine-3-carboxamide (820 mg, 1.97 mmol) in DMF (12 mL) was added tert-butyl (3R)-3-(methanesulfonyloxy)piperidine-1-carboxylate (1.10 g, 3.94 mmol). The resulting solution was stirred at 60°C overnight at 60°C and concentrated under vacuum. The residue was applied onto a silica gel column eluting with ethyl acetate/petroleum ether (3/2) and ethyl acetate. The appropriate fractions were combined and concentrated under vacuum. This resulted in 330 mg (24%) of tert-butyl 3-[3-[2-(difluoromethoxy)-5-(methylsulfanyl)phenyl]-4-[pyrazolo[1,5-a]pyrimidine-3-amido]-1H-pyrazol-1-yl]piperidine-1-carboxylate as light yellow oil. TLC: ethyl acetate, R_{f} = 0.4.

A mixture of tert-butyl (3S)-3-[3-[2-(difluoromethoxy)-5-(methylsulfanyl)phenyl]-4-[pyrazolo[1,5-a]pyrimidine-3-amido]-1H-pyrazol-1-yl]piperidine-1-carboxylate (330 mg, 0.550 mmol), dichloromethane (6.0 mL), trifluoroacetic acid (4.0 mL) was stirred for 4 h at room temperature. The resulting mixture was concentrated under vacuum. This resulted in 400 mg (crude) of N-[3-[2-(difluoromethoxy)-5-(methylsulfanyl)phenyl]-1-[(3S)-piperidin-3-yl]-1H-pyrazol-4-yl]pyrazolo[1,5-a]pyrimidine-3-carboxamide as light yellow oil. TLC: dichloromethane/methanol = 5/1, R_{f} = 0.4.

A mixture of N-[3-[2-(difluoromethoxy)-5-(methylsulfanyl)phenyl]-1-[(3S)-piperidin-3-yl]-1H-pyrazol-4-yl]pyrazolo[1,5-a]pyrimidine-3-carboxamide (400 mg, 0.801 mmol), methanol (10 mL), formaldehyde (700 mg, 37% aqueous solution). The mixture was stirred at room temperature overnight. NaBH₃CN (202 mg, 3.214 mmol) was added. The resulting solution was stirred for another 4 h at room temperature and concentrated under vacuum. The residue was applied onto a silica gel column eluting with dichloromethane/methanol (20/1-10/1). The appropriate fractions were combined and concentrated under vacuum. The crude product was purified by Prep-HPLC with the following conditions: Column, XBridge Shield RP18 OBD Column, 19^{∗}150mm, 5um; mobile phase, Water with 10 mM NH₄HCO₃ and MeCN (20.0% MeCN up to 55.0% MeCN in 9 min); Detector, UV 254nm. This resulted in 19.5 mg (5%) of N-[3-[2-(difluoromethoxy)-5-(methylsulfanyl)phenyl]-1-[(3S)-1-methylpiperidin-3-yl]-1H-pyrazol-4-yl]pyrazolo[1,5-a]pyrimidine-3-carboxamide as a light yellow solid. LC/MS (Method H, ESI): [M+H]⁺ = 514.2, R_{T}= 1.37 min; ¹H NMR (300 MHz, CD₃OD-*d₄*): δ (ppm) 9.08 (dd, *J* = 7.1, 1.7 Hz, 1H), 8.64 (dd, *J* = 4.2, 1.5 Hz, 1H), 8.63 (s, 1H), 8.38 (s, 1H), 7.49-7.43 (m, 2H), 7.33 (d, *J* = 8.7 Hz, 1H), 7.19 (dd, *J* = 7.2, 4.2 Hz, 1H), 6.74 (t, *J* = 74.1 Hz, 1H), 4.50-4.36 (m, 1H), 3.28-3.19 (m, 1H), 2.95-2.83 (m, 1H), 2.52 (s, 3H), 2.50-2.44 (m, 1H), 2.39 (s, 3H), 2.30-2.10 (m, 2H), 2.04-1.70 (m, 3H).

### Example 25

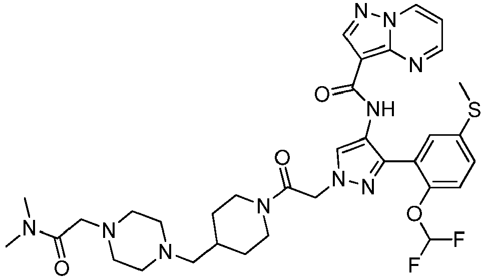

N-(3-(2-(difluoromethoxy)-5-(methylthio)phenyl)-1-(2-(4-((4-(2-(dimethylamino)-2-oxoethyl)piperazin-1-yl)methyl)piperidin-1-yl)-2-oxoethyl)-1H-pyrazol-4-yl)pyrazolo[1,5-a]pyrimidine-3-carboxamide

Into a 100-mL round-bottom flask purged and maintained with an inert atmosphere of nitrogen, was placed 2-bromo-N,N-dimethylacetamide (1.10 g, 6.63 mmol), N,N-dimethylformamide (30 mL), tert-butyl piperazine-1-carboxylate (1.24 g, 6.66 mmol), DIPEA (2.58 g, 20.0 mmol). The resulting solution was stirred for 5 h at room temperature and concentrated under vacuum. The residue was applied onto a silica gel column eluting with dichloromethane/methanol (10/1). This resulted in 1.61 g (89%) of tert-butyl 4-[(dimethylcarbamoyl)methyl]piperazine-1-carboxylate as a light yellow solid. TLC: MeOH/DCM = 1/5. R_{f} = 0.4.

A solution of tert-butyl 4-[(dimethylcarbamoyl)methyl]piperazine-1-carboxylate (1.61 g, 5.90 mmol) in dichloromethane (20 mL) and trifluoroacetic acid (10 mL) was stirred for 4 h at room temperature. The resulting mixture was concentrated under vacuum. This resulted in 900 mg (crude) of N,N-dimethyl-2-(piperazin-1-yl)acetamide as yellow oil. TLC: MeOH/DCM = 1/5, R_{f} = 0.1.

To a solution of TFA salt of N,N-dimethyl-2-(piperazin-1-yl)acetamide (1.75 g) and tert-butyl 4-formylpiperidine-1-carboxylate (3.27 g, 15.3 mmol) in MeOH (20 mL) was added NaBH₃CN (960 mg, 15.276 mmol). The resulting solution was stirred at room temperature overnight and concentrated under vacuum. The residue was applied onto a silica gel column eluting with dichloromethane/methanol (8%MeOH). This resulted in 1.31 g (35%) of tert-butyl 4-([4-[(dimethylcarbamoyl)methyl]piperazin-1-yl]methyl)piperidine-1-carboxylate as colorless oil. TLC: dichloromethane/methanol = 8/1, R_{f} = 0.4.

Into a 50-mL round-bottom flask, was placed tert-butyl 4-([4-[(dimethylcarbamoyl)methyl]piperazin-1-yl]methyl)piperidine-1-carboxylate (1.20 g, 3.26 mmol), saturated HCl solution in dioxane (4.0 mL). The resulting solution was stirred for 3 h at room temperature. The resulting mixture was concentrated under vacuum. This resulted in 850 mg (97%) of N,N-dimethyl-2-[4-(piperidin-4-ylmethyl)piperazin-1-yl]acetamide HCl salt as a white solid.

Into a 50-mL round-bottom flask, was placed 2-[3-[2-(difluoromethoxy)-5-(methylsulfanyl)phenyl]-4-[pyrazolo[1,5-a]pyrimidine-3-amido]-1H-pyrazol-1-yl]acetic acid (150 mg, 0.316 mmol), N,N-dimethylformamide (20 mL), EDC.HCl (121 mg, 0.632 mmol). This was followed by the addition of HOBt (85.4 mg, 0.632 mmol). To this was added DIPEA (164 mg, 1.27 mmol), N,N-dimethyl-2-[4-(piperidin-4-ylmethyl)piperazin-1-yl]acetamide HCl salt (170 mg). The resulting solution was stirred overnight at room temperature. The resulting mixture was concentrated under vacuum. The residue was applied onto a silica gel column eluting with DCM/MeOH (8%MeOH). The crude product was further purified by Prep-HPLC with the following conditions: Column, XBridge Prep C₁₈ OBD Column, 19^{∗}150mm, 5um; mobile phase, ACN/H₂O (10 mM NH₄HCO₃) = 18% increasing to ACN/H₂O (10 mM NH₄HCO₃) = 41% within 8 min; Detector, UV 254 nm to give 3.6 mg (2%) of N-[3-[2-(difluoromethoxy)-5-(methylsulfanyl)phenyl]-1-[2-[4-([4-[(dimethylcarbamoyl)methyl]piperazin-1-yl]methyl)piperidin-1-yl]-2-oxoethyl]-1H-pyrazol-4-yl]pyrazolo[1,5-a]pyrimidine-3-carboxamide as a white solid. LC/MS (Method A, ESI): [M+H]⁺ = 725.3, R_{T} = 2.37 min; ¹H NMR (400 MHz, CD₃OD-*d₄*): δ (ppm) 9.09 (dd, *J* = 7.2, 1.6 Hz, 1H), 8.66-8.65 (m, 2H), 8.36 (s, 1 H), 7.55 (d, *J* = 2.4 Hz, 1H), 7.45 (dd, *J* = 8.8, 2.4 Hz, 1H), 7.35 (d, *J* = 8.4 Hz, 1H), 7.20 (dd, *J* = 7.2, 4.4 Hz, 1H), 6.74 (t, *J* = 74 Hz, 1H), 5.31-5.17 (m, 2H), 4.53-4.50 (m, 1H), 4.05-4.02 (m, 1H), 3.37-3.33 (m, 1H), 3.32-3.24 (m, 2H), 3.20-3.15 (m, 1H), 3.11 (s, 3H), 2.95 (s, 3 H), 2.77-2.74 ((m, 1H), 2.59-2.53 (m, 10 H), 2.27-2.25 (m, 2H), 1.92-1.83 (m, 3H), 1.20-1.14 (m, 2H).

### Example 46

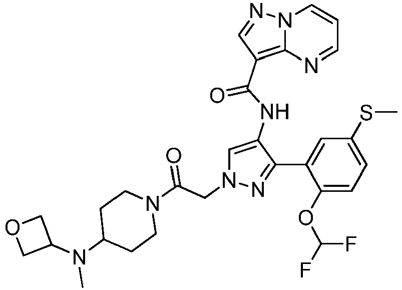

N-(3-(2-(difluoromethoxy)-5-(methylthio)phenyl)-1-(2-(4-(methyl(oxetan-3-yl)amino)piperidin-1-yl)-2-oxoethyl)-1H-pyrazol-4-yl)pyrazolo[1,5-a]pyrimidine-3-carboxamide

Into a 1000-mL round-bottom flask, was placed piperidin-4-one hydrochloride (50 g, 368.753 mmol), dichloromethane (200 mL), sodium bicarbonate (62.2 g, 0.741 mol). The reaction mixture was stirred for 4 h at room temperature, and the reaction mixture was cooled by ice bath. Neat 2-bromoacetyl bromide (74.1 g, 367 mmol) was added dropwise to the cold reaction mixture under ice bath cooling. The reaction was allowed to warm to room temperature and stirred overnight at room temperature. The solids were filtered out. The filtrate was concentrated under vacuum. The residue was applied onto a silica gel column eluting with ethyl acetate/petroleum ether (1/1). This resulted in 23.8 g (29%) of 1-(2-bromoacetyl)piperidin-4-one. LC/MS (Method I, ESI): [M+H]⁺= 220.0, R_{T}= 0.54 min.

Into a 500-mL round-bottom flask, was placed N-[3-[2-(difluoromethoxy)-5-(methylsulfanyl)phenyl]-1H-pyrazol-4-yl]pyrazolo[1,5-a]pyrimidine-3-carboxamide (10.0 g, 24.0 mmol), N,N-dimethylformamide (250 mL), Cs₂CO₃ (15.7 g, 48.1 mmol), 1-(2-bromoacetyl)piperidin-4-one (10.5 g, 47.9 mmol). The resulting solution was stirred for 80 min at 60°C and concentrated under vacuum. The residue was dissolved in ethyl acetate (200 mL), washed successively with water (2x100 mL), brine (1x100 mL), dried and concentrated under reduced pressure. This resulted in 11.2 g (84%) of N-[3-[2-(difluoromethoxy)-5-(methylsulfanyl)phenyl]-1-[2-oxo-2-(4-oxopiperidin-1-yl)ethyl]-1H-pyrazol-4-yl]pyrazolo[1,5-a]pyrimidine-3-carboxamide as a solid. LC/MS (Method I, ESI): [M+H]⁺= 556.0, R_{T}= 0.92 min.

Into a 50-mL round-bottom flask purged and maintained with an inert atmosphere of nitrogen, was placed N-[3-[2-(difluoromethoxy)-5-(methylsulfanyl)phenyl]-1-[2-oxo-2-(4-oxopiperidin-1-yl)ethyl]-1H-pyrazol-4-yl]pyrazolo[1,5-a]pyrimidine-3-carboxamide (170 mg, 0.306 mmol), ethanol (8.0 mL), oxetan-3-amine (45.0 mg, 0.616 mmol), Ti(OiPr)₄ (177 mg, 0.623 mmol). The mixture solution was stirred 3 h at 60°C. The solution was cooled to room temperature. Then AcOH (0.03 mL, 0.524 mmol) and NaBH₃CN (39.0 mg, 0.621 mmol) was added into mixture solution. The resulting solution was stirred for 5 h at 60°C and concentrated under vacuum. The residue was applied onto a silica gel column eluting with dichloromethane/methanol (9/1). The appropriate fractions were combined and concentrated under vacuum. This resulted in 107 mg (57%) of N-[3-[2-(difluoromethoxy)-5-(methylsulfanyl)phenyl]-1-(2-[4-[(oxetan-3-yl)amino]piperidin-1-yl]-2-oxoethyl)-1H-pyrazol-4-yl]pyrazolo[1,5-a]pyrimidine-3-carboxamide as a light yellow solid. LC/MS (Method I, ESI): [M+H]⁺ = 613.0, R_{T} = 0.81 min.

Into a 25-mL round-bottom flask purged and maintained with an inert atmosphere of nitrogen, was placed N-[3-[2-(difluoromethoxy)-5-(methylsulfanyl)phenyl]-1-(2-[4-[(oxetan-3-yl)amino]piperidin-1-yl]-2-oxoethyl)-1H-pyrazol-4-yl]pyrazolo[1,5-a]pyrimidine-3-carboxamide (86.0 mg, 0.140 mmol), methanol (5.0 mL), formaldehyde (186 mg, 37% aqueous, 2.30 mmol), NaBH₃CN (13.4 mg, 0.213 mmol). The resulting solution was stirred for 5 h at 25°C and concentrated under vacuum. The residue was applied onto a silica gel column eluting with dichloromethane/methanol (9/1). The crude product was further purified by Prep-HPLC with the following conditions: Column, XBridge Prep C₁₈ OBD Column, 19^{∗}150mm, 5um; mobile phase, ACN/H₂O (10 mM NH₄HCO₃) = 18% increasing to ACN/H₂O (10 mM NH₄HCO₃) = 41% within 8 min; Detector, UV 254 nm) to give 12.4 mg (14%) of N-[3-[2-(difluoromethoxy)-5-(methylsulfanyl)phenyl]-1-(2-[4-[methyl(oxetan-3-yl)amino]piperidin-1-yl]-2-oxoethyl)-1H-pyrazol-4-yl]pyrazolo[1,5-a]pyrimidine-3-carboxamide as a white solid. LC/MS (Method D, ESI): [M+H]⁺ = 627.1, R_{T} = 1.45 min; ¹H NMR (400 MHz, CD₃OD-*d₄*): δ (ppm) 9.10 (dd, *J* = 7.2, 2.0 Hz, 1H), 8.66-8.65 (m, 2H), 8.37 (s, 1H), 7.55 (d, *J* = 2.4 Hz, 1H), 7.46 (dd, *J* = 8.8, 2.4 Hz, 1H), 7.36 (d, J = 8.8 Hz, 1H), 7.21 (dd, *J* = 7.2, 4.4 Hz, 1H), 6.76 (t, *J* = 73.6 Hz, 1H), 5.34-5.17 (m, 1H), 4.69-4.61 (m,5H), 4.13-4.09 (m, 2H), 3.21-3.14 (m, 1H), 2.73-2.67 (m, 2H), 2.54 (s, 3H), 2.30-2.22 (m, 3H), 1.85-1.76 (m, 2H), 1.58-1.46 (m, 2H).

### Example 55

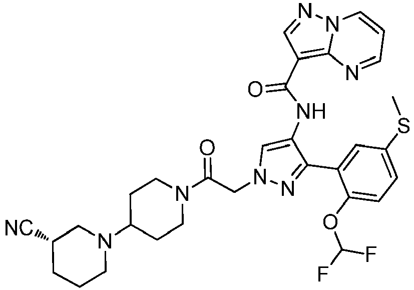

N-[1-(2-[4-[(3S)-3-cyanopiperidin-1-yl]piperidin-1-yl]-2-oxoethyl)-3-[2-(difluoromethoxy)-5-(methylsulfanyl)phenyl]-1H-pyrazol-4-yl]pyrazolo[1,5-a]pyrimidine-3-carboxamide

The (S)-isomer N-[1-(2-[4-[(3S)-3-cyanopiperidin-1-yl]piperidin-1-yl]-2-oxoethyl)-3-[2-(difluoromethoxy)-5-(methylsulfanyl)phenyl]-1H-pyrazol-4-yl]pyrazolo[1,5-a]pyrimidine-3-carboxamide was prepared by the same fashion from N-[3-[2-(difluoromethoxy)-5-(methylsulfanyl)phenyl]-1-[2-oxo-2-(4-oxopiperidin-1-yl)ethyl]-1H-pyrazol-4-yl]pyrazolo[1,5-a]pyrimidine-3-carboxamide and (3S)-piperidine-3-carbonitrile. LC/MS (Method A, ESI): [M+H]⁺ = 650.3, R_{T} = 1.50 min; ¹H NMR (300 MHz, CD₃OD-*d₄*): δ (ppm) 9.07 (dd, *J* = 6.9, 1.5 Hz, 1H), 8.65 - 8.63 (m, 2H), 8.35 (s, 1H), 7.54 (d, *J* = 2.4 Hz, 1H), 7.44 (dd, J = 8.6, 2.6 Hz,1H), 7.34 (d, *J* = 8.4 Hz, 1H), 7.18 (dd, *J* = 6.9, 4.2 Hz, 1 H), 6.73 (t, *J* = 74.1Hz, 1H), 5.27 (d, *J* = 16.2 Hz, 1H), 5.17 (d, *J* = 16.8 Hz, 1H), 4.59 - 4.56 (m, 1H), 4.08 - 3.98 (m, 1H), 3.30-3.19 (m, 1H), 2.89 - 2.85 (m, 2H), 2.77 - 2.69 (m, 5 H), 2.52 (s, 3 H), 1.86 - 1.50 (m, 8H).

### Example 58

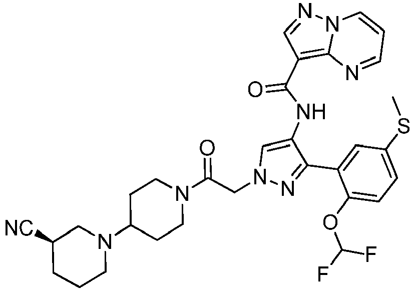

N-[1-(2-[4-[(3R)-3-cyanopiperidin-1-yl]piperidin-1-yl]-2-oxoethyl)-3-[2-(difluoromethoxy)-5-(methylsulfanyl)phenyl]-1H-pyrazol-4-yl]pyrazolo[1,5-a]pyrimidine-3-carboxamide

To a solution of N-[3-[2-(difluoromethoxy)-5-(methylsulfanyl)phenyl]-1-[2-oxo-2-(4-oxopiperidin-1-yl)ethyl]-1H-pyrazol-4-yl]pyrazolo[1,5-a]pyrimidine-3-carboxamide (1.00 g, 1.80 mmol) in ethanol (15 mL) was added Ti(OiPr)₄ (1.02 g, 3.60 mmol) and (3R)-piperidine-3-carbonitrile (595 mg, 5.40 mmol). The resulting solution was stirred at 60°C overnight. Then AcOH (0.1 mL, 1.75 mmol) and NaBH₃CN (114 mg, 1.81 mmol) were added. The resulting solution was stirred for 5 h at 60°C, and concentrated under vacuum. The residue was applied onto a silica gel column eluting with DCM/MeOH (92/8). The crude product (700 mg) was further purified by Prep-HPLC with the following conditions: Column, XBridge Shield RP18 OBD Column, 19^{∗}150mm, 5um; mobile phase, ACN/H₂O (10 mM NH₄HCO₃) = 24% increasing to ACN/H₂O (10 mM NH₄HCO₃) = 54% within 7 min; Detector, UV 254 nm. This resulted in 366.5 mg (31%) of N-[1-(2-[4-[(3R)-3-cyanopiperidin-1-yl]piperidin-1-yl]-2-oxoethyl)-3-[2-(difluoromethoxy)-5-(methylsulfanyl)phenyl]-1H-pyrazol-4-yl]pyrazolo[1,5-a]pyrimidine-3-carboxamide as a white solid. LC/MS (Method A, ESI): [M+H]⁺ = 650.3, R_{T}= 1.51 min; ¹H NMR (400 MHz, CD₃OD-*d₄*): δ (ppm) 8.96 (dd, *J* = 7.2, 1.6 Hz, 1H), 8.53 - 8.51 (m, 2H), 8.25 (s, 1H), 7.43 (d, *J* = 2.4 Hz, 1H), 7.33 (dd, *J* = 8.8, 2.4 Hz, 1H), 7.24 (d, *J* = 8.8 Hz, 1H), 7.09 (dd, *J* = 7.0, 4.2 Hz, 1H), 6.64 (t, *J* = 74.0 Hz, 1H), 5.17 (d, *J* = 16.4 Hz, 1H), 5.06 (d, *J* = 16.4 Hz, 1H), 4.45 - 4.40 (m, 1H), 4.00 - 3.87 (m, 1H), 3.07 - 2.95 (m, 1H), 2.78 - 2.45 (m, 7H), 2.41 (s, 3H), 1.76 - 1.47 (m, 8 H).

### Example 63

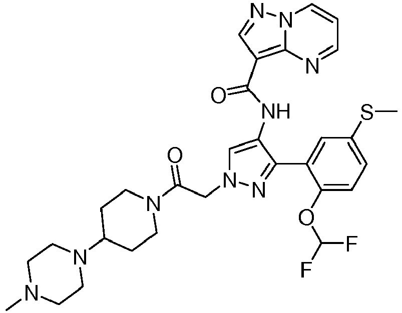

N-[3-[2-(difluoromethoxy)-5-(methylsulfanyl)phenyl]-1-[2-[4-(4-methylpiperazin-1-yl)piperidin-1-yl]-2-oxoethyl]-1H-pyrazol-4-yl]pyrazolo[1,5-a]pyrimidine-3-carboxamide

To a solution of N-[3-[2-(difluoromethoxy)-5-(methylsulfanyl)phenyl]-1-[2-oxo-2-(4-oxopiperidin-1-yl)ethyl]-1H-pyrazol-4-yl]pyrazolo[1,5-a]pyrimidine-3-carboxamide (150 mg, 0.270 mmol) in ethanol (10 mL) was added 1-methylpiperazine (54.1 mg, 0.539 mmol) and Ti(OiPr)₄ (156 mg, 0.549 mmol). The resulting solution was stirred for 2 h at 60°C. Then AcOH (0.031 mL, 0.524 mmol) and NaBH₃CN (34.1 mg, 0.541 mmol) was added. The resulting solution was allowed to react with stirring for an additional 2 h at 60°C. The reaction was then quenched by the addition of 2 mL of water. The resulting mixture was concentrated under vacuum. The residue was applied onto a silica gel column eluting with dichloromethane/methanol (4/1). The collected fractions were combined and concentrated under vacuum. Then the residue was purified by Prep-HPLC with the following conditions: Column, XBridge Prep Phenyl OBD Column, 19^{∗}150mm, 5um; mobile phase, Waters (0.05%NH₃H₂O) and ACN (20% ACN up to 50.0% in 12 min); Detector, UV 254 nm to give 71.7 mg (42%) of N-[3-[2-(difluoromethoxy)-5-(methylsulfanyl)phenyl]-1-[2-[4-(4-methylpiperazin-1-yl)piperidin-1-yl]-2-oxoethyl]-1H-pyrazol-4-yl]pyrazolo[1,5-a]pyrimidine-3-carboxamide as a yellow solid. LC/MS (Method J, ESI): [M+H]⁺ = 640.3, RT= 2.67 min; ¹H NMR (300 MHz, CD₃OD-*d₄*): δ (ppm) 8.98 (dd, *J* = 6.9, 1.5 Hz, 1H), 8.53 - 8.51 (m, 2H), 8.25 (s, 1H), 7.42 (d, *J* = 2.4 Hz, 1H), 7.33 (dd, J = 8.7, 2.4 Hz, 1H), 7.23 (d, *J* = 8.4 Hz, 1H), 7.07 (dd, J = 6.9, 4.2 Hz, 1H), 6.63 (t, *J* = 74.1 Hz, 1H), 5.16 (d, *J* = 16.2 Hz, 1H), 5.05 (d, *J* = 16.2 Hz, 1H), 4.43 - 4.40 (m, 1H), 3.96 - 3.94 (m, 1H), 3.24 - 3.08 (m, 1H), 2.64 - 2.54 (m, 5H), 2.54 - 2.41 (m, 8 H), 2.17 (s, 3H), 1.95 - 1.80 (m, 2H), 1.55 - 1.29 (m, 2H).

### Example 71

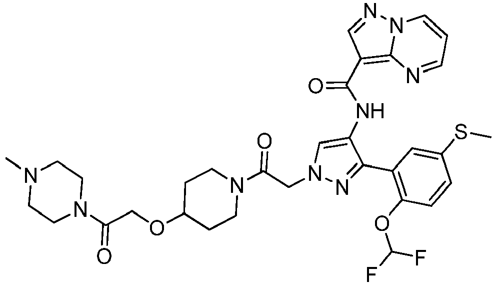

N-(3-(2-(difluoromethoxy)-5-(methylthio)phenyl)-1-(2-(4-(2-(4-methylpiperazin-1-yl)-2-oxoethoxy)piperidin-1-yl)-2-oxoethyl)-1H-pyrazol-4-yl)pyrazolo[1,5-a]pyrimidine-3-carboxamide

Sodium hydride (1.00 g, 60% in mineral oil, 25.00 mmol) was added in portions to a solution of tert-butyl 4-hydroxypiperidine-1-carboxylate (5.00 g, 24.84 mmol) in anhydrous THF (25 mL) at room temperature under nitrogen. After hydrogen gas evolution ceased, ethyl 2-bromoacetate (8.40 g, 50.3 mmol) was added dropwise. The resulting solution was stirred overnight at room temperature. The reaction was then quenched by the addition of water. The resulting solution was extracted with ethyl acetate and the organic layers combined. The organic layers were washed with brine, dried over sodium sulfate, filtered and concentrated under reduced pressure. The residue was applied onto a silica gel column eluting with ethyl acetate/petroleum ether (1/4). This resulted in 5.00 g (70%) of tert-butyl 4-(2-ethoxy-2-oxoethoxy)piperidine-1-carboxylate as colorless oil. TLC: ethyl acetate/hexane =1/4, R_{f} = 0.3.

Into a 100-mL round-bottom flask, was placed tert-butyl 4-(2-ethoxy-2-oxoethoxy)piperidine-1-carboxylate (5.00 g, 17.4 mmol), sodium hydroxide (3.00 g, 75.0 mmol), water (20 mL), ethanol (20 mL). The resulting solution was stirred for 2 days at room temperature. The resulting mixture was concentrated under vacuum. The pH value of the solution was adjusted to 2 with HCl (3 mol/L). The resulting solution was extracted with ethyl acetate (2x) and the organic layers combined and concentrated under vacuum. This resulted in 4.00 g (89%) of 2-([1-[(tert-butoxy)carbonyl]piperidin-4-yl]oxy)acetic acid as an oil. TLC: DCM/MeOH=5/1, R_{f} = 0.4.

Into a 100-mL round-bottom flask, was placed 2-([1-[(tert-butoxy)carbonyl]piperidin-4-yl]oxy)acetic acid (2.00 g, 7.71 mmol), N,N-dimethylformamide (30 mL), 1-methylpiperazine (1.55 g, 15.5 mmol), HATU (5.90 g, 15.5 mmol), DIPEA (3.99 g, 30.9 mmol). The resulting solution was stirred for 3 h at room temperature. The resulting mixture was concentrated under vacuum. The residue was applied onto a silica gel column eluting with dichloromethane. This resulted in 3.00 g (crude) of tert-butyl 4-[2-(4-methylpiperazin-1-yl)-2-oxoethoxy]piperidine-1-carboxylate as yellow oil. LC/MS (Method E, ESI): [M+H]⁺ = 342.0, R_{T}= 0.79 min.

Into a 100-mL round-bottom flask, was placed tert-butyl 4-[2-(4-methylpiperazin-1-yl)-2-oxoethoxy]piperidine-1-carboxylate (3.00 g, 8.79 mmol), saturated HCl solution in dioxane (10 mL). The resulting solution was stirred overnight at room temperature and concentrated under vacuum. This resulted in 3.00 g (crude) desired product. TLC: MeOH/DCM = 1/5, R_{f} = 0.05.

Into a 50-mL round-bottom flask, was placed 2-[3-[2-(difluoromethoxy)-5-(methylsulfanyl)phenyl]-4-[pyrazolo[1,5-a]pyrimidine-3-amido]-1H-pyrazol-1-yl]acetic acid (300 mg, 0.632 mmol), N,N-dimethylformamide (15 mL), 1-(4-methylpiperazin-1-yl)-2-(piperidin-4-yloxy)ethan-1-one (263 mg, 1.09 mmol), EDC.HCl (243 mg, 1.27 mmol), HOBt (171 mg, 1.27 mmol), DIPEA (327 mg, 2.53 mmol). The resulting solution was stirred overnight at room temperature. The resulting mixture was concentrated under vacuum. The residue was applied onto a silica gel column eluting with dichloromethane/methanol (9/1). The crude product (300 mg) was purified by Prep-HPLC with the following conditions: Column, XBridge Prep RP18 OBD Column, 19^{∗}150mm, 5um; mobile phase A: Water with 10 mM NH₄HCO₃, mobile phase B: ACN; (18% B increasing 40% B in 7 min; Detector, UV 254 nm. This resulted in 90.4 mg (20%) of N-[3-[2-(difluoromethoxy)-5-(methylsulfanyl)phenyl]-1-(2-[4-[2-(4-methylpiperazin-1-yl)-2-oxoethoxy]piperidin-1-yl]-2-oxoethyl)-1H-pyrazol-4-yl]pyrazolo[1,5-a]pyrimidine-3-carboxamide as a light yellow solid. LC/MS (Method F, ESI): [M+H]⁺ = 698.4, R_{T} = 2.52 min; ¹H NMR (300 MHz, CD₃OD-*d₄*): δ (ppm) 9.04 (dd, *J* = 7.2, 1.5Hz, 1H), 8.62-8.61 (m, 2H), 8.33 (s, 1H), 7.52 (s, 1H), 7.41 (dd, *J* = 8.7, 2.4Hz, 1H), 7.32 (d, *J* = 8.7 Hz, 1H), 7.15 (dd, *J* = 6.9, 4.2 Hz, 1H), 6.72 (t, *J* = 74.1 Hz, 1H), 5.29-5.22 (m, 2H), 4.35-4.12 (m, 2H), 3.70-3.47 (m, 9H), 2.50 (s, 3H), 2.42-2.30 (m, 4H), 2.24 (d, *J* = 10.2 Hz, 3H), 1.92-1.79 (m, 3H), 1.59-1.41 (m, 1H).

### Example 76

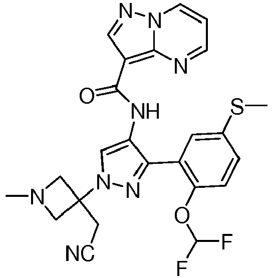

N-(1-(3-(cyanomethyl)-1-methylazetidin-3-yl)-3-(2-(difluoromethoxy)-5-(methylthio)phenyl)-1H-pyrazol-4-yl)pyrazolo[1,5-a]pyrimidine-3-carboxamide

Into a 30-mL sealed tube, was placed N-[1-[3-(cyanomethyl)azetidin-3-yl]-3-[2-(difluoromethoxy)-5-(methylsulfanyl)phenyl]-1H-pyrazol-4-yl]pyrazolo[1,5-a]pyrimidine-3-carboxamide (100 mg, 0.196 mmol), dichloromethane (6.0 mL), HCHO (100mg, 37% aqueous solution). The resulting solution was stirred overnight at room temperature. NaBH(OAc)₃ (63.0 mg, 0.297 mmol) was added. The resulting solution was allowed to react, with stirring, for an additional 6 h at room temperature. The resulting mixture was concentrated under vacuum. The resulting mixture was washed with H₂O. The resulting solution was extracted with of dichloromethane and the organic layers combined and dried over anhydrous sodium sulfate and concentrated under vacuum. The residue was applied onto a silica gel column eluting with dichloromethane/methanol (95/5). The crude product (100 mg) was purified by Prep-HPLC with the following conditions: Column, XBridge Prep C₁₈ OBD Column, 19^{∗}150mm, 5um; mobile phase, Mobile Phase A: Water with 10 mM NH₄HCO₃, Mobile Phase B: ACN,12% B increasing to 82% B within 8 min; Detector, UV 254 nm. This resulted in 15.9 mg (15%) of N-[1-[3-(cyanomethyl)-1-methylazetidin-3-yl]-3-[2-(difluoromethoxy)-5-(methylsulfanyl)phenyl]-1H-pyrazol-4-yl]pyrazolo[1,5-a]pyrimidine-3-carboxamide as a white solid. LC/MS (Method F, ESI): [M+H]⁺ = 525.2, R_{T}= 2.07 min; ¹H NMR (400 MHz, DMSO-*d₆*): δ (ppm) 9.79 (s, 1H), 9.35 (d, *J* = 6.8, 1H), 8.70-8.68 (m, 2H), 8.55 (s, 1H), 7.46-7.41 (m, 2H), 7.38-7.30 (m, 2H), 7.19 (t, *J* = 74.0 Hz, 1H), 3.64 (d, *J* = 9.2 Hz, 2H), 3.59 (d, *J* = 8.8 Hz, 2H), 3.52 (s, 2H), 2.51 (s, 3H), 2.36 (s, 3H).

### Example 77

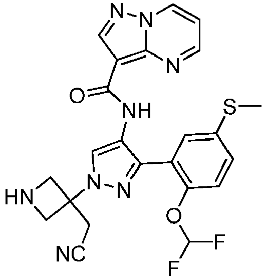

N-(1-(3-(cyanomethyl)azetidin-3-yl)-3-(2-(difluoromethoxy)-5-(methylthio)phenyl)-1H-pyrazol-4-yl)pyrazolo[1,5-a]pyrimidine-3-carboxamide

Into a 100-mL round-bottom flask, was placed N-[3-[2-(difluoromethoxy)-5-(methylsulfanyl)phenyl]-1H-pyrazol-4-yl]pyrazolo[1,5-a]pyrimidine-3-carboxamide (2 g, 4.803 mmol), tert-butyl 3-(cyanomethylidene)azetidine-1-carboxylate (933 mg, 4.80 mmol), CH₃CN (30 mL), DBU (512 mg, 3.36 mmol). The resulting solution was stirred for 7 h at 50°C and concentrated under vacuum. The residue was applied onto a silica gel column eluting with ethyl acetate/petroleum ether (1/1). This resulted in 1.5 g (51%) of tert-butyl 3-(cyanomethyl)-3-[3-[2-(difluoromethoxy)-5-(methylsulfanyl)phenyl]-4-[pyrazolo[1,5-a]pyrimidine-3-amido]-1H-pyrazol-1-yl]azetidine-1-carboxylate as a light yellow solid. LC/MS (Method G, ESI): [M+H]⁺ = 611.2, RT = 1.18 min.

A solution of tert-butyl 3-(cyanomethyl)-3-[3-[2-(difluoromethoxy)-5-(methylsulfanyl)phenyl]-4-[pyrazolo[1,5-a]pyrimidine-3-amido]-1H-pyrazol-1-yl]azetidine-1-carboxylate (200 mg, 0.328 mmol) in dichloromethane (8.0 mL) and trifluoroacetic acid (4.0 mL) was stirred for 5 h at room temperature and concentrated under vacuum. The residue was applied onto a silica gel column eluting with DCM/MeOH (9/1). The crude product (100 mg) was purified by Prep-HPLC with the following conditions: Column, XBridge Prep C₁₈ OBD Column, 19^{∗}150mm, 5um; mobile phase, A:Water with 10 mM NH₄HCO₃, Mobile Phase B: ACN,12% B increasing to 82% B within 8 min; Detector, UV 254 nm to give 19.8 mg (12%) of N-[1-[3-(cyanomethyl)azetidin-3-yl]-3-[2-(difluoromethoxy)-5-(methylsulfanyl)phenyl]-1H-pyrazol-4-yl]pyrazolo[1,5-a]pyrimidine-3-carboxamide as a white solid. LC/MS (Method F, ESI): [M+H]⁺ = 511.1, R_{T}= 2.03 min; ¹H NMR (400 MHz, DMSO-*d₆*): δ (ppm) 9.80 (s, 1H), 9.35 (d, *J* = 6.8 Hz, 1H), 8.69 (d, *J* = 3.2 Hz, 1H), 8.68 (s, 1H), 8.55 (s, 1H), 7.47 (d, *J* = 6.4 Hz, 1H), 7.46 (s, 1H), 7.41 (d, *J* = 6.8 Hz, 1H), 7.29 (dd, J = 6.8, 4.4 Hz, 1H), 7.19 (t, J = 73.2 Hz, 1H), 4.05 (d, *J* = 8.8 Hz, 2H), 3.68 (d, *J* = 8.8 Hz, 2H), 3.57 (s, 2H), 2.51 (s, 3H).

### Example 81

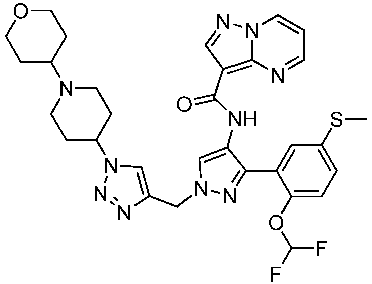

N-(3-(2-(difluoromethoxy)-5-(methylthio)phenyl)-1-((1-(1-(tetrahydro-2H-pyran-4-yl)piperidin-4-yl)-1H-1,2,3-triazol-4-yl)methyl)-1H-pyrazol-4-yl)pyrazolo[1,5-a]pyrimidine-3-carboxamide

3-Chloroprop-1-yne (351 mg, 4.711 mmol) was added to a mixture of N-[3-[2-(difluoromethoxy)-5-(methylsulfanyl)phenyl]-1H-pyrazol-4-yl]pyrazolo[1,5-a]pyrimidine-3-carboxamide (1.00 g, 2.40 mmol) and Cs₂CO₃ (1.57 g, 4.81 mmol) in DMF (30 mL). The reaction mixture was stirred at 50°C for 3 h and concentrated under vacuum. The residue was applied onto a silica gel column eluting with ethyl acetate/petroleum ether (3/2). The appropriate fractions were combined and concentrated under vacuum. This resulted in 600 mg (55%) of N-[3-[2-(difluoromethoxy)-5-(methylsulfanyl)phenyl]-1-(prop-2-yn-1-yl)-1H-pyrazol-4-yl]pyrazolo[1,5-a]pyrimidine-3-carboxamide as a yellow solid. LC/MS (Method B, ESI): [M+H]⁺ = 455.0. R_{T} =1.62 min.

To a solution of tert-butyl 4-bromopiperidine-1-carboxylate (1.00 g, 3.79 mmol) in DMF (20 mL) was added NaN₃ (739 mg, 11.4 mmol) and NaI (113 mg, 0.754 mmol). The resulting solution was stirred at 60°C for 20 h and poured into saturated sodium bicarbonate (50 mL), extracted with ethyl acetate (3x) and the organic layers combined. The organic extracts were washed with brine, dried over sodium sulfate and concentrated under vacuum. The residue was applied onto a silica gel column eluting with ethyl acetate/petroleum ether (1/19). The appropriate fractions were combined and concentrated under vacuum. This resulted in 620 mg (72%) of tert-butyl 4-azidopiperidine-1-carboxylate as yellow oil. TLC: PE/EA=4/1, R_{f}= 0.3.

Into a 50-mL round-bottom flask, was placed N-[3-[2-(difluoromethoxy)-5-(methylsulfanyl)phenyl]-1-(prop-2-yn-1-yl)-1H-pyrazol-4-yl]pyrazolo[1,5-a]pyrimidine-3-carboxamide (500 mg, 1.10 mmol), N,N-dimethylformamide (10 mL), tert-butyl 4-azidopiperidine-1-carboxylate (125 mg, 0.552 mmol), CuI (38.0 mg, 0.200 mmol), DIPEA (284 mg, 2.20 mmol). The resulting solution was stirred for 15 h at 25°C and concentrated under vacuum. The residue was applied onto a silica gel column eluting with ethyl acetate. The appropriate fractions were combined and concentrated under vacuum. This resulted in 228 mg (30%) of tert-butyl 4-[4-([3-[2-(difluoromethoxy)-5-(methylsulfanyl)phenyl]-4-[pyrazolo[1,5-a]pyrimidine-3-amido]-1H-pyrazol-1-yl]methyl)-1H-1,2,3-triazol-1-yl]piperidine-1-carboxylate as a yellow solid. LC/MS (Method I, ESI): [M+H]⁺ = 681.2. R_{T} =1.52 min.

A mixture of tert-butyl 4-[4-([3-[2-(difluoromethoxy)-5-(methylsulfanyl)phenyl]-4-[pyrazolo[1,5-a]pyrimidine-3-amido]-1H-pyrazol-1-yl]methyl)-1H-1,2,3-triazol-1-yl]piperidine-1-carboxylate (200 mg, 0.294 mmol) in methanol (10 mL) and concentrated hydrochloric acid (3.0 mL) was stirred for 15 h at 25 °C. The resulting mixture was concentrated under vacuum. This resulted in 220 mg (crude) of N-[3-[2-(difluoromethoxy)-5-(methylsulfanyl)phenyl]-1-[[1-(piperidin-4-yl)-1H-1,2,3-triazol-4-yl]methyl]-1H-pyrazol-4-yl]pyrazolo[1,5-a]pyrimidine-3-carboxamide as a yellow solid. LC/MS (Method D, ESI): [M+H]⁺ = 581.2. R_{T} = 1.41 min.

A mixture of N-[3-[2-(difluoromethoxy)-5-(methylsulfanyl)phenyl]-1-[[1-(piperidin-4-yl)-1H-1,2,3-triazol-4-yl]methyl]-1H-pyrazol-4-yl]pyrazolo[1,5-a]pyrimidine-3-carboxamide (193 mg, 0.332 mmol), oxan-4-one (133 mg, 1.33 mmol) in DCM (15 mL) was stirred for 15 h at 25°C. NaBH(OAc)₃ (706 mg, 3.33 mmol) was added. The resulting solution was for an additional 4 h at 25°C. The resulting mixture was concentrated under vacuum. The residue was applied onto a silica gel column eluting with dichloromethane/methanol (9/1). The collected fractions were combined and concentrated under vacuum. This resulted in 51.9 mg (23%) of N-[3-[2-(difluoromethoxy)-5-(methylsulfanyl)phenyl]-1-([1-[1-(oxan-4-yl)piperidin-4-yl]-1H-1,2,3-triazol-4-yl]methyl)-1H-pyrazol-4-yl]pyrazolo[1,5-a]pyrimidine-3-carboxamide as an off-white solid. LC/MS (Method C, ESI): [M+H]⁺ = 665.0, R_{T} = 1.48 min; ¹H NMR (300 MHz, CD₃OD-*d₄*): δ (ppm) 9.07 (dd, *J* = 7.1, 1.7 Hz, 1H), 8.63 (dd, *J* = 4.2, 1.5 Hz, 1H), 8.62 (s, 1H), 8.38 (s, 1H), 8.09 (s, 1H), 7.49 (d, *J* = 2.4 Hz, 1H), 7.43 (dd, *J* = 8.7, 2.4 Hz, 1H), 7.33 (d, *J* = 8.4 Hz, 1H), 7.18 (dd, J = 7.2, 4.2 Hz, 1H), 6.72 (t, *J* = 74.1 Hz, 1H), 5.52 (s, 2H), 4.64-4.45 (m, 1H), 4.03-3.98 (m, 2H), 3.45-3.33 (m, 2H), 3.19-3.14 (m, 2H), 2.68-2.54 (m, 1H), 2.51 (s, 3H), 2.49-2.42 (m, 2H), 2.29-2.06 (m, 4H), 1.86-1.83 (m, 2H), 1.62-1.56 (m, 2H).

### Example 101

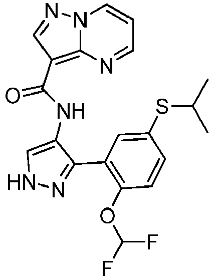

N-[3-[2-(difluoromethoxy)-5-(propan-2-ylsulfanyl)phenyl]-1H-pyrazol-4-yl]pyrazolo[1,5-a]pyrimidine-3-carboxamide

To a solution of N-[5-[5-bromo-2-(difluoromethoxy)phenyl]-1-[[2-(trimethylsilyl)ethoxy]methyl]-1H-pyrazol-4-yl]pyrazolo[1,5-a]pyrimidine-3-carboxamide (200 mg, 0.345 mmol) in dioxane (5.0 mL) was added (propan-2-ylsulfanyl)sodium (102 mg, 1.04 mmol), Pd₂(dba)₃.CHCl₃ (31.6 mg, 0.0305 mmol), XantPhos (39.9 mg, 0.0690 mmol) under nitrogen atmosphere. The resulting solution was stirred overnight at 100°C under N2 atmosphere. The resulting mixture was concentrated under vacuum. The residue was applied onto a silica gel column eluting with ethyl acetate/petroleum ether (1/1). This resulted in 103 mg (52%) of N-[5-[2-(difluoromethoxy)-5-(propan-2-ylsulfanyl)phenyl]-1-[[2-(trimethylsilyl)ethoxy-]methyl]-1H-pyrazol-4-yl]pyrazolo[1,5-a]pyrimidine-3-carboxamide as a light yellow solid. LC/MS (Method G, ESI): [M+H]⁺= 575.3, R_{T}= 1.18 min.

To a solution of N-[5-[2-(difluoromethoxy)-5-(propan-2-ylsulfanyl)phenyl]-1-[[2-(trimethylsilyl)ethoxy]methyl]-1H-pyrazol-4-yl]pyrazolo[1,5-a]pyrimidine-3-carboxamide (103 mg, 0.179 mmol) in dichloromethane (5.0 mL) was added TFA (3.1 mL). The resulting solution was stirred for 3 h at room temperature, and concentrated under vacuum. The crude product was purified by Prep-HPLC with the following conditions: Column, XBridge Prep C₁₈ OBD column, 19^{∗}150mm, 5um; mobile phase, CH₃CN/H₂O = 20% increasing to CH₃CN/H₂O = 46% in 6 min; Detector, UV 254 nm to give 13.6 mg (17%) of N-[3-[2-(difluoromethoxy)-5-(propan-2-ylsulfanyl)phenyl]-1H-pyrazol-4-yl]pyrazolo[1,5-a]pyrimidine-3-carboxamide as a off-white solid. LC/MS (Method A, ESI): [M+H]⁺ = 445.2, R_{T}= 1.84 min; ¹H NMR (300 MHz, DMSO-*d₆*): δ (ppm) 13.04 (s, 1H), 9.68 (s, 1H), 9.33 (dd, *J* = 7.1, 1.4 Hz, 1H), 8.68 - 8.65 (m, 2H), 8.27 (s, 1H), 7.56 - 7.53 (m, 2H), 7.37 (d, *J* = 8.4 Hz, 1H), 7.28 (dd, *J* = 7.2, 4.2 Hz, 1H), 7.23 (t, *J* = 73.5 Hz, 1H), 3.50 - 3.45 (m, 1H), 1.23 (d, *J* = 6.6 Hz, 6H).

### Example 129

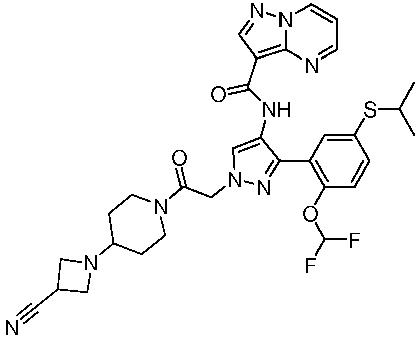

N-(1-[2-[4-(3-cyanoazetidin-1-yl)piperidin-1-yl]-2-oxoethyl]-3-[2-(difluoromethoxy)-5-(propan-2-ylsulfanyl)phenyl]-1H-pyrazol-4-yl)pyrazolo[1,5-a]pyrimidine-3-carboxamide

To a solution of tert-butyl 4-oxopiperidine-l-carboxylate (1.00 g, 5.02 mmol) in methanol (10 mL) was added NaOAc (412 mg, 5.02 mmol) and azetidine-3-carbonitrile hydrochloride (714 mg, 6.02 mmol). The mixture was stirred overnight at room temperature, then NaBH₃CN (315 mg, 5.02 mmol) was added. The resulting solution was stirred for 2 h at room temperature and concentrated under vacuum. The residue was dissolved in EtOAc and washed with water and brine. The organic phase was dried over anhydrous sodium sulfate and concentrated under vacuum. The residue was applied onto a silica gel column eluting with dichloride methane /methanol (95/5). This resulted in 1.41 g (crude) of tert-butyl 4-(3-cyanoazetidin-1-yl)piperidine-1-carboxylate as a white solid. LC/MS (Method I, ESI): [M+H]⁺ = 266.4, R_{T}= 0.71 min.

To a solution of tert-butyl 4-(3-cyanoazetidin-1-yl)piperidine-1-carboxylate (1.41 g, 5.30 mmol) in dioxane (4.0 mL), a solution of HCl in dioxane (4.0 ml, 15% w/w) was added. The resulting solution was stirred for 3 h at room temperature. The resulting mixture was concentrated under vacuum. This resulted in 1.32 g (crude) of 1-(piperidin-4-yl)azetidine-3-carbonitrile hydrochloride as a white solid. LC/MS (Method G, ESI): [M+H]⁺= 166.2, R_{T}= 0.17 min; ¹H-NMR (300 MHz, CD₃OD-*d₄*): δ (ppm) 4.52 - 4.30 (m, 4H), 3.67 - 3.54 (m, 4H), 3.08 (t, *J* = 12.75 Hz, 2H), 2.39 - 2.17 (m, 2H), 1.18 - 1.69 (m, 2H).

To a solution of 2-[3-[2-(difluoromethoxy)-5-(propan-2-ylsulfanyl)phenyl]-4-[pyrazolo[1,5-a]pyrimidine-3-amido]-1H-pyrazol-1-yl]acetic acid (1.11 g, 2.19 mmol) in N,N-dimethylformamide (7.0 mL) was added EDC.HCl (842 mg, 4.39 mmol), HOBt (592 mg, 4.38 mmol) and DIPEA (1131 mg, 8.75 mmol). The reaction mixture was stirred for 0.5 hour at room temperature and then 1-(piperidin-4-yl)azetidine-3-carbonitrile hydrochloride (663 mg, 3.286 mmol) was added. The resulting solution was stirred for 12 h at 45°C. The resulting solution was diluted with 50 mL of ethyl acetate. The resulting mixture was washed with water and brine. The organic phase was dried over anhydrous sodium sulfate and concentrated under vacuum. The residue was applied onto a silica gel column eluting with dichloromethane/methanol (95/5). Then was purified by Prep-HPLC with the following conditions: Column, XBridge Prep C₁₈ OBD Column, 19^{∗}150mm, 5um; mobile phase, Water (10 mM NH₄HCO₃) and ACN (28.0% ACN up to 42.0% in 13 min); Detector, UV 254/220nm. The obtained product was crystallized in ethanol to afford 419.8 mg (30%) of N-(1-[2-[4-(3-cyanoazetidin-1-yl)piperidin-1-yl]-2-oxoethyl]-3-[2-(difluoromethoxy)-5-(propan-2-ylsulfanyl)phenyl]-1H-pyrazol-4-yl)pyrazolo[1,5-a]pyrimidine-3-carboxamide as a yellow solid. LC/MS (Method D, ESI): [M+H]⁺= 650.3, R_{T}= 1.72 min; ¹H-NMR (300 MHz, CD₃OD-*d₄*): δ (ppm) 9.09 (dd, *J* = 7.2, 1.8 Hz, 1H), 8.64 - 8.62 (m, 2H), 8.34 (s, 1H), 7.67 (d, *J* = 2.4 Hz, 1H), 7.58 (dd, *J* = 8.6, 2.4 Hz, 1H), 7.35 (d, *J* = 8.4 Hz, 1H), 7.19 (dd, *J* = 6.9, 4.2 Hz, 1H), 6.79 (t, *J* = 73.8 Hz, 1H), 5.27 - 5.20 (m, 2H), 4.24 - 4.20 (m, 1H), 3.95 - 3.90 (m, 1H), 3.67 - 3.51 (m, 2H), 3.50 - 3.36 (m, 4H), 3.29 - 3.17 (m, 1H), 3.05-3.08 (m, 1H), 2.49-2.45 (m, 1H), 1.90 - 1.68 (m, 2H), 1.28 - 1.18 (m, 8H).

### Example 144

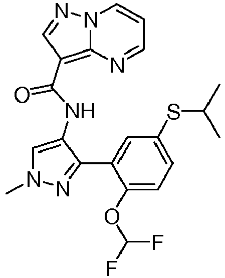

N-[3-[2-(difluoromethoxy)-5-(propan-2-ylsulfanyl)phenyl]-1-methyl-1H-pyrazol-4-yl]pyrazolo[1,5-a]pyrimidine-3-carboxamide

To a solution N-[3-[5-bromo-2-(difluoromethoxy)phenyl]-1-methyl-1H-pyrazol-4-yl]pyrazolo[1,5-a]pyrimidine-3-carboxamide (350 mg, 0.756 mmol) in toluene (10 mL) added (propan-2-ylsulfanyl)sodium (222 mg, 2.26 mmol), Pd₂(dba)₃.CHCl₃ (34.1 mg, 0.0331 mmol) and XantPhos (34.1 mg, 0.0591 mmol) under nitrogen atmosphere. The resulting solution was stirred overnight at 85°C in an oil bath. This reaction was repeated twice. The resulting mixture was concentrated under vacuum. The residue was applied onto a silica gel column eluting with ethyl acetate/petroleum ether. The crude product was purified by re-crystallization from ethyl acetate to give 356.8 mg (52%) of N-[3-[2-(difluoromethoxy)-5-(propan-2-ylsulfanyl)phenyl]-1-methyl-1H-pyrazol-4-yl]pyrazolo[1,5-a]pyrimidine-3-carboxamide as a yellow solid. (Method A, ESI, *m*/*z*): [M+H]⁺ = 459.2 R_{T} = 2.02 min; 1H NMR (400 MHz, CDCl₃): δ (ppm) 9.88 (s, H), 8.79 (dd, *J* = 6.8, 1.6 Hz, 1H), 8.74 (s, 1H), 8.59 (dd, *J* = 4.0, 1.6 Hz, 1H), 8.32 (s, 1H), 7.72 (d, *J* = 2.4 Hz, 1H), 7.47 (dd, *J* = 8.4, 2.4 Hz, 1H), 7.29 (d, *J* = 2.8 Hz, 1H), 7.02 (dd, J = 6.8, 4.0 Hz, 1H), 6.50 (t, *J* = 74.4 Hz, 1H), 4.02 (s, 3H), 3.46 - 3.39 (m, 1H), 1.31 (d, *J* = 6.4 Hz, 6H).

### Example 150

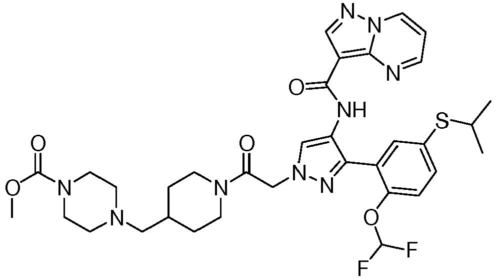

methyl 4-[[1-(2-[3-[2-(difluoromethoxy)-5-(propan-2-ylsulfanyl)phenyl]-4-[pyrazolo[1,5-a]pyrimidine-3-amido]-1H-pyrazol-1-yl]acetyl)piperidin-4-yl]methyl]piperazine-1-carboxylate

To a solution of 2-[3-[2-(difluoromethoxy)-5-(propan-2-ylsulfanyl)phenyl]-4-[pyrazolo[1,5-a]pyrimidine-3-amido]-1H-pyrazol-1-yl]acetic acid (200 mg, 0.398 mmol) and methyl 4-(piperidin-4-ylmethyl)piperazine-1-carboxylate (192 mg, 0.796 mmol) in N,N-dimethylformamide (10 mL) added EDC.HCl (153 mg, 0.796 mmol), HOBt (108 mg, 0.796 mmol), DIPEA (206 mg, 1.59 mmol) at room temperature. The resulting solution was stirred for 12 h at 45°C. The resulting solution was diluted with 50 mL of ethyl acetate. The resulting mixture was washed with H₂O and brine. The mixture was dried over anhydrous sodium sulfate and concentrated under vacuum. The residue was applied onto a silica gel column eluting with ethyl acetate/THF (90/10). The crude product was re-crystallized from methanol/isopropyl ether in the ratio of 1/5 to give 136.8 mg (47%) of methyl 4-[[1-(2-[3-[2-(difluoromethoxy)-5-(propan-2-ylsulfanyl)phenyl]-4-[pyrazolo[1,5-a]pyrimidine-3-amido]-1H-pyrazol-1-yl]acetyl)piperidin-4-yl]methyl]piperazine-1-carboxylate as a white solid. LC/MS (Method D, ESI): [M+H]⁺=726.4, R_{T}= 1.85 min; ¹H-NMR (300 MHz, CD₃OD-*d₄*): δ (ppm) 9.09 (dd, *J* = 6.9, 1.8 Hz, 1H), 8.63 - 8.61 (m, 2H), 8.34 (s, 1H), 7.67 (d, *J* = 2.1 Hz, 1H), 7.56 (dd, *J* = 8.6, 2.4 Hz, 1H), 7.35 (d, *J* = 8.4 Hz, 1H), 7.18 (dd, *J* = 6.9, 4.2 Hz, 1H), 6.79(t, *J* = 73.8 Hz, 1H), 5.26 (d, *J* = 16.5 Hz, 1H), 5.15 (d, *J* = 16.5 Hz, 1H), 5.38 - 5.07 (m, 2H), 4.53 - 4.48 (m, 1H), 4.08 - 3.98 (m, 1H), 3.68 (s, 3H), 3.56 - 3.38 (m, 5H), 3..22 - 3.15 (m, 1H), 2.79 - 2.70 (m, 1H), 2.38 (t, *J* = 5.1 Hz, 4H), 2.23 (d, *J* = *6.9* Hz, 2H), 1.91 - 1.83 (m, 3H), 1.27 (d, *J* = 6.6 Hz, 6H), 1.22 - 1.12 (m, 2H).

### Example 156

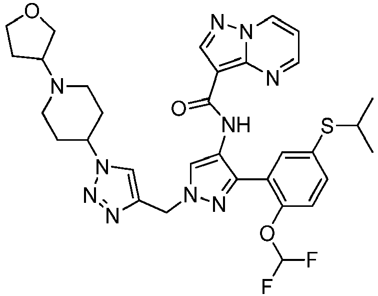

N-[3-[2-(difluoromethoxy)-5-(propan-2-ylsulfanyl)phenyl]-1-([1-[1-(oxolan-3-yl)piperidin-4-yl]-1H-1,2,3-triazol-4-yl]methyl)-1H-pyrazol-4-yl]pyrazolo[1,5-a]pyrimidine-3-carboxamide

To a suspension of N-[3-[5-bromo-2-(difluoromethoxy)phenyl]-1H-pyrazol-4-yl]pyrazolo[1,5-a]pyrimidine-3-carboxamide (2.00 g, 4.45 mmol) and Cs₂CO₃ (1.74 g, 5.34 mmol) in N,N-dimethylformamide (30 mL) was added dropwise 3-chloroprop-1-yne (400 mg, 5.37 mmol) at room temperature. The resulting solution was stirred for 12 h at room temperature. The resulting mixture was diluted with 50 mL of ethyl acetate. The resulted mixture was washed with water and brine and further dried over anhydrous sodium sulfate and concentrated under vacuum. The residue was applied onto a silica gel column eluting with dichloromethane/ethyl acetate (4/6) to give 1.83 g (84%) of N-[3-[5-bromo-2-(difluoromethoxy)phenyl]-1-(prop-2-yn-1-yl)-1H-pyrazol-4-yl]pyrazolo[1,5-a]pyrimidine-3-carboxamide as a yellow solid. LC/MS (Method I, ESI): [M+H]⁺ = 487.1, R_{T}= 1.14 min.

To a solution of tert-butyl 4-(methanesulfonyloxy)piperidine-1-carboxylate (1.50 g, 5.37 mmol) in N,N-dimethylformamide (15 mL) was added sodium azide (771 mg, 11.9 mmol) at room temperature. The resulting solution was stirred for 12 h at 100°C in an oil bath. The resulting mixture was diluted with 100 ml of ethyl acetate. The ethyl acetate solution was washed with water and brine, dried over anhydrous sodium sulfate, filtered and concentrated under vacuum. This resulted in 1.30 g (crude) of tert-butyl 4-azidopiperidine-1-carboxylate as yellow oil. The crude product was used directly without any further purification.

To a solution of N-[3-[5-bromo-2-(difluoromethoxy)phenyl]-1-(prop-2-yn-1-yl)-1H-pyrazol-4-yl]pyrazolo[1,5-a]pyrimidine-3-carboxamide (2.00 g, 4.11 mmol) in N,N-dimethylformamide (80 mL) added tert-butyl 4-azidopiperidine-1-carboxylate (1.86 g, 8.22 mmol), CuI (779 mg, 4.11 mmol) and DIPEA (1.06 g, 8.20 mmol) at room temperature under N₂ atmosphere. The resulting solution was stirred for 12 h at room temperature under N₂ atmosphere and diluted with 200 ml of ethyl acetate. The resulting mixture was washed with water and brine. The organic phase was dried over anhydrous sodium sulfate and concentrated under vacuum. The residue was applied onto a silica gel column eluting with dichloromethane/methanol (96/4) to give 1.02 g (35%) of tert-butyl 4-[4-([3-[5-bromo-2-(difluoromethoxy)phenyl]-4-[pyrazolo[1,5-a]pyrimidine-3-amido]-1H-pyrazol-1-yl]methyl)-1H-1,2,3-triazol-1-yl]piperidine-1-carboxylate as a brown solid. LC/MS (Method G ESI): [M+H]⁺ = 657.2, R_{T}= 0.99 min.

To a solution of tert-butyl 4-[4-([3-[5-bromo-2-(difluoromethoxy)phenyl]-4-[pyrazolo[1,5-a]pyrimidine-3-amido]-1H-pyrazol-1-yl]methyl)-1H-1,2,3-triazol-1-yl]piperidine-1-carboxylate (300 mg, 0.420 mmol) in toluene (15 mL) was added (propan-2-ylsulfanyl)sodium (124 mg, 1.26 mmol), Pd₂(dba)₃.CHCl₃ (21.8 mg, 0.0211 mmol) and XantPhos (24.3 mg, 0.0419 mmol) under N₂ atmosphere. The resulting solution was stirred for 12 h at 80°C in an oil bath under N₂ atmosphere. The resulting mixture was concentrated under vacuum. The residue was applied onto a silica gel column eluting with dichloromethane/methanol (95/5) to obtain 273 mg (92%) of tert-butyl 4-[4-([3-[2-(difluoromethoxy)-5-(propan-2-ylsulfanyl)phenyl]-4-[pyrazolo[1,5-a]pyrimidine-3-amido]-1H-pyrazol-1-yl]methyl)-1H-1,2,3-triazol-1-yl]piperidine-1-carboxylate as a yellow solid. LC/MS (Method G, ESI): [M+H]⁺= 709.4, R_{T} = 1.16 min.

To a solution of tert-butyl 4-[4-([3-[2-(difluoromethoxy)-5-(propan-2-ylsulfanyl)phenyl]-4-[pyrazolo[1,5-a]pyrimidine-3-amido]-1H-pyrazol-1-yl]methyl)-1H-1,2,3-triazol-1-yl]piperidine-1-carboxylate (273 mg, 0.385 mmol) in dioxane (5.0 mL) was added a solution of HCl in dioxane (5.0 mL, 15%, w/w) at room temperature. The reaction mixture was stirred for 2 hours. The solids were collected by filtration. This resulted in 257 mg (crude) of N-[3-[2-(difluoromethoxy)-5-(propan-2-ylsulfanyl)phenyl]-1-[[1-(piperidin-4-yl)-1H-1,2,3-triazol-4-yl]methyl]-1H-pyrazol-4-yl]pyrazolo[1,5-a]pyrimidine-3-carboxamide hydrochloride as a yellow solid. LC/MS (Method G, ESI): [M+H]⁺ = 609.4, R_{T} = 0.74 min.

To a solution of N-[3-[2-(difluoromethoxy)-5-(propan-2-ylsulfanyl)phenyl]-1-[[1-(piperidin-4-yl)-1H-1,2,3-triazol-4-yl]methyl]-1H-pyrazol-4-yl]pyrazolo[1,5-a]pyrimidine-3-carboxamide (257 mg, 0.422 mmol) in dichloromethane (15 mL) added oxolan-3-one (145 mg, 1.69 mmol) and NaOAc (36.1 mg, 0.442 mmol). The resulting solution was stirred for 4 h at room temperature. Then NaBH(OAc)₃ (179 mg, 0.844 mmol) was added one portion. The resulting solution was allowed to react with stirring for an additional 12 h at room temperature. The resulting mixture was concentrated under vacuum. The residue was applied onto a silica gel column eluting with dichloromethane/methanol (95/5). The crude product (178 mg) was purified by Prep-HPLC with the following conditions: Column, XBridge Shield RP18 OBD Column, 19^{∗}150mm, 5um; mobile phase, Water (10 mM NH₄HCO₃) and ACN (25.0% ACN up to 50.0% in 8 min); Detector, UV 220 nm, 254 nm to give 53.1 mg (18%) of N-[3-[2-(difluoromethoxy)-5-(propan-2-ylsulfanyl)phenyl]-1-([1-[1-(oxolan-3-yl)piperidin-4-yl]-1H-1,2,3-triazol-4-yl]methyl)-1H-pyrazol-4-yl]pyrazolo[1,5-a]pyrimidine-3-carboxamide as a white solid. LC/MS (Method M, ESI): [M+H]⁺ = 679.4, R_{T}= 2.69 min; ¹H NMR (400 MHz,CD₃OD-*d₄*): δ (ppm) 9.10 (dd, *J* = 6.8, 1.6 Hz, 1H), 8.65 - 8.64 (m, 2H), 8.39 (s, 1H), 8.12 (s, 1H), 7.64 (d, *J* = 2.4 Hz, 1H), 7.57 (dd, *J* = 8.4, 2.4 Hz, 1H), 7.35 (d, J = 8.8 Hz, 1H), 7.22 (dd, *J* = 7.0, 4.2 Hz, 1H), 6.80 (t, J = 73.6 Hz, 1H), 5.54 (s, 2H), 4.68 - 4.48 (m, 1H), 4.03 - 3.84 (m, 2H), 3.85 - 3.61 (m, 2H), 3.54 - 3.39 (m, 1H), 3.24 - 3.02 (m, 2H), 2.97 - 2.92 (m, 1H), 2.39 - 2.36 (m, 2H), 2.23 - 2.14 (m, 5H), 1.90 - 1.83 (m, 1H), 1.28 (d, *J* = 6.8 Hz, 6H).

### Example 159

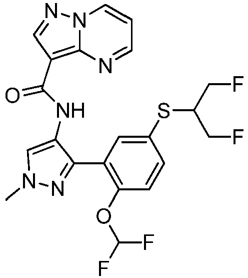

N-[3-[2-(difluoromethoxy)-5-[(1,3-difluoropropan-2-yl)sulfanyl]phenyl]-1-methyl-1H-pyrazol-4-yl]pyrazolo[1,5-a]pyrimidine-3-carboxamide

To a suspension of sodium hydride (260 mg, 10.8 mmol) in toluene (40 ml) was added dropwise tris(propan-2-yl)silanethiol (1.23 g, 6.47 mmol) at room temperature under N2 atmosphere. The mixture was stirred for 1 hour at this temperature until the mixture turn clear. Then N-[3-[5-bromo-2-(difluoromethoxy)phenyl]-1-methyl-1H-pyrazol-4-yl]pyrazolo[1,5-a]pyrimidine-3-carboxamide (2.50 g, 5.40 mmol), Pd₂(dba)₃.CHCl₃ (184 mg, 0.178 mmol) and Xantphos (250 mg, 0.432 mmol) was added at room temperature under nitrogen atmosphere. The resulting solution was stirred for 30 min at 90°C under N₂ atmosphere. The reaction was then quenched by the addition of 50 mL of aqueous NH₄Cl solution. The resulting solution was diluted with 100 mL of EA. The organic layer was separated and washed with 3x50 mL of water and 2x50 mL of brine. The mixture was dried over anhydrous sodium sulfate and concentrated under vacuum. The residue was applied onto a silica gel column eluting with ethyl acetate/petroleum ether (1/1) to give 2.35 g (76%) of N-[3-[2-(difluoromethoxy)-5-[[tris(propan-2-yl)silyl]sulfanyl]phenyl]-1-methyl-1H-pyrazol-4-yl]pyrazolo[1,5-a]pyrimidine-3-carboxamide as a off-white solid. TLC: PE/EA = 1/1, R_{f} = 0.4.

To a solution of 1,3-difluoropropan-2-ol (102 mg, 1.04 mmol) and DIPEA (134 mg, 1.04 mmol) in dichloromethane (15 mL) was added dropwise methanesulfonyl chloride (144 mg, 1.25 mmol) at 0 °C. Then the resulting solution was stirred for 2 h at room temperature, and washed with water and brine dried over anhydrous sodium sulfate and concentrated under vacuum. This resulted in 112 mg (crude product) of 1,3-difluoropropan-2-yl trifluoromethanesulfonate as a yellow oil. The resulted product was used directly without any purification.

To a solution of N-[3-[2-(difluoromethoxy)-5-[[tris(propan-2-yl)silyl]sulfanyl]phenyl]-1-methyl-1H-pyrazol-4-yl]pyrazolo[1,5-a]pyrimidine-3-carboxamide (150 mg, 0.262 mmol) in N,N-dimethylformamide (10 mL) was added 1,3-difluoropropan-2-yl trifluoromethanesulfonate (110 mg, 0.482 mmol). Then tetrabutylammonium fluoride tetrahydrofuran solution (0.26 mL, 1M in THF, 0.26 mmol) was added dropwise at room temperature. The resulting solution was stirred for 12 h at this temperature. The resulting solution was diluted with 50 mL of ethyl acetate and washed with H₂O and brine. The organic phase was dried over anhydrous sodium sulfate and concentrated under vacuum. The crude product was purified by Prep-HPLC with the following conditions: Column, XBridge Prep C₁₈ OBD Column, 19^{∗}150mm, 5um; mobile phase, A:Water (10 mM NH₄HCO₃) mobile phase B: ACN; (33% B increasing to 67% B in 9 min; Detector, UV 254 nm to give 13.9 mg (11%) of N-[3-[2-(difluoromethoxy)-5-[(1,3-difluoropropan-2-yl)sulfanyl]phenyl]-1-methyl-1H-pyrazol-4-yl]pyrazolo[1,5-a]pyrimidine-3-carboxamide as a white solid. LC/MS (Method D, ESI): [M+H]⁺= 495.2, R_{T}= 1.71 min; ¹H NMR (300 MHz,DMSO-*d₆*): δ (ppm) 9.69 (s, 1H), 9.33 (dd, *J* = 7.0, 1.6 Hz, 1H), 8.68 - 8.66 (m, 2H), 8.30 (s, 1H), 7.70 - 7.66 (m, 2H), 7.41 (d, *J* = 8.4 Hz, 1H), 7.29 (dd, *J* = 6.9, 4.2 Hz, 1H), 7.28 (t, *J* = 73.2 Hz, 1H), 4.70 (d, *J* = 5.7 Hz, 2H), 4.54 (d, *J* = 5.4 Hz, 2H), 3.94 (s, 3H), 3.89 - 3.76 (m, 1H).

### Example 160

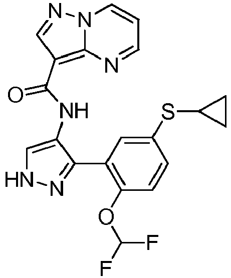

N-[3-[5-(cyclopropylsulfanyl)-2-(difluoromethoxy)phenyl]-1H-pyrazol-4-yl]pyrazolo[1,5-a]pyrimidine-3-carboxamide

To a suspension of sodium hydride (310 mg, 12.9 mmol) in toluene (80 mL) was added dropwise cyclopropanethiol (960 mg, 12.9 mmol) at room temperature under nitrogen atmosphere. The resulting suspension was stirred for 6 h at this temperature under nitrogen. Then N-[5-[5-bromo-2-(difluoromethoxy)phenyl]-1-[[2-(trimethylsilyl)ethoxy]methyl]-1H-pyrazol-4-yl]pyrazolo[1,5-a]pyrimidine-3-carboxamide (5.00 g, 8.63 mmol), Pd₂(dba)₃.CHCl₃ (450 mg, 0.435 mmol) and XantPhos (500 mg, 0.864 mmol) were added under nitrogen atmosphere. The resulting solution was allowed to react with stirring for an additional 12 h while the temperature was maintained at 85°C in an oil bath under N₂ atmosphere. The resulting mixture was concentrated under vacuum. The residue was applied onto a silica gel column eluting with ethyl acetate/hexane (1/1) to give 3.87 g (78%) of N-[5-5-(cyclopropylsulfanyl)-2-(difluoromethoxy)phenyl]-1-[[2-(trimethylsilyl)ethoxy]methyl]-1H-pyrazol-4-yl]pyrazolo[1,5-a]pyrimidine-3-carboxamide as a yellow solid. LC/MS (Method G, ESI): [M+H]⁺= 573.2, R_{T}= 1.12 min.

To a solution of N-[5-[5-(cyclopropylsulfanyl)-2-(difluoromethoxy)phenyl]-1-[[2-(trimethylsilyl)ethoxy]methyl]-1H-pyrazol-4-yl]pyrazolo[1,5-a]pyrimidine-3-carboxamide (120 mg, 0.210 mmol) in methanol (12 mL) was added concentrated HCl (6 mL, 12 M) at room temperature. The resulting solution was stirred for 2 h at room temperature. The resulting mixture was concentrated under vacuum. The residue was applied onto a silica gel column eluting with PE/EA. The product (150 mg) was further purified by Prep-HPLC with the following conditions: Column, XBridge Shield RP18 OBD Column, 19^{∗}150mm, 5um; mobile phase, ACN/H₂O (10 mM NH₄HCO₃) = 21% increasing to ACN/H₂O (10 mM NH₄HCO₃) = 50% within 9 min; Detector, UV 254 nm to obtain 49.7 mg (54%) of N-[3-[5-(cyclopropylsulfanyl)-2-(difluoromethoxy)phenyl]-1H-pyrazol-4-yl]pyrazolo[1,5-a]pyrimidine-3-carboxamide as an off-white solid. LC/MS (Method N, ESI): [M+H]⁺ = 443.2, R_{T}= 3.12 min; ¹H NMR (400 MHz, CD₃OD-*d₄*): δ (ppm) 9.09 (dd, *J* = 7.2, 1.6 Hz, 1H), 8.66 - 8.64 (m, 2H), 8.27 (s, 1 H), 7.62 (d, *J =* 2.4 Hz, 1H), 7.53 (dd, *J* = 8.8, 2.4 Hz, 1 H), 7.36 (d, *J* = 8.8 Hz, 1H), 7.22 (dd, *J* = 6.8, 4.4 Hz, 1H), 6.74 (t, *J* = 73.8 Hz, 1 H), 2.31 - 2.23 (m, 1 H), 1.12 - 1.02 (m, 2 H), 0.70 - 0.64 (m, 2 H).

### Example 163

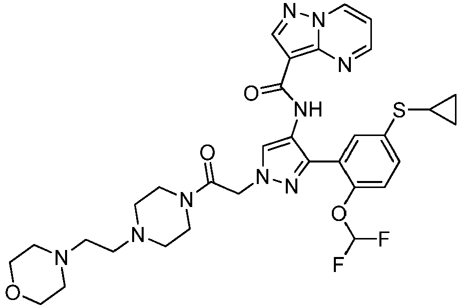

N-[3-[5-(cyclopropylsulfanyl)-2-(difluoromethoxy)phenyl]-1-(2-[4-[2-(morpholin-4-yl)ethyl]piperazin-1-yl]-2-oxoethyl)-1H-pyrazol-4-yl]pyrazolo[1,5-a]pyrimidine-3-carboxamide

To a solution of 4-[2-(piperazin-1-yl)ethyl]morpholine (500 mg, 2.51 mmol) and DIPEA (81.0 mg, 0.627 mmol) in dichloromethane (15 mL) was added dropwise a solution of 2-bromoacetyl bromide (660 mg, 3.27 mmol) in DCM (5.0 ml) at 0°C. The resulting solution was stirred for 10 min at 0°C. The pH value of the solution was adjusted to 2 with hydrogen chloride. The resulting solution was extracted with 3x50 mL of water and the water layers combined. Sodium bicarbonate was employed to adjust the pH to 8. The resulting solution was extracted with 3x100 ML of dichloromethane and the organic layers combined and and dried with Na₂SO₄. The solids were filtered out. The resulting mixture was concentrated under vacuum to give 745 mg (93%) of 2-bromo-1-[4-[2-(morpholin-4-yl)ethyl]piperazin-1-yl]ethan-1-one as a yellow solid. LC/MS (Method G, ESI): [M+H]+ = 322.1, RT= 0.15 min.

To a solution of N-[3-[5-(cyclopropylsulfanyl)-2-(difluoromethoxy)phenyl]-1H-pyrazol-4-yl]pyrazolo[1,5-a]pyrimidine-3-carboxamide (100 mg, 0.226 mmol) in N,N-dimethylformamide (10 mL) was added Cs₂CO₃ (147 mg, 0.451 mmol). Then 2-bromo-1-[4-[2-(morpholin-4-yl)ethyl]piperazin-1-yl]ethan-1-one (360 mg, 1.12 mmol) was added in several batches. The resulting solution was stirred for 6 h at 60°C. The resulting mixture was concentrated under vacuum. The residue was applied onto a silica gel column eluting with dichloromethane/methanol (4/1). The collected fractions were combined and concentrated under vacuum. The product (80 mg) was purified by Prep-HPLC with the following conditions: Column, XBridge Shield RP18 OBD Column, 19^{∗}150mm, 5um; mobile phase, Water with 10 mM NH₄HCO₃ and MeCN (13.0% MeCN up to 50.0% in 13 min); Detector, UV 254 nm. to give 7.61 mg (5%) of N-[3-[5-(cyclopropylsulfanyl)-2-(difluoromethoxy)phenyl]-1-(2-[4-[2-(morpholin-4-yl)ethyl]piperazin-1-yl]-2-oxoethyl)-1H-pyrazol-4-yl]pyrazolo[1,5-a]pyrimidine-3-carboxamide as a light yellow solid. LC/MS (Method D, ESI): [M+H]⁺ = 682.3, R_{T} = 1.54 min; ¹H NMR (400 MHz, CD₃OD-*d₄*) : δ (ppm) 9.09 (dd, *J* = 7.2, 1.6 Hz, 1H), 8.66 - 8.65 (m, 2H), 8.37 (s, 1H), 7.65 (d, *J* = 2.4 Hz, 1H), 7.56 (dd, *J* = 8.4, 2.4 Hz, 1H), 7.36 (d, *J* = 8.8 Hz, 1H), 7.21 (dd, *J* = 6.8, 4.4 Hz, 1H), 6.75 (t, *J* = 74.0 Hz, 1H), 5.25 (s, 2H), 3.72 - 3.67 (m, 8H), 2.61 - 2.55 (m, 12H), 2.30 - 2.26 (m, 1H), 1.13 - 1.08 (m, 2H), 0.70 - 0.63 (m, 2H).

### Example 167

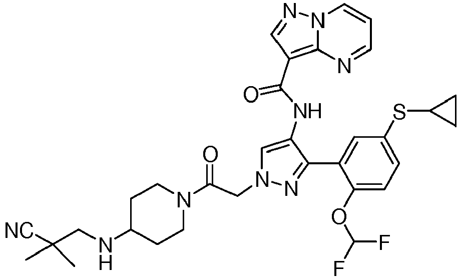

N-[1-(2-[4-[(2-cyano-2,2-dimethylethyl)amino]piperidin-1-yl]-2-oxoethyl)-3-[5-(cyclopropylsulfanyl)-2-(difluoromethoxy)phenyl]-1H-pyrazol-4-yl]pyrazolo[1,5-a]pyrimidine-3-carboxamide

To a solution of N-[3-[5-(cyclopropylsulfanyl)-2-(difluoromethoxy)phenyl]-1H-pyrazol-4-yl]pyrazolo[1,5-a]pyrimidine-3-carboxamide (700 mg, 1.58 mmol) in N,N-dimethylformamide (20 mL) was added Cs₂CO₃ (1.01 g, 3.07 mmol). Then 1-(2-bromoacetyl)piperidin-4-one (694 mg, 3.15 mmol) was added in several batches. The resulting solution was stirred for 2 h at 60°C in an oil bath. The reaction mixture was cooled. The resulting solution was diluted with 100 mL of H₂O. The resulting solution was extracted with 3x100 mL of dichloromethane and the organic layers combined and concentrated under vacuum. The residue was applied onto a silica gel column eluting with dichloromethane/methanol (20/1∼10/1). The collected fractions were combined and concentrated under vacuum. This resulted in 950 mg of N-[3-[5-(cyclopropylsulfanyl)-2-(difluoromethoxy)phenyl]-1-[2-oxo-2-(4-oxopiperidin-1-yl)ethyl]-1H-pyrazol-4-yl]pyrazolo[1,5-a]pyrimidine-3-carboxamide as a light yellow solid. LC/MS (Method I, ESI): [M+H]⁺ = 582.2, R_{T}= 0.99 min.

To a solution of N-[3-[5-(cyclopropylsulfanyl)-2-(difluoromethoxy)phenyl]-1-[2-oxo-2-(4-oxopiperidin-1-yl)ethyl]-1H-pyrazol-4-yl]pyrazolo[1,5-a]pyrimidine-3-carboxamide (150 mg, 0.258 mmol) in dichloromethane (10 mL) was added 3-amino-2,2-dimethylpropanenitrile (38.1 mg, 0.387 mmol). The resulting solution was stirred overnight at room temperature. Then NaBH(OAc)₃ (82.1 mg, 0.387 mmol) was added. The resulting solution was allowed to react with stirring for an additional 2 h at room temperature. The resulting mixture was concentrated under vacuum. The residue was applied onto a silica gel column eluting with dichloromethane/methanol (10/1). The collected fractions were combined and concentrated under vacuum. The crude product was purified by Prep-HPLC with the following conditions: Column, XBridge Shield RP18 OBD Column, 19^{∗}150mm, 5um; mobile phase, Water with 10 mM NH₄HCO₃ and MeCN (15.0% MeCN up to 50.0% in 10 min); Detector, UV 254nm to give in 64.2 mg (38%) of N-[1-(2-[4-[(2-cyano-2,2-dimethylethyl)amino]piperidin-1-yl]-2-oxoethyl)-3-[5-(cyclopropylsulfanyl)-2-(difluoromethoxy)phenyl]-1H-pyrazol-4-yl]pyrazolo[1,5-a]pyrimidine-3-carboxamide as a light yellow solid. LC/MS (Method K, ESI): [M+H]⁺ = 664.4, R_{T} = 2.66 min. ¹H NMR (300 MHz, CD₃OD-*d₄*): δ (ppm) 9.08 (dd, *J* = 6.9, 1.5 Hz, 1H), 8.64 - 8.62 (m, 2H), 8.35 (s, 1H), 7.63 (d, *J* = 2.4 Hz, 1H), 7.52 (dd, *J* = 8.7, 2.4 Hz, 1H), 7.34 (d, *J* = 8.7 Hz, 1H), 7.18 (dd, *J* = 6.9, 4.2 Hz, 1H), 6.74 (t, *J* = 74.1 Hz, 1H), 5.33 - 5.17 (m, 2H), 4.42 - 4.28 (m, 1H), 4.08 - 3.92 (m, 1 H), 3.30 - 3.20 (m, 1H), 3.02 - 2.89 (m, 1 H), 2.88 - 2.68 (m, 3 H), 2.32 - 2.20 (m, 1 H), 2.08 - 1.90 (m, 2 H), 1.51 - 1.25 (m, 8 H), 1.15 - 1.04 (m, 2 H), 0.71 - 0.55 (m, 2 H).

### Example 175

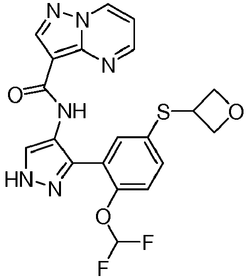

N-[3-[2-(difluoromethoxy)-5-(oxetan-3-ylsulfanyl)phenyl]-1H-pyrazol-4-yl]pyrazolo[1,5-a]pyrimidine-3-carboxamide

To a solution of N-[5-[5-bromo-2-(difluoromethoxy)phenyl]-1-[[2-(trimethylsilyl)ethoxy]methyl]-1H-pyrazol-4-yl]pyrazolo[1,5-a]pyrimidine-3-carboxamide (200 mg, 0.345 mmol) in toluene (16 mL) was added Pd₂(dba)₃.CHCl₃ (40.1 mg, 0.0387 mmol), Xantphos (36.0 mg, 0.0622 mmol), potassium carbonate (143 mg, 1.04 mmol) and oxetane-3-thiol (156 mg, 1.73 mmol) under nitrogen atmosphere. The resulting solution was stirred overnight at 100°C in an oil bath. The resulting mixture was concentrated under vacuum. The residue was applied onto a silica gel column eluting with ethyl acetate/PE (70%EA) to give 120 mg (59%) of N-[5-[2-(difluoromethoxy)-5-(oxetan-3-ylsulfanyl)phenyl]-1-[[2-(trimethylsilyl)ethoxy]methyl]-1H-pyrazol-4-yl]pyrazolo[1,5-a]pyrimidine-3-carboxamide as yellow oil. LC/MS(Method G, ESI): [M+H]⁺ = 581.1, R_{T}= 0.99 min.

To a solution of N-[5-[2-(difluoromethoxy)-5-(oxetan-3-ylsulfanyl)phenyl]-1-[[2-(trimethylsilyl)ethoxy]methyl]-1H-pyrazol-4-yl]pyrazolo[1,5-a]pyrimidine-3-carboxamide (120 mg, 0.204 mmol) in dichloromethane (15 mL) was added trifluoroacetic acid (6.0 mL). The resulting solution was stirred overnight at room temperature. The resulting mixture was concentrated under vacuum. The crude product (120 mg) was purified by Prep-HPLC with the following conditions: Column, XBridge Prep Phenyl OBD Column, 19^{∗}150mm, 5um; mobile phase, Waters (0.05% NH₃H₂O) and ACN (20.0% ACN up to 50.0% in 12 min); Detector, UV 220 nm, 254 nm. This resulted in 18.7 mg (20%) of N-[3-[2-(difluoromethoxy)-5-(oxetan-3-ylsulfanyl)phenyl]-1H-pyrazol-4-yl]pyrazolo[1,5-a]pyrimidine-3-carboxamide as a white solid. LC/MS (Method A, ESI): [M+H]⁺ = 459.1, R_{T}= 1.59 min; ¹H NMR (300 MHz, CD₃OD-*d₄*): δ (ppm) 9.08 (dd, *J* = 6.9, 1.5 Hz, 1H), 8.66 - 8.63 (m, 2H), 8.28 (s, 1H), 7.50 - 7.36 (m, 3H), 7.19 (dd, *J* = 6.9, 4.2 Hz, 1H), 6.78 (t, *J* = 73.8 Hz, 1H), 5.15 - 5.01 (m, 2H), 4,62 - 4.57 (m, 3H).

### Example 179

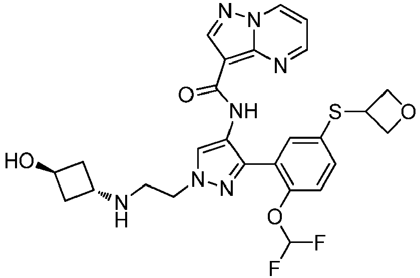

N-[3-[2-(difluoromethoxy)-5-(oxetan-3-ylsulfanyl)phenyl]-1-(2-[[trans-3-hydroxycyclobutyl]amino]ethyl)-1H-pyrazol-4-yl]pyrazolo[1,5-a]pyrimidine-3-carboxamide

To a solution of N-[3-[2-(difluoromethoxy)-5-(oxetan-3-ylsulfanyl)phenyl]-1H-pyrazol-4-yl]pyrazolo[1,5-a]pyrimidine-3-carboxamide (500 mg, 1.09 mmol) in tetrahydrofuran (14 mL) was added Cs₂CO₃ (1.42 g, 4.37 mmol) and 1,2-dibromoethane (4.083 g, 21.7 mmol). The resulting solution was stirred for 4.5 h at 80°C in an oil bath. The resulting mixture was concentrated under vacuum. The residue was dissolved in H₂O and dichloromethane. The organic layers combined and dried over anhydrous sodium sulfate and concentrated under vacuum to obtain 520 mg (84%) of N-[1-(2-bromoethyl)-3-[2-(difluoromethoxy)-5-(oxetan-3-ylsulfanyl)phenyl]-1H-pyrazol-4-yl]pyrazolo[1,5-a]pyrimidine-3-carboxamide as a light yellow solid. LC/MS (Method A, ESI): [M+H]⁺ = 565.0, R_{T}= 1.39 min.

To a solution of N-[1-(2-bromoethyl)-3-[2-(difluoromethoxy)-5-(oxetan-3-ylsulfanyl)phenyl]-1H-pyrazol-4-yl]pyrazolo[1,5-a]pyrimidine-3-carboxamide (520 mg, 0.920 mmol) in CH₃CN (18 mL) was added DIPEA (595 mg, 4.60 mmol) and trans-3-aminocyclobutan-1-ol (241 mg, 2.76 mmol). The resulting solution was stirred overnight at 80°C in an oil bath. The resulting mixture was concentrated under vacuum. The residue was applied onto a silica gel column eluting with DCM/MeOH (85/15). Then the crude product was further purified by Prep-HPLC with the following conditions: Column, XBridge Shield RP18 OBD Column, 19^{∗}150mm, 5um; mobile phase, ACN/H₂O (10 mM NH₄HCO₃) = 12% increasing to ACN/H₂O(10 mM NH₄HCO₃) = 38% within 8 min; Detector, UV 254 nm to give 356.7 mg (68%) of N-[3-[2-(difluoromethoxy)-5-(oxetan-3-ylsulfanyl)phenyl]-1-(2-[[trans-3-hydroxycyclobutyl]amino]ethyl)-1H-pyrazol-4-yl]pyrazolo[1,5-a]pyrimidine-3-carboxamide as a light yellow solid. LC/MS (Method O, ESI): [M+H]⁺ = 572.2, R_{T}= 0.98 min. ¹H NMR (300 MHz, CD₃OD-*d₄*): δ (ppm) 9.10 (d, *J* = 7.2 Hz, 1H), 8.66 - 8.63 (m, 2H), 8.32 (s, 1H), 7.51 (d, *J* = 2.1 Hz, 1H), 7.44 (dd, *J* = 8.6, 2.3 Hz, 1H), 7.36 (d, *J* = 8.4 Hz, 1H), 7.19 (dd, *J* = 7.2, 4.2 Hz, 1H), 6.80 (t, *J* = 73.8 Hz, 1H), 5.06 - 5.03 (m, 2 H), 4.67 - 4.52 (m, 3H), 4.42 - 4.32 (m, 3H), 3.55 - 3.45 (m, 1H), 3.05 (t, *J* = 6.2 Hz, 2H), 2.18 - 2.13 (m, 4H).

### Example 193

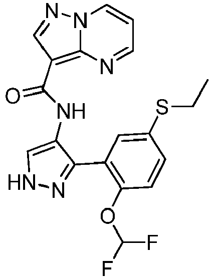

N-[3-[2-(difluoromethoxy)-5-(ethylsulfanyl)phenyl]-1H-pyrazol-4-yl]pyrazolo[1,5-a]pyrimidine-3-carboxamide

To a solution of N-[5-[5-bromo-2-(difluoromethoxy)phenyl]-1-[[2-(trimethylsilyl)ethoxy]methyl]-1H-pyrazol-4-yl]pyrazolo[1,5-a]pyrimidine-3-carboxamide (200 mg, 0.345 mmol) in toluene (5 mL) was added Pd₂(dba)₃.CHCl₃ (31.6 mg, 0.0305 mmol), XantPhos (39.9 mg, 0.0690 mmol) and (ethylsulfanyl)sodium (145 mg, 1.73 mmol) under nitrogen atmosphere. The resulting solution was stirred for 14 h at 90°C in an oil bath under N₂ atmosphere. The resulting mixture was concentrated under vacuum. The residue was applied onto a silica gel column eluting with ethyl acetate/petroleum ether (1/1) to obtain 101 mg (52%) of N-[5-[2-(difluoromethoxy)-5-(ethylsulfanyl)phenyl]-1-[[2-(trimethylsilyl)ethoxy]methyl]-1H-pyrazol-4-yl]pyrazolo[1,5-a]pyrimidine-3-carboxamide as a light yellow solid. LC/MS (Method A, ESI): [M+H]⁺ =561.3, R_{T}= 1.77 min.

To a solution of N-[5-[2-(difluoromethoxy)-5-(ethylsulfanyl)phenyl]-1-[[2-(trimethylsilyl)ethoxy]methyl]-1H-pyrazol-4-yl]pyrazolo[1,5-a]pyrimidine-3-carboxamide (101 mg, 0.178 mmol) in dichloromethane (5.0 mL) was added CF₃COOH (0.51 mL) at room temperature. The resulting solution was stirred for 2 h at room temperature. The resulting mixture was concentrated under vacuum. The residue was purified by Prep-HPLC with the following conditions: Column, XBridge Prep C₁₈ OBD, 19^{∗}150mm, 5um; mobile phase, CH₃CN/H₂O = 25% increasing to CH₃CN/H₂O = 44% within 8 min; Detector, UV 254 nm to give 33.3 mg (43%) of N-[3-[2-(difluoromethoxy)-5-(ethylsulfanyl)phenyl]-1H-pyrazol-4-yl]pyrazolo[1,5-a]pyrimidine-3-carboxamide as a light yellow solid. LC/MS (Method L, ESI): [M+H]⁺= 431.1, R_{T}= 3.15min; ¹H NMR (300 MHz, CD₃OD-*d₄*): δ (ppm) 9.09 (d, *J* = 6.9 Hz, 1H), 8.71 - 8.61 (m, 2H), 8.28 (s, 1H), 7.54 (s, 1H), 7.48 (d, *J* = 8.7 Hz, 1H), 7.32 (d, *J* = 8.4 Hz, 1H), 7.17 (dd, *J* = 6.6, 4.5 Hz, 1H), 6.76 (t, *J* = 73.7 Hz, 1H), 2.94 (q, *J* = 7.2 Hz, 2H), 1.27 (t, *J* = 7.2 Hz, 3H).

The following representative compounds of Formula (I) were prepared using procedures similar to those described in the Schemes and Examples herein.

| Ex. | Structure | Name |
|---|---|---|
| 1 | 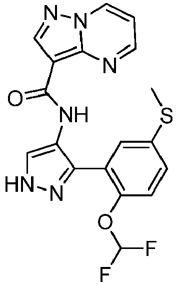 | N-[3-[2-(difluoromethoxy)-5-methylsulfanyl-phenyl]-1H-pyrazol-4-yl]pyrazolo[1,5-a]pyrimidine-3-carboxamide |
| | | or |
| | | N-(3-(2-(difluoromethoxy)-5-(methylthio)phenyl)-1H-pyrazol-4-yl)pyrazolo[1,5-a]pyrimidine-3-carboxamide |
| 2 | 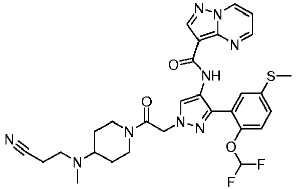 | N-[1-[2-[4-[2-cyanoethyl(methyl)amino]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 3 | 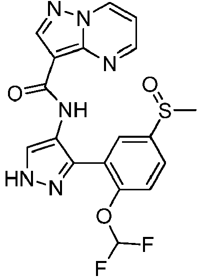 | N-[3-[2-(difluoromethoxy)-5-methylsulfinyl-phenyl]-1H-pyrazol-4-yl]pyrazolo[1,5-a]pyrimidine-3-carboxamide |
| 4 | 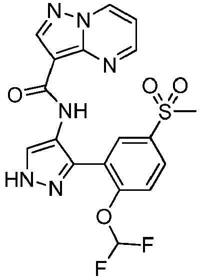 | N-[3-[2-(difluoromethoxy)-5-methylsulfonyl-phenyl]-1H-pyrazol-4-yl]pyrazolo[1,5-a]pyrimidine-3-carboxamide |
| 5 | 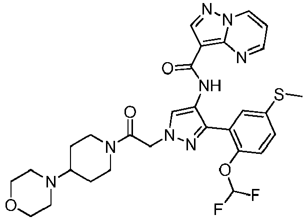 | N-[3-[2-(difluoromethoxy)-5-methylsulfanyl-phenyl]-1-[2-(4-morpholino-1-piperidyl)-2-oxoethyl]pyrazol-4-yl]pyrazolo[1,5-a]pyrimidine-3-carboxamide |
| | | Or |
| | | N-(3-(2-(difluoromethoxy)-5-(methylthio)phenyl) - 1-(2-(4-morpholinopiperidin-1-yl)-2-oxoethyl)-1H-pyrazol-4-yl)pyrazolo[1,5-a]pyrimidine-3-carboxamide |
| 6 | 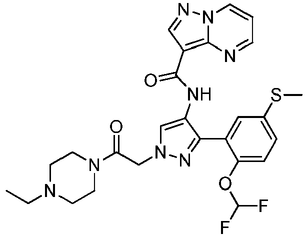 | N-[3-[2-(difluoromethoxy)-5-methylsulfanyl-phenyl]-1-[2-(4-ethylpiperazin-1-yl)-2-oxoethyl]pyrazol-4-yl]pyrazolo[1,5-a]pyrimidine-3-carboxamide |
| 7 | 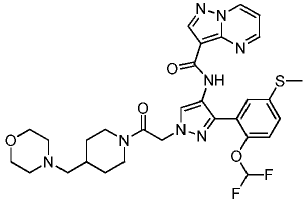 | N-[3-[2-(difluoromethoxy)-5-methylsulfanyl-phenyl]-1-[2-[4-(morpholinomethyl)-1-piperidyl]-2-oxo-ethyl]pyrazol-4-yl]pyrazolo[1,5-a]pyrimidine-3-carboxamide |
| 8 | 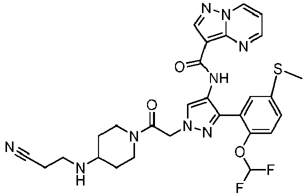 | N-[1-[2-[4-(2-cyanoethylamino)-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 9 | 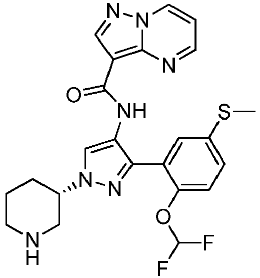 | N-[3-[2-(difluoromethoxy)-5-methylsulfanyl-phenyl]-1-[(3S)-3-piperidyl]pyrazol-4-yl]pyrazolo[1,5-a]pyrimidine-3-carboxamide |
| 10 | 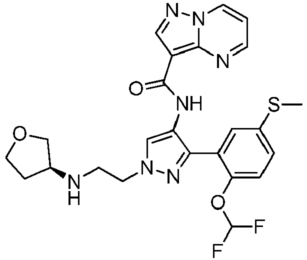 | N-[3-[2-(difluoromethoxy)-5-methylsulfanyl-phenyl]-1-[2-[[(3S)-tetrahydrofuran-3-yl]amino]ethyl]pyrazol-4-yl]pyrazolo[1,5-a]pyrimidine-3-carboxamide |
| 11 | 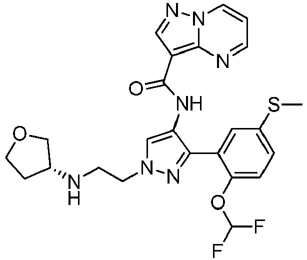 | N-[3-[2-(difluoromethoxy)-5-methylsulfanyl-phenyl]-1-[2-[[(3R)-tetrahydrofuran-3-yl]amino]ethyl]pyrazol-4-yl]pyrazolo[1,5-a]pyrimidine-3-carboxamide |
| | | Or |
| | | (R)-N-(3-(2-(difluoromethoxy)-5-(methylthio)phenyl)-1-(2-((tetrahydrofuran-3-yl)amino)ethyl)-1H-pyrazol-4-yl)pyrazolo[1,5-a]pyrimidine-3-carboxamide |
| 12 | 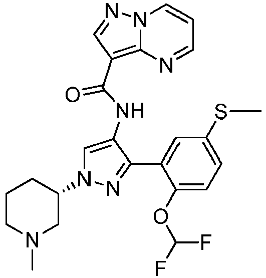 | N-[3-[2-(difluoromethoxy)-5-methylsulfanyl-phenyl]-1-[(3S)-1-methyl-3-piperidyl]pyrazol-4-yl]pyrazolo[1,5-a]pyrimidine-3-carboxamide |
| | | Or |
| | | (S)-N-(3-(2-(difluoromethoxy)-5-(methylthio)phenyl)-1-(1-methylpiperidin-3-yl)-1H-pyrazol-4-yl)pyrazolo[1,5-a]pyrimidine-3-carboxamide |
| 13 | 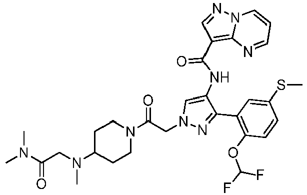 | N-[3-[2-(difluoromethoxy)-5-methylsulfanyl-phenyl]-1-[2-[4-[[2-(dimethylamino)-2-oxo-ethyl]-methyl-amino]-1-piperidyl]-2-oxo-ethyl]pyrazol-4-yl]pyrazolo[1,5-a]pyrimidine-3-carboxamide |
| 14 | 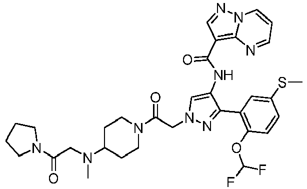 | N-[3-[2-(difluoromethoxy)-5-methylsulfanyl-phenyl]-1-[2-[4-[methyl-(2-oxo-2-pyrrolidin-1-yl-ethyl)amino]-1-piperidyl]-2-oxo-ethyl]pyrazol-4-yl]pyrazolo[1,5-a]pyrimidine-3-carboxamide |
| 15 | 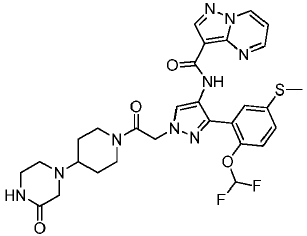 | N-[3-[2-(difluoromethoxy)-5-methylsulfanyl-phenyl]-1-[2-oxo-2-[4-(3-oxopiperazin-1-yl)-1-piperidyl]ethyl]pyrazol-4-yl]pyrazolo[1,5-a]pyrimidine-3-carboxamide |
| 16 | 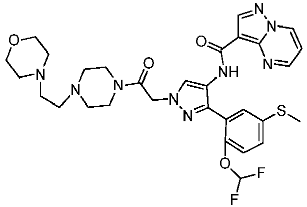 | N-[3-[2-(difluoromethoxy)-5-methylsulfanyl-phenyl]-1-[2-[4-(2-morpholinoethyl)piperazin-1-yl]-2-oxo-ethyl]pyrazol-4-yl]pyrazolo[1,5-a]pyrimidine-3-carboxamide |
| 17 | 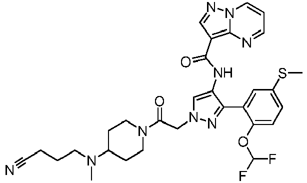 | N-[1-[2-[4-[3-cyanopropyl(methyl)amino]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 18 | 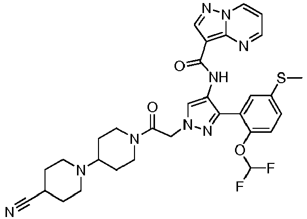 | N-[ 1-[2-[4-(4-cyano-1-piperidyl)-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 19 | 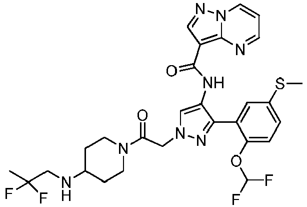 | N-[3-[2-(difluoromethoxy)-5-methylsulfanyl-phenyl]-1-[2-[4-(2,2-difluoropropylamino)-1-piperidyl]-2-oxo-ethyl]pyrazol-4-yl]pyrazolo[1,5-a]pyrimidine-3-carboxamide |
| 20 | 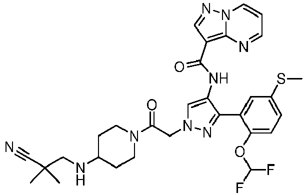 | N-[1-[2-[4-[(2-cyano-2-methyl-propyl)amino]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 21 | 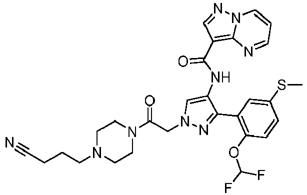 | N-[1-[2-[4-(3-cyanopropyl)piperazin-1-yl]-2-oxoethyl]-3-[2-(difluoromethoxy)-5-methylsulfanyl-phenyl]pyrazol-4-yl]pyrazolo[1,5-a]pyrimidine-3-carboxamide |
| 22 | 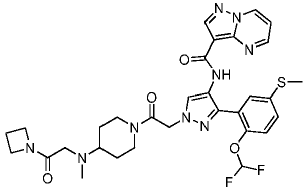 | N-[ 1-[2-[4-[[2-(azetidin-1-yl)-2-oxo-ethyl]-methyl-amino]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 23 | 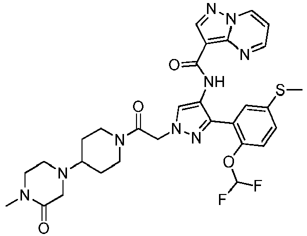 | N-[3-[2-(difluoromethoxy)-5-methylsulfanyl-phenyl]-1-[2-[4-(4-methyl-3-oxo-piperazin-1-yl)-1-piperidyl]-2-oxo-ethyl]pyrazol-4-yl]pyrazolo[1,5-a]pyrimidine-3-carboxamide |
| 24 | 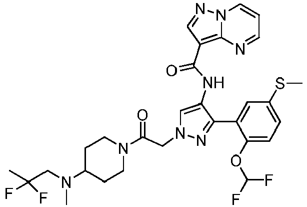 | N-[3-[2-(difluoromethoxy)-5-methylsulfanyl-phenyl]-1-[2-[4-[2,2-difluoropropyl(methyl)amino]-1-piperidyl]-2-oxoethyl]pyrazol-4-yl]pyrazolo[1,5-a]pyrimidine-3-carboxamide |
| 25 | 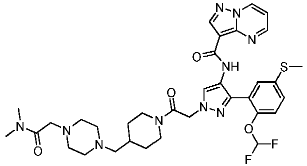 | N-[3-[2-(difluoromethoxy)-5-methylsulfanyl-phenyl]-1-[2-[4-[[4-[2-(dimethylamino)-2-oxoethyl]piperazin-1-yl]methyl]-1-piperidyl]-2-oxoethyl]pyrazol-4-yl]pyrazolo[1,5-a]pyrimidine-3-carboxamide |
| | | Or |
| | | N-(3-(2-(difluoromethoxy)-5-(methylthio)phenyl) -1-(2-(4-((4-(2-(dimethylamino)-2-oxoethyl)piperazin-1-yl)methyl)piperidin-1-yl)-2-oxoethyl)-1H-pyrazol-4-yl)pyrazolo[1,5-a]pyrimidine-3-carboxamide |
| 26 | 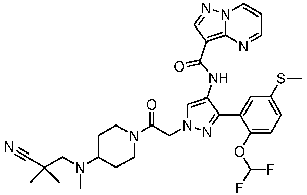 | N-[1-[2-[4-[(2-cyano-2-methyl-propyl)-methyl-amino]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 27 | 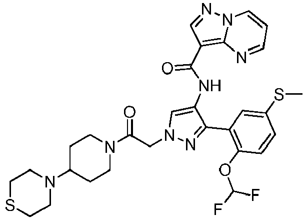 | N-[3-[2-(difluoromethoxy)-5-methylsulfanyl-phenyl]-1-[2-oxo-2-(4-thiomorpholino-1-piperidyl)ethyl]pyrazol-4-yl]pyrazolo[1,5-a]pyrimidine-3-carboxamide |
| 28 | 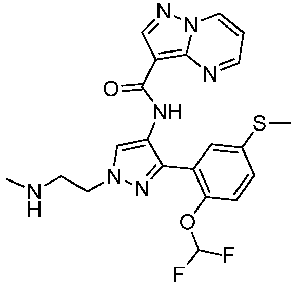 | N-[3-[2-(difluoromethoxy)-5-methylsulfanyl-phenyl]-1-[2-(methylamino)ethyl]pyrazol-4-yl]pyrazolo[1,5-a]pyrimidine-3-carboxamide |
| 29 | 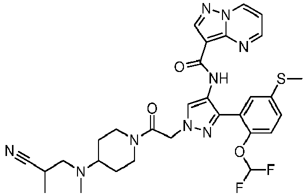 | N-[1-[2-[4-[2-cyanopropyl(methyl)amino]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 30 | 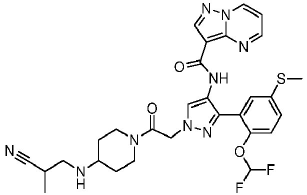 | N-[1-[2-[4-(2-cyanopropylamino)-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 31 | 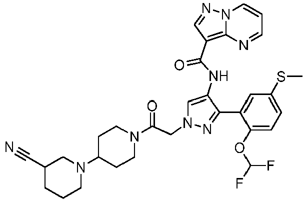 | N-[ 1-[2-[4-(3-cyano-1-piperidyl)-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 32 | 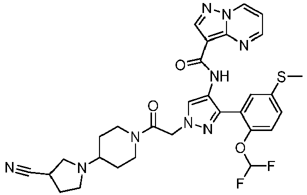 | N-[1-[2-[4-(3-cyanopyrrolidin-1-yl)-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 33 | 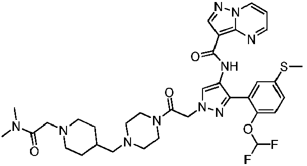 | N-[3-[2-(difluoromethoxy)-5-methylsulfanyl-phenyl]-1-[2-[4-[[1-[2-(dimethylamino)-2-oxoethyl]-4-piperidyl]methyl]piperazin-1-yl]-2-oxoethyl]pyrazol-4-yl]pyrazolo[1,5-a]pyrimidine-3-carboxamide |
| 34 | 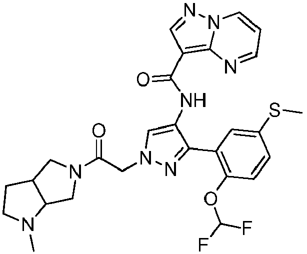 | N-[3-[2-(difluoromethoxy)-5-methylsulfanyl-phenyl]-1-[2-(1-methyl-2,3,3a,4,6,6a-hexahydropyrrolo[2,3-c]pyrrol-5-yl)-2-oxoethyl]pyrazol-4-yl]pyrazolo[1,5-a]pyrimidine-3-carboxamide |
| 35 | 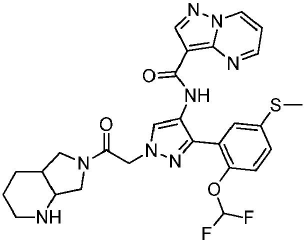 | N-[1-[2-(1,2,3,4,4a,5,7,7a-octahydropyrrolo[3,4-b]pyridin-6-yl)-2-oxo-ethyl]-3-[2-(difluoromethoxy)-5-methylsulfanyl-phenyl]pyrazol-4-yl]pyrazolo[1,5-a]pyrimidine-3-carboxamide |
| 36 | 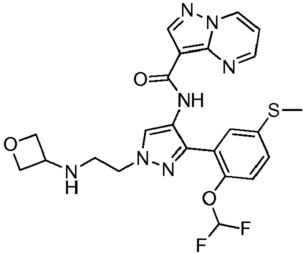 | N-[3-[2-(difluoromethoxy)-5-methylsulfanyl-phenyl]-1-[2-(oxetan-3-ylamino)ethyl]pyrazol-4-yl]pyrazolo[1,5-a]pyrimidine-3-carboxamide |
| 37 | 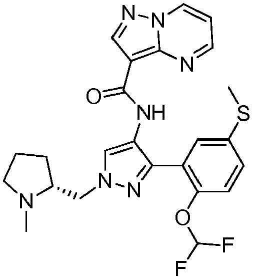 | N-[3-[2-(difluoromethoxy)-5-methylsulfanyl-phenyl]-1-[[(2R)-1-methylpyrrolidin-2-yl]methyl]pyrazol-4-yl]pyrazolo[1,5-a]pyrimidine-3-carboxamide |
| 38 | 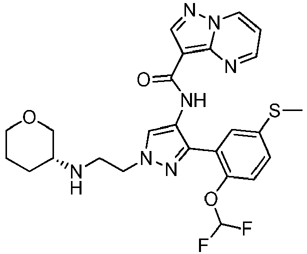 | N-[3-[2-(difluoromethoxy)-5-methylsulfanyl-phenyl]-1-[2-[[(3R)-tetrahydropyran-3-yl]amino]ethyl]pyrazol-4-yl]pyrazolo[1,5-a]pyrimidine-3-carboxamide |
| 39 | 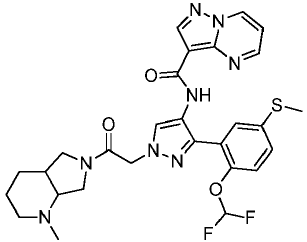 | N-[3-[2-(difluoromethoxy)-5-methylsulfanyl-phenyl]-1-[2-(1-methyl-3,4,4a,5,7,7a-hexahydro-2H-pyrrolo[3,4-b]pyridin-6-yl)-2-oxoethyl]pyrazol-4-yl]pyrazolo[1,5-a]pyrimidine-3-carboxamide |
| 40 | 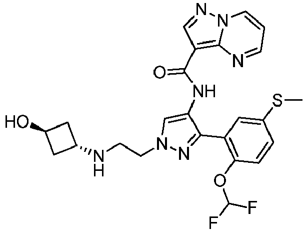 | N-[3-[2-(difluoromethoxy)-5-methylsulfanyl-phenyl]-1-[2-[(3-hydroxycyclobutyl)amino]ethyl]pyrazol-4-yl]pyrazolo[1,5-a]pyrimidine-3-carboxamide |
| 41 | 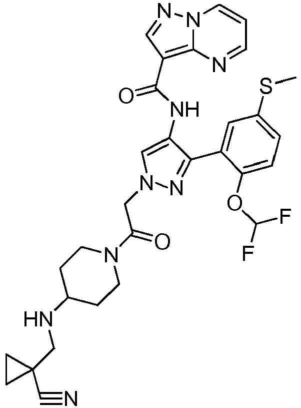 | N-[1-[2-[4-[(1-cyanocyclopropyl)methylamino]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 42 | 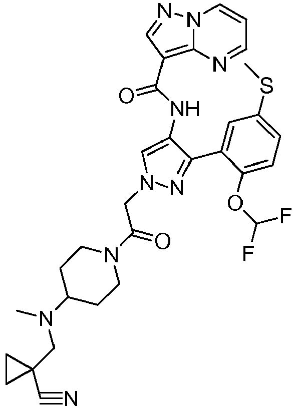 | N-[1-[2-[4-[(1-cyanocyclopropyl)methyl-methyl-amino]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 43 | 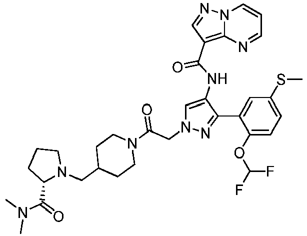 | N-[3-[2-(difluoromethoxy)-5-methylsulfanyl-phenyl]-1-[2-[4-[[(2S)-2-(dimethylcarbamoyl)pyrrolidin-1-yl]methyl]-1-piperidyl]-2-oxo-ethyl]pyrazol-4-yl]pyrazolo[1,5-a]pyrimidine-3-carboxamide |
| 44 | 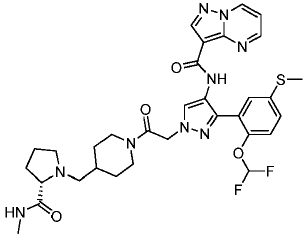 | N-[3-[2-(difluoromethoxy)-5-methylsulfanyl-phenyl]-1-[2-[4-[[(2S)-2-(methylcarbamoyl)pyrrolidin-1-yl] methyl]-1-piperidyl]-2-oxo-ethyl]pyrazol-4-yl]pyrazolo[1,5-a]pyrimidine-3-carboxamide |
| 45 | 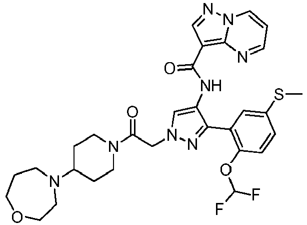 | N-[3-[2-(difluoromethoxy)-5-methylsulfanyl-phenyl]-1-[2-[4-(1,4-oxazepan-4-yl)-1-piperidyl]-2-oxo-ethyl]pyrazol-4-yl]pyrazolo[1,5-a]pyrimidine-3-carboxamide |
| 46 | 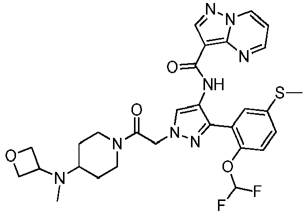 | N-[3-[2-(difluoromethoxy)-5-methylsulfanyl-phenyl]-1-[2-[4-[methyl(oxetan-3-yl)amino]-1-piperidyl]-2-oxo-ethyl]pyrazol-4-yl]pyrazolo[1,5-a]pyrimidine-3-carboxamide |
| | | Or |
| | | N-(3-(2-(difluoromethoxy)-5-(methylthio)phenyl)-1-(2-(4-(methyl(oxetan-3-yl)amino)piperidin-1-yl)-2-oxoethyl)-1H-pyrazol-4-yl)pyrazolo[1,5-a]pyrimidine-3-carboxamide |
| 47 | 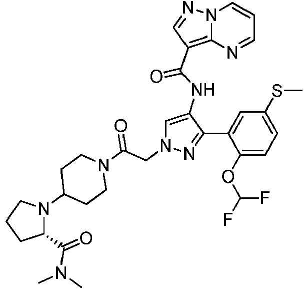 | N-[3-[2-(difluoromethoxy)-5-methylsulfanyl-phenyl]-1-[2-[4-[(2S)-2-(dimethylcarbamoyl)pyrrolidin-1-yl]-1-piperidyl]-2-oxo-ethyl]pyrazol-4-yl]pyrazolo[1,5-a]pyrimidine-3-carboxamide |
| 48 | 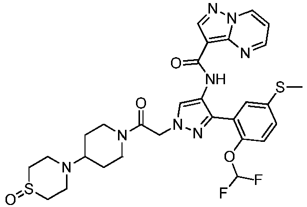 | N-[3-[2-(difluoromethoxy)-5-methylsulfanyl-phenyl]-1-[2-oxo-2-[4-(1-oxo-1,4-thiazinan-4-yl)-1-piperidyl]ethyl]pyrazol-4-yl]pyrazolo[1,5-a]pyrimidine-3-carboxamide |
| 49 | 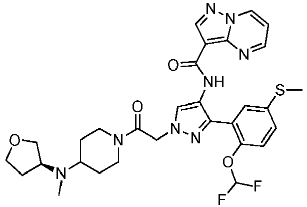 | N-[3-[2-(difluoromethoxy)-5-methylsulfanyl-phenyl]-1-[2-[4-[methyl-[(3S)-tetrahydrofuran-3-yl]amino]-1-piperidyl]-2-oxo-ethyl]pyrazol-4-yl]pyrazolo[1,5-a]pyrimidine-3-carboxamide |
| 50 | 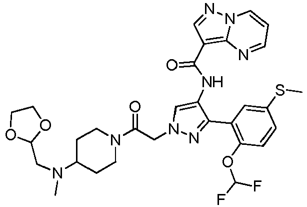 | N-[3-[2-(difluoromethoxy)-5-methylsulfanyl-phenyl]-1-[2-[4-[1,3-dioxolan-2-ylmethyl(methyl)amino]-1-piperidyl]-2-oxoethyl]pyrazol-4-yl]pyrazolo[1,5-a]pyrimidine-3-carboxamide |
| 51 | 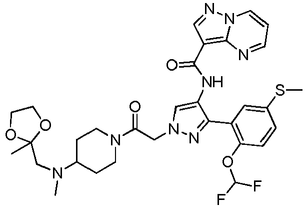 | N-[3-[2-(difluoromethoxy)-5-methylsulfanyl-phenyl]-1-[2-[4-[methyl-[(2-methyl-1,3-dioxolan-2-yl)methyl]amino]-1-piperidyl]-2-oxo-ethyl]pyrazol-4-yl]pyrazolo[1,5-a]pyrimidine-3-carboxamide |
| 52 | 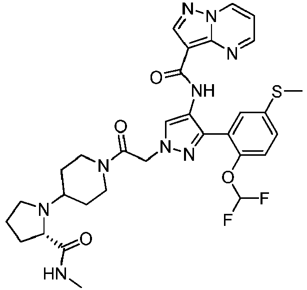 | N-[3-[2-(difluoromethoxy)-5-methylsulfanyl-phenyl]-1-[2-[4-[(2S)-2-(methylcarbamoyl)pyrrolidin-1-yl]-1-piperidyl]-2-oxo-ethyl]pyrazol-4-yl]pyrazolo[1,5-a]pyrimidine-3-carboxamide |
| 53 | 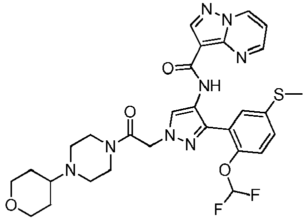 | N-[3-[2-(difluoromethoxy)-5-methylsulfanyl-phenyl]-1-[2-oxo-2-(4-tetrahydropyran-4-ylpiperazin-1 -yl)ethyl]pyrazol-4-yl]pyrazolo[1,5-a]pyrimidine-3-carboxamide |
| 54 | 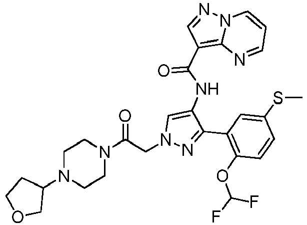 | N-[3-[2-(difluoromethoxy)-5-methylsulfanyl-phenyl]-1-[2-oxo-2-(4-tetrahydrofuran-3-ylpiperazin-1-yl)ethyl]pyrazol-4-yl]pyrazolo[1,5-a]pyrimidine-3-carboxamide |
| 55 | 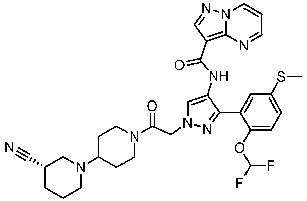 | N-[1-[2-[4-[(3S)-3-cyano-1-piperidyl]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| | | Or |
| | | N-[1-(2-[4-[(3S)-3-cyanopiperidin-1-yl]piperidin-1-yl] -2-oxoethyl)-3 - [2- (difluoromethoxy)-5-(methylsulfanyl)phenyl]-1H-pyrazol-4-yl]pyrazolo[1,5-a]pyrimidine-3-carboxamide |
| 56 | 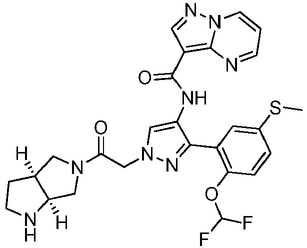 | N-[1-[2-[(3aS,6aS)-2,3,3a,4,6,6a-hexahydro-1H-pyrrolo[2,3-c]pyrrol-5-yl]-2-oxo-ethyl]-3-[2-(difluoromethoxy)-5-methylsulfanyl-phenyl]pyrazol-4-yl]pyrazolo[1,5-a]pyrimidine-3-carboxamide |
| 57 | 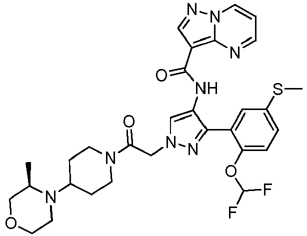 | N-[3-[2-(difluoromethoxy)-5-methylsulfanyl-phenyl]-1-[2-[4-[(3R)-3-methylmorpholin-4-yl]-1-piperidyl]-2-oxo-ethyl]pyrazol-4-yl]pyrazolo[1,5-a]pyrimidine-3-carboxamide |
| 58 | 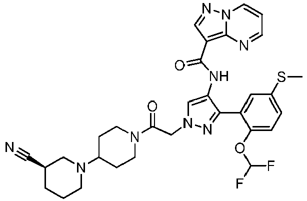 | N-[ 1-[2-[4-[(3R)-3-cyano-1-piperidyl]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| | | Or |
| | | N-[1-(2-[4-[(3R)-3-cyanopiperidin-1-yl]piperidin-1-yl]-2-oxoethyl)-3-[2-(difluoromethoxy)-5-(methylsulfanyl)phenyl]-1H-pyrazol-4-yl]pyrazolo[1,5-a]pyrimidine-3-carboxamide |
| 59 | 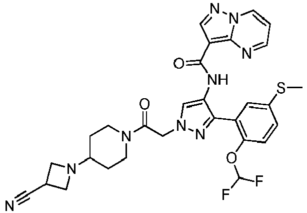 | N-[1-[2-[4-(3-cyanoazetidin-1-yl)-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 60 | 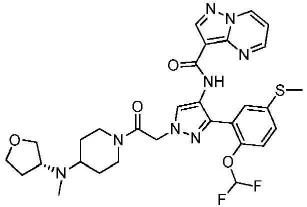 | N-[3-[2-(difluoromethoxy)-5-methylsulfanyl-phenyl]-1-[2-[4-[methyl-[(3R)-tetrahydrofuran-3-yl]amino]-1-piperidyl]-2-oxo-ethyl]pyrazol-4-yl]pyrazolo[1,5-a]pyrimidine-3-carboxamide |
| 61 | 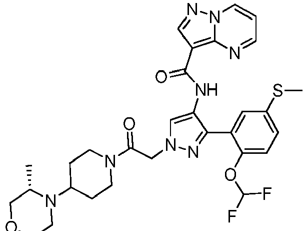 | N-[3-[2-(difluoromethoxy)-5-methylsulfanyl-phenyl]-1-[2-[4-[(3S)-3-methylmorpholin-4-yl]-1-piperidyl]-2-oxo-ethyl]pyrazol-4-yl]pyrazolo[1,5-a]pyrimidine-3-carboxamide |
| 62 | 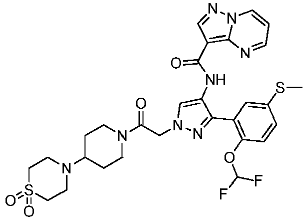 | N-[3-[2-(difluoromethoxy)-5-methylsulfanyl-phenyl]-1-[2-[ 4-(1,1-dioxo-1,4-thiazinan-4-yl)-1-piperidyl]-2-oxo-ethyl]pyrazol-4-yl]pyrazolo[1,5-a]pyrimidine-3-carboxamide |
| 63 | 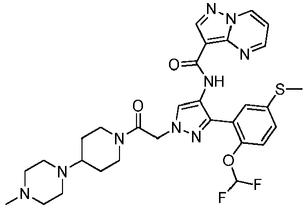 | N-[3-[2-(difluoromethoxy)-5-methylsulfanyl-phenyl]-1-[2-[4-(4-methylpiperazin-1-yl)-1-piperidyl]-2-oxo-ethyl]pyrazol-4-yl]pyrazolo[1,5-a]pyrimidine-3-carboxamide |
| | | Or |
| | | N-[3-[2-(difluoromethoxy)-5-(methylsulfanyl)phenyl]-1-[2-[4-(4-methylpiperazin-1-yl)piperidin-1-yl]-2-oxoethyl]-1H-pyrazol-4-yl]pyrazolo[1,5-a]pyrimidine-3-carboxamide |
| 64 | 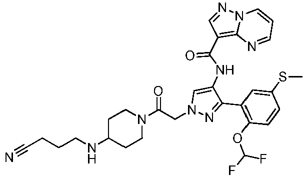 | N-[1-[2-[4-(3-cyanopropylamino)-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 65 | 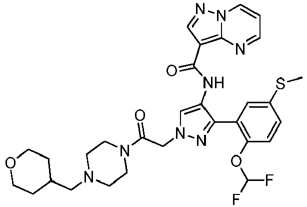 | N-[3-[2-(difluoromethoxy)-5-methylsulfanyl-phenyl]-1-[2-oxo-2-[4-(tetrahydropyran-4-ylmethyl)piperazin-1-yl]ethyl]pyrazol-4-yl]pyrazolo[1,5-a]pyrimidine-3-carboxamide |
| 66 | 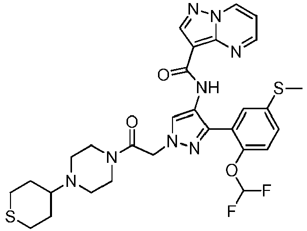 | N-[3-[2-(difluoromethoxy)-5-methylsulfanyl-phenyl]-1-[2-oxo-2-(4-tetrahydrothiopyran-4-ylpiperazin-1 -yl)ethyl]pyrazol-4-yl]pyrazolo[1,5-a]pyrimidine-3-carboxamide |
| 67 | 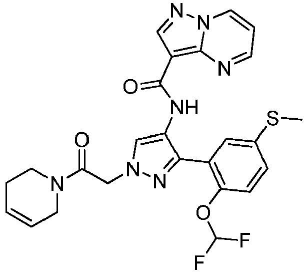 | N-[3-[2-(difluoromethoxy)-5-methylsulfanyl-phenyl]-1-[2-(3,6-dihydro-2H-pyridin-1-yl)-2-oxoethyl]pyrazol-4-yl]pyrazolo[1,5-a]pyrimidine-3-carboxamide |
| 68 | 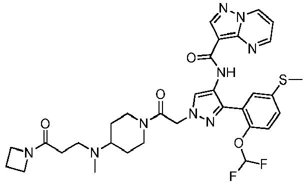 | N-[1-[2-[4-[[3-(azetidin-1-yl)-3-oxo-propyl]-methyl-amino]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 69 | 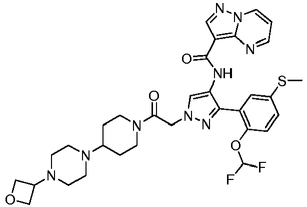 | N-[3-[2-(difluoromethoxy)-5-methylsulfanyl-phenyl]-1-[2-[4-[4-(oxetan-3-yl)piperazin-1-yl]-1-piperidyl]-2-oxo-ethyl]pyrazol-4-yl]pyrazolo[1,5-a]pyrimidine-3-carboxamide |
| 70 | 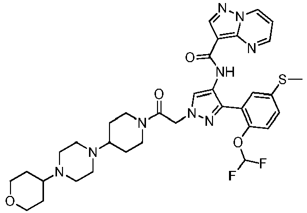 | N-[3-[2-(difluoromethoxy)-5-methylsulfanyl-phenyl]-1-[2-oxo-2-[4-(4-tetrahydropyran-4-ylpiperazin-1-yl)-1-piperidyl]ethyl]pyrazol-4-yl]pyrazolo[1,5-a]pyrimidine-3-carboxamide |
| 71 | 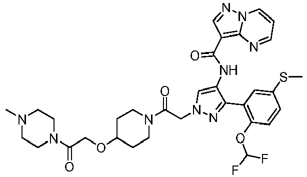 | N-[3-[2-(difluoromethoxy)-5-methylsulfanyl-phenyl]-1-[2-[4-[2-(4-methylpiperazin-1-yl)-2-oxo-ethoxy]-1-piperidyl]-2-oxo-ethyl]pyrazol-4-yl]pyrazolo[1,5-a]pyrimidine-3-carboxamide |
| | | Or |
| | | N-(3-(2-(difluoromethoxy)-5-(methylthio)phenyl)-1-(2-(4-(2-(4-methylpiperazin-1-yl)-2-oxoethoxy)piperidin-1-yl)-2-oxoethyl)-1H-pyrazol-4-yl)pyrazolo[1,5-a]pyrimidine-3-carboxamide |
| 72 | 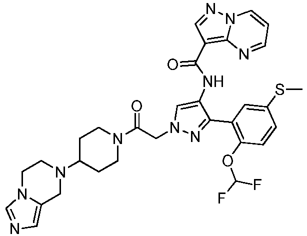 | N-[3-[2-(difluoromethoxy)-5-methylsulfanyl-phenyl]-1-[2-[4-(6,8-dihydro-5H-imidazo[1,5-a]pyrazin-7-yl)-1-piperidyl]-2-oxo-ethyl]pyrazol-4-yl]pyrazolo[1,5-a]pyrimidine-3-carboxamide |
| 73 | 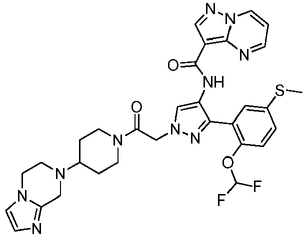 | N-[3-[2-(difluoromethoxy)-5-methylsulfanyl-phenyl]-1-[2-[4-(6,8-dihydro-5H-imidazo[1,2-a]pyrazin-7-yl)-1-piperidyl]-2-oxo-ethyl]pyrazol-4-yl]pyrazolo[1,5-a]pyrimidine-3-carboxamide |
| 74 | 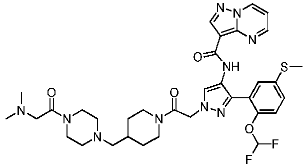 | N-[3-[2-(difluoromethoxy)-5-methylsulfanyl-phenyl]-1-[2-[4-[[4-[2-(dimethylamino)acetyl]piperazin-1-yl]methyl]-1-piperidyl]-2-oxo-ethyl]pyrazol-4-yl]pyrazolo[1,5-a]pyrimidine-3-carboxamide |
| 75 | 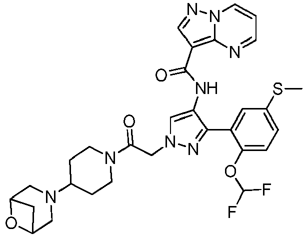 | N-[3-[2-(difluoromethoxy)-5-methylsulfanyl-phenyl]-1-[2-[4-(6-oxa-3-azabicyclo[3.1.1]heptan-3-yl)-1-piperidyl]-2-oxo-ethyl]pyrazol-4-yl]pyrazolo[1,5-a]pyrimidine-3-carboxamide |
| 76 | 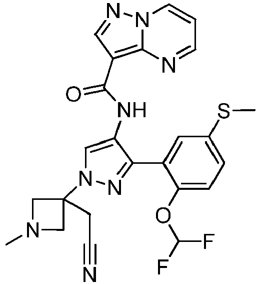 | N-[1-[3-(cyanomethyl)-1-methyl-azetidin-3-yl]-3-[2-(difluoromethoxy)-5-methylsulfanyl-phenyl]pyrazol-4-yl]pyrazolo[1,5-a]pyrimidine-3-carboxamide |
| | | Or |
| | | N-(1-(3-(cyanomethyl)-1-methylazetidin-3-yl)-3-(2-(difluoromethoxy)-5-(methylthio)phenyl)-1H-pyrazol-4-yl)pyrazolo[1,5-a]pyrimidine-3-carboxamide |
| 77 | 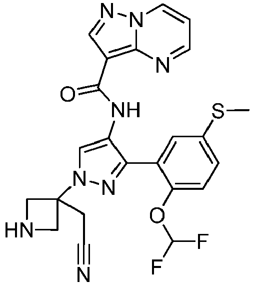 | N-[1-[3-(cyanomethyl)azetidin-3-yl]-3-[2-(difluoromethoxy)-5-methylsulfanyl-phenyl]pyrazol-4-yl]pyrazolo[1,5-a]pyrimidine-3-carboxamide |
| | | Or |
| | | N-( 1-(3-(cyanomethyl)azetidin-3-yl)-3-(2-(difluoromethoxy)-5-(methylthio)phenyl)-1H-pyrazol-4-yl)pyrazolo[1,5-a]pyrimidine-3-carboxamide |
| 78 | 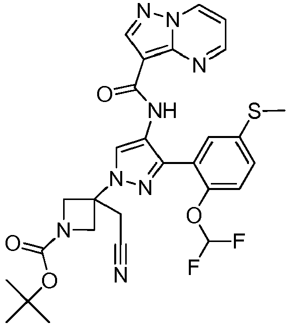 | tert-butyl 3-(cyanomethyl)-3-[3-[2-(difluoromethoxy)-5-methylsulfanyl-phenyl]-4-(pyrazolo[1,5-a]pyrimidine-3-carbonylamino)pyrazol-1-yl]azetidine-1-carboxylate |
| 79 | 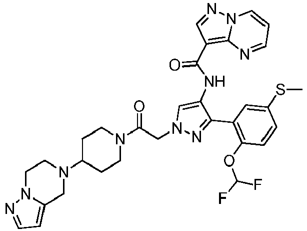 | N-[3-[2-(difluoromethoxy)-5-methylsulfanyl-phenyl]-1-[2-[4-(6,7-dihydro-4H-pyrazolo[1,5-a]pyrazin-5-yl)-1-piperidyl]-2-oxo-ethyl]pyrazol-4-yl]pyrazolo[1,5-a]pyrimidine-3-carboxamide |
| 80 | 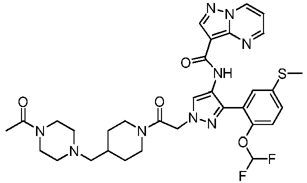 | N-[1-[2-[4-[(4-acetylpiperazin-1-yl)methyl]-1-piperidyl]-2-oxo-ethyl]-3-[2-(difluoromethoxy)-5-methylsulfanyl-phenyl]pyrazol-4-yl]pyrazolo[1,5-a]pyrimidine-3-carboxamide |
| 81 | 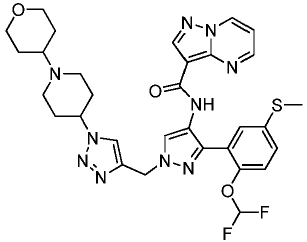 | N-[3-[2-(difluoromethoxy)-5-methylsulfanyl-phenyl]-1-[[1-(1-tetrahydropyran-4-yl-4-piperidyl)triazol-4-yl]methyl]pyrazol-4-yl]pyrazolo[1,5-a]pyrimidine-3-carboxamide |
| | | Or |
| | | N-(3-(2-(difluoromethoxy)-5-(methylthio)phenyl)-1-((1-(1-(tetrahydro-2H-pyran-4-yl)piperidin-4-yl)-1H-1,2,3-triazol-4-yl)methyl)-1H-pyrazol-4-yl)pyrazolo[1,5-a]pyrimidine-3-carboxamide |
| 82 | 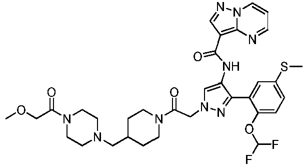 | N-[3-[2-(difluoromethoxy)-5-methylsulfanyl-phenyl]-1-[2-[4-[[4-(2-methoxyacetyl)piperazin-1-yl]methyl]-1-piperidyl]-2-oxo-ethyl]pyrazol-4-yl]pyrazolo[1,5-a]pyrimidine-3-carboxamide |
| 83 | 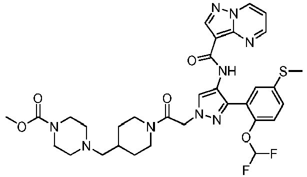 | methyl 4-[[1-[2-[3-[2-(difluoromethoxy)-5-methylsulfanyl-phenyl]-4-(pyrazolo[1,5-a]pyrimidine-3-carbonylamino)pyrazol-1-yl]acetyl]-4-piperidyl]methyl]piperazine-1-carboxylate |
| 84 | 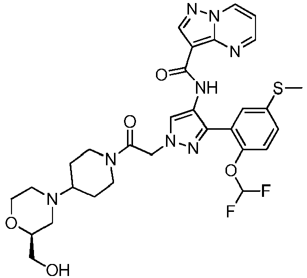 | N-[3-[2-(difluoromethoxy)-5-methylsulfanyl-phenyl]-1-[2-[4-[(2S)-2-(hydroxymethyl)morpholin-4-yl]-1-piperidyl]-2-oxo-ethyl]pyrazol-4-yl]pyrazolo[1,5-a]pyrimidine-3-carboxamide |
| 85 | 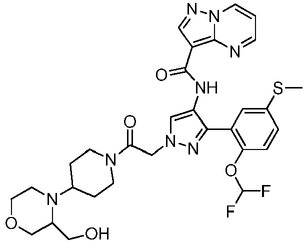 | N-[3-[2-(difluoromethoxy)-5-methylsulfanyl-phenyl]-1-[2-[4-[3-(hydroxymethyl)morpholin-4-yl]-1-piperidyl]-2-oxo-ethyl]pyrazol-4-yl]pyrazolo[1,5-a]pyrimidine-3-carboxamide |
| 86 | 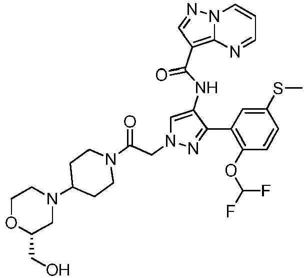 | N-[3-[2-(difluoromethoxy)-5-methylsulfanyl-phenyl]-1-[2-[4-[(2R)-2-(hydroxymethyl)morpholin-4-yl]-1-piperidyl]-2-oxo-ethyl]pyrazol-4-yl]pyrazolo[1,5-a]pyrimidine-3-carboxamide |
| 87 | 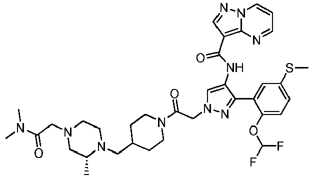 | N-[3-[2-(difluoromethoxy)-5-methylsulfanyl-phenyl]-1-[2-[4-[[(2R)-4-[2-(dimethylamino)-2-oxo-ethyl]-2-methyl-piperazin-1-yl]methyl]-1-piperidyl]-2-oxo-ethyl]pyrazol-4-yl]pyrazolo[1,5-a]pyrimidine-3-carboxamide |
| 88 | 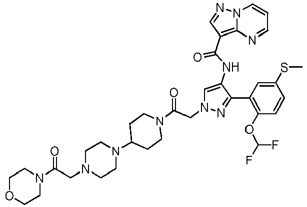 | N-[3-[2-(difluoromethoxy)-5-methylsulfanyl-phenyl]-1-[2-[4-[4-(2-morpholino-2-oxoethyl)piperazin-1-yl]-1-piperidyl]-2-oxoethyl]pyrazol-4-yl]pyrazolo[1,5-a]pyrimidine-3-carboxamide |
| 89 | 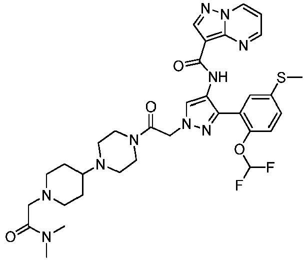 | N-[3-[2-(difluoromethoxy)-5-methylsulfanyl-phenyl]-1-[2-[4-[1-[2-(dimethylamino)-2-oxoethyl]-4-piperidyl]piperazin-1-yl]-2-oxoethyl]pyrazol-4-yl]pyrazolo[1,5-a]pyrimidine-3-carboxamide |
| 90 | 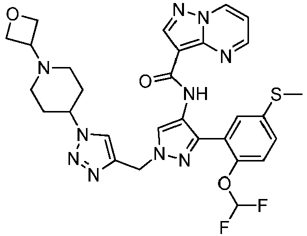 | N-[3-[2-(difluoromethoxy)-5-methylsulfanyl-phenyl]-1-[[1-[1-(oxetan-3-yl)-4-piperidyl]triazol-4-yl]methyl]pyrazol-4-yl]pyrazolo[1,5-a]pyrimidine-3-carboxamide |
| 91 | 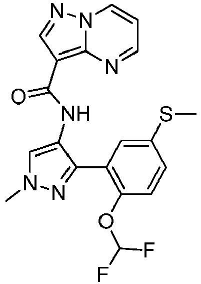 | N-[3-[2-(difluoromethoxy)-5-methylsulfanyl-phenyl]-1-methyl-pyrazol-4-yl]pyrazolo[1,5-a]pyrimidine-3-carboxamide |
| 92 | 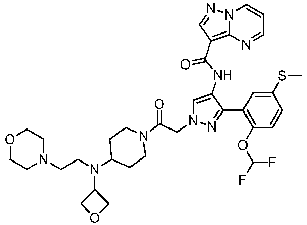 | N-[3-[2-(difluoromethoxy)-5-methylsulfanyl-phenyl]-1-[2-[4-[2-morpholinoethyl(oxetan-3-yl)amino]-1-piperidyl]-2-oxo-ethyl]pyrazol-4-yl]pyrazolo[1,5-a]pyrimidine-3-carboxamide |
| 93 | 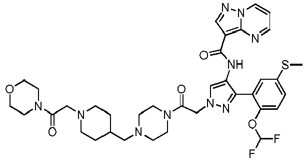 | N-[3-[2-(difluoromethoxy)-5-methylsulfanyl-phenyl]-1-[2-[4-[[1-(2-morpholino-2-oxo-ethyl)-4-piperidyl]methyl]piperazin-1-yl]-2-oxoethyl]pyrazol-4-yl]pyrazolo[1,5-a]pyrimidine-3-carboxamide |
| 94 | 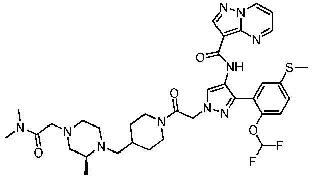 | N-[3-[2-(difluoromethoxy)-5-methylsulfanyl-phenyl]-1-[2-[4-[[(2S)-4-[2-(dimethylamino)-2-oxo-ethyl]-2-methyl-piperazin-1-yl]methyl]-1-piperidyl]-2-oxo-ethyl]pyrazol-4-yl]pyrazolo[1,5-a]pyrimidine-3-carboxamide |
| 95 | 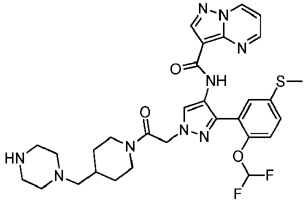 | N-[3-[2-(difluoromethoxy)-5-methylsulfanyl-phenyl]-1-[2-oxo-2-[4-(piperazin-1-ylmethyl)-1-piperidyl]ethyl]pyrazol-4-yl]pyrazolo[1,5-a]pyrimidine-3-carboxamide |
| 96 | 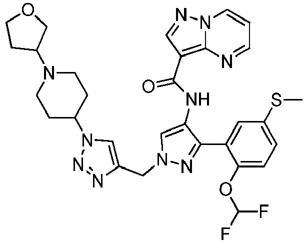 | N-[3-[2-(difluoromethoxy)-5-methylsulfanyl-phenyl]-1-[[1-(1-tetrahydrofuran-3-yl-4-piperidyl)triazol-4-yl]methyl]pyrazol-4-yl]pyrazolo[1,5-a]pyrimidine-3-carboxamide |
| 97 | 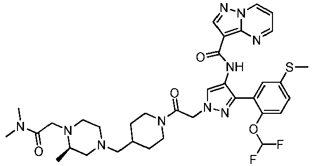 | N-[3-[2-(difluoromethoxy)-5-methylsulfanyl-phenyl]-1-[2-[4-[[(3R)-4-[2-(dimethylamino)-2-oxo-ethyl]-3-methyl-piperazin-1-yl]methyl]-1-piperidyl]-2-oxo-ethyl]pyrazol-4-yl]pyrazolo[1,5-a]pyrimidine-3-carboxamide |
| 98 | 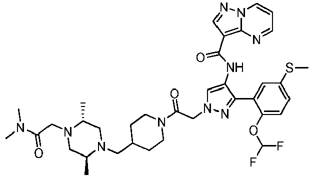 | N-[3-[2-(difluoromethoxy)-5-methylsulfanyl-phenyl]-1-[2-[4-[[(2S,5R)-4-[2-(dimethylamino)-2-oxo-ethyl]-2,5-dimethyl-piperazin-1-yl]methyl]-1-piperidyl]-2-oxo-ethyl]pyrazol-4-yl]pyrazolo[1,5-a]pyrimidine-3-carboxamide |
| 99 | 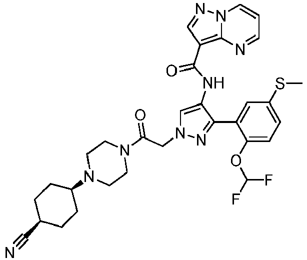 | N-[1-[2-[4-(4-cyanocyclohexyl)piperazin-1-yl]-2-oxo-ethyl]-3-[2-(difluoromethoxy)-5-methylsulfanyl-phenyl]pyrazol-4-yl]pyrazolo[1,5-a]pyrimidine-3-carboxamide |
| 100 | 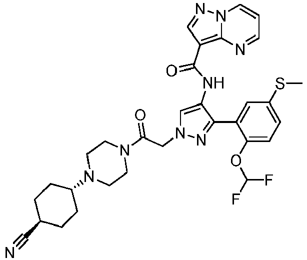 | N-[1-[2-[4-(4-cyanocyclohexyl)piperazin-1-yl]-2-oxo-ethyl]-3-[2-(difluoromethoxy)-5-methylsulfanyl-phenyl]pyrazol-4-yl]pyrazolo[1,5-a]pyrimidine-3-carboxamide |
| 101 | 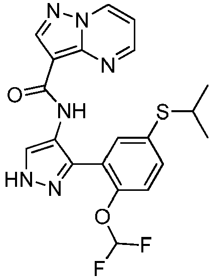 | N-[3-[2-(difluoromethoxy)-5-isopropylsulfanyl-phenyl]-1H-pyrazol-4-yl]pyrazolo[1,5-a]pyrimidine-3-carboxamide |
| | | Or |
| | | N-[3-[2-(difluoromethoxy)-5-(propan-2-ylsulfanyl)phenyl]-1H-pyrazol-4-yl]pyrazolo[1,5-a]pyrimidine-3-carboxamide |
| 102 | 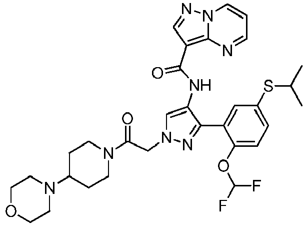 | N-[3-[2-(difluoromethoxy)-5-isopropylsulfanyl-phenyl]-1-[2-(4-morpholino-1-piperidyl)-2-oxoethyl]pyrazol-4-yl]pyrazolo[1,5-a]pyrimidine-3-carboxamide |
| 103 | 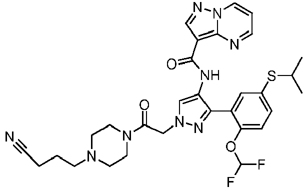 | N-[ 1-[2-[4-(3-cyanopropyl)piperazin-1-yl]-2-oxo-ethyl]-3-[2-(difluoromethoxy)-5-isopropylsulfanyl-phenyl]pyrazol-4-yl]pyrazolo[1,5-a]pyrimidine-3-carboxamide |
| 104 | 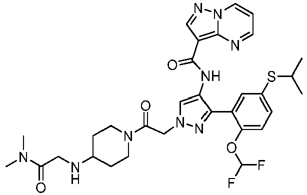 | N-[3-[2-(difluoromethoxy)-5-isopropylsulfanyl-phenyl]-1-[2-[4-[[2-(dimethylamino)-2-oxo-ethyl]amino]-1-piperidyl]-2-oxo-ethyl]pyrazol-4-yl]pyrazolo[1,5-a]pyrimidine-3-carboxamide |
| 105 | 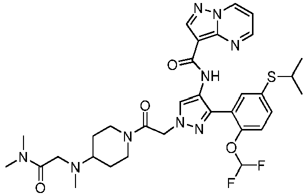 | N-[3-[2-(difluoromethoxy)-5-isopropylsulfanyl-phenyl]-1-[2-[4-[[2-(dimethylamino)-2-oxo-ethyl]-methyl-amino]-1-piperidyl]-2-oxo-ethyl]pyrazol-4-yl]pyrazolo[1,5-a]pyrimidine-3-carboxamide |
| 106 | 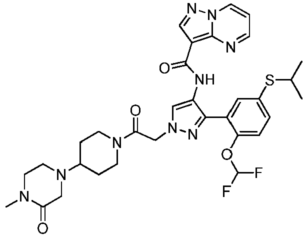 | N-[3-[2-(difluoromethoxy)-5-isopropylsulfanyl-phenyl]-1-[2-[4-(4-methyl-3-oxo-piperazin-1-yl)-1-piperidyl]-2-oxo-ethyl]pyrazol-4-yl]pyrazolo[1,5-a]pyrimidine-3-carboxamide |
| 107 | 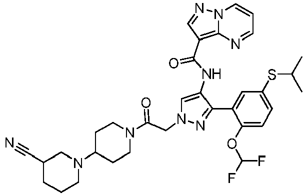 | N-[1-[2-[4-(3-cyano-1-piperidyl)-1-piperidyl]-2-oxo-ethyl] -3- [2-(difluoromethoxy)-5-isopropylsulfanyl-phenyl]pyrazol-4-yl]pyrazolo[1,5-a]pyrimidine-3-carboxamide |
| 108 | 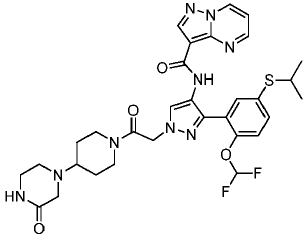 | N-[3-[2-(difluoromethoxy)-5-isopropylsulfanyl-phenyl]-1-[2-oxo-2-[4-(3-oxopiperazin-1-yl)-1-piperidyl]ethyl]pyrazol-4-yl]pyrazolo[1,5-a]pyrimidine-3-carboxamide |
| 109 | 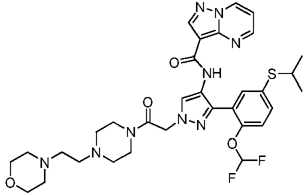 | N-[3-[2-(difluoromethoxy)-5-isopropylsulfanyl-phenyl]-1-[2-[4-(2-morpholinoethyl)piperazin-1-yl]-2-oxo-ethyl]pyrazol-4-yl]pyrazolo[1,5-a]pyrimidine-3-carboxamide |
| 110 | 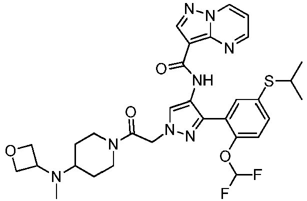 | N-[3-[2-(difluoromethoxy)-5-isopropylsulfanyl-phenyl]-1-[2-[4-[methyl(oxetan-3-yl)amino]-1-piperidyl]-2-oxo-ethyl]pyrazol-4-yl]pyrazolo[1,5-a]pyrimidine-3-carboxamide |
| 111 | 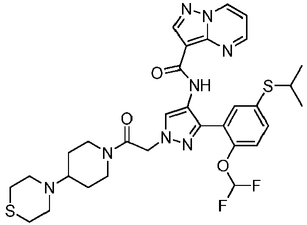 | N-[3-[2-(difluoromethoxy)-5-isopropylsulfanyl-phenyl]-1-[2-oxo-2-(4-thiomorpholino-1-piperidyl)ethyl]pyrazol-4-yl]pyrazolo[1,5-a]pyrimidine-3-carboxamide |
| 112 | 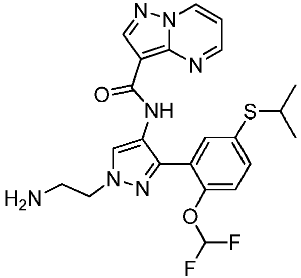 | N-[1-(2-aminoethyl)-3-[2-(difluoromethoxy)-5-isopropylsulfanyl-phenyl]pyrazol-4-yl]pyrazolo[1,5-a]pyrimidine-3-carboxamide |
| 113 | 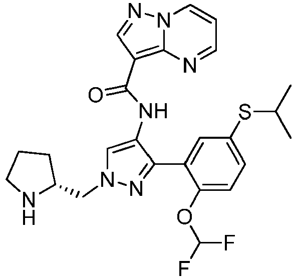 | N-[3-[2-(difluoromethoxy)-5-isopropylsulfanyl-phenyl]-1-[[(2R)-pyrrolidin-2-yl]methyl]pyrazol-4-yl]pyrazolo[1,5-a]pyrimidine-3-carboxamide |
| 114 | 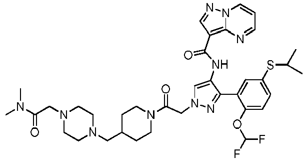 | N-[3-[2-(difluoromethoxy)-5-isopropylsulfanyl-phenyl]-1-[2-[4-[[4-[2-(dimethylamino)-2-oxo-ethyl]piperazin-1-yl]methyl]-1-piperidyl]-2-oxo-ethyl]pyrazol-4-yl]pyrazolo[1,5-a]pyrimidine-3-carboxamide |
| 115 | 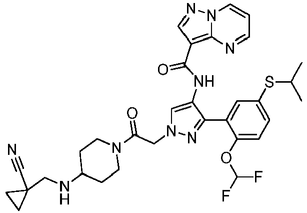 | N-[1-[2-[4-[(1-cyanocyclopropyl)methylamino]-1-piperidyl]-2-oxo-ethyl]-3-[2-(difluoromethoxy)-5-isopropylsulfanyl-phenyl]pyrazol-4-yl]pyrazolo[1,5-a]pyrimidine-3-carboxamide |
| 116 | 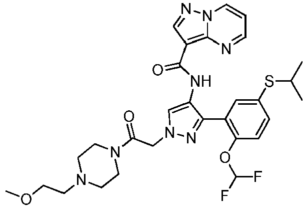 | N-[3-[2-(difluoromethoxy)-5-isopropylsulfanyl-phenyl]-1-[2-[4-(2-methoxyethyl)piperazin-1-yl]-2-oxo-ethyl]pyrazol-4-yl]pyrazolo[1,5-a]pyrimidine-3-carboxamide |
| 117 | 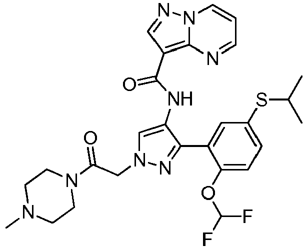 | N-[3-[2-(difluoromethoxy)-5-isopropylsulfanyl-phenyl]-1-[2-(4-methylpiperazin-1-yl)-2-oxo-ethyl]pyrazol-4-yl]pyrazolo[1,5-a]pyrimidine-3-carboxamide |
| 118 | 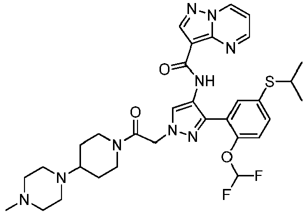 | N-[3-[2-(difluoromethoxy)-5-isopropylsulfanyl-phenyl]-1-[2-[4-(4-methylpiperazin-1-yl)-1-piperidyl]-2-oxo-ethyl]pyrazol-4-yl]pyrazolo[1,5-a]pyrimidine-3-carboxamide |
| 119 | 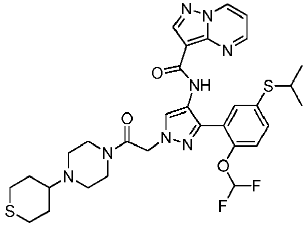 | N-[3-[2-(difluoromethoxy)-5-isopropylsulfanyl-phenyl]-1-[2-oxo-2-(4-tetrahydrothiopyran-4-ylpiperazin-1-yl)ethyl]pyrazol-4-yl]pyrazolo[1,5-a]pyrimidine-3-carboxamide |
| 120 | 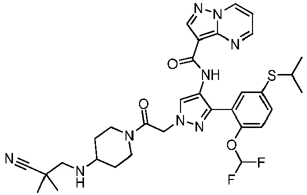 | N-[1-[2-[4-[(2-cyano-2-methyl-propyl)amino]-1-piperidyl]-2-oxo-ethyl]-3-[2-(difluoromethoxy)-5-isopropylsulfanyl-phenyl]pyrazol-4-yl]pyrazolo[1,5-a]pyrimidine-3-carboxamide |
| 121 | 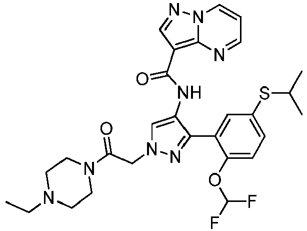 | N-[3-[2-(difluoromethoxy)-5-isopropylsulfanyl-phenyl]-1-[2-(4-ethylpiperazin-1-yl)-2-oxo-ethyl]pyrazol-4-yl]pyrazolo[1,5-a]pyrimidine-3-carboxamide |
| 122 | 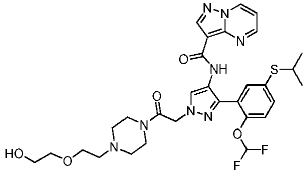 | N-[3-[2-(difluoromethoxy)-5-isopropylsulfanyl-phenyl]-1-[2-[4-[2-(2-hydroxyethoxy)ethyl]piperazin-1-yl]-2-oxo-ethyl]pyrazol-4-yl]pyrazolo[1,5-a]pyrimidine-3-carboxamide |
| 123 | 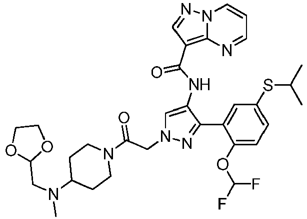 | N-[3-[2-(difluoromethoxy)-5-isopropylsulfanyl-phenyl]-1-[2-[4-[1,3-dioxolan-2-ylmethyl(methyl)amino]-1-piperidyl]-2-oxo-ethyl]pyrazol-4-yl]pyrazolo[1,5-a]pyrimidine-3-carboxamide |
| 124 | 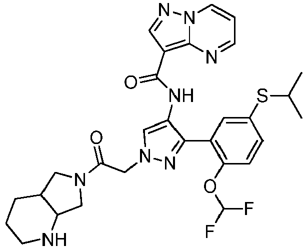 | N-[1-[2-(1,2,3,4,4a,5,7,7a-octahydropyrrolo[3,4-b]pyridin-6-yl)-2-oxo-ethyl]-3-[2-(difluoromethoxy)-5-isopropylsulfanyl-phenyl]pyrazol-4-yl]pyrazolo[1,5-a]pyrimidine-3-carboxamide |
| 125 | 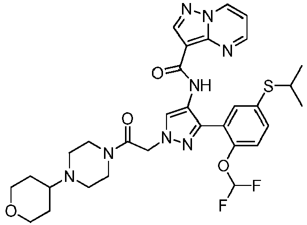 | N-[3-[2-(difluoromethoxy)-5-isopropylsulfanyl-phenyl]-1-[2-oxo-2-(4-tetrahydropyran-4-ylpiperazin-1 -yl)ethyl]pyrazol-4-yl]pyrazolo[1,5-a]pyrimidine-3-carboxamide |
| 126 | 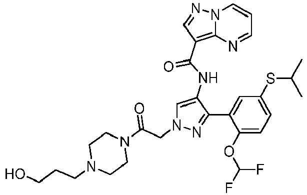 | N-[3-[2-(difluoromethoxy)-5-isopropylsulfanyl-phenyl]-1-[2-[4-(3-hydroxypropyl)piperazin-1-yl]-2-oxo-ethyl]pyrazol-4-yl]pyrazolo[1,5-a]pyrimidine-3-carboxamide |
| 127 | 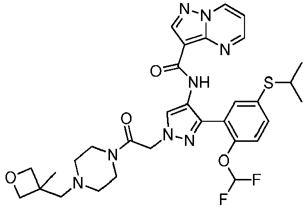 | N-[3-[2-(difluoromethoxy)-5-isopropylsulfanyl-phenyl]-1-[2-[4-[(3-methyloxetan-3-yl)methyl]piperazin-1-yl]-2-oxo-ethyl]pyrazol-4-yl]pyrazolo[1,5-a]pyrimidine-3-carboxamide |
| 128 | 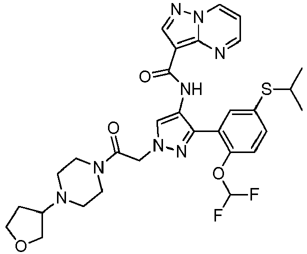 | N-[3-[2-(difluoromethoxy)-5-isopropylsulfanyl-phenyl]-1-[2-oxo-2-(4-tetrahydrofuran-3-ylpiperazin-1-yl)ethyl]pyrazol-4-yl]pyrazolo[1,5-a]pyrimidine-3-carboxamide |
| 129 | 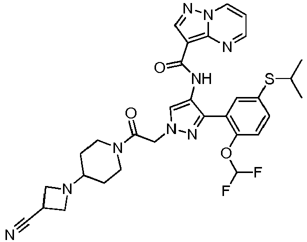 | N-[ 1-[2-[4-(3-cyanoazetidin-1-yl)-1-piperidyl]-2-oxo-ethyl]-3-[2-(difluoromethoxy)-5-isopropylsulfanyl-phenyl]pyrazol-4-yl]pyrazolo[1,5-a]pyrimidine-3-carboxamide |
| | | Or |
| | | N-(1-[2-[4-(3-cyanoazetidin-1-yl)piperidin-1-yl]-2-oxoethyl]-3-[2-(difluoromethoxy)-5-(propan-2-ylsulfanyl)phenyl]-1H-pyrazol-4-yl)pyrazolo[1,5-a]pyrimidine-3-carboxamide |
| 130 | 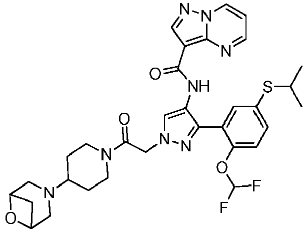 | N-[3-[2-(difluoromethoxy)-5-isopropylsulfanyl-phenyl]-1-[2-[4-(6-oxa-3-azabicyclo[3.1.1]heptan-3-yl)-1-piperidyl]-2-oxo-ethyl]pyrazol-4-yl]pyrazolo[1,5-a]pyrimidine-3-carboxamide |
| 131 | 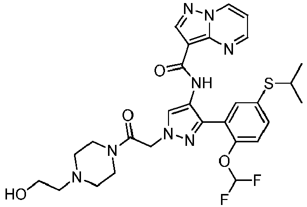 | N-[3-[2-(difluoromethoxy)-5-isopropylsulfanyl-phenyl]-1-[2-[4-(2-hydroxyethyl)piperazin-1-yl]-2-oxo-ethyl]pyrazol-4-yl]pyrazolo[1,5-a]pyrimidine-3-carboxamide |
| 132 | 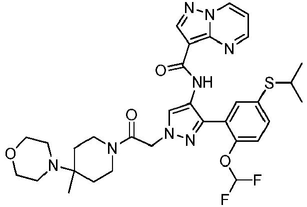 | N-[3-[2-(difluoromethoxy)-5-isopropylsulfanyl-phenyl]-1-[2-(4-methyl-4-morpholino-1-piperidyl)-2-oxo-ethyl]pyrazol-4-yl]pyrazolo[1,5-a]pyrimidine-3-carboxamide |
| 133 | 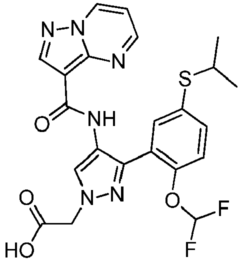 | 2-[3-[2-(difluoromethoxy)-5-isopropylsulfanyl-phenyl]-4-(pyrazolo[1,5-a]pyrimidine-3-carbonylamino)pyrazol-1-yl]acetic acid |
| 134 | 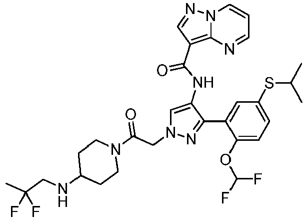 | N-[3-[2-(difluoromethoxy)-5-isopropylsulfanyl-phenyl]-1-[2-[4-(2,2-difluoropropylamino)-1-piperidyl]-2-oxo-ethyl]pyrazol-4-yl]pyrazolo[1,5-a]pyrimidine-3-carboxamide |
| 135 | 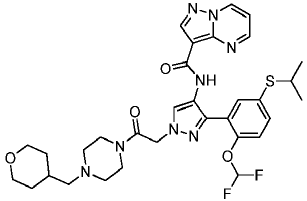 | N-[3-[2-(difluoromethoxy)-5-isopropylsulfanyl-phenyl]-1-[2-oxo-2-[4-(tetrahydropyran-4-ylmethyl)piperazin-1-yl]ethyl]pyrazol-4-yl]pyrazolo[1,5-a]pyrimidine-3-carboxamide |
| 136 | 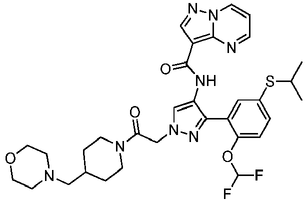 | N-[3-[2-(difluoromethoxy)-5-isopropylsulfanyl-phenyl]-1-[2-[4-(morpholinomethyl)-1-piperidyl]-2-oxo-ethyl]pyrazol-4-yl]pyrazolo[1,5-a]pyrimidine-3-carboxamide |
| 137 | 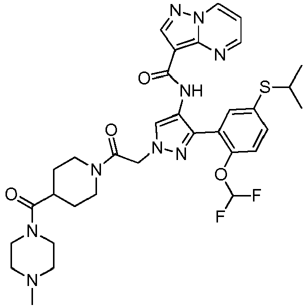 | N-[3-[2-(difluoromethoxy)-5-isopropylsulfanyl-phenyl]-1-[2-[4-(4-methylpiperazine-1-carbonyl)-1-piperidyl]-2-oxo-ethyl]pyrazol-4-yl]pyrazolo[1,5-a]pyrimidine-3-carboxamide |
| 138 | 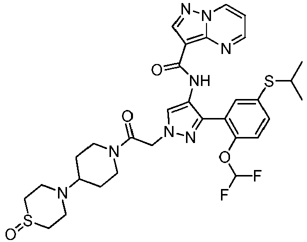 | N-[3-[2-(difluoromethoxy)-5-isopropylsulfanyl-phenyl]-1-[2-oxo-2-[4-(1-oxo-1,4-thiazinan-4-yl)-1-piperidyl]ethyl]pyrazol-4-yl]pyrazolo[1,5-a]pyrimidine-3-carboxamide |
| 139 | 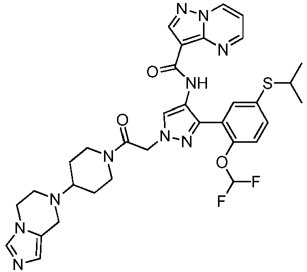 | N-[3-[2-(difluoromethoxy)-5-isopropylsulfanyl-phenyl]-1-[2-[4-(6,8-dihydro-5H-imidazo[1,5-a]pyrazin-7-yl)-1-piperidyl]-2-oxo-ethyl]pyrazol-4-yl]pyrazolo[1,5-a]pyrimidine-3-carboxamide |
| 140 | 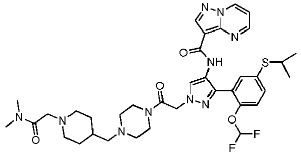 | N-[3-[2-(difluoromethoxy)-5-isopropylsulfanyl-phenyl]-1-[2-[4-[[1-[2-(dimethylamino)-2-oxo-ethyl]-4-piperidyl]methyl]piperazin-1-yl]-2-oxo-ethyl]pyrazol-4-yl]pyrazolo[1,5-a]pyrimidine-3-carboxamide |
| 141 | 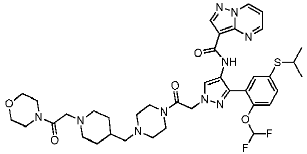 | N-[3-[2-(difluoromethoxy)-5-isopropylsulfanyl-phenyl]-1-[2-[4-[[1-(2-morpholino-2-oxo-ethyl)-4-piperidyl]methyl]piperazin-1-yl]-2-oxo-ethyl]pyrazol-4-yl]pyrazolo[1,5-a]pyrimidine-3-carboxamide |
| 142 | 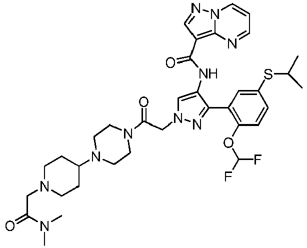 | N-[3-[2-(difluoromethoxy)-5-isopropylsulfanyl-phenyl]-1-[2-[4-[1-[2-(dimethylamino)-2-oxo-ethyl]-4-piperidyl]piperazin-1-yl]-2-oxo-ethyl]pyrazol-4-yl]pyrazolo[1,5-a]pyrimidine-3-carboxamide |
| 143 | 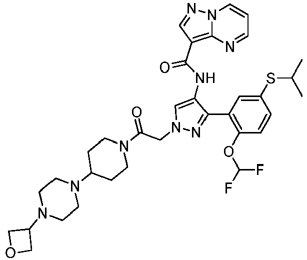 | N-[3-[2-(difluoromethoxy)-5-isopropylsulfanyl-phenyl]-1-[2-[4-[4-(oxetan-3-yl)piperazin-1-yl]-1-piperidyl]-2-oxo-ethyl]pyrazol-4-yl]pyrazolo[1,5-a]pyrimidine-3-carboxamide |
| 144 | 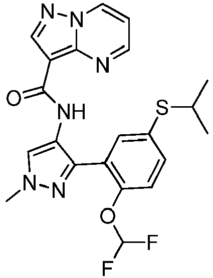 | N-[3-[2-(difluoromethoxy)-5-isopropylsulfanyl-phenyl]-1-methyl-pyrazol-4-yl]pyrazolo[1,5-a]pyrimidine-3-carboxamide |
| | | Or |
| | | N-[3-[2-(difluoromethoxy)-5-(propan-2-ylsulfanyl)phenyl]-1-methyl-1H-pyrazol-4-yl]pyrazolo[1,5-a]pyrimidine-3-carboxamide |
| 145 | 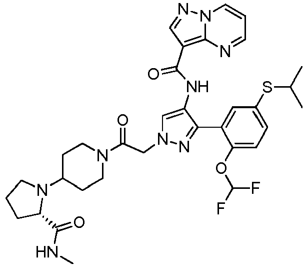 | N-[3-[2-(difluoromethoxy)-5-isopropylsulfanyl-phenyl]-1-[2-[4-[(2S)-2-(methylcarbamoyl)pyrrolidin-1-yl]-1-piperidyl]-2-oxo-ethyl]pyrazol-4-yl]pyrazolo[1,5-a]pyrimidine-3-carboxamide |
| 146 | 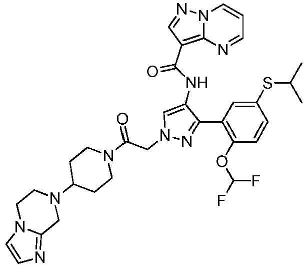 | N-[3-[2-(difluoromethoxy)-5-isopropylsulfanyl-phenyl]-1-[2-[4-(6,8-dihydro-5H-imidazo[1,2-a]pyrazin-7-yl)-1-piperidyl]-2-oxo-ethyl]pyrazol-4-yl]pyrazolo[1,5-a]pyrimidine-3-carboxamide |
| 147 | 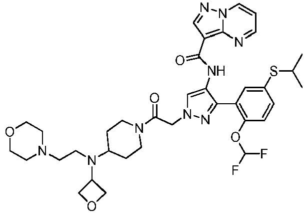 | N-[3-[2-(difluoromethoxy)-5-isopropylsulfanyl-phenyl]-1-[2-[4-[2-morpholinoethyl(oxetan-3-yl)amino]-1-piperidyl]-2-oxo-ethyl]pyrazol-4-yl]pyrazolo[1,5-a]pyrimidine-3-carboxamide |
| 148 | 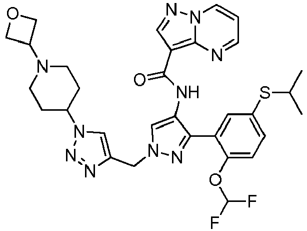 | N-[3-[2-(difluoromethoxy)-5-isopropylsulfanyl-phenyl]-1-[[1-[1-(oxetan-3-yl)-4-piperidyl]triazol-4-yl]methyl]pyrazol-4-yl]pyrazolo[1,5-a]pyrimidine-3-carboxamide |
| 149 | 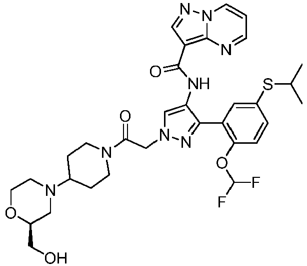 | N-[3-[2-(difluoromethoxy)-5-isopropylsulfanyl-phenyl]-1-[2-[4-[(2S)-2-(hydroxymethyl)morpholin-4-yl]-1-piperidyl]-2-oxo-ethyl]pyrazol-4-yl]pyrazolo[1,5-a]pyrimidine-3-carboxamide |
| 150 | 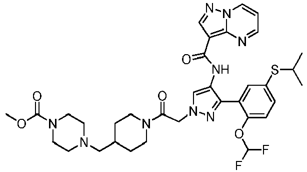 | methyl 4-[[1-[2-[3-[2-(difluoromethoxy)-5-isopropylsulfanyl-phenyl]-4-(pyrazolo[1,5-a]pyrimidine-3-carbonylamino)pyrazol-1-yl] acetyl]-4-piperidyl] methyl]piperazine-1-carboxylate |
| | | or |
| | | methyl 4-[[1-(2-[3-[2-(difluoromethoxy)-5-(propan-2-ylsulfanyl)phenyl]-4-[pyrazolo[1,5-a]pyrimidine-3-amido]-1H-pyrazol-1-yl]acetyl)piperidin-4-yl]methyl]piperazine-1-carboxylate |
| 151 | 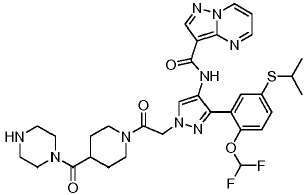 | N-[3-[2-(difluoromethoxy)-5-isopropylsulfanyl-phenyl]-1-[2-oxo-2-[4-(piperazine-1-carbonyl)-1-piperidyl]ethyl]pyrazol-4-yl]pyrazolo[1,5-a]pyrimidine-3-carboxamide |
| 152 | 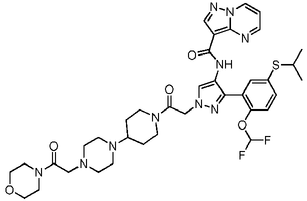 | N-[3-[2-(difluoromethoxy)-5-isopropylsulfanyl-phenyl]-1-[2-[4-[4-(2-morpholino-2-oxo-ethyl)piperazin-1-yl]-1-piperidyl]-2-oxo-ethyl]pyrazol-4-yl]pyrazolo[1,5-a]pyrimidine-3-carboxamide |
| 153 | 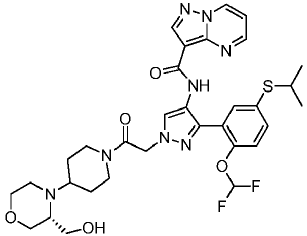 | N-[3-[2-(difluoromethoxy)-5-isopropylsulfanyl-phenyl]-1-[2-[4-[(3R)-3-(hydroxymethyl)morpholin-4-yl]-1-piperidyl]-2-oxo-ethyl]pyrazol-4-yl]pyrazolo[1,5-a]pyrimidine-3-carboxamide |
| 154 | 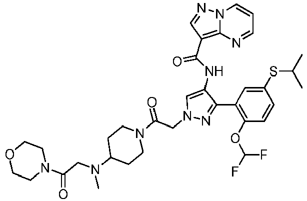 | N-[3-[2-(difluoromethoxy)-5-isopropylsulfanyl-phenyl]-1-[2-[4-[methyl-(2-morpholino-2-oxo-ethyl)amino]-1-piperidyl]-2-oxo-ethyl]pyrazol-4-yl]pyrazolo[1,5-a]pyrimidine-3-carboxamide |
| 155 | 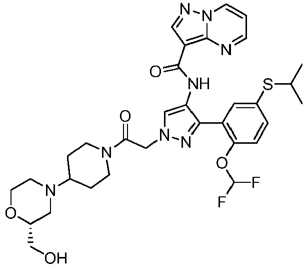 | N-[3-[2-(difluoromethoxy)-5-isopropylsulfanyl-phenyl]-1-[2-[4-[(2R)-2-(hydroxymethyl)morpholin-4-yl]-1-piperidyl]-2-oxo-ethyl]pyrazol-4-yl]pyrazolo[1,5-a]pyrimidine-3-carboxamide |
| 156 | 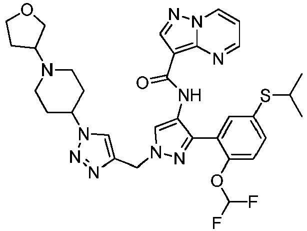 | N-[3-[2-(difluoromethoxy)-5-isopropylsulfanyl-phenyl]-1-[[1-(1-tetrahydrofuran-3-yl-4-piperidyl)triazol-4-yl]methyl]pyrazol-4-yl]pyrazolo[1,5-a]pyrimidine-3-carboxamide |
| | | Or |
| | | N-[3-[2-(difluoromethoxy)-5-(propan-2-ylsulfanyl)phenyl]-1-([1-[1-(oxolan-3-yl)piperidin-4-yl]-1H-1,2,3-triazol-4-yl]methyl)-1H-pyrazol-4-yl]pyrazolo[1,5-a]pyrimidine-3-carboxamide |
| 157 | 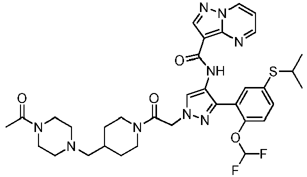 | N-[1-[2-[4-[(4-acetylpiperazin-1-yl)methyl]-1-piperidyl]-2-oxo-ethyl]-3-[2-(difluoromethoxy)-5-isopropylsulfanyl-phenyl]pyrazol-4-yl]pyrazolo[1,5-a]pyrimidine-3-carboxamide |
| 158 | 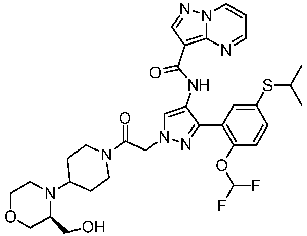 | N-[3-[2-(difluoromethoxy)-5-isopropylsulfanyl-phenyl]-1-[2-[4-[(3S)-3-(hydroxymethyl)morpholin-4-yl]-1-piperidyl]-2-oxo-ethyl]pyrazol-4-yl]pyrazolo[1,5-a]pyrimidine-3-carboxamide |
| 159 | 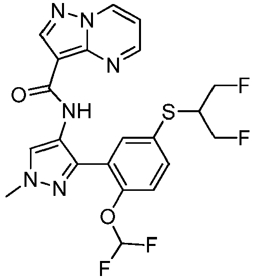 | N-[3-[2-(difluoromethoxy)-5-[2-fluoro-1-(fluoromethyl)ethyl]sulfanyl-phenyl]-1-methyl-pyrazol-4-yl]pyrazolo[1,5-a]pyrimidine-3-carboxamide |
| | | Or |
| | | N-[3-[2-(difluoromethoxy)-5-[(1,3-difluoropropan-2-yl)sulfanyl]phenyl]-1-methyl-1H-pyrazol-4-yl]pyrazolo[1,5-a]pyrimidine-3-carboxamide |
| 160 | 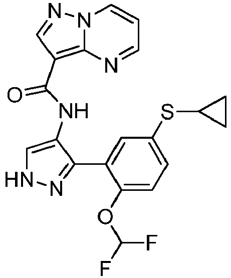 | N-[3-[5-cyclopropylsulfanyl-2-(difluoromethoxy)phenyl]-1H-pyrazol-4-yl]pyrazolo[1,5-a]pyrimidine-3-carboxamide |
| | | Or |
| | | N-[3-[5-(cyclopropylsulfanyl)-2-(difluoromethoxy)phenyl]-1H-pyrazol-4-yl]pyrazolo[1,5-a]pyrimidine-3-carboxamide |
| 161 | 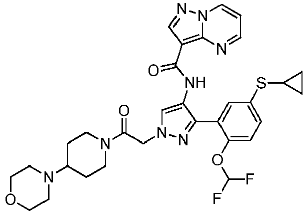 | N-[3-[5-cyclopropylsulfanyl-2-(difluoromethoxy)phenyl]-1-[2-(4-morpholino-1-piperidyl)-2-oxo-ethyl]pyrazol-4-yl]pyrazolo[1,5-a]pyrimidine-3-carboxamide |
| 162 | 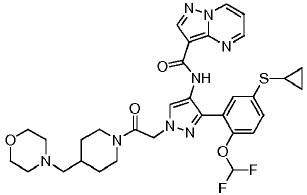 | N-[3-[5-cyclopropylsulfanyl-2-(difluoromethoxy)phenyl]-1-[2-[4-(morpholinomethyl)-1-piperidyl]-2-oxo-ethyl]pyrazol-4-yl]pyrazolo[1,5-a]pyrimidine-3-carboxamide |
| 163 | 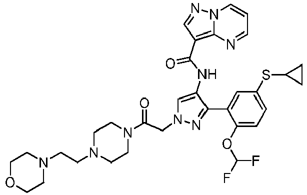 | N-[3-[5-cyclopropylsulfanyl-2-(difluoromethoxy)phenyl]-1-[2-[4-(2-morpholinoethyl)piperazin-1-yl]-2-oxo-ethyl]pyrazol-4-yl]pyrazolo[1,5-a]pyrimidine-3-carboxamide |
| | | Or |
| | | N-[3-[5-(cyclopropylsulfanyl)-2-(difluoromethoxy)phenyl]-1-(2-[4-[2-(morpholin-4-yl)ethyl]piperazin-1-yl]-2-oxoethyl)-1H-pyrazol-4-yl]pyrazolo[1,5-a]pyrimidine-3-carboxamide |
| 164 | 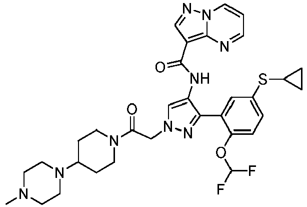 | N-[3-[5-cyclopropylsulfanyl-2-(difluoromethoxy)phenyl]-1-[2-[4-(4-methylpiperazin-1-yl)-1-piperidyl]-2-oxo-ethyl]pyrazol-4-yl]pyrazolo[1,5-a]pyrimidine-3-carboxamide |
| 165 | 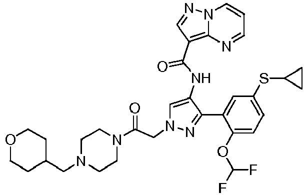 | N-[3-[5-cyclopropylsulfanyl-2-(difluoromethoxy)phenyl]-1-[2-oxo-2-[4-(tetrahydropyran-4-ylmethyl)piperazin-1-yl]ethyl]pyrazol-4-yl]pyrazolo[1,5-a]pyrimidine-3-carboxamide |
| 166 | 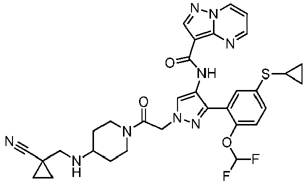 | N-[1-[2-[4-[(1-cyanocyclopropyl)methylamino]-1-piperidyl]-2-oxo-ethyl]-3-[5-cyclopropylsulfanyl-2-(difluoromethoxy)phenyl]pyrazol-4-yl]pyrazolo[1,5-a]pyrimidine-3-carboxamide |
| 167 | 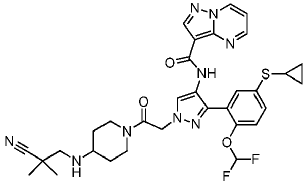 | N-[1-[2-[4-[(2-cyano-2-methyl-propyl)amino]-1-piperidyl]-2-oxo-ethyl]-3-[5-cyclopropylsulfanyl-2-(difluoromethoxy)phenyl]pyrazol-4-yl]pyrazolo[1,5-a]pyrimidine-3-carboxamide |
| | | Or |
| | | N-[1-(2-[4-[(2-cyano-2,2-dimethylethyl)amino]piperidin-1-yl]-2-oxoethyl)-3-[5-(cyclopropylsulfanyl)-2-(difluoromethoxy)phenyl]-1H-pyrazol-4-yl]pyrazolo[1,5-a]pyrimidine-3-carboxamide |
| 168 | 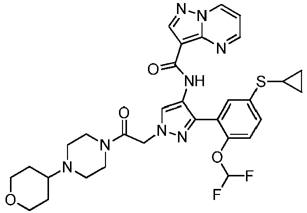 | N-[3-[5-cyclopropylsulfanyl-2-(difluoromethoxy)phenyl]-1-[2-oxo-2-(4-tetrahydropyran-4-ylpiperazin-1-yl)ethyl]pyrazol-4-yl]pyrazolo[1,5-a]pyrimidine-3-carboxamide |
| 169 | 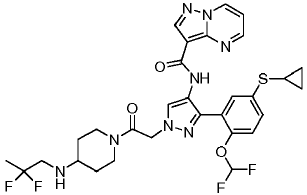 | N-[3-[5-cyclopropylsulfanyl-2-(difluoromethoxy)phenyl]-1-[2-[4-(2,2-difluoropropylamino)-1-piperidyl]-2-oxo-ethyl]pyrazol-4-yl]pyrazolo[1,5-a]pyrimidine-3-carboxamide |
| 170 | 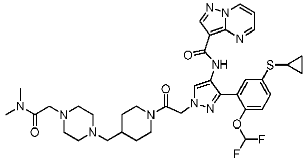 | N-[3-[5-cyclopropylsulfanyl-2-(difluoromethoxy)phenyl]-1-[2-[4-[[4-[2-(dimethylamino)-2-oxo-ethyl]piperazin-1-yl]methyl]-1-piperidyl]-2-oxo-ethyl]pyrazol-4-yl]pyrazolo[1,5-a]pyrimidine-3-carboxamide |
| 171 | 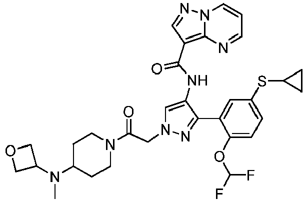 | N-[3-[5-cyclopropylsulfanyl-2-(difluoromethoxy)phenyl]-1-[2-[4-[methyl(oxetan-3-yl)amino]-1-piperidyl]-2-oxo-ethyl]pyrazol-4-yl]pyrazolo[1,5-a]pyrimidine-3-carboxamide |
| 172 | 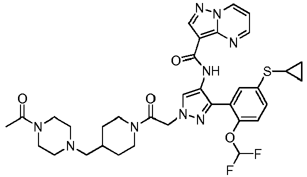 | N-[1-[2-[4-[(4-acetylpiperazin-1-yl)methyl]-1-piperidyl]-2-oxo-ethyl]-3-[5-cyclopropylsulfanyl-2-(difluoromethoxy)phenyl]pyrazol-4-yl]pyrazolo[1,5-a]pyrimidine-3-carboxamide |
| 173 | 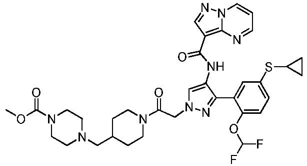 | methyl 4-[[1-[2-[3-[5-cyclopropylsulfanyl-2-(difluoromethoxy)phenyl]-4-(pyrazolo[1,5-a]pyrimidine-3-carbonylamino)pyrazol-1-yl] acetyl]-4-piperidyl] methyl]piperazine-1-carboxylate |
| 174 | 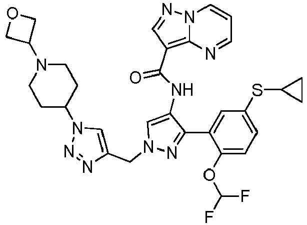 | N-[3-[5-cyclopropylsulfanyl-2-(difluoromethoxy)phenyl]-1-[[1-[1-(oxetan-3-yl)-4-piperidyl]triazol-4-yl]methyl]pyrazol-4-yl]pyrazolo[1,5-a]pyrimidine-3-carboxamide |
| 175 | 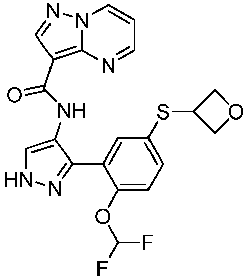 | N-[3-[2-(difluoromethoxy)-5-(oxetan-3-ylsulfanyl)phenyl]-1H-pyrazol-4-yl]pyrazolo[1,5-a]pyrimidine- 3-carboxamide |
| | | Or |
| | | N-[3-[2-(difluoromethoxy)-5-(oxetan-3-ylsulfanyl)phenyl]-1H-pyrazol-4-yl]pyrazolo[1,5-a]pyrimidine-3-carboxamide |
| 176 | 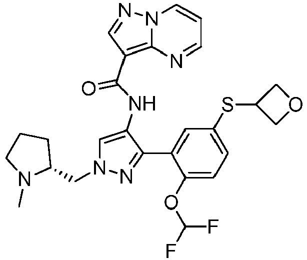 | N-[3-[2-(difluoromethoxy)-5-(oxetan-3-ylsulfanyl)phenyl]-1-[[(2R)-1-methylpyrrolidin-2-yl]methyl]pyrazol-4-yl]pyrazolo[1,5-a]pyrimidine-3-carboxamide |
| 177 | 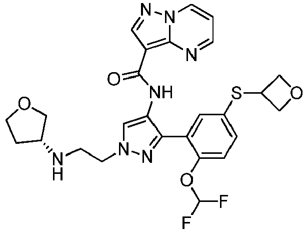 | N-[3-[2-(difluoromethoxy)-5-(oxetan-3-ylsulfanyl)phenyl]-1-[2-[[(3R)-tetrahydrofuran-3-yl]amino]ethyl]pyrazol-4-yl]pyrazolo[1,5-a]pyrimidine- 3-carboxamide |
| 178 | 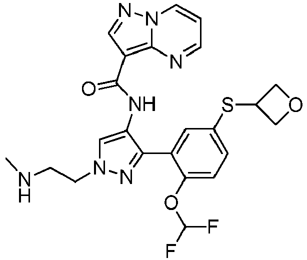 | N-[3-[2-(difluoromethoxy)-5-(oxetan-3-ylsulfanyl)phenyl]-1-[2-(methylamino)ethyl]pyrazol-4-yl]pyrazolo[1,5-a]pyrimidine- 3-carboxamide |
| 179 | 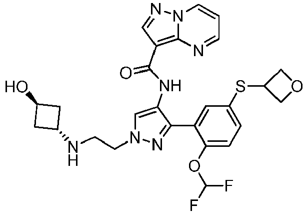 | N-[3-[2-(difluoromethoxy)-5-(oxetan-3-ylsulfanyl)phenyl]-1-[2-[(3-hydroxycyclobutyl)amino]ethyl]pyrazol-4-yl]pyrazolo[1,5-a]pyrimidine-3-carboxamide |
| | | Or |
| | | N-[3-[2-(difluoromethoxy)-5-(oxetan-3-ylsulfanyl)phenyl]-1-(2-[[trans-3-hydroxycyclobutyl] amino]ethyl)-1H-pyrazol-4-yl]pyrazolo[1,5-a]pyrimidine-3-carboxamide |
| 180 | 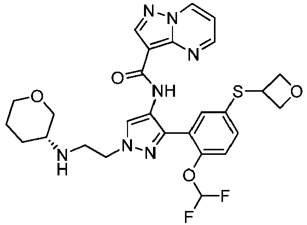 | N-[3-[2-(difluoromethoxy)-5-(oxetan-3-ylsulfanyl)phenyl]-1-[2-[[(3R)-tetrahydropyran-3-yl]amino]ethyl]pyrazol-4-yl]pyrazolo[1,5-a]pyrimidine- 3-carboxamide |
| 181 | 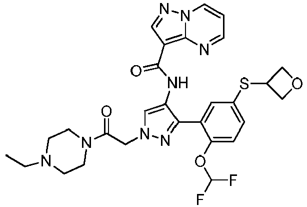 | N-[3-[2-(difluoromethoxy)-5-(oxetan-3-ylsulfanyl)phenyl]-1-[2-(4-ethylpiperazin-1-yl)-2-oxo-ethyl]pyrazol-4-yl]pyrazolo[1,5-a]pyrimidine-3-carboxamide |
| 182 | 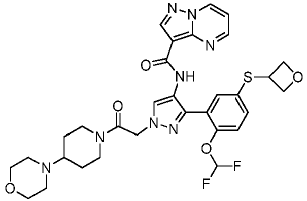 | N-[3-[2-(difluoromethoxy)-5-(oxetan-3-ylsulfanyl)phenyl]-1-[2-(4-morpholino-1-piperidyl)-2-oxo-ethyl]pyrazol-4-yl]pyrazolo[1,5-a]pyrimidine-3-carboxamide |
| 183 | 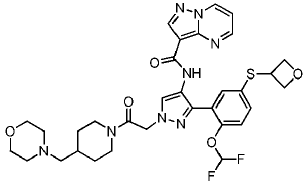 | N-[3-[2-(difluoromethoxy)-5-(oxetan-3-ylsulfanyl)phenyl] -1 - [2- [4- (morpholinomethyl) -1 - piperidyl]-2-oxo-ethyl]pyrazol-4-yl]pyrazolo[1,5-a]pyrimidine- 3-carboxamide |
| 184 | 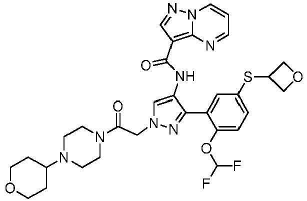 | N-[3-[2-(difluoromethoxy)-5-(oxetan-3-ylsulfanyl)phenyl]-1-[2-oxo-2-(4-tetrahydropyran-4-ylpiperazin-1-yl)ethyl]pyrazol-4-yl]pyrazolo[1,5-a]pyrimidine- 3-carboxamide |
| 185 | 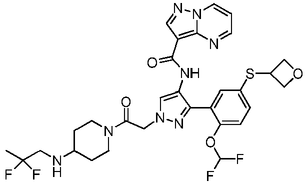 | N-[3-[2-(difluoromethoxy)-5-(oxetan-3-ylsulfanyl)phenyl]-1-[2-[4-(2,2-difluoropropylamino)-1-piperidyl]-2-oxoethyl]pyrazol-4-yl]pyrazolo[1,5-a]pyrimidine-3-carboxamide |
| 186 | 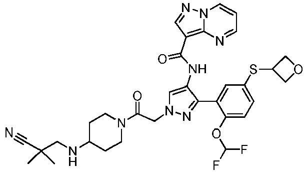 | N-[1-[2-[4-[(2-cyano-2-methyl-propyl)amino]-1-piperidyl]-2-oxo-ethyl]-3-[2-(difluoromethoxy)-5-(oxetan-3-ylsulfanyl)phenyl]pyrazol-4-yl]pyrazolo[1,5-a]pyrimidine-3-carboxamide |
| 187 | 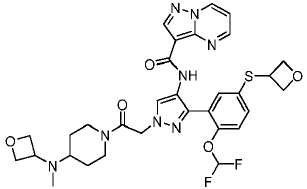 | N-[3-[2-(difluoromethoxy)-5-(oxetan-3-ylsulfanyl)phenyl]-1-[2-[4-[methyl(oxetan-3-yl)amino]-1-piperidyl]-2-oxo-ethyl]pyrazol-4-yl]pyrazolo[1,5-a]pyrimidine-3-carboxamide |
| 188 | 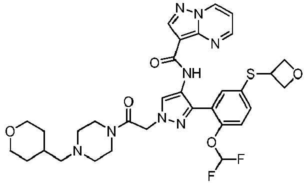 | N-[3-[2-(difluoromethoxy)-5-(oxetan-3-ylsulfanyl)phenyl]-1-[2-oxo-2-[4-(tetrahydropyran-4-ylmethyl)piperazin-1-yl]ethyl]pyrazol-4-yl]pyrazolo[1,5-a]pyrimidine-3-carboxamide |
| 189 | 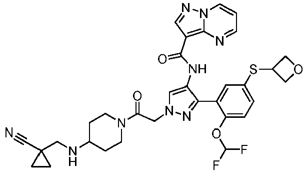 | N-[1-[2-[4-[(1-cyanocyclopropyl)methylamino]-1-pipendyl]-2-oxo-ethyl]-3-[2-(difluoromethoxy)-5-(oxetan-3-ylsulfanyl)phenyl]pyrazol-4-yl]pyrazolo[1,5-a]pyrimidine-3-carboxamide |
| 190 | 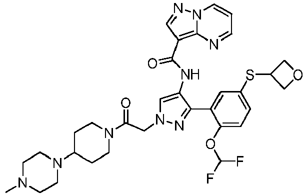 | N-[3-[2-(difluoromethoxy)-5-(oxetan-3-ylsulfanyl)phenyl]-1-[2-[4-(4-methylpiperazin-1-yl)-1-piperidyl]-2-oxo-ethyl]pyrazol-4-yl]pyrazolo[1,5-a]pyrimidine-3-carboxamide |
| 191 | 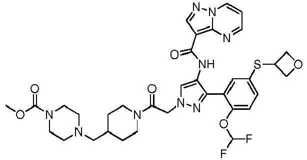 | methyl 4-[[1-[2-[3-[2-(difluoromethoxy)-5-(oxetan-3-ylsulfanyl)phenyl]-4-(pyrazolo[1,5-a]pyrimidine-3-carbonylamino)pyrazol-1-yl]acetyl]-4-piperidyl]methyl]piperazine-1-carboxylate |
| 192 | 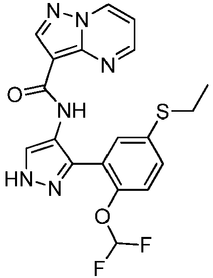 | N-[3-[2-(difluoromethoxy)-5-ethylsulfanylphenyl]-1H-pyrazol-4-yl]pyrazolo[1,5-a]pyrimidine-3-carboxamide |
| | | Or |
| | | N-[3-[2-(difluoromethoxy)-5-(ethylsulfanyl)phenyl]-1H-pyrazol-4-yl]pyrazolo[1,5-a]pyrimidine-3-carboxamide |
| 193 | 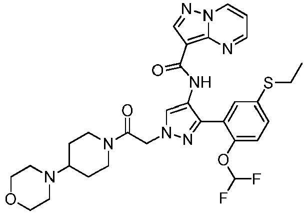 | N-[3-[2-(difluoromethoxy)-5-ethylsulfanylphenyl]-1-[2-(4-morpholino-1-piperidyl)-2-oxoethyl]pyrazol-4-yl]pyrazolo[1,5-a]pyrimidine-3-carboxamide |
| 194 | 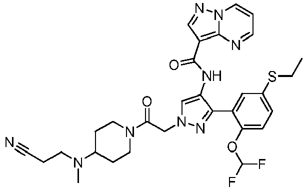 | N-[1-[2-[4-[2-cyanoethyl(methyl)amino]-1-pipendyl]-2-oxo-ethyl]-3-[2-(difluoromethoxy)-5-ethylsulfanyl-phenyl]pyrazol-4-yl]pyrazolo[1,5-a]pyrimidine- 3-carboxamide |

### Enzymatic Assays

### JAK Enzyme Assays were carried out as follows:

The activity of the isolated recombinant JAK1 and JAK2 kinase domain was measured by monitoring phosphorylation of a peptide derived from JAK3 (Val-Ala-Leu-Val-Asp-Gly-Tyr-Phe-Arg-Leu-Thr-Thr, fluorescently labeled on the N-terminus with 5-carboxyfluorescein) using the Caliper LabChip® technology (Caliper Life Sciences, Hopkinton, MA). To determine inhibition constants (*K*ᵢ), compounds were diluted serially in DMSO and added to 50 µL kinase reactions containing purified enzyme (1.5 nM JAK1, or 0.2 nM JAK2), 100 mM HEPES buffer (pH 7.2), 0.015% Brij-35, 1.5 µM peptide substrate, ATP (25 µM), 10 mM MgCl₂, 4 mM DTT at a final DMSO concentration of 2%. Reactions were incubated at 22 °C in 384-well polypropylene microtiter plates for 30 minutes and then stopped by addition of 25 µL of an EDTA containing solution (100 mM HEPES buffer (pH 7.2), 0.015% Brij-35, 150 mM EDTA), resulting in a final EDTA concentration of 50 mM. After termination of the kinase reaction, the proportion of phosphorylated product was determined as a fraction of total peptide substrate using the Caliper LabChip® 3000 according to the manufacturer's specifications. *K*ᵢ values were then determined using the Morrison tight binding model (Morrison, J.F., Biochim. Biophys. Acta. 185:269-296 (1969); William, J.W. and Morrison, J.F., Meth. Enzymol., 63:437-467 (1979)) modified for ATP-competitive inhibition [*K*ᵢ = *K*_{i,app} / (1 + [ATP] / *K*_{m,app})]. Data for representative compounds is shown in Table 2.

### JAK1 Pathway Assay in Cell Lines was carried out as follows:

Inhibitor potency (EC₅₀) was determined in cell-based assays designed to measure JAK1 dependent STAT phosphorylation. As noted above, inhibition of IL-4, IL-13, and IL-9 signaling by blocking the Jak/Stat signaling pathway can alleviate asthmatic symptoms in pre-clinical lung inflammation models (Mathew et al., 2001, J Exp Med 193(9): 1087-1096; Kudlacz et. al., 2008, Eur J. Pharmacol 582(1-3): 154-161).

In one assay approach, TF-1 human erythroleukemia cells obtained from the American Type Culture Collection (ATCC; Manassas, VA) were used to measure JAK1-dependent STAT6 phosphorylation downstream of IL-13 stimulation. Prior to use in the assays, TF-1 cells were starved of GM-CSF overnight in OptiMEM medium (Life Technologies, Grand Island, NY) supplemented with 0.5% charcoal/dextran stripped fetal bovine serum (FBS), 0.1 mM non-essential amino acids (NEAA), and 1 mM sodium pyruvate. The assays were run in 384-well plates in serum-free OptiMEM medium using 300,000 cells per well. In a second assay approach, BEAS-2B human bronchial epithelial cells obtained from ATCC were plated at 100,000 cells per well of a 96-well plate one day prior to the experiment. The BEAS-2B assay was run in complete growth medium (bronchial epithelial basal medium plus bulletkit; Lonza; Basel, Switzerland).

Test compounds were serially diluted 1:2 in DMSO and then diluted 1:50 in medium just before use. Diluted compounds were added to the cells, for a final DMSO concentration of 0.2%, and incubated for 30 min (for the TF-1 assay) or 1 hr (for the BEAS-2B assay) at 37 °C. Then, cells were stimulated with human recombinant cytokine at their respective EC₉₀ concentrations, as previously determined for each individual lot. Cells were stimulated with IL-13 (R&D Systems, Minneapolis, MN) for 15 min at 37°C. The TF-1 cell reactions were stopped by the direct addition of 10x lysis buffer (Cell Signaling Technologies, Danvers, MA), whereas the BEAS-2B cell incubations were halted by the removal of medium and addition of 1x lysis buffer. The resultant samples were frozen in the plates at -80 °C. Compound mediated inhibition of STAT6 phosphorylation was measured in the cell lysates using MesoScale Discovery (MSD) technology (Gaithersburg, MD). EC₅₀ values were determined as the concentration of compound required for 50% inhibition of STAT phosphorylation relative to that measured for the DMSO control. Data for representative compounds is shown in Table 2.

**Table 2:**

| Ex | JAK1 Ki (uM) | JAK2 Ki (uM) | P-STAT6 BEAS2B+IL13 IC50 (uM) | LCMS (ESI) m/z [M+H]+ | LCMS Method | LCMS RT (Min) |
|---|---|---|---|---|---|---|
| 1 | 0.000079 | 0.000041 | 0.0026 | 417.1 | A | 1.81 |
| 2 | 0.00028 | 0.00042 | 0.025 | 624.1 | C | 2.53 |
| 3 | 0.0017 | 0.00057 | 0.43 | 433 | C | 2.07 |
| 4 | 0.00045 | 0.00016 | 0.035 | 449 | C | 2.3 |
| 5 | 0.00029 | 0.0004 | 0.015 | 627.4 | A | 1.81 |
| 6 | 0.00049 | 0.00061 | 0.018 | 571.2 | A | 1.81 |
| 7 | 0.0003 | 0.00049 | 0.0048 | 641.3 | A | 2.39 |
| 8 | 0.00029 | 0.00039 | 0.018 | 610.2 | A | 2 |
| 9 | 0.00044 | 0.00028 | 0.0052 | 500.2 | A | 2.43 |
| 10 | 0.00049 | 0.00027 | 0.0088 | 530.2 | A | 2.37 |
| 11 | 0.00032 | 0.00021 | 0.0059 | 530.2 | A | 1.47 |
| 12 | 0.00041 | 0.00032 | 0.006 | 514.2 | H | 1.37 |
| 13 | 0.00044 | 0.0009 | 0.057 | 656.3 | A | 2.38 |
| 14 | 0.00032 | 0.00068 | 0.089 | 682.3 | F | 2.81 |
| 15 | 0.00028 | 0.00038 | 0.31 | 640.2 | F | 1.31 |
| 16 | 0.00039 | 0.0005 | 0.018 | 656.3 | A | 1.63 |
| 17 | 0.00027 | 0.00053 | 0.005 | 638.3 | A | 1.47 |
| 18 | 0.00024 | 0.00037 | 0.006 | 650.3 | A | 1.48 |
| 19 | 0.00024 | 0.00039 | 0.0074 | 635.2 | A | 1.49 |
| 20 | 0.0002 | 0.00033 | 0.011 | 638.3 | A | 1.48 |
| 21 | 0.00027 | 0.00033 | 0.0091 | 610.2 | A | 1.44 |
| 22 | 0.00032 | 0.00062 | 0.13 | 668.1 | F | 1.02 |
| 23 | 0.00024 | 0.00031 | 0.061 | 654.3 | A | 1.8 |
| 24 | 0.00022 | 0.00032 | 0.0049 | 649.1 | F | 1.06 |
| 25 | 0.00031 | 0.00051 | 0.018 | 725.4 | A | 1.39 |
| 26 | 0.00024 | 0.00039 | 0.012 | 652.3 | D | 1.94 |
| 27 | 0.00023 | 0.00032 | 0.0051 | 643.2 | A | 1.49 |
| 28 | 0.00022 | 0.00026 | 0.003 | 474.1 | F | 0.98 |
| 29 | 0.00026 | 0.00037 | 0.009 | 638.3 | D | 1.82 |
| 30 | 0.00029 | 0.00038 | 0.0087 | 624.2 | F | 1.09 |
| 31 | 0.00019 | 0.00025 | 0.0083 | 650.3 | A | 1.48 |
| 32 | 0.00023 | 0.00033 | 0.011 | 636.2 | J | 2.8 |
| 33 | 0.00038 | 0.00073 | 0.035 | 725.4 | A | 1.32 |
| 34 | 0.00028 | 0.0003 | 0.0053 | 583.2 | F | 0.98 |
| 35 | 0.00039 | 0.0004 | 0.019 | 583.2 | A | 1.43 |
| 36 | 0.00036 | 0.00029 | 0.0098 | 516.1 | F | 1.98 |
| 37 | 0.00022 | 0.00048 | 0.0099 | 514.2 | A | 2.51 |
| 38 | 0.00023 | 0.00019 | 0.02 | 544.2 | A | 1.5 |
| 39 | 0.0003 | 0.00033 | 0.019 | 597.1 | D | 1.6 |
| 40 | 0.00028 | 0.00025 | 0.0095 | 530.1 | D | 1.4 |
| 41 | 0.00027 | 0.00045 | 0.02 | 636.3 | A | 1.46 |
| 42 | 0.00017 | 0.00026 | 0.013 | 650.3 | P | 3.14 |
| 43 | 0.00035 | 0.00087 | 0.076 | 696.2 | D | 1.59 |
| 44 | 0.00038 | 0.00072 | 0.073 | 682.1 | D | 1.63 |
| 45 | 0.00032 | 0.00068 | 0.044 | 641.1 | J | 2.92 |
| 46 | 0.00025 | 0.00044 | 0.022 | 627.1 | D | 1.45 |
| 47 | 0.00052 | 0.0013 | 0.35 | 682.3 | A | 1.47 |
| 48 | 0.00029 | 0.00044 | 0.41 | 659.1 | J | 2.48 |
| 49 | 0.00028 | 0.00062 | 0.015 | 641.1 | F | 2 |
| 50 | 0.0005 | 0.00096 | 0.018 | 657.1 | D | 1.58 |
| 51 | 0.00043 | 0.00097 | 0.026 | 671.1 | D | 1.72 |
| 52 | 0.00029 | 0.00044 | 0.043 | 667.6 | A | 1.49 |
| 53 | 0.00027 | 0.00032 | 0.012 | 627.2 | D | 1.49 |
| 54 | 0.00023 | 0.00031 | 0.012 | 613.2 | D | 1.47 |
| 55 | 0.00027 | 0.00052 | 0.011 | 650.3 | A | 1.5 |
| 56 | 0.00041 | 0.00048 | 0.084 | 569.2 | A | 1.43 |
| 57 | 0.00026 | 0.00043 | 0.018 | 641.3 | A | 2.04 |
| 58 | 0.0002 | 0.00033 | 0.013 | 650.3 | A | 1.51 |
| 59 | 0.0002 | 0.00034 | 0.015 | 622.2 | A | 2.4 |
| 60 | 0.00031 | 0.00052 | 0.014 | 641.2 | D | 1.51 |
| 61 | 0.00025 | 0.00048 | 0.013 | 641.3 | A | 2.41 |
| 62 | 0.00027 | 0.00038 | 0.16 | 675.3 | A | 2.1 |
| 63 | 0.00035 | 0.0007 | 0.022 | 640.3 | J | 2.67 |
| 64 | 0.00033 | 0.00048 | 0.052 | 624.3 | A | 2.31 |
| 65 | 0.00041 | 0.00049 | 0.015 | 641.2 | J | 2.69 |
| 66 | 0.0002 | 0.00024 | 0.005 | 643.2 | A | 2.25 |
| 67 | 0.00019 | 0.00027 | 0.0089 | 540.2 | A | 1.82 |
| 68 | 0.00047 | 0.00085 | 0.3 | 682.3 | J | 2.76 |
| 69 | 0.00035 | 0.00071 | 0.05 | 682.4 | A | 2.38 |
| 70 | 0.00042 | 0.00082 | 0.038 | 710.3 | J | 2.71 |
| 71 | 0.00059 | 0.0011 | 0.083 | 698.4 | F | 2.39 |
| 72 | 0.0004 | 0.00036 | 0.11 | 663.3 | D | 1.44 |
| 73 | 0.00023 | 0.00025 | 0.039 | 663.3 | J | 2.8 |
| 74 | 0.0003 | 0.00058 | 0.05 | 725.4 | A | 1.89 |
| 75 | 0.00028 | 0.00038 | 0.012 | 639.3 | A | 2.05 |
| 76 | 0.0014 | 0.001 | 0.06 | 525.2 | F | 2.07 |
| 77 | 0.00047 | 0.00033 | 0.027 | 511.1 | F | 2.03 |
| 78 | 0.00092 | 0.00072 | 0.069 | 611.2 | F | 3.65 |
| 79 | 0.00039 | 0.00044 | 0.035 | 663 | A | 2.63 |
| 80 | 0.00026 | 0.00043 | 0.016 | 682.3 | D | 1.51 |
| 81 | 0.00035 | 0.00023 | 0.0081 | 665 | C | 1.48 |
| 82 | 0.00029 | 0.00041 | 0.034 | 712.3 | D | 1.51 |
| 83 | 0.0003 | 0.00044 | 0.01 | 698.3 | F | 1.01 |
| 84 | 0.00037 | 0.00053 | 0.044 | 657.3 | A | 1.41 |
| 85 | 0.00033 | 0.00038 | 0.11 | 657.3 | A | 1.42 |
| 86 | 0.00031 | 0.00042 | 0.12 | 657.4 | A | 1.93 |
| 87 | 0.00039 | 0.00061 | 0.035 | 739 | A | 2.33 |
| 88 | 0.00032 | 0.00049 | 0.47 | 753.4 | A | 1.67 |
| 89 | 0.00051 | 0.00078 | 0.2 | 711.4 | A | 2.32 |
| 90 | 0.00032 | 0.00014 | 0.022 | 637.3 | D | 2.22 |
| 91 | 0.00013 | 0.000071 | 0.0039 | 431.1 | A | 1.81 |
| 92 | 0.00061 | 0.00077 | 1 | 726.4 | K | 2.63 |
| 93 | 0.00044 | 0.00071 | 0.026 | 767.4 | A | 2.35 |
| 94 | 0.00046 | 0.00063 | 0.062 | 739.5 | A | 1.43 |
| 95 | 0.00037 | 0.00068 | 0.15 | 640.4 | D | 1.45 |
| 96 | 0.0003 | 0.0002 | 0.014 | 651.4 | D | 1.94 |
| 97 | 0.00038 | 0.00062 | 0.03 | 739.5 | D | 1.58 |
| 98 | 0.00064 | 0.0012 | 0.037 | 753.4 | D | 1.63 |
| 99 | 0.00038 | 0.00056 | 0.0095 | 650.3 | D | 1.61 |
| 100 | 0.00041 | 0.0006 | 0.0094 | 650.3 | D | 1.62 |
| 101 | 0.0002 | 0.000093 | 0.0037 | 445.2 | A | 1.84 |
| 102 | 0.00038 | 0.00085 | 0.02 | 655.4 | A | 2 |
| 103 | 0.00033 | 0.00075 | 0.041 | 638.3 | A | 1.56 |
| 104 | 0.00042 | 0.0012 | 0.16 | 670.1 | H | 1.07 |
| 105 | 0.00043 | 0.0013 | 0.072 | 684.1 | H | 1.07 |
| 106 | 0.0004 | 0.00079 | 0.11 | 682.3 | A | 1.53 |
| 107 | 0.00075 | 0.0017 | 0.056 | 678.4 | A | 1.87 |
| 108 | 0.00037 | 0.00094 | 0.85 | 668.4 | K | 4.02 |
| 109 | 0.00041 | 0.00078 | 0.033 | 684.3 | D | 1.58 |
| 110 | 0.00041 | 0.001 | 0.027 | 655.3 | D | 1.64 |
| 111 | 0.00038 | 0.001 | 0.019 | 671.2 | D | 1.88 |
| 112 | 0.00039 | 0.00039 | 0.018 | 488.2 | A | 2.57 |
| 113 | 0.00064 | 0.00078 | 0.02 | 528.3 | A | 2.08 |
| 114 | 0.0016 | 0.0039 | 0.055 | 753.4 | D | 1.7 |
| 115 | 0.00065 | 0.0016 | 0.034 | 664.3 | J | 2.77 |
| 116 | 0.00051 | 0.001 | 0.026 | 630 | A | 1.58 |
| 117 | 0.0007 | 0.0014 | 0.021 | 585.3 | D | 1.65 |
| 118 | 0.0006 | 0.0018 | 0.04 | 668.3 | H | 1 |
| 119 | 0.00047 | 0.00096 | 0.03 | 671.3 | D | 1.91 |
| 120 | 0.0004 | 0.0012 | 0.027 | 666 | K | 2.74 |
| 121 | 0.0007 | 0.0013 | 0.031 | 599.4 | A | 2.2 |
| 122 | 0.00041 | 0.00085 | 0.042 | 659.5 | A | 1.54 |
| 123 | 0.00079 | 0.002 | 0.038 | 685.3 | J | 2.68 |
| 124 | 0.00092 | 0.0011 | 0.059 | 611.4 | A | 1.58 |
| 125 | 0.00047 | 0.00094 | 0.017 | 655.3 | D | 1.83 |
| 126 | 0.00037 | 0.00084 | 0.043 | 629.3 | D | 1.54 |
| 127 | 0.00048 | 0.00097 | 0.035 | 655.4 | K | 2.64 |
| 128 | 0.00058 | 0.0012 | 0.047 | 641.3 | A | 2.33 |
| 129 | 0.00042 | 0.00099 | 0.026 | 650.3 | D | 1.72 |
| 130 | 0.00057 | 0.001 | 0.019 | 667.4 | A | 1.89 |
| 131 | 0.00041 | 0.0008 | 0.03 | 615.3 | A | 2.55 |
| 132 | 0.00067 | 0.0012 | 0.036 | 669.3 | H | 1.1 |
| 133 | | | | 503.2 | D | 1.2 |
| 134 | 0.00051 | 0.0011 | 0.027 | 663.4 | K | 2.86 |
| 135 | 0.00043 | 0.00087 | 0.024 | 669.4 | J | 2.65 |
| 136 | 0.00049 | 0.0012 | 0.02 | 669.4 | H | 1.1 |
| 137 | 0.0005 | 0.0015 | 0.066 | 696.5 | H | 1.1 |
| 138 | 0.00022 | 0.00017 | 0.25 | 687.3 | D | 1.47 |
| 139 | 0.00051 | 0.00084 | 0.14 | 691.5 | A | 2.1 |
| 140 | 0.00063 | 0.0017 | 0.051 | 753.4 | D | 1.72 |
| 141 | 0.00078 | 0.0019 | 0.036 | 795.4 | D | 1.67 |
| 142 | 0.00088 | 0.002 | 0.11 | 739.4 | D | 2.45 |
| 143 | 0.00092 | 0.0025 | 0.089 | 710.4 | D | 2.46 |
| 144 | 0.00025 | 0.00014 | 0.0056 | 459.1 | A | 2.02 |
| 145 | 0.0006 | 0.0016 | 0.1 | 696.4 | D | 1.73 |
| 146 | 0.00037 | 0.00072 | 0.17 | 691.4 | D | 1.61 |
| 147 | 0.00073 | 0.0018 | 1 | 754.4 | O | 1.15 |
| 148 | 0.0004 | 0.00024 | 0.055 | 665.4 | K | 2.7 |
| 149 | 0.00052 | 0.0012 | 0.05 | 685.4 | D | 1.51 |
| 150 | 0.0006 | 0.0014 | 0.015 | 726.4 | D | 1.86 |
| 151 | 0.00054 | 0.0023 | 0.96 | 682.5 | D | 2.26 |
| 152 | 0.00052 | 0.0013 | 0.29 | 781.5 | O | 1.14 |
| 153 | 0.00041 | 0.00091 | 0.11 | 685.4 | D | 2.29 |
| 154 | 0.00042 | 0.0015 | 0.081 | 726.4 | D | 1.63 |
| 155 | 0.00031 | 0.00099 | 0.048 | 685.3 | D | 1.52 |
| 156 | 0.00036 | 0.00029 | 0.021 | 679.4 | M | 2.69 |
| 157 | 0.00027 | 0.00077 | 0.047 | 710.4 | D | 1.65 |
| 158 | 0.00059 | 0.001 | 0.091 | 685.4 | D | 1.54 |
| 159 | 0.00016 | 0.000097 | 0.0039 | 495.2 | D | 1.71 |
| 160 | 0.000098 | 0.000056 | 0.0073 | 443.2 | N | 3.12 |
| 161 | 0.0003 | 0.00064 | 0.016 | 653.3 | A | 2.58 |
| 162 | 0.00042 | 0.00098 | 0.015 | 667.3 | D | 2.02 |
| 163 | 0.00043 | 0.00094 | 0.024 | 682.3 | D | 1.54 |
| 164 | 0.00055 | 0.0015 | 0.032 | 666.4 | A | 1.44 |
| 165 | 0.0004 | 0.00067 | 0.02 | 667.4 | A | 1.88 |
| 166 | 0.00029 | 0.00072 | 0.017 | 662.4 | A | 2.63 |
| 167 | 0.00028 | 0.00067 | 0.011 | 664.4 | K | 2.66 |
| 168 | 0.00029 | 0.00046 | 0.014 | 653.4 | A | 1.55 |
| 169 | 0.00043 | 0.0008 | 0.02 | 661.3 | A | 1.57 |
| 170 | 0.00039 | 0.00078 | 0.025 | 751.4 | D | 1.63 |
| 171 | 0.00043 | 0.00078 | 0.022 | 653.4 | A | 1.53 |
| 172 | 0.00045 | 0.00091 | 0.032 | 708.4 | D | 1.62 |
| 173 | 0.00033 | 0.00083 | 0.017 | 724.4 | D | 1.81 |
| 174 | 0.00027 | 0.00021 | 0.011 | 663.4 | A | 1.55 |
| 175 | 0.00023 | 0.00011 | 0.0095 | 459.1 | A | 1.59 |
| 176 | 0.00038 | 0.0014 | 0.028 | 556.3 | A | 2.66 |
| 177 | 0.00081 | 0.00048 | 0.018 | 572.2 | A | 2.34 |
| 178 | 0.00036 | 0.00064 | 0.009 | 516.2 | A | 1.7 |
| 179 | 0.00074 | 0.00059 | 0.026 | 572.2 | O | 0.98 |
| 180 | 0.001 | 0.00076 | 0.014 | 586.3 | K | 2.84 |
| 181 | 0.0008 | 0.0018 | 0.083 | 613.2 | A | 2.1 |
| 182 | 0.00092 | 0.0019 | 0.091 | 669.3 | D | 1.4 |
| 183 | 0.00065 | 0.0015 | 0.033 | 683.4 | A | 2.47 |
| 184 | 0.00056 | 0.0011 | 0.074 | 669.4 | A | 1.38 |
| 185 | 0.00071 | 0.0017 | 0.047 | 677.4 | O | 2.04 |
| 186 | 0.00065 | 0.0018 | 0.09 | 680.4 | O | 2.08 |
| 187 | 0.00054 | 0.0013 | 0.25 | 669.3 | O | 1.02 |
| 188 | 0.00062 | 0.0013 | 0.048 | 683.4 | A | 2.49 |
| 189 | 0.001 | 0.003 | 0.77 | 678.4 | A | 1.41 |
| 190 | 0.0011 | 0.0038 | 0.17 | 682.3 | O | 0.96 |
| 191 | 0.00044 | 0.001 | 0.049 | 740.4 | A | 1.42 |
| 192 | 0.00012 | 0.000055 | 0.002 | 431.1 | L | 3.15 |
| 193 | 0.00036 | 0.00057 | 0.017 | 641.3 | A | 2.51 |
| 194 | 0.00031 | 0.00063 | 0.025 | 638.3 | A | 1.53 |

### LRRK2 Assay

The LRRK2 assay conditions to measure IC50 values follow procedures carried out under Life TechnologiesTM Adapta® Assay Conditions using SelectScreen Profiling (75 µM ATP; 10-point curves with test compound starting at 10 µM with 3-fold dilutions (10 µm - 0.508 nM)).

The following table shows potency of compounds against JAK1 and LRRK2. 5-Sulfanylphenyl compounds D, E, F, G have greater selectivity for JAK1 over LRRK2 than their 5-chlorophenyl matched pair A. Similarly, 5-sulfanylphenyl compound H has greater selectivity for JAK1 over LRRK2 than its 5-chlorophenyl matched pair B, and 5-sulfanylphenyl compound I has greater selectivity for JAK1 over LRRK2 than its 5-chlorophenyl matched pair C. A high degree of JAK1 over LRRK2 selectivity is maintained for 5-sulfanylphenyl compounds J, K, L, M, and N.

| Compound | Structure | JAK1_Ki (uM) | LRRK2 IC50 (uM) | Ratio (LRRK2 IC50: JAK1 Ki) |
|---|---|---|---|---|
| A | 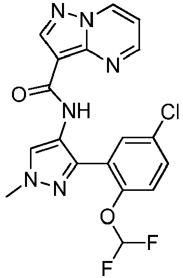 | 0.00018 | 0.0809 | 449 |
| B | 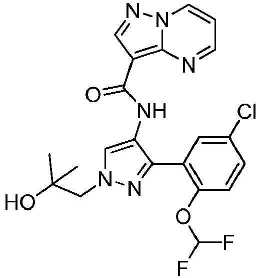 | 0.00040 | 0.431 | 1078 |
| C | 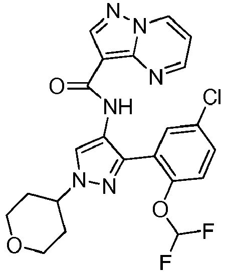 | 0.00069 | 1.03 | 1493 |
| D | 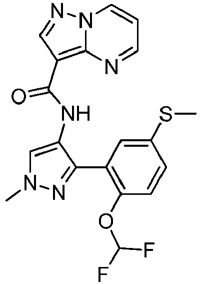 | 0.00013 | 0.193 | 1485 |
| E | 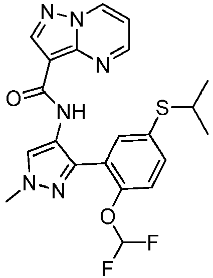 | 0.00025 | 2.22 | 8880 |
| F | 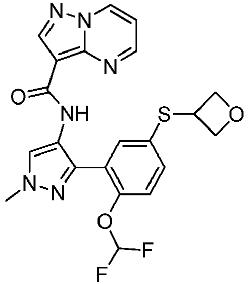 | 0.00029 | | |
| G | 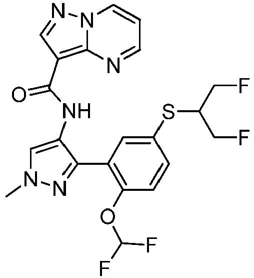 | 0.00016 | | |
| H | 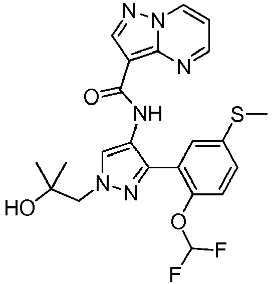 | | | |
| I | 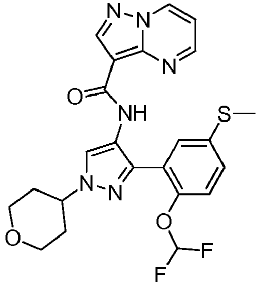 | 0.00067 | | |
| J | 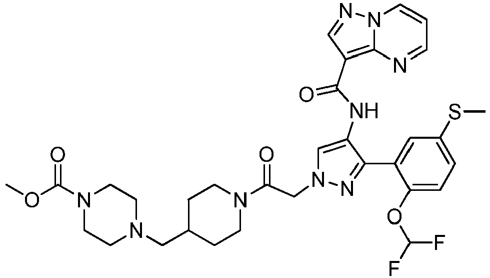 | 0.0003 | 2.45 | 8167 |
| K | 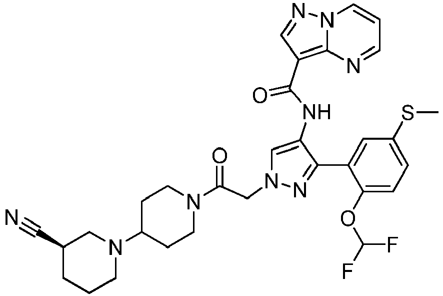 | 0.0002 | 1.7 | 8500 |
| L | 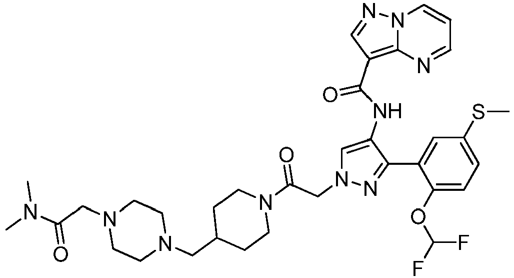 | 0.00031 | 2.00 | 6453 |
| M | 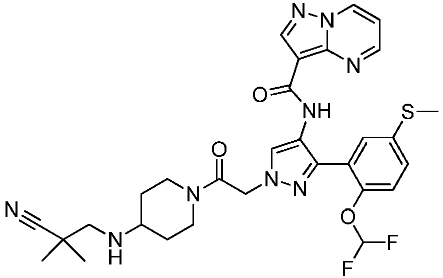 | 0.0002 | 3.35 | 16750 |
| N | 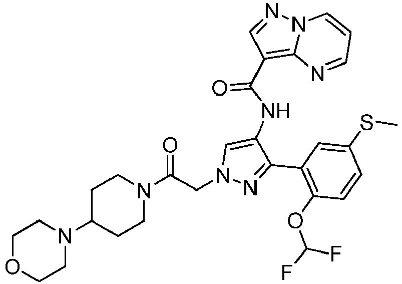 | 0.00029 | 1.85 | 6379 |

Additionally, Figure 1 compares the inhibition of LRRK2 between compounds of Formula (00A) (points on the right) and corresponding compounds wherein the group -S(O)ₙ-R² is replaced with Cl (points on the left). The Y-axis represents % inhibition of LRRK2 at a test concentration of 0.1 uM. The linked points represent matched pairs that differ only between the group -S(O)ₙ-R² and Cl. Each compound of Formula (00A) demonstrated reduced inhibition of LRRK2 compared to the corresponding compound wherein the group -S(O)ₙ-R² was replaced with Cl.

## Claims

1. A compound of Formula (00A): 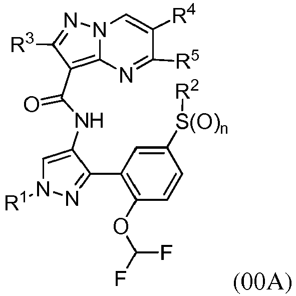 or a salt thereof, wherein:
R¹ is H, C₁-C₆alkyl, C₂-C₆alkenyl, C₂-C₆alkynyl, -(C₀-C₃alkyl)CN, -(C₀-C₃alkyl)OR^{a},-(C₀-C₃alkyl)R^{a}, -(C₀-C₃alkyl)SR^{a}, -(C₀-C₃alkyl)NR^{a}R^{b}, -(C₀-C₃alkyl)OCF₃, -(C₀-C₃alkyl)CF₃, -(C₀-C₃alkyl)NO₂, -(C₀-C₃alkyl)C(O)R^{a}, -(C₀-C₃alkyl)C(O)OR^{a}, -(C₀-C₃alkyl)C(O)NR^{a}R^{b},-(C₀-C₃alkyl)NR^{a}C(O)R^{b}, -(C₀-C₃alkyl)S(O)₁₋₂R^{a}, -(C₀-C₃alkyl)NR^{a}S(O)₁₋₂R^{b}, -(C₀-C₃alkyl)S(O)₁₋₂NR^{a}R^{b}, -(C₀-C₃alkyl)(5-6-membered heteroaryl) or -(C₀-C₃alkyl)phenyl, wherein R¹ is optionally substituted by one or more groups independently selected from the group consisting of halogen, C₁-C₃alkyl, oxo, -CF₃, -(C₀-C₃alkyl)OR^{c} and -(C₀-C₃alkyl)NR^{c}R^{d};
R^{a} is independently hydrogen, C₁-C₆alkyl, C₃-C₆ cycloalkyl, 3-10 membered heterocyclyl, 5-6 membered heteroaryl, -C(O)R^{c}, -C(O)OR^{c}, -C(O)NR^{c}R^{d}, -NR^{c}C(O)R^{d},-S(O)₁₋₂R^{c}, -NR^{c}S(O)₁₋₂R^{d} or -S(O)₁₋₂NR^{c}R^{d}, wherein any C₃-C₆ cycloalkyl, 3-10 membered heterocyclyl, and 5-6 membered heteroaryl of R^{a} is optionally substituted with one or more groups R^{e};
R^{b} is independently hydrogen or C₁-C₃alkyl, wherein said alkyl is optionally substituted by one or more groups independently selected from the group consisting of halogen and oxo; or
R^{c} and R^{d} are independently selected from the group consisting of hydrogen, 3-6 membered heterocyclyl, C₃-C₆ cycloalkyl, and C₁-C₃alkyl, wherein any 3-6 membered heterocyclyl, C₃-C₆ cycloalkyl, and C₁-C₃alkyl of R^{c} and R^{d} is optionally substituted by one or more groups independently selected from the group consisting of halogen and oxo; or R^{c} and R^{d} are taken together with the atom to which they are attached to form a 3-6-membered heterocyclyl, optionally substituted by one or more groups independently selected from the group consisting of halogen, oxo, -CF₃ and C₁-C₃alkyl;
each R^{e} is independently selected from the group consisting of oxo, OR^{f}, NR^{f}R^{g}, halogen, 3-10 membered heterocyclyl, C₃-C₆ cycloalkyl, and C₁-C₆alkyl, wherein any C₃-C₆ cycloalkyl and C₁-C₆alkyl of R^{e} is optionally substituted by one or more groups independently selected from the group consisting of OR^{f}, NR^{f}R^{g}, halogen, 3-10 membered heterocyclyl, oxo, and cyano, and wherein any 3-10 membered heterocyclyl of R^{e} is optionally substituted by one or more groups independently selected from the group consisting of halogen, oxo, cyano, -CF₃, NR^{h}R^{k}, 3-6 membered heterocyclyl, and C₁-C₃alkyl that is optionally substituted by one or more groups independently selected from the group consisting of halogen, oxo, OR^{f}, and NR^{h}R^{k};
R^{f} and R^{g} are each independently selected from the group consisting of hydrogen, C₁-C₆alkyl, 3-6 membered heterocyclyl, and C₃-C₆ cycloalkyl, wherein any C₁-C₆alkyl, 3-6 membered heterocyclyl, and C₃-C₆ cycloalkyl of R^{f} and R^{g} is optionally substituted by one or more R^{m};
each R^{m} is independently selected from the group consisting of halogen, cyano, oxo, C₃-C₆cycloalkyl, 3-6 membered heterocyclyl, hydroxy, and NR^{h}R^{k}, wherein any C₃-C₆cycloalkyl and 3-6 membered heterocyclyl of R^{m} is optionally substituted with one or more groups independently selected from the group consisting of halogen, oxo, cyano, and C₁-C₃alkyl;
R^{h} and R^{k} are each independently selected from the group consisting of hydrogen and C₁-C₆alkyl that is optionally substituted by one or more groups independently selected from the group consisting of halogen, cyano, 3-6 membered heterocyclyl, and oxo; or R^{h} and R^{k} are taken together with the atom to which they are attached to form a 3-6-membered heterocyclyl that is optionally substituted by one or more groups independently selected from the group consisting of halogen, cyano, oxo, -CF₃ and C₁-C₃alkyl that is optionally substituted by one or more groups independently selected from the group consisting of halogen and oxo;
R² is C₁-C₆alkyl, C₂-C₆alkenyl, C₂-C₆alkynyl, C₃-C₆ cycloalkyl, 3-6-membered heterocyclyl, (C₃-C₆ cycloalkyl)C₁-C₆alkyl, (3-6-membered heterocyclyl)C₁-C₆alkyl, -C(O)(C₃-C₆ cycloalkyl), or -C(O)(3-6-membered heterocyclyl), wherein R² is optionally substituted with one or more halo;
n is 0, 1, or 2;
R³ is H or NH₂;
R⁴ is H or CH₃; and
R⁵ is H or NH₂.

2. The compound of claim 1 wherein R¹ is H, C₁-C₆alkyl, -(C₀-C₃alkyl)R^{a}, -(C₀-C₃alkyl)NR^{a}R^{b}, -(C₀-C₃alkyl)C(O)R^{a}, or -(C₀-C₃alkyl)C(O)OR^{a}, or a salt thereof.

3. The compound of claim 1 wherein R¹ is H, or a salt thereof.

4. The compound of claim 1 wherein R¹ is C₁-C₆alkyl, C₂-C₆alkenyl, C₂-C₆alkynyl, -(C₀-C₃alkyl)CN, -(C₀-C₃alkyl)OR^{a}, -(C₀-C₃alkyl)R^{a}, -(C₀-C₃alkyl)SR^{a}, -(C₀-C₃alkyl)NR^{a}R^{b}, -(Co-C₃alkyl)OCF₃, -(C₀-C₃alkyl)CF₃, -(C₀-C₃alkyl)NO₂, -(C₀-C₃alkyl)C(O)R^{a}, -(Co-C₃alkyl)C(O)OR^{a}, -(C₀-C₃alkyl)C(O)NR^{a}R^{b}, -(C₀-C₃alkyl)NR^{a}C(O)R^{b}, -(C₀-C₃alkyl)S(O)₁₋₂R^{a}, -(C₀-C₃alkyl)NR^{a}S(O)₁₋₂R^{b}, -(C₀-C₃alkyl)S(O)₁₋₂NR^{a}R^{b}, -(C₀-C₃alkyl)(5-6-membered heteroaryl) or -(C₀-C₃alkyl)phenyl, wherein R¹ is optionally substituted by one or more groups independently selected from the group consisting of halogen, C₁-C₃alkyl, oxo, -CF₃, -(Co-C₃alkyl)OR^{c} and -(C₀-C₃alkyl)NR^{c}R^{d}.

5. The compound of claim 1 or a salt thereof, wherein R¹ is selected from the group consisting of H, methyl,

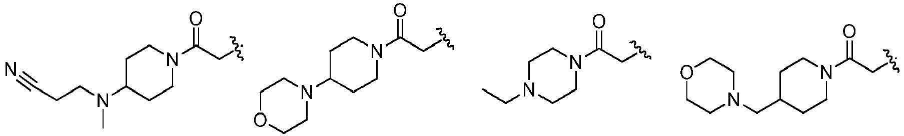

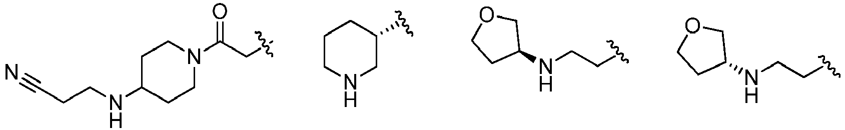

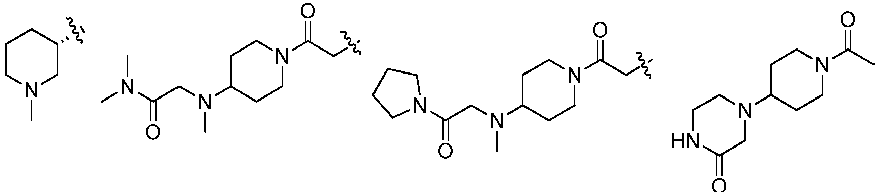

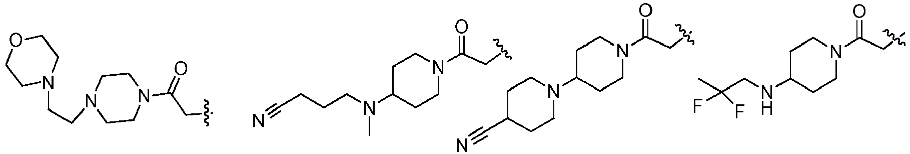

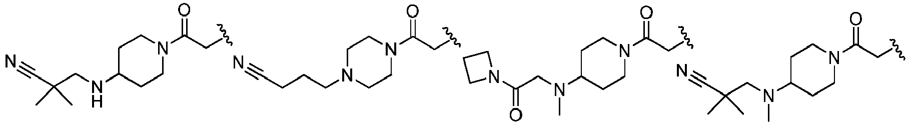

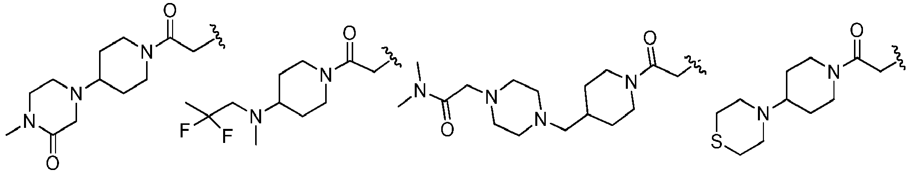

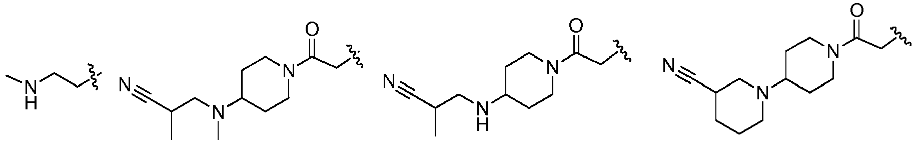

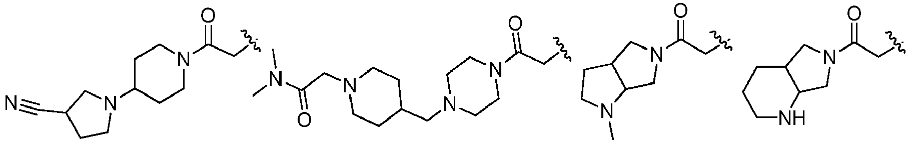

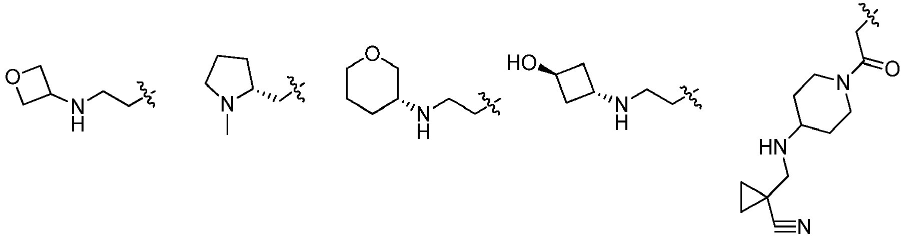

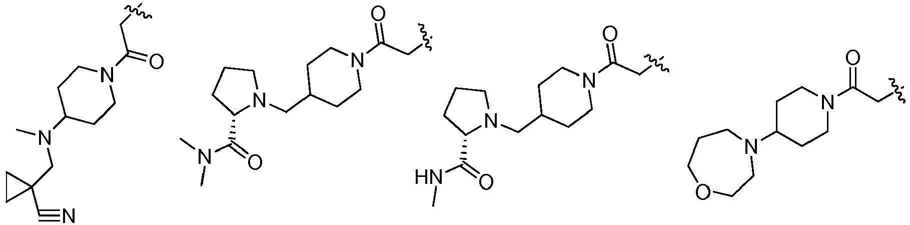

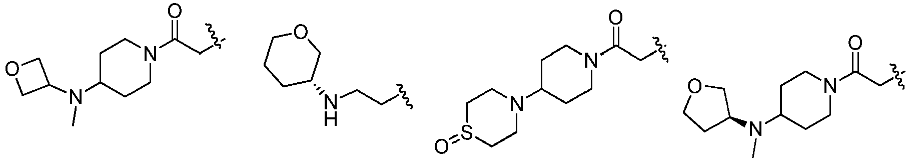

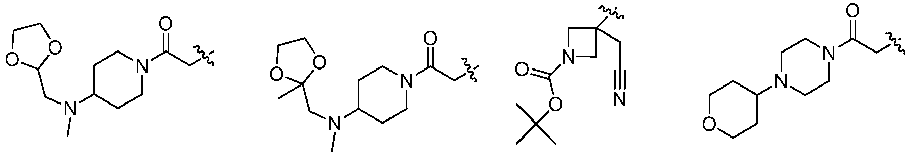

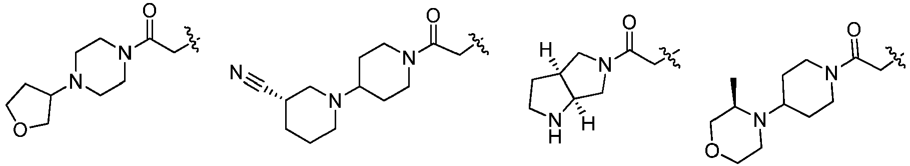

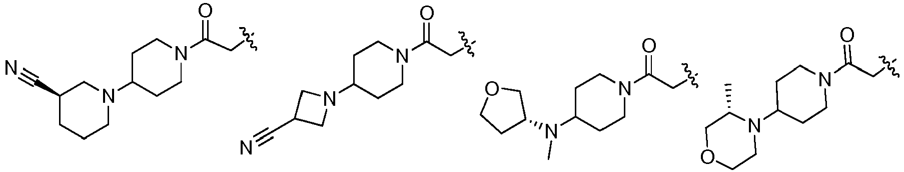

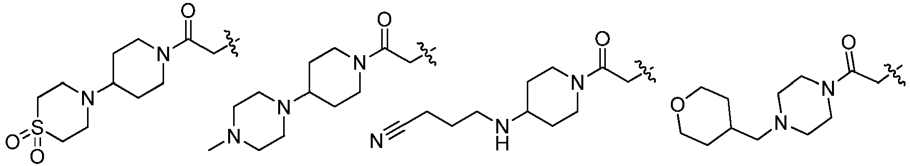

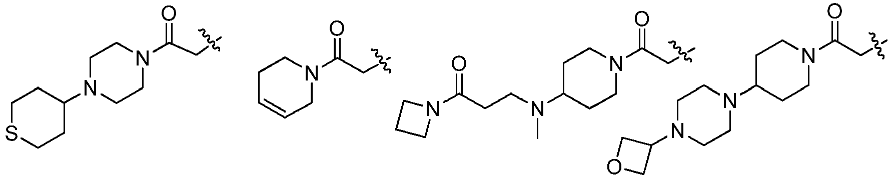

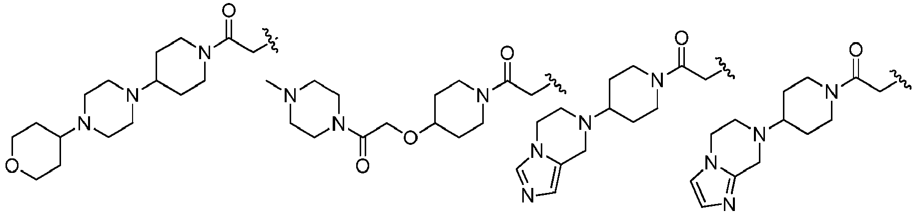

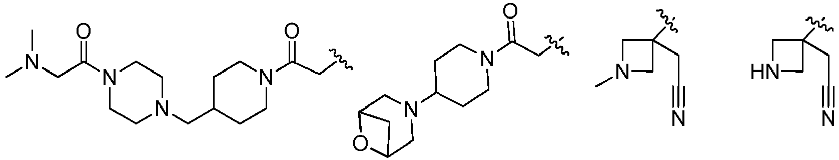

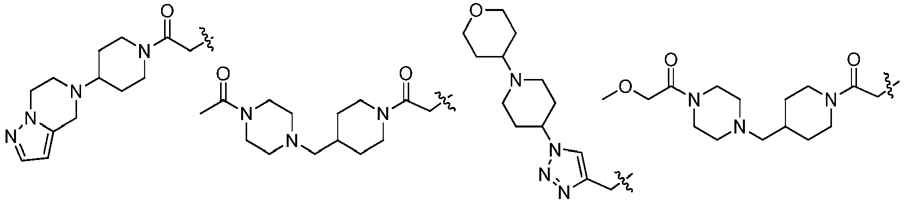

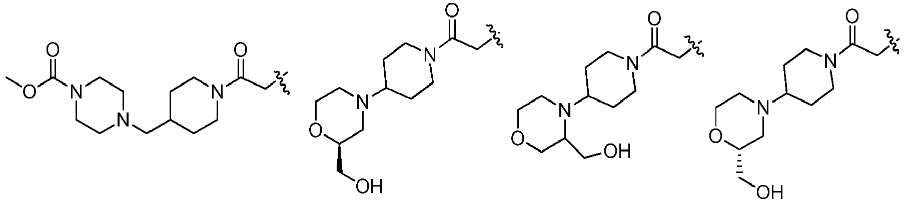

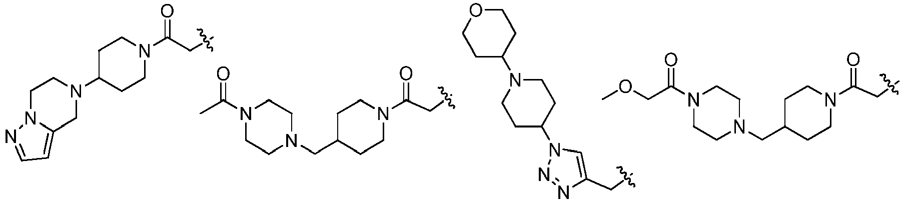

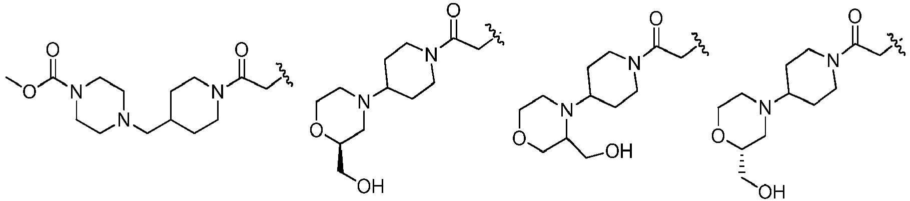

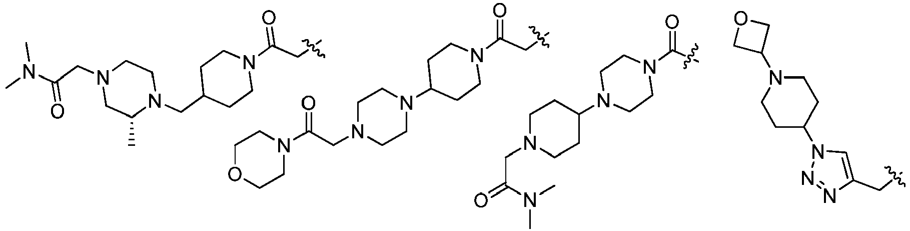

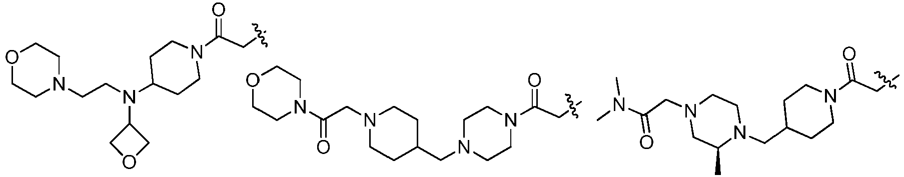

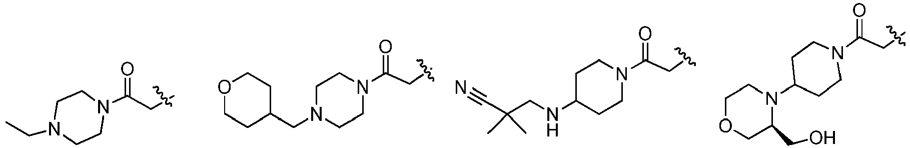

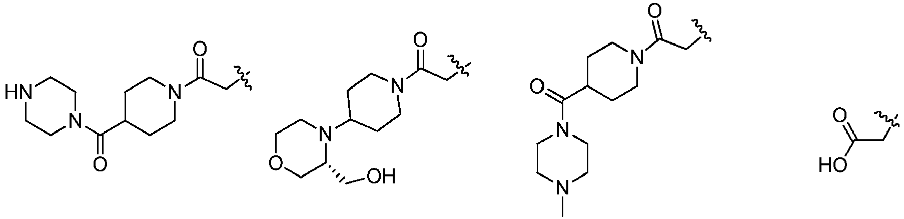

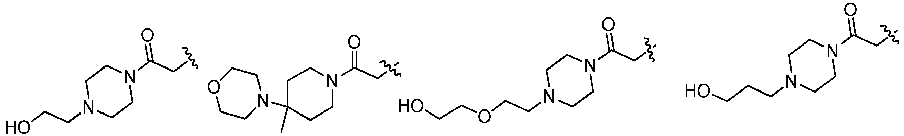

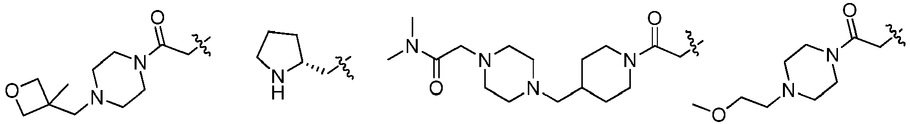

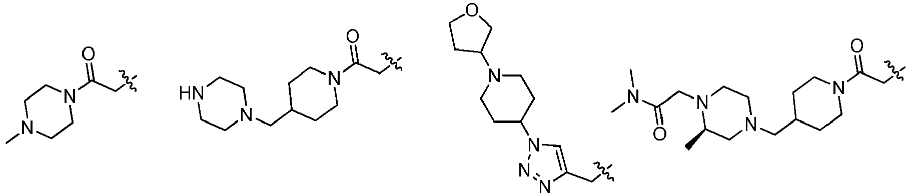

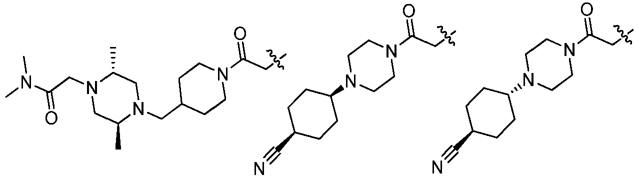

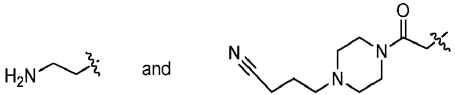

6. The compound of any one of claims 1-5 wherein R² is C₁-C₆alkyl, C₃-C₆ cycloalkyl, or 3-6-membered heterocyclyl, or a salt thereof.

7. The compound of any one of claims 1-5 wherein R² is methyl, ethyl, isopropyl, cyclopropyl, or oxetanyl, or a salt thereof.

8. The compound of any one of claims 1-7 wherein n is 1, or a salt thereof.

9. The compound of any one of claims 1-7 wherein n is 2, or a salt thereof.

10. The compound of claim 1 which is
a) a compound of Formula (00B): 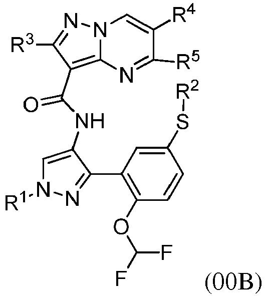 or a salt thereof;
b) a compound of Formula (00C): 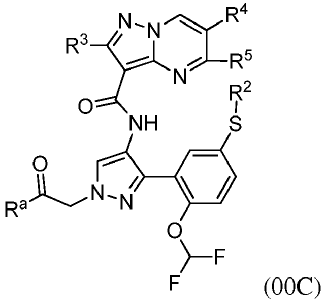 or a salt thereof;
c) a compound of Formula (00D): 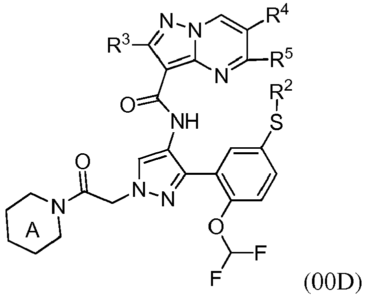 wherein the ring A is optionally substituted with one or more groups R^{e}; or a salt thereof;
d) a compound of Formula (00E): 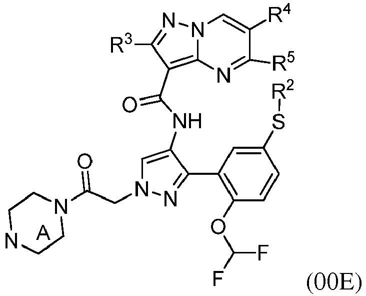 wherein the ring A is optionally substituted with one or more groups R^{e}; or a salt thereof;
e) a compound of Formula (00F): 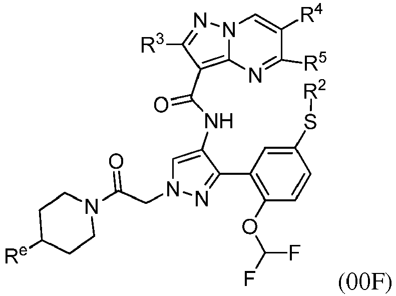 or a salt thereof: or
f) a compound of Formula (00G): 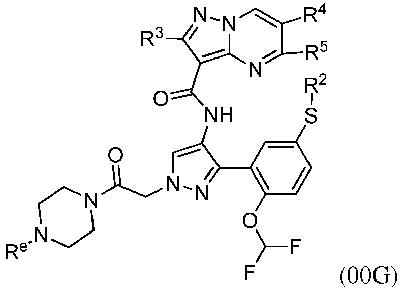 or a salt thereof.

11. The compound of claim 10 wherein R² is C₁-C₆alkyl, C₃-C₆ cycloalkyl, or 3-6-membered heterocyclyl, or a salt thereof.

12. The compound of claim 10 wherein R² is methyl, ethyl, isopropyl, cyclopropyl, or oxetanyl, or a salt thereof.

13. The compound of claim 10-12 wherein R^{e} is selected from the group consisting of methyl, ethyl,

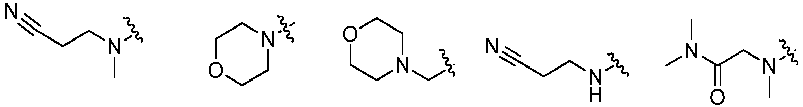

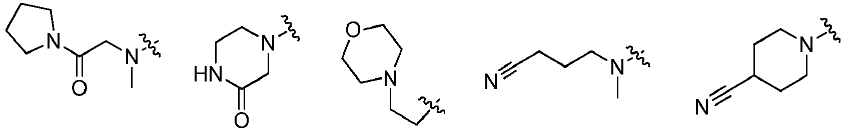

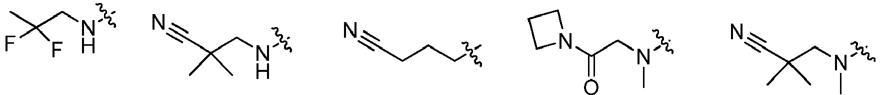

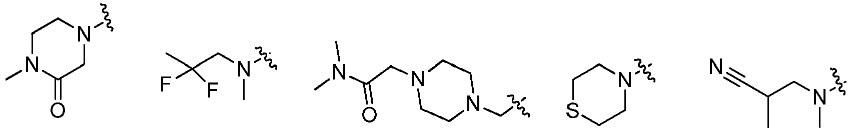

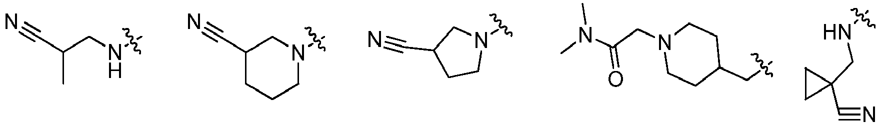

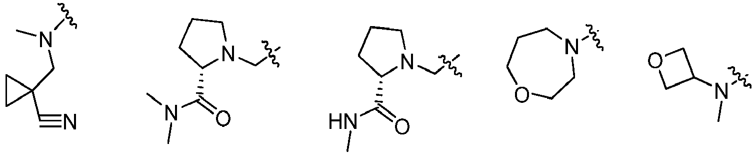

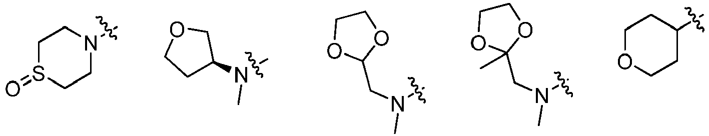

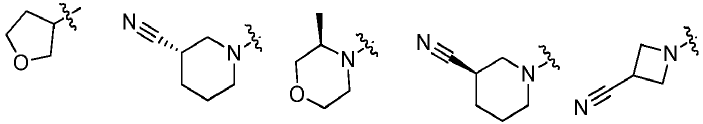

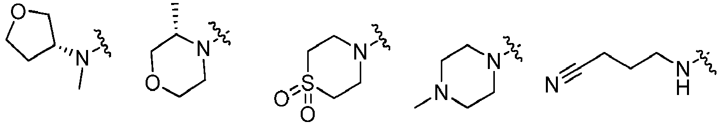

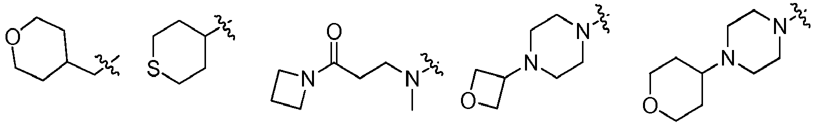

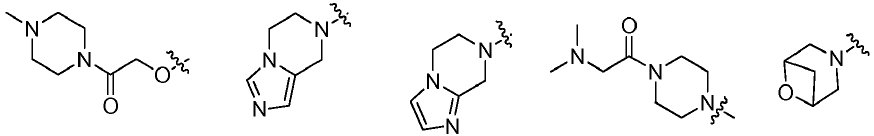

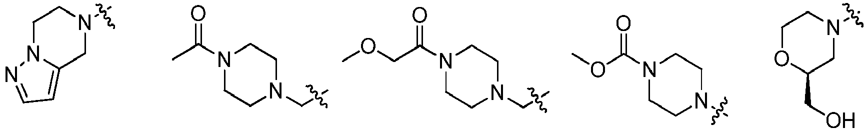

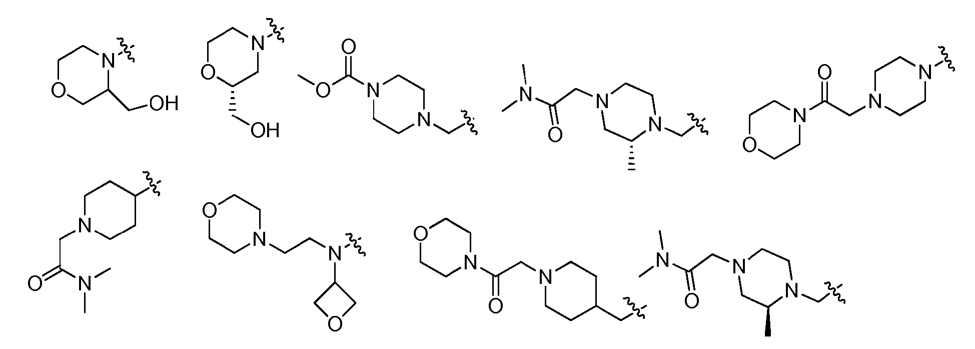

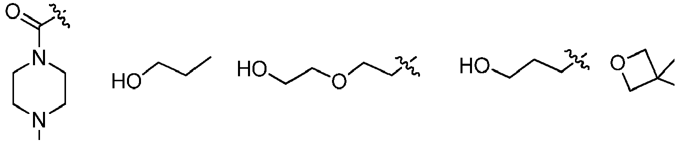

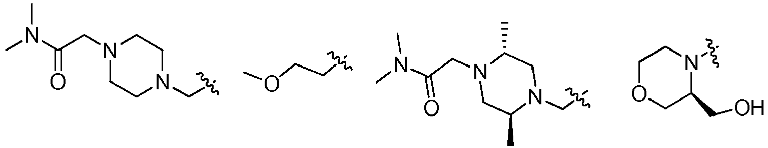

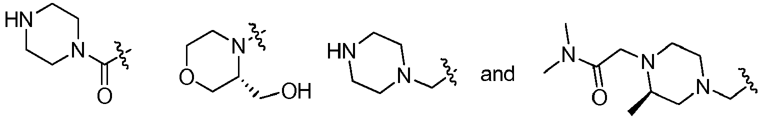

14. A compound according to claim 1 selected from: 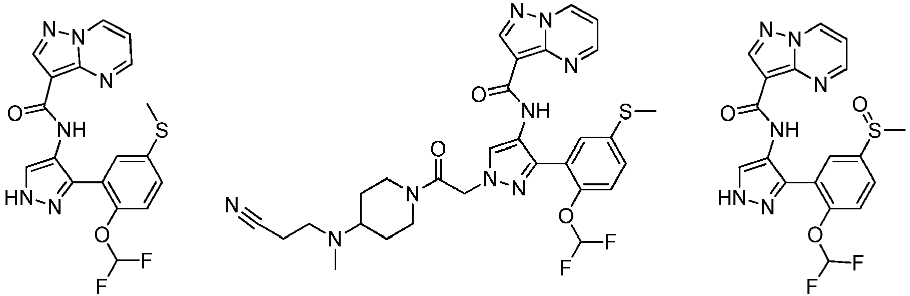 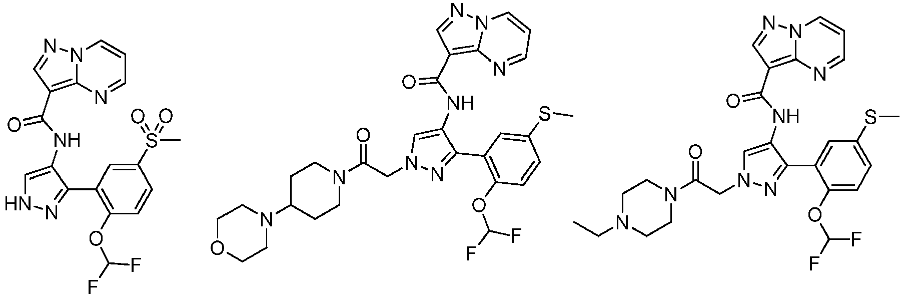 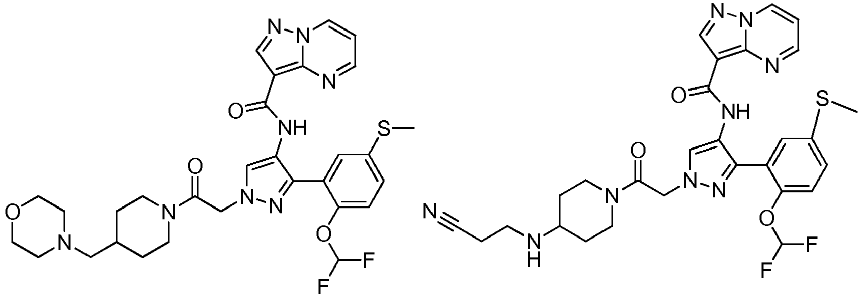 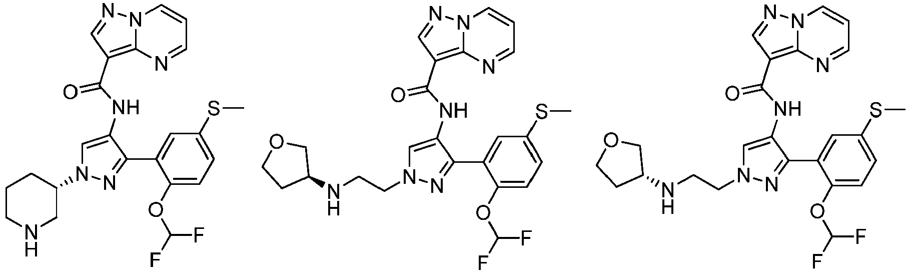 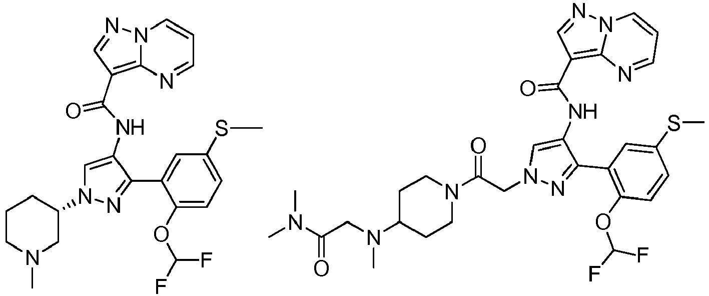 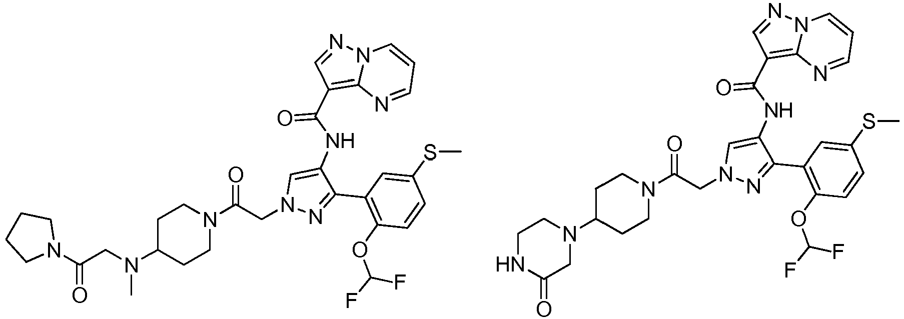 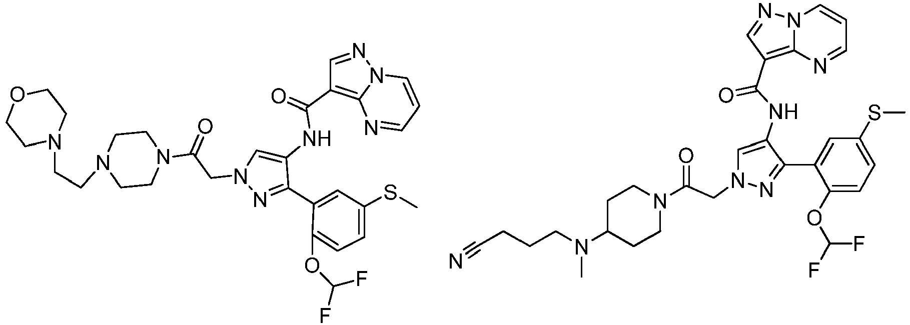 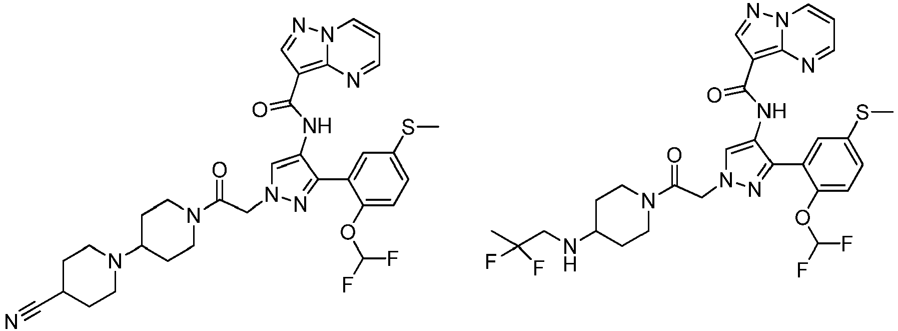 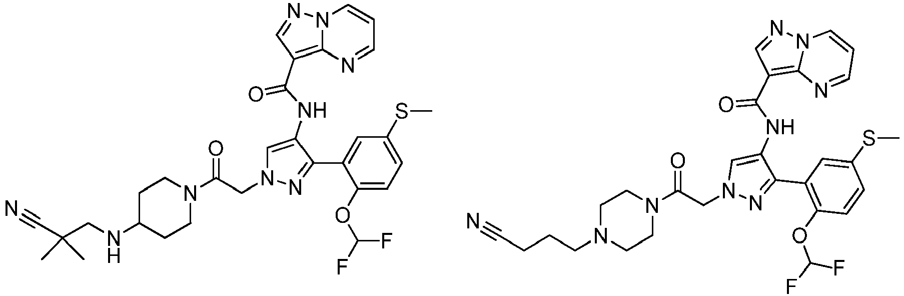 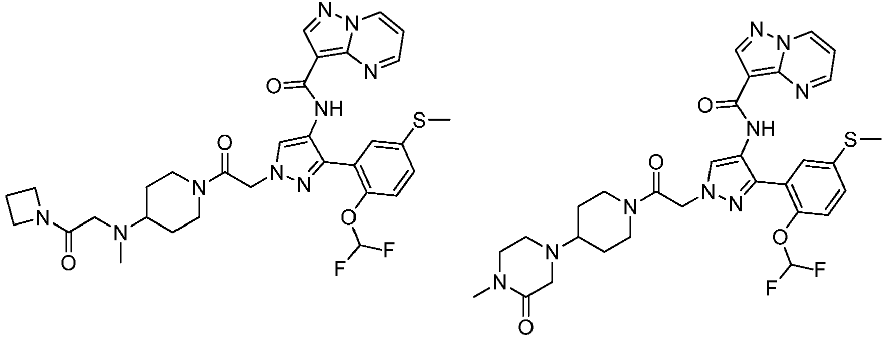 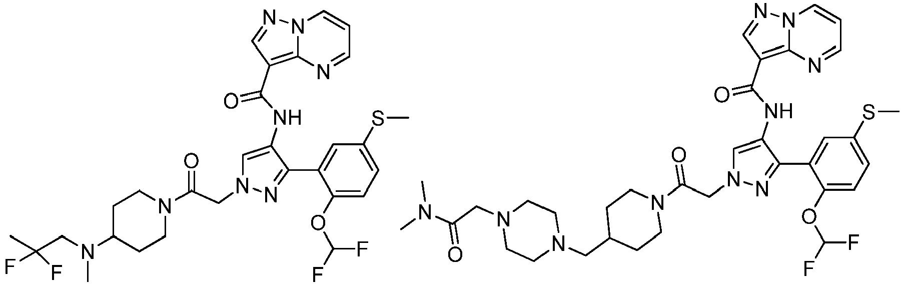 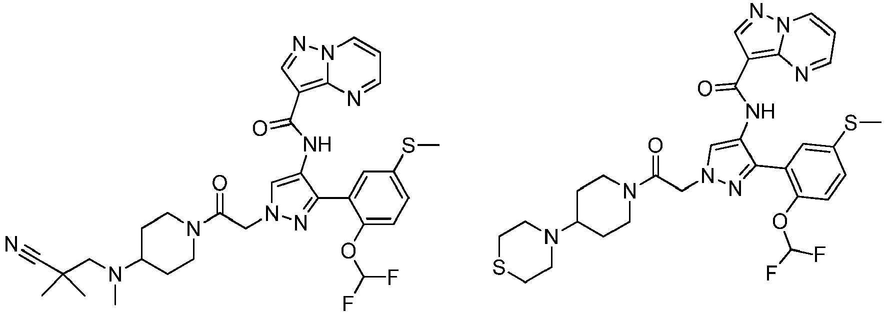 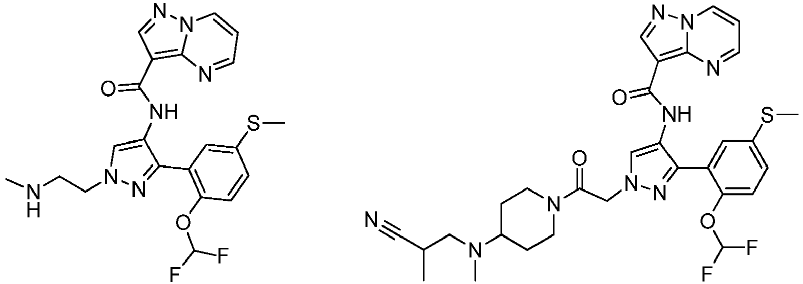 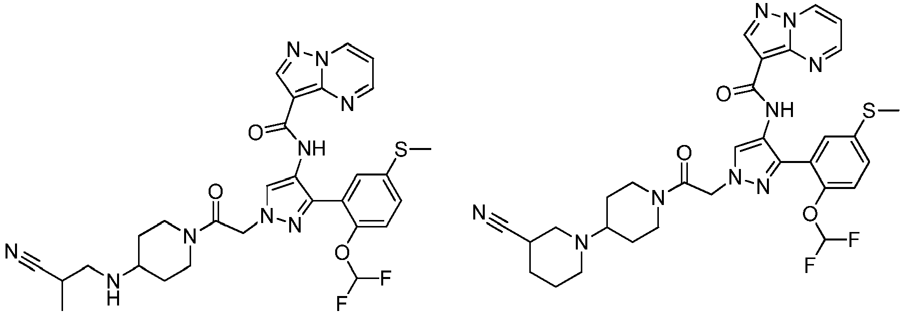 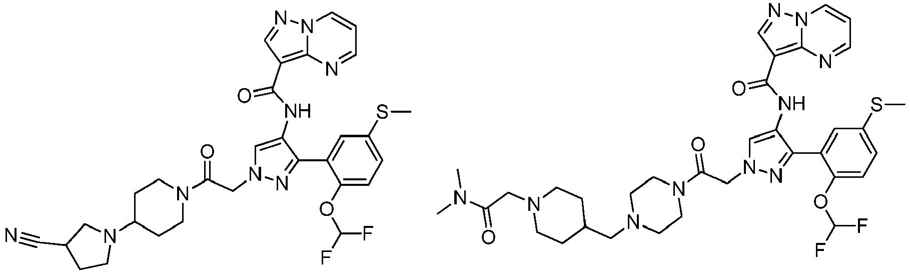 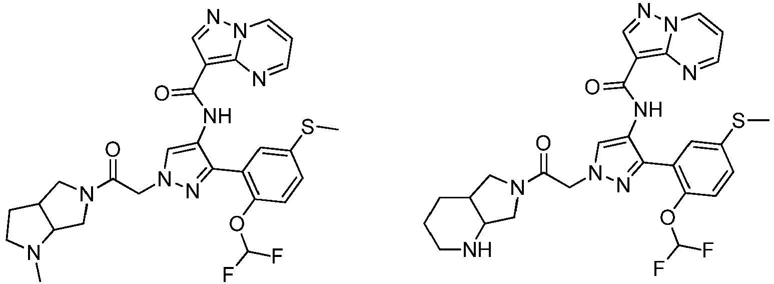 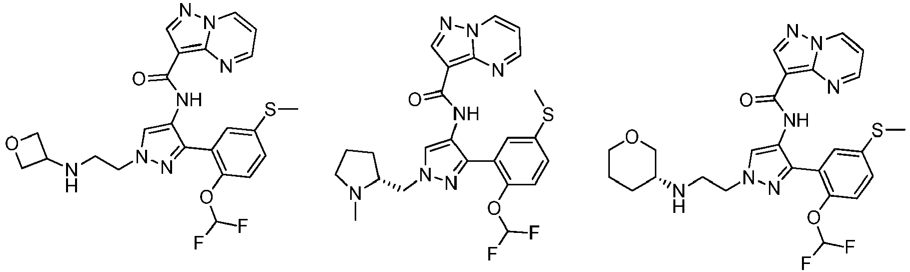 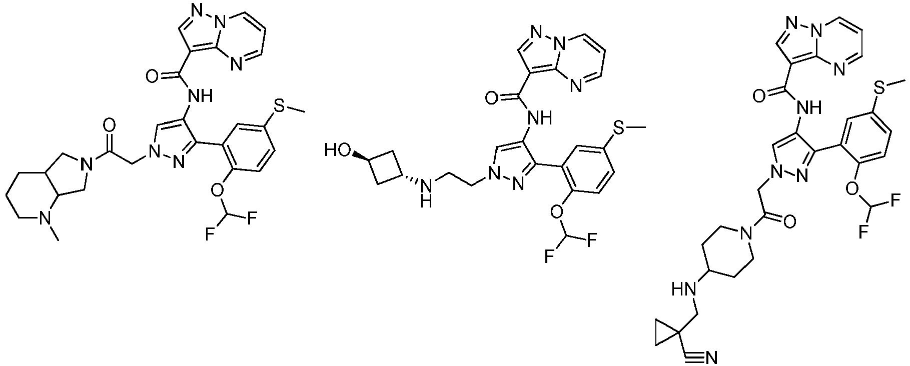 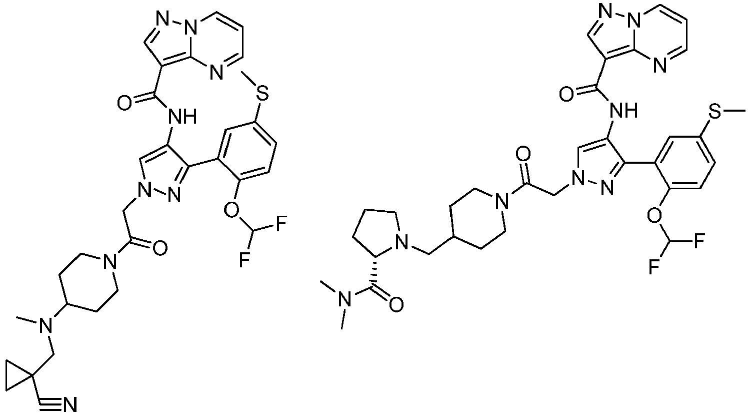 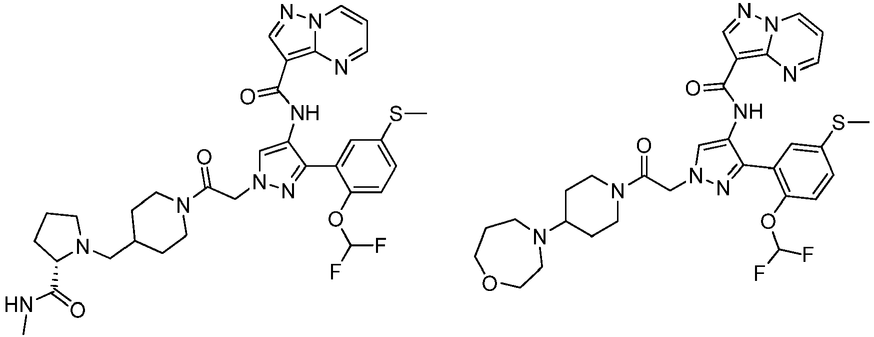 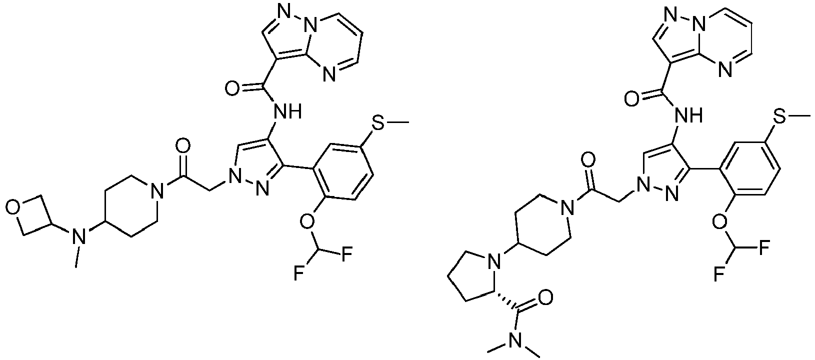 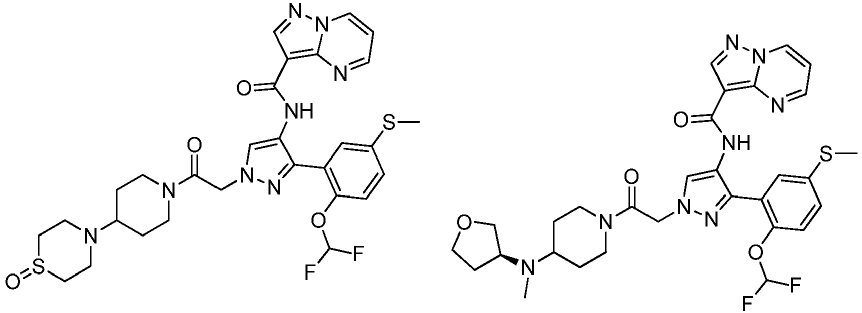 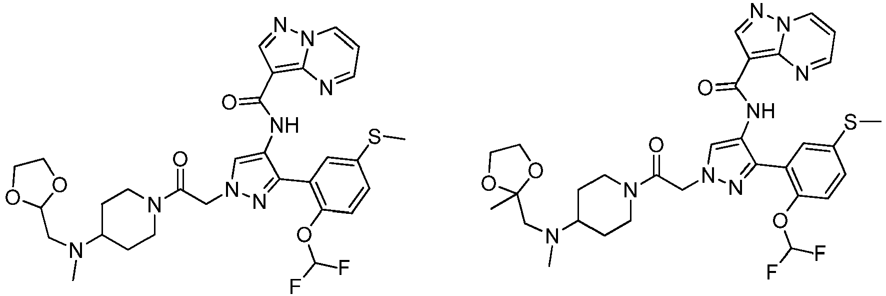 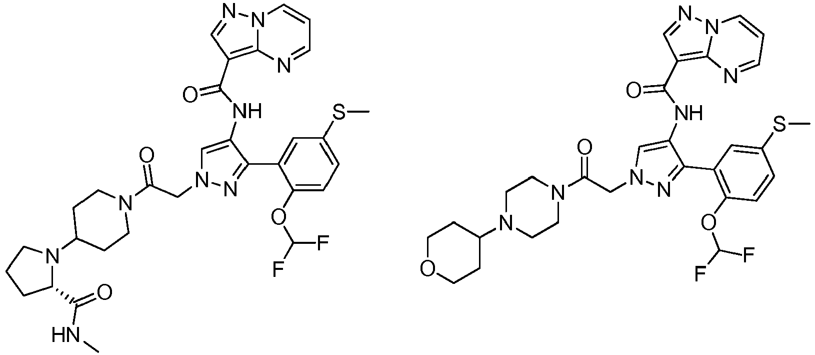 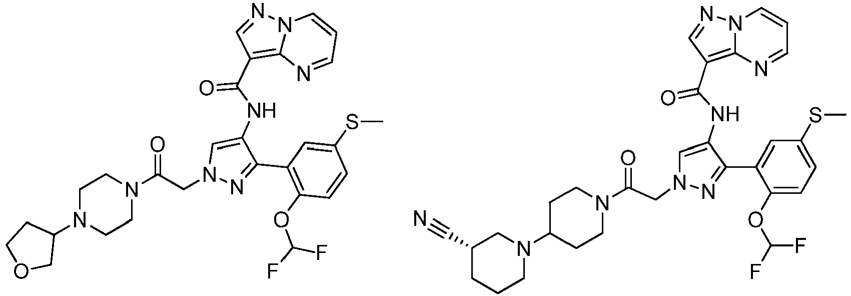 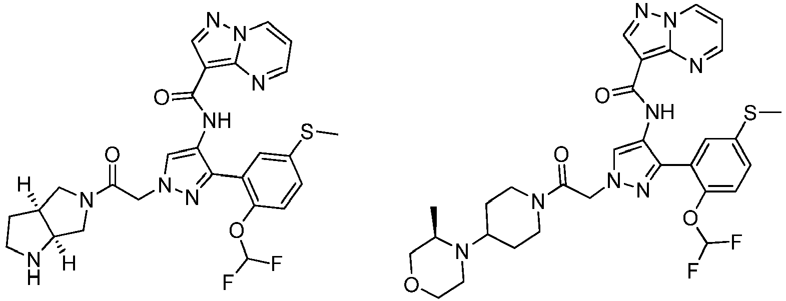 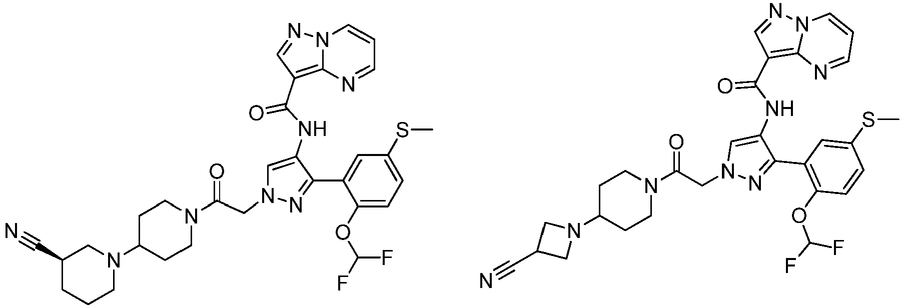 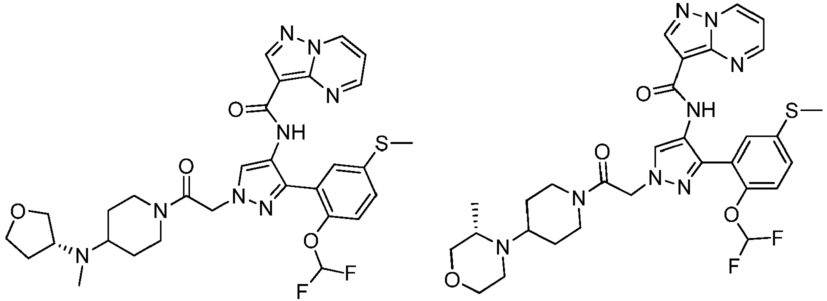 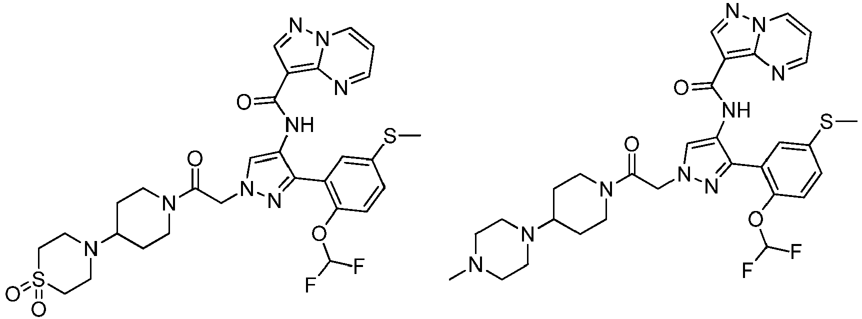 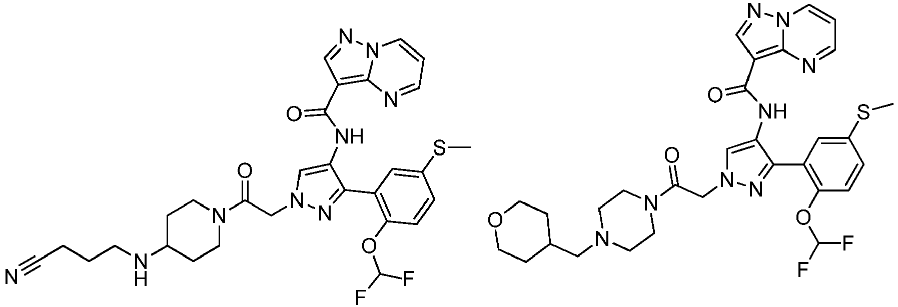 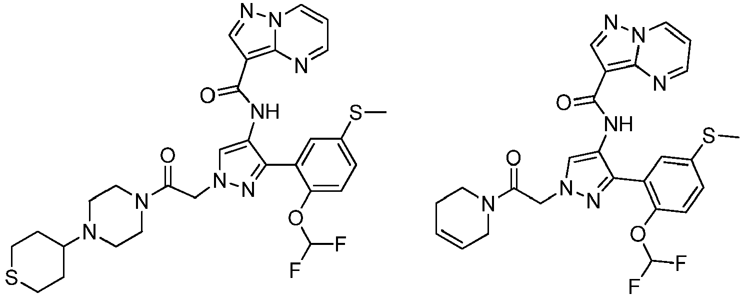 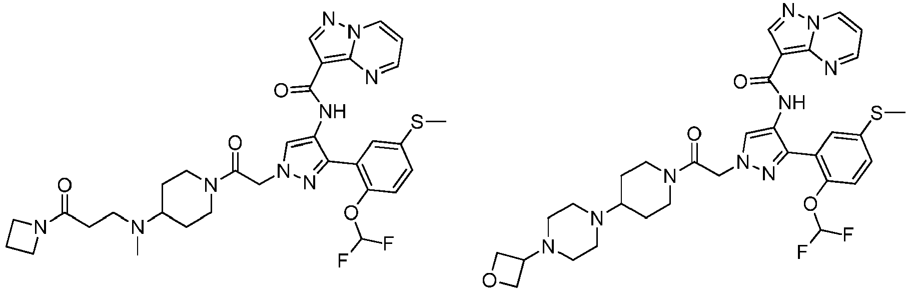 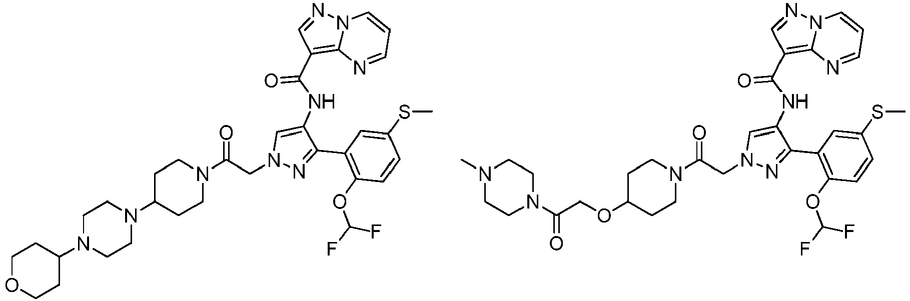 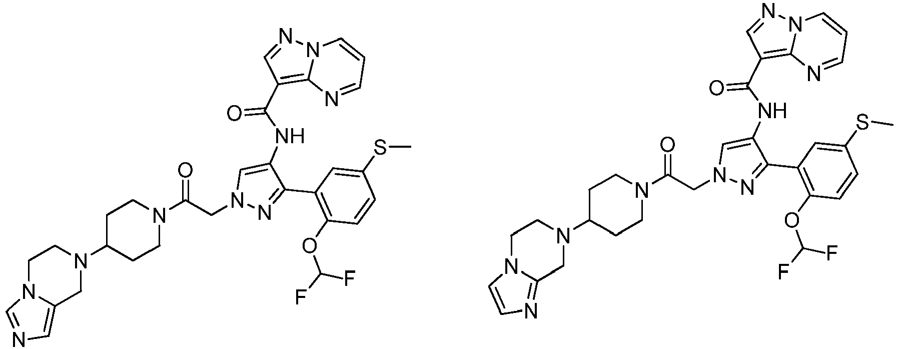 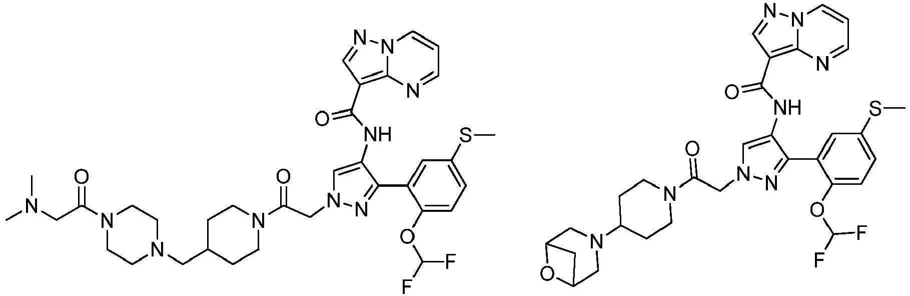 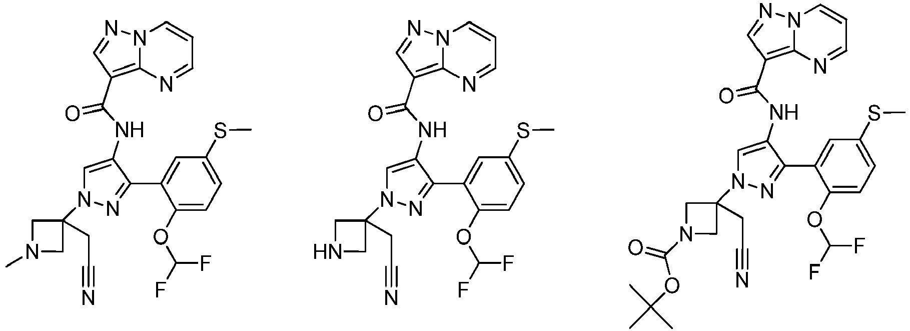 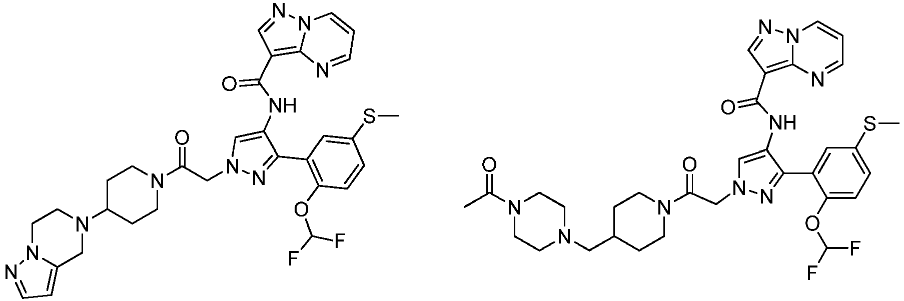 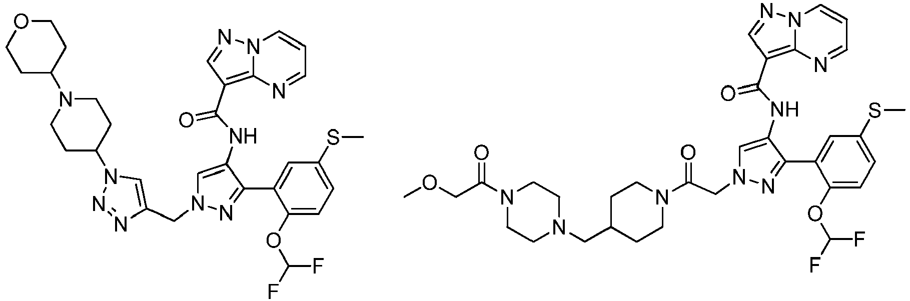 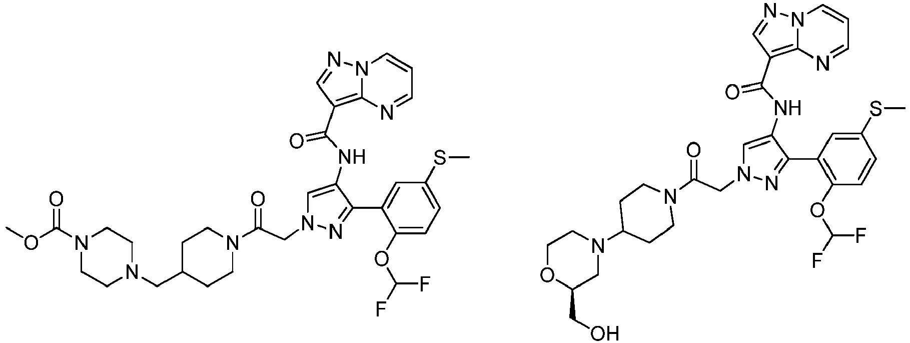 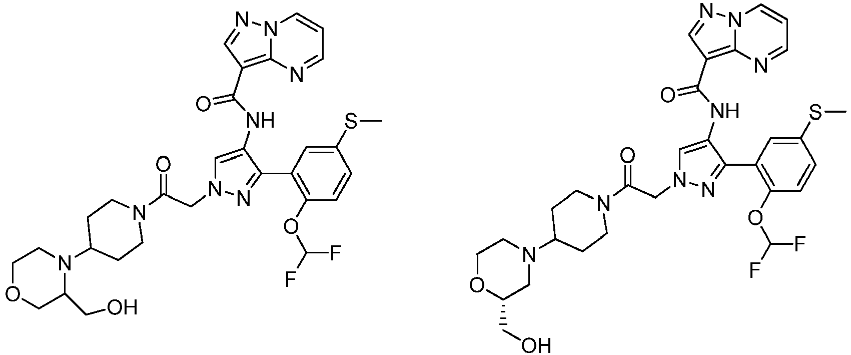 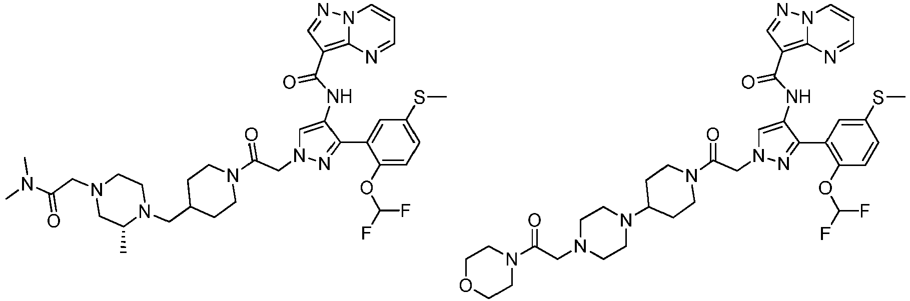 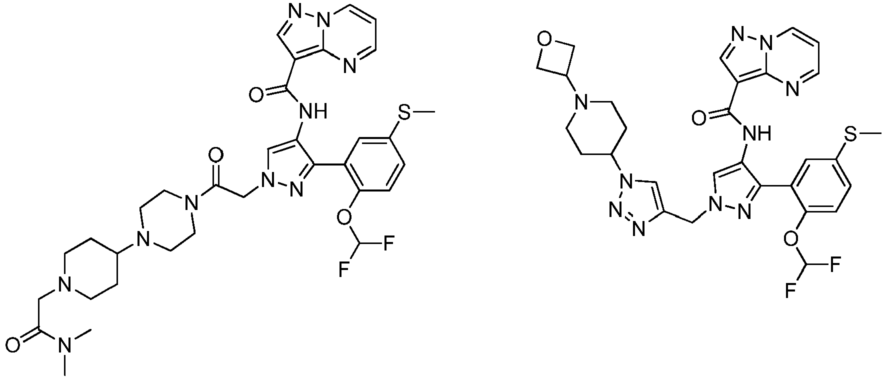 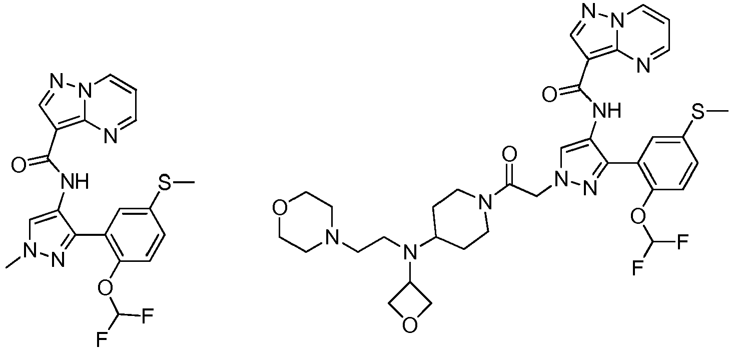 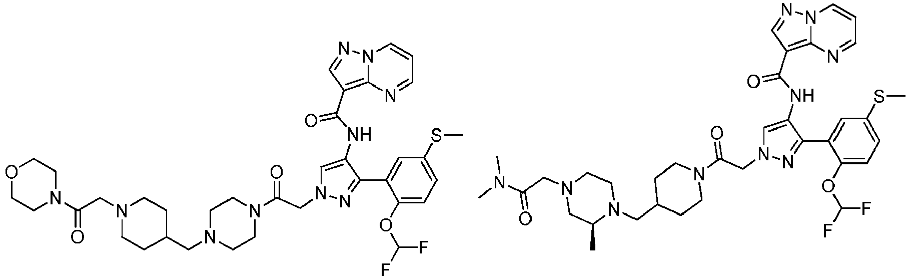 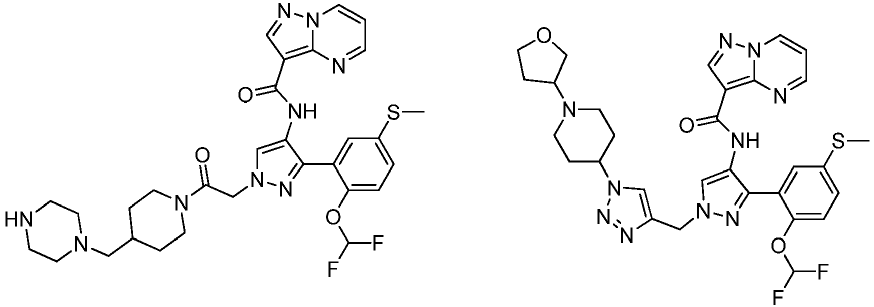 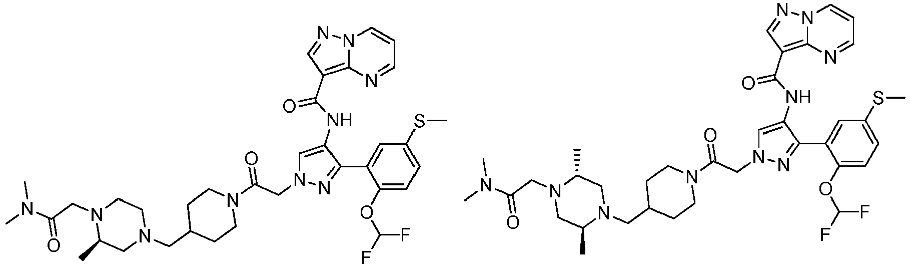 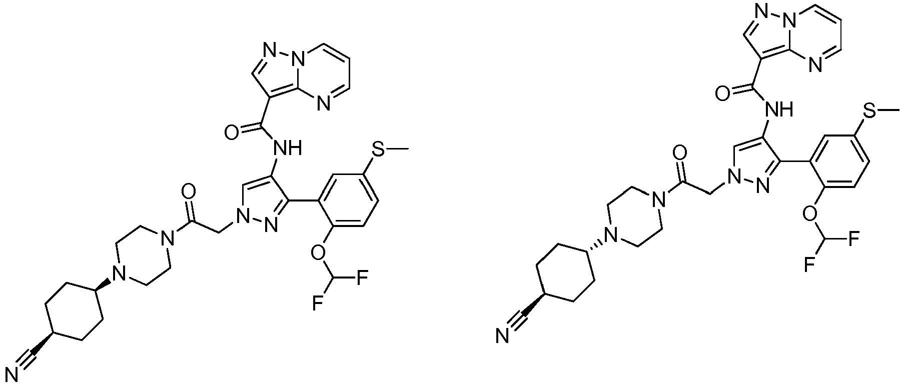 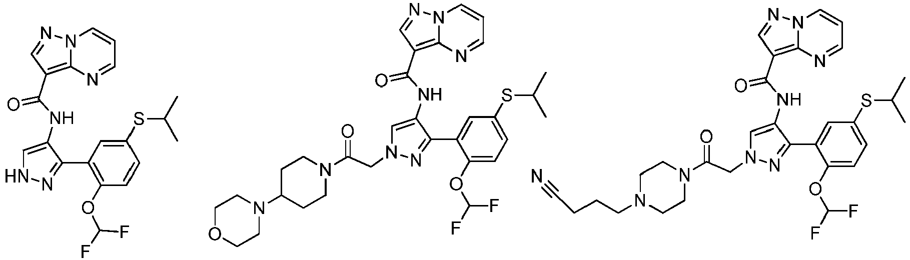 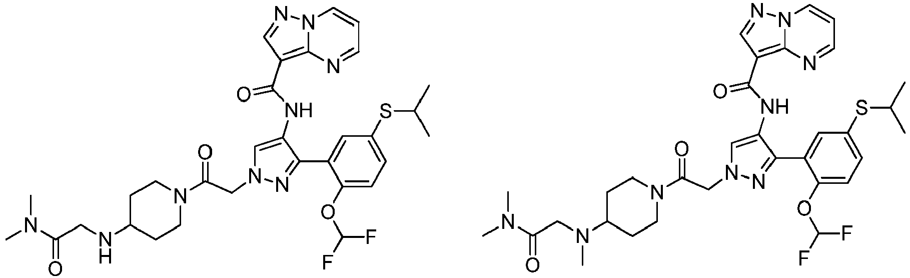 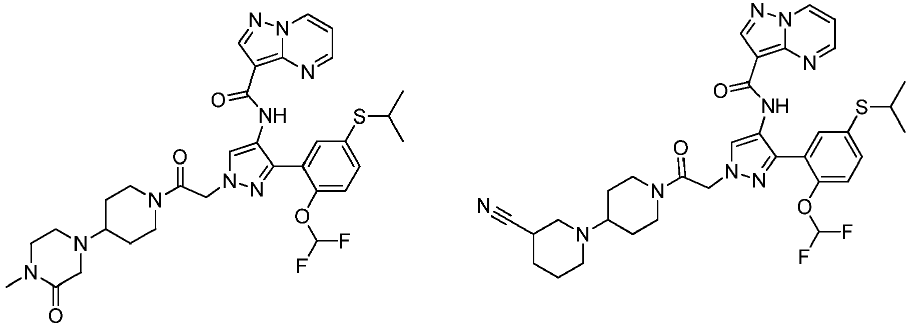 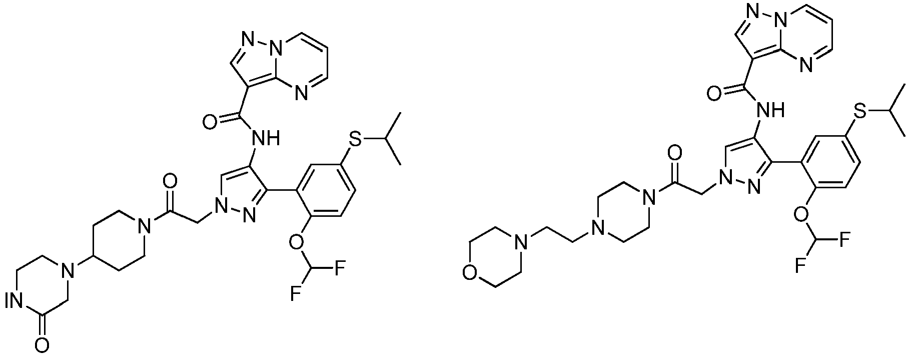 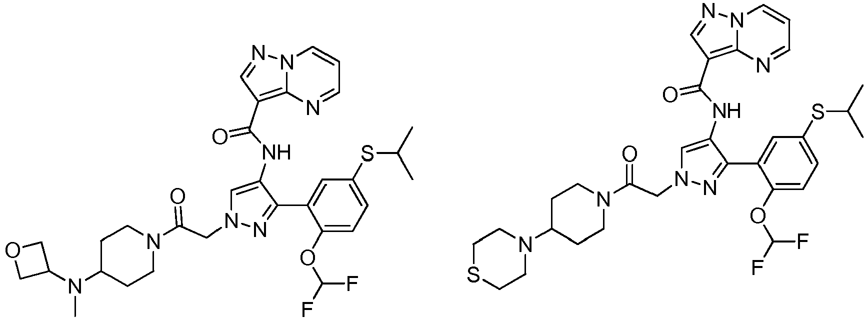 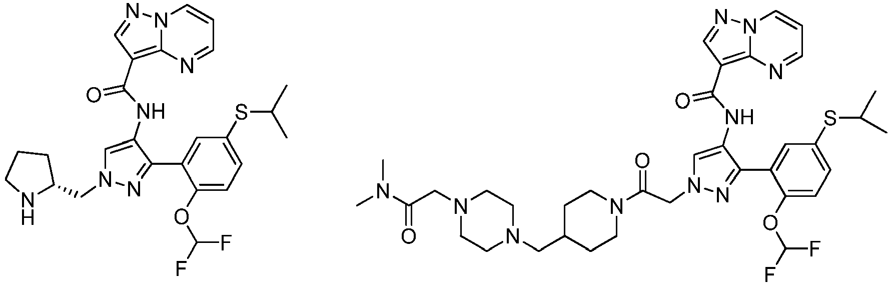 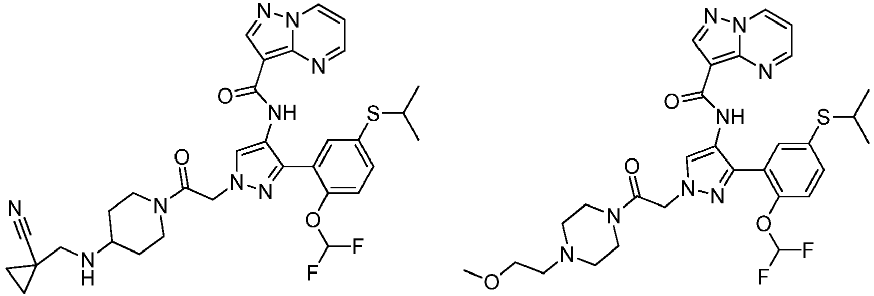 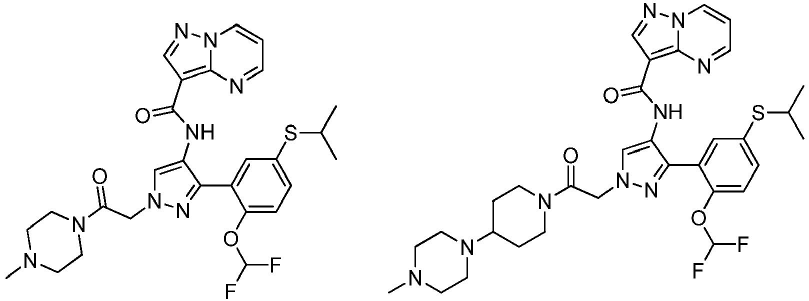 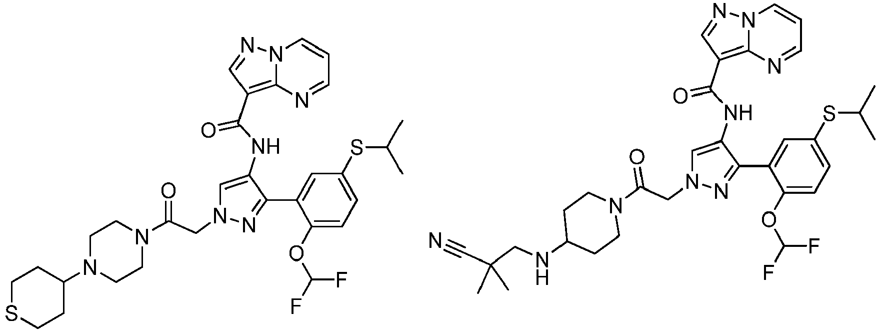 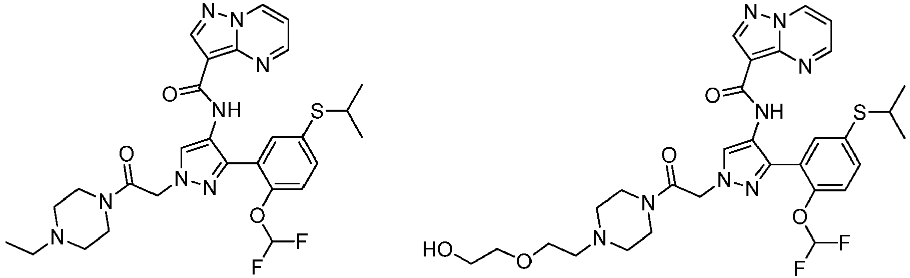 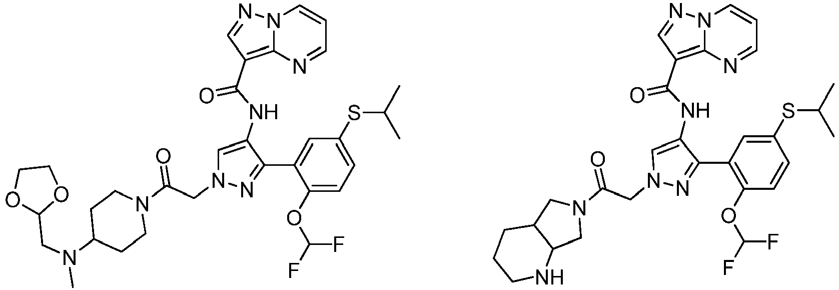 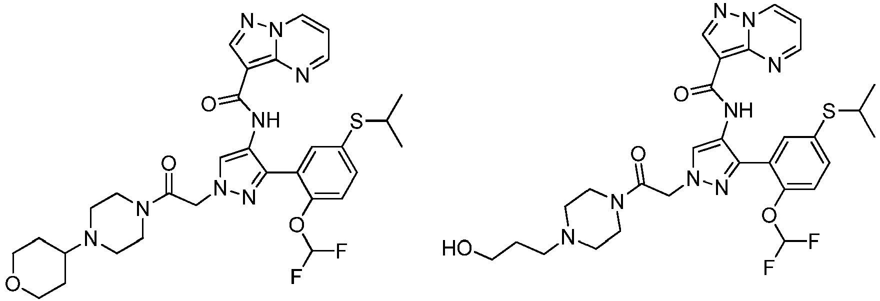 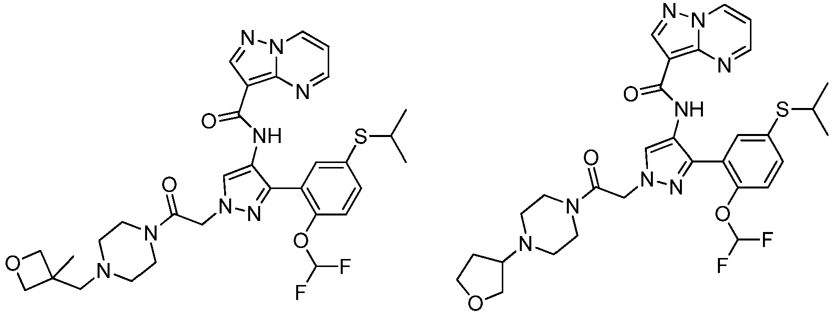 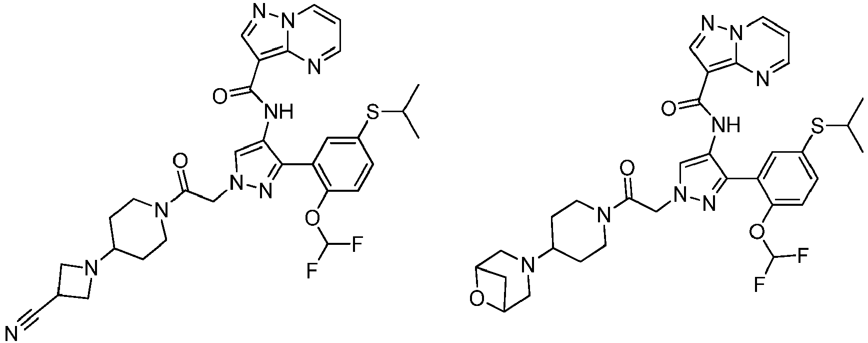 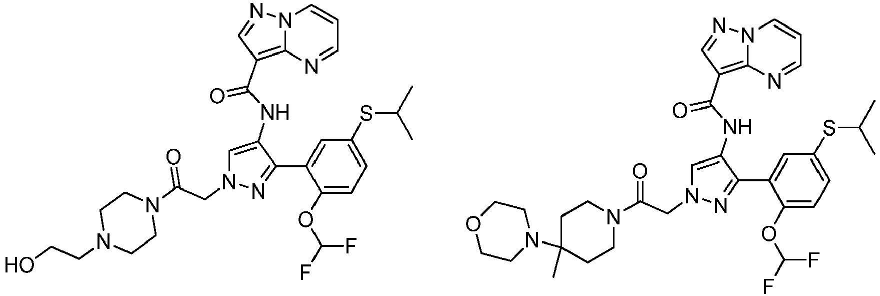 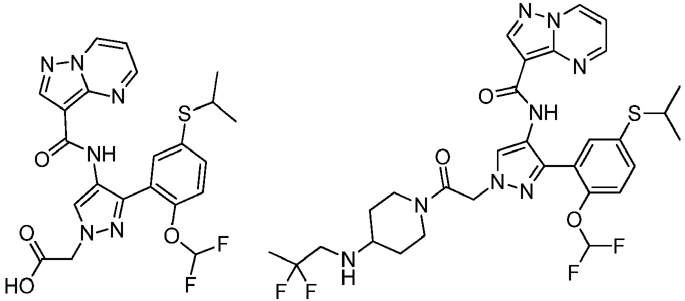 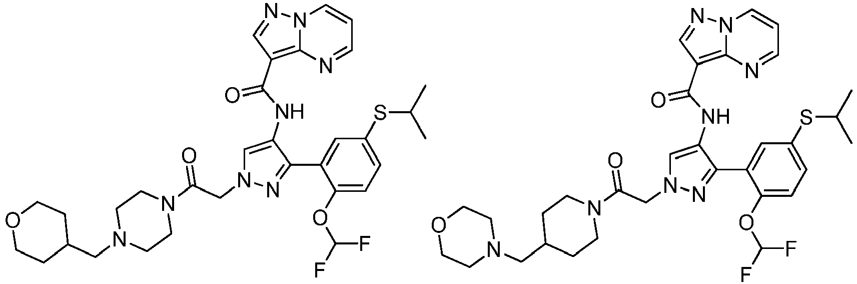 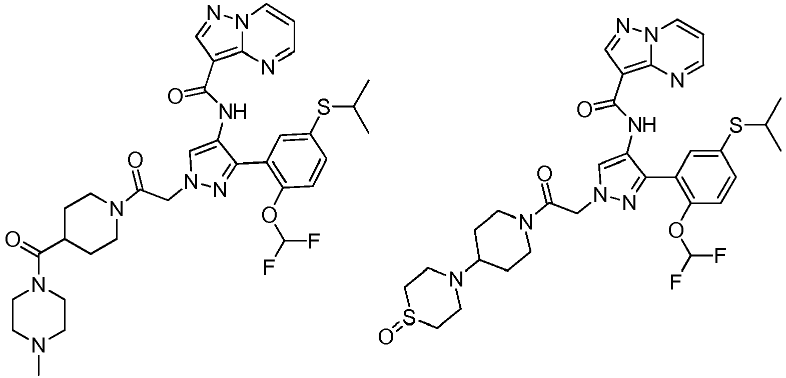 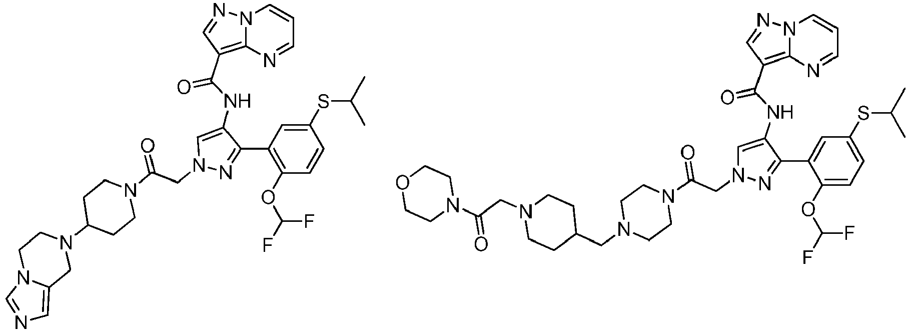 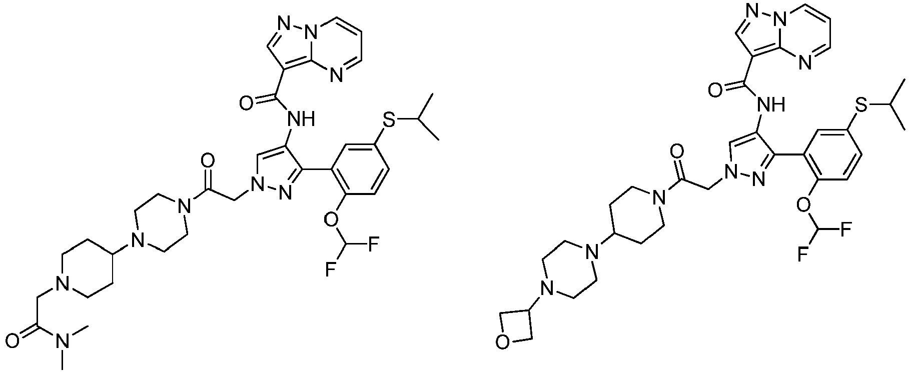 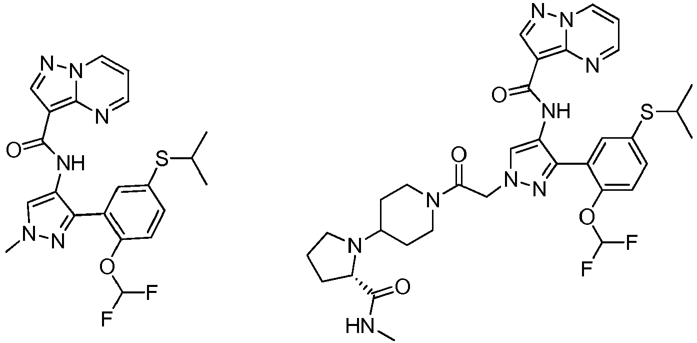 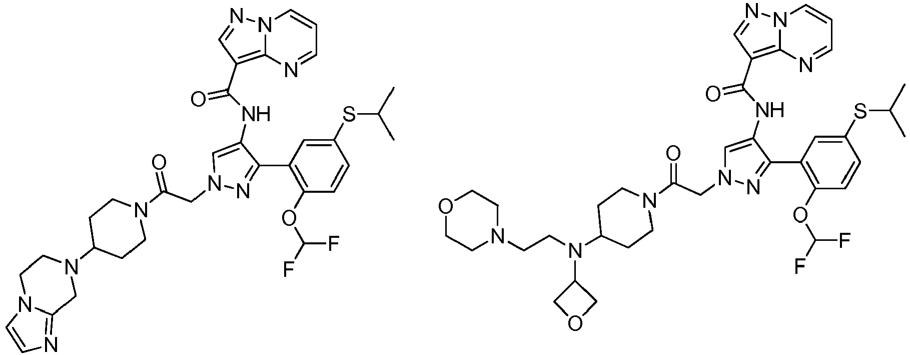 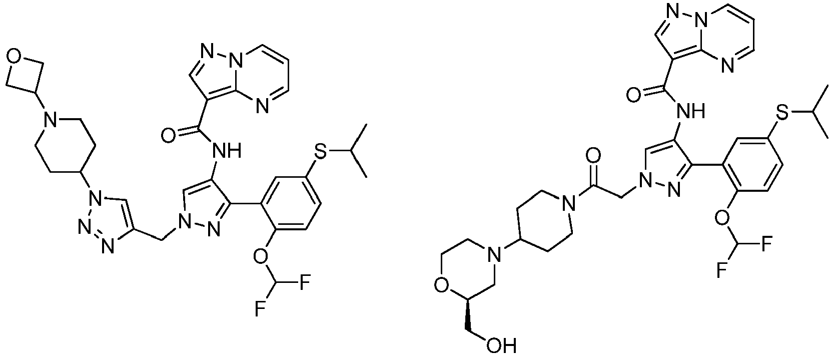 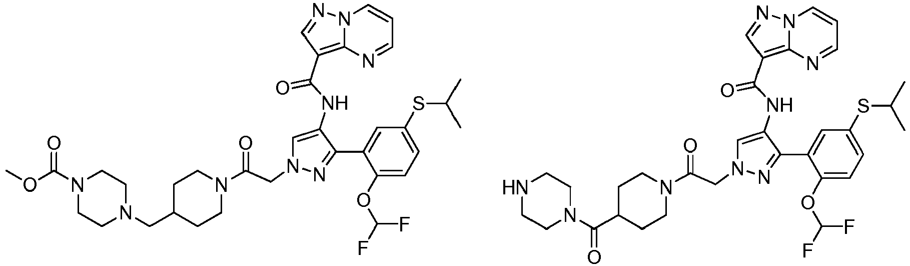 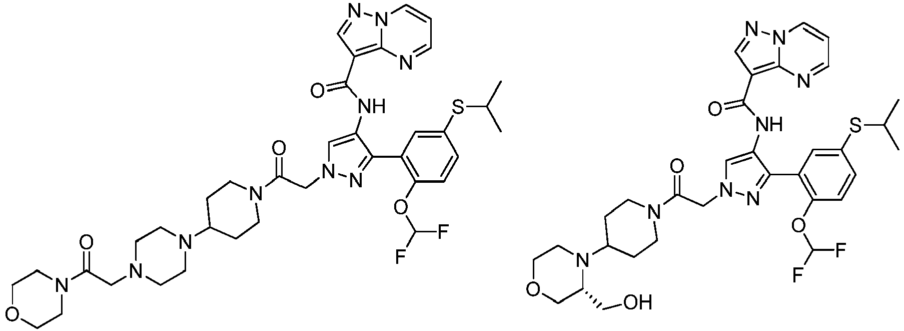 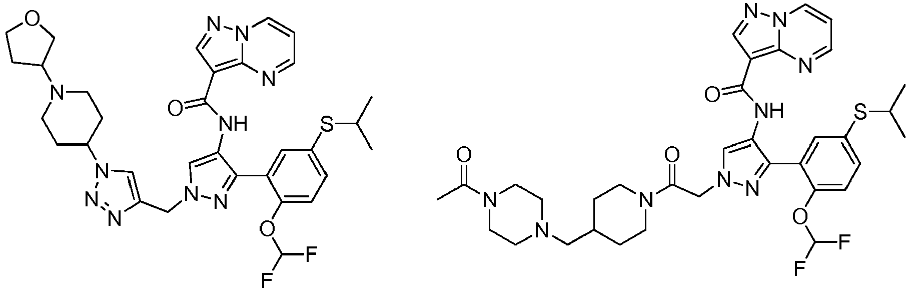 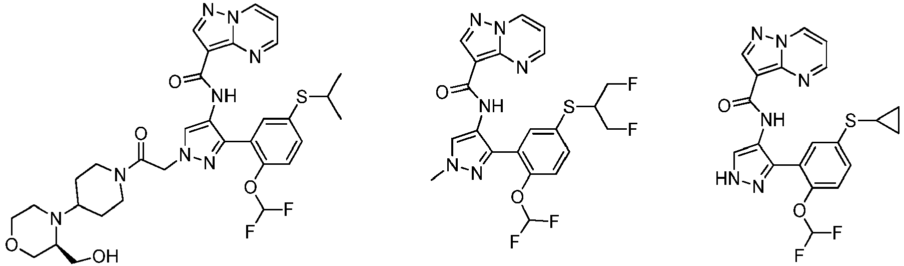 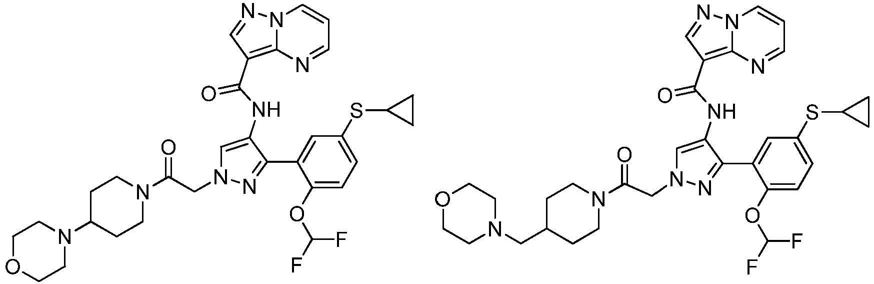 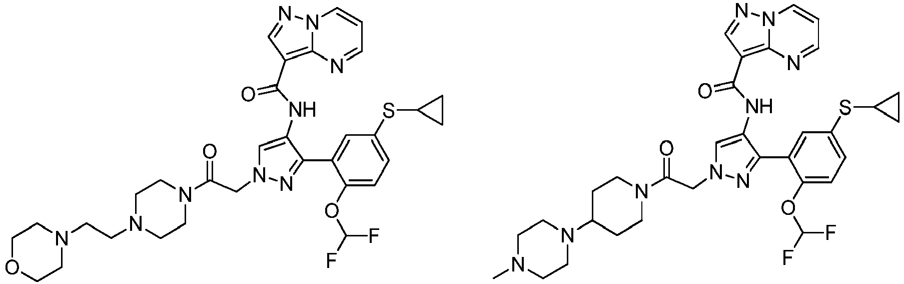 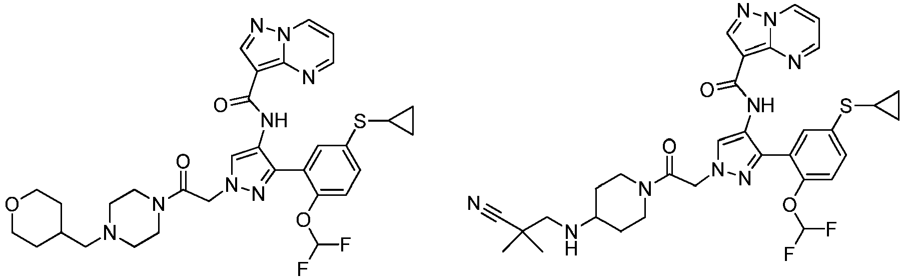 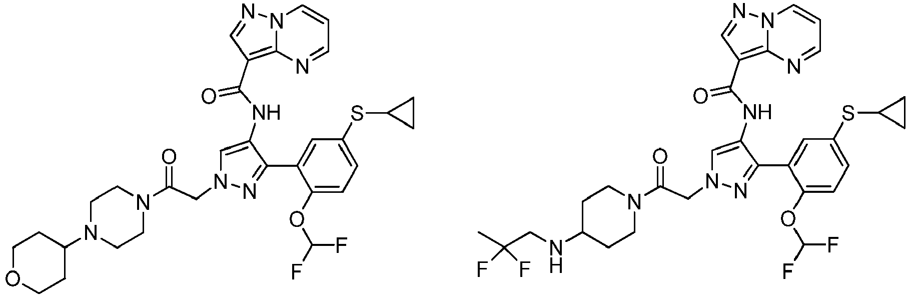 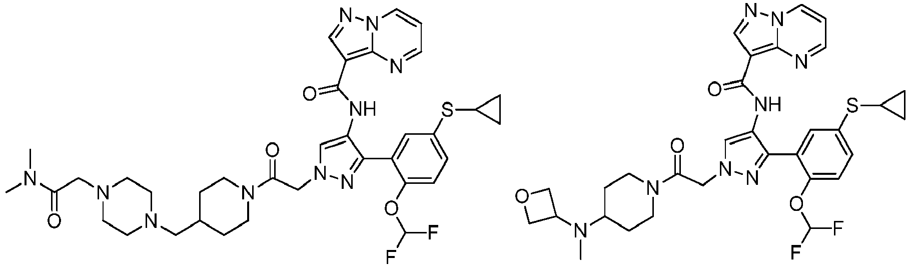 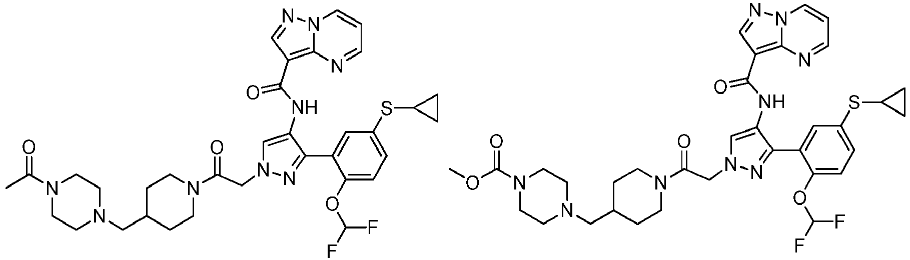 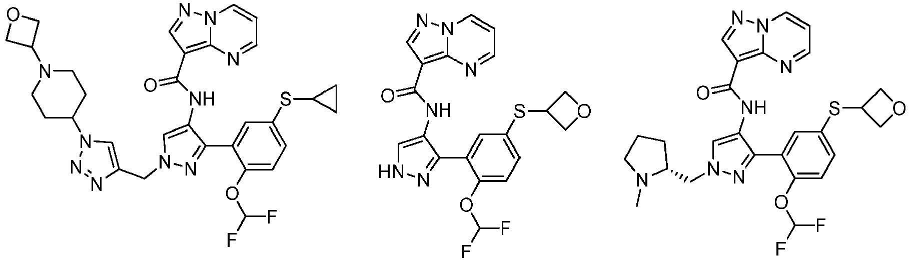 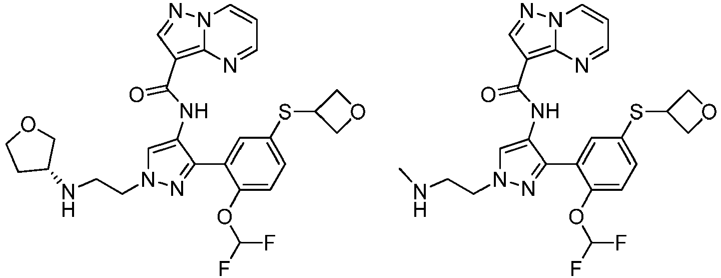 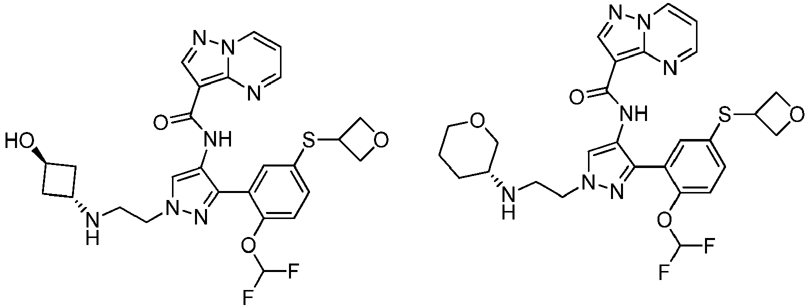 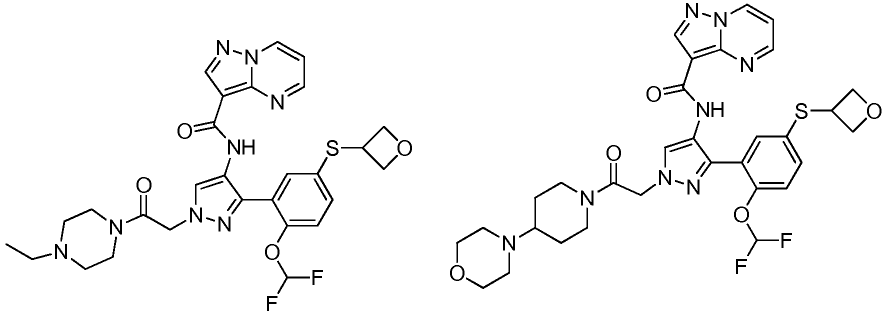 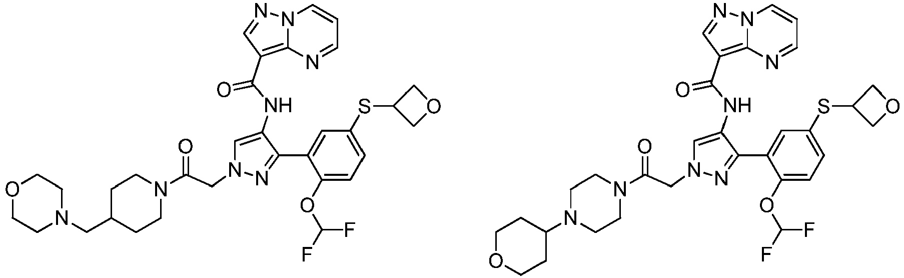 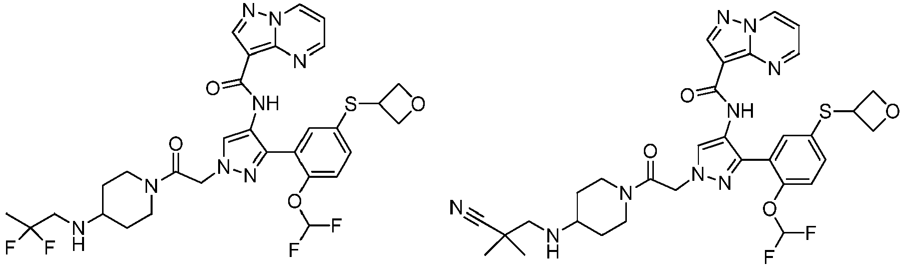 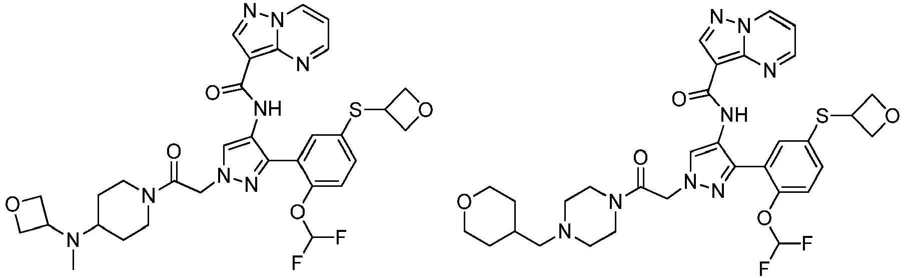 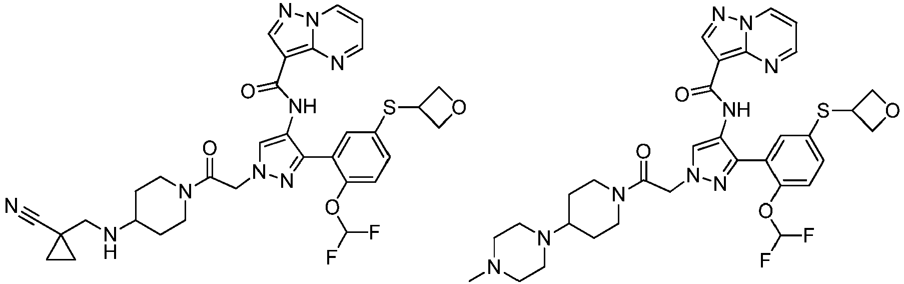 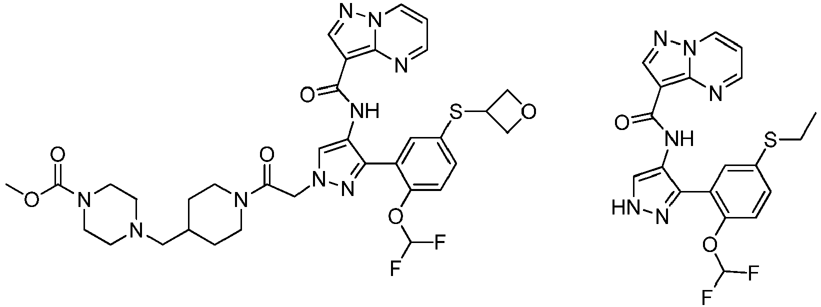 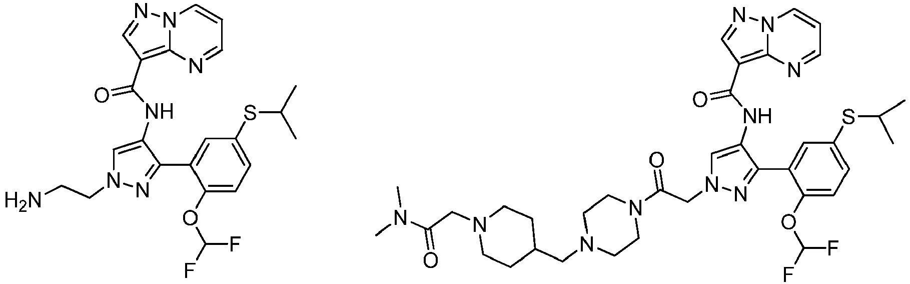 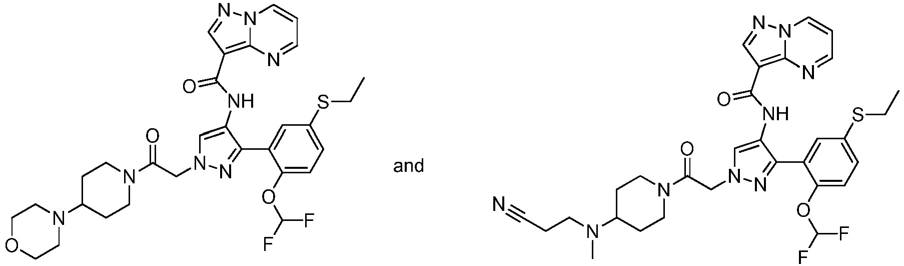 or a salt thereof.

15. A compound according to claim 1 selected from: 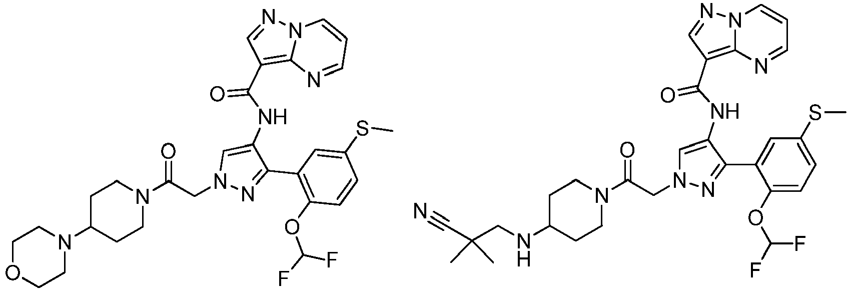 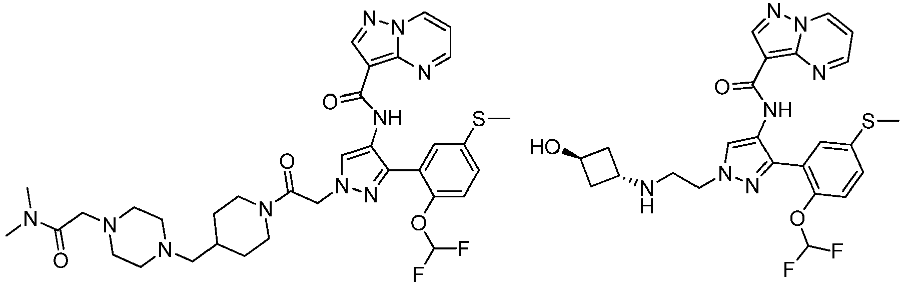 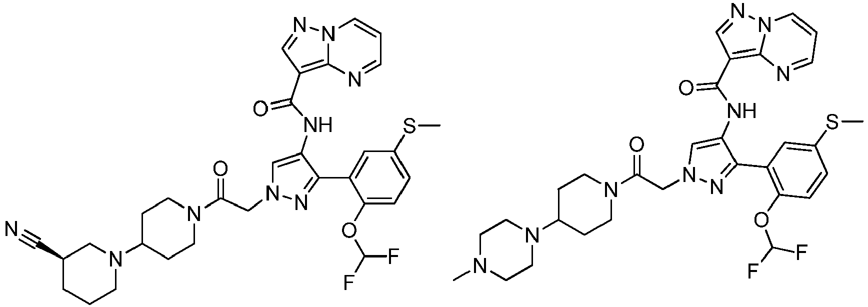 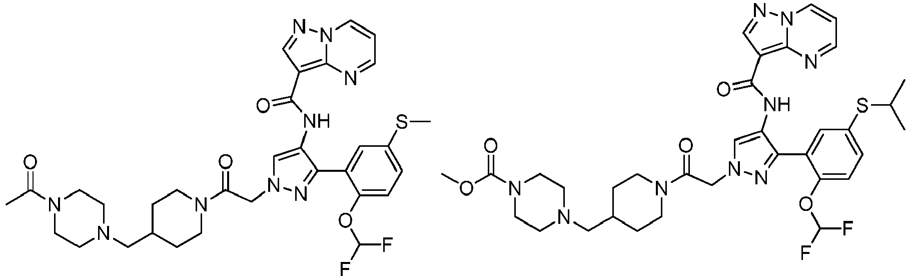 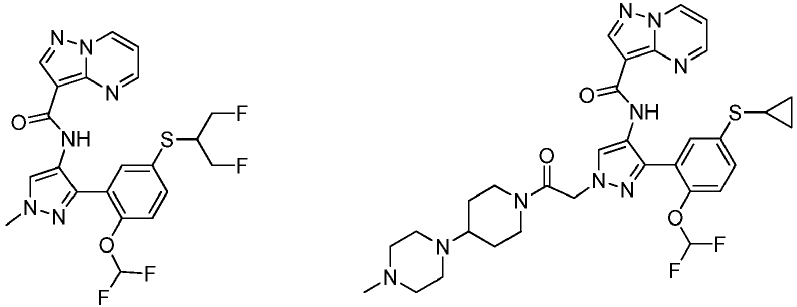 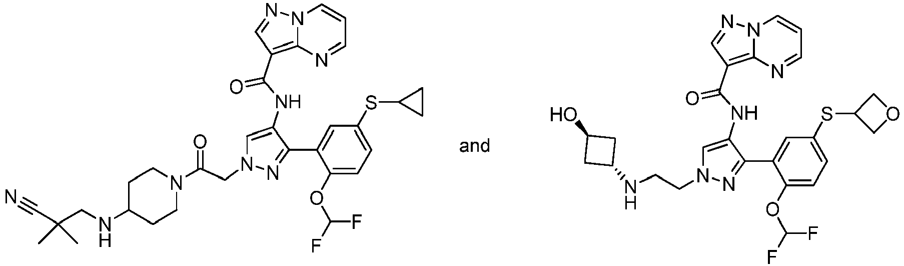 or a salt thereof.

16. A pharmaceutical composition comprising a compound of any of claims 1-15 or a salt thereof, and a pharmaceutically acceptable carrier, diluent or excipient.

17. A compound of any of claims 1-15 or a pharmaceutically acceptable salt thereof, for use in the treatment of an inflammatory disease.

## Patentansprüche

1. Verbindung der Formel (00A): 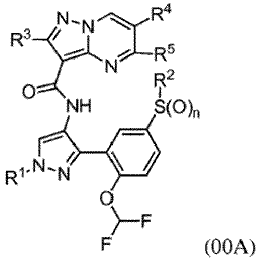 oder ein Salz davon, wobei:
R¹ H, C₁-C₆-Alkyl, C₂-C₆-Alkenyl, C₂-C₆-Alkinyl, -(C₀-C₃-Alkyl)CN, -(C₀-C₃-Alkyl)OR^{a}, -(C₀-C₃-Alkyl)R^{a}, -(C₀-C₃-Alkyl)SR^{a}, -(C₀-C₃-Alkyl)NR^{a}R^{b}, -(C₀-C₃-Alkyl)OCF₃, -(C₀-C₃-Alkyl)CF₃, -(C₀-C₃-Alkyl)NO₂, -(C₀-C₃-Alkyl)C(O)R^{a}, -(C₀-C₃-Alkyl)C(O)OR^{a}, -(C₀-C₃-Alkyl)C(O)NR^{a}R^{b}, -(C₀-C₃-Alkyl)NR^{a}C(O)R^{b}, -(C₀-C₃-Alkyl)S(O)₁₋₂R^{a}, -(C₀-C₃-Alkyl)NR^{a}S(O)₁₋₂R^{b}, -(C₀-C₃-Alkyl)S(O)₁₋₂NR^{a}R^{b}, -(C₀-C₃-Alkyl)(5-6-gliedriges Heteroaryl) oder -(C₀-C₃-Alkyl)phenyl ist, wobei R¹ gegebenenfalls durch eine oder mehrere Gruppen substituiert ist, die unabhängig voneinander ausgewählt sind aus der Gruppe, bestehend aus Halogen, C₁-C₃-Alkyl, Oxo, -CF₃, -(C₀-C₃-Alkyl)OR^{c} und -(C₀-C₃-Alkyl)NR^{c}R^{d};
R^{a} unabhängig voneinander Wasserstoff, C₁-C₆-Alkyl, C₃-C₆-Cycloalkyl, 3-10-gliedriges Heterocyclyl, 5-6-gliedriges Heteroaryl, -C(O)R^{c}, -C(O)OR^{c}, -C(O)NR^{c}R^{d}, -NR^{c}C(O)R^{d}, -S(O)₁₋₂R^{c}, -NR^{c}S(O)₁₋₂R^{d} oder -S(O)₁₋₂NR^{c}R^{d} ist, wobei C₃-C₆-Cycloalkyl, 3-10-gliedriges Heterocyclyl und 5-6-gliedriges Heteroaryl von R^{a} gegebenenfalls mit einer oder mehreren Gruppen R^{e} substituiert ist;
R^{b} unabhängig voneinander Wasserstoff oder C₁-C₃-Alkyl ist, wobei das Alkyl gegebenenfalls durch eine oder mehrere Gruppen substituiert ist, die unabhängig voneinander ausgewählt sind aus der Gruppe, bestehend aus Halogen und Oxo; oder
R^{c} und R^{d} unabhängig voneinander ausgewählt sind aus der Gruppe, bestehend aus Wasserstoff, 3-6-gliedrigem Heterocyclyl, C₃-C₆-Cycloalkyl und C₁-C₃-Alkyl, wobei 3-6-gliedriges Heterocyclyl, C₃-C₆-Cycloalkyl und C₁-C₃-Alkyl von R^{c} und R^{d} gegebenenfalls durch eine oder mehrere Gruppen substituiert sind, die unabhängig voneinander ausgewählt sind aus der Gruppe, bestehend aus Halogen und Oxo; oder R^{c} und R^{d} zusammen mit dem Atom, an das sie gebunden sind, ein 3-6-gliedriges Heterocyclyl bilden, das gegebenenfalls durch eine oder mehrere Gruppen substituiert ist, die unabhängig voneinander ausgewählt sind aus der Gruppe, bestehend aus Halogen, Oxo, -CF₃ und C₁-C₃-Alkyl;
jeder R^{e} unabhängig voneinander ausgewählt ist aus der Gruppe, bestehend aus Oxo, OR^{f}, NR^{f}R^{g}, Halogen, 3-10-gliedrigem Heterocyclyl, C₃-C₆-Cycloalkyl und C₁-C₆-Alkyl, wobei C₃-C₆-Cycloalkyl und C₁-C₆-Alkyl von R^{e} gegebenenfalls durch eine oder mehrere Gruppen substituiert sind, die unabhängig voneinander ausgewählt sind aus der Gruppe, bestehend aus OR^{f}, NR^{f}R^{g}, Halogen, 3-10-gliedrigem Heterocyclyl, Oxo und Cyano, und wobei 3-10-gliedriges Heterocyclyl von R^{e} gegebenenfalls durch eine oder mehrere Gruppen substituiert ist, die unabhängig voneinander ausgewählt sind aus der Gruppe, bestehend aus Halogen, Oxo, Cyano, -CF₃, NR^{h}R^{k}, 3-6-gliedrigem Heterocyclyl und C₁-C₃-Alkyl, das gegebenenfalls durch eine oder mehrere Gruppen substituiert ist, die unabhängig voneinander ausgewählt sind aus der Gruppe, bestehend aus Halogen, Oxo, OR^{f} und NR^{h}R^{k};
R^{f} und R^{g} jeweils unabhängig voneinander ausgewählt sind aus der Gruppe, bestehend aus Wasserstoff, C₁-C₆-Alkyl, 3-6-gliedrigem Heterocyclyl und C₃-C₆-Cycloalkyl, wobei C₁-C₆-Alkyl, 3-6-gliedriges Heterocyclyl und C₃-C₆-Cycloalkyl von R^{f} und R^{g} gegebenenfalls durch einen oder mehrere R^{m} substituiert sind;
jeder R^{m} unabhängig voneinander ausgewählt ist aus der Gruppe, bestehend aus Halogen, Cyano, Oxo, C₃-C₆-Cycloalkyl, 3-6-gliedrigem Heterocyclyl, Hydroxy und NR^{h}R^{k}, wobei C₃-C₆-Cycloalkyl und 3-6gliedriges Heterocyclyl von R^{m} gegebenenfalls mit einer oder mehreren Gruppen substituiert sind, die unabhängig voneinander ausgewählt sind aus der Gruppe, bestehend aus Halogen, Oxo, Cyano und C₁-C₃-Alkyl;
R^{h} und R^{k} jeweils unabhängig voneinander ausgewählt sind aus der Gruppe, bestehend aus Wasserstoff und C₁-C₆-Alkyl, das gegebenenfalls durch eine oder mehrere Gruppen substituiert ist, die unabhängig voneinander ausgewählt sind aus der Gruppe, bestehend aus Halogen, Cyano, 3-6-gliedrigem Heterocyclyl und Oxo; oder R^{h} und R^{k} zusammen mit dem Atom, an das sie gebunden sind, ein 3-6-gliedriges Heterocyclyl bilden, das gegebenenfalls durch eine oder mehrere Gruppen substituiert ist, die unabhängig voneinander ausgewählt sind aus der Gruppe, bestehend aus Halogen, Cyano, Oxo, -CF₃ und C₁-C₃-Alkyl, das gegebenenfalls durch eine oder mehrere Gruppen substituiert ist, die unabhängig voneinander ausgewählt sind aus der Gruppe, bestehend aus Halogen und Oxo;
R² C₁-C₆-Alkyl, C₂-C₆-Alkenyl, C₂-C₆-Alkinyl, C₃-C₆-Cycloalkyl, 3-6-gliedriges Heterocyclyl, (C₃-C₆-Cycloalkyl)C₁-C₆-alkyl, (3-6-gliedriges Heterocyclyl)C₁-C₆-alkyl, -C(O)(C₃-C₆-Cycloalkyl) oder -C(O)(3-6-gliedriges Heterocyclyl) ist, wobei R² gegebenenfalls mit einem oder mehreren Halogen(en) substituiert ist;
n 0, 1 oder 2 ist;
R³ H oder NH₂ ist;
R⁴ H oder CH₃ ist und
R⁵ H oder NH₂ ist.

2. Verbindung nach Anspruch 1, wobei R¹ H, C₁-C₆-Alkyl, -(C₀-C₃-Alkyl)R^{a}, -(C₀-C₃-Alkyl)NR^{a}R^{b}, -(C₀-C₃-Alkyl)C(O)R^{a} oder -(C₀-C₃-Alkyl)C(O)OR^{a} ist, oder ein Salz davon.

3. Verbindung nach Anspruch 1, wobei R¹ H ist, oder ein Salz davon.

4. Verbindung nach Anspruch 1, wobei R¹ C₁-C₆-Alkyl, C₂-C₆-Alkenyl, C₂-C₆-Alkinyl, -(C₀-C₃-Alkyl)CN, -(C₀-C₃-Alkyl)OR^{a}, -(C₀-C₃-Alkyl)R^{a}, -(C₀-C₃-Alkyl)SR^{a}, -(C₀-C₃-Alkyl)NR^{a}R^{b}, -(C₀-C₃-Alkyl)OCF₃, -(C₀-C₃-Alkyl)CF₃, -(C₀-C₃-Alkyl)NO₂, -(C₀-C₃-Alkyl)C(O)R^{a}, -(C₀-C₃-Alkyl)C(O)OR^{a}, -(C₀-C₃-Alkyl)C(O)NR^{a}R^{b}, -(C₀-C₃-Alkyl)NR^{a}C(O)R^{b}, -(C₀-C₃-Alkyl)S(O)₁₋₂R^{a}, -(C₀-C₃-Alkyl)NR^{a}S(O)₁₋₂R^{b}, -(C₀-C₃-Alkyl)S(O)₁₋₂NR^{a}R^{b}, -(C₀-C₃-Alkyl)(5-6-gliedriges Heteroaryl) oder -(C₀-C₃-Alkyl)phenyl ist, wobei R¹ gegebenenfalls durch eine oder mehrere Gruppen substituiert ist, die unabhängig voneinander ausgewählt sind aus der Gruppe, bestehend aus Halogen, C₁-C₃-Alkyl, Oxo, -CF₃, -(C₀-C₃-Alkyl)OR^{c} und -(C₀-C₃-Alkyl)NR^{c}R^{d}.

5. Verbindung nach Anspruch 1 oder ein Salz davon, wobei R¹ ausgewählt ist aus der Gruppe, bestehend aus H, Methyl,

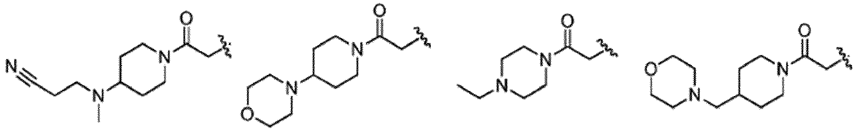

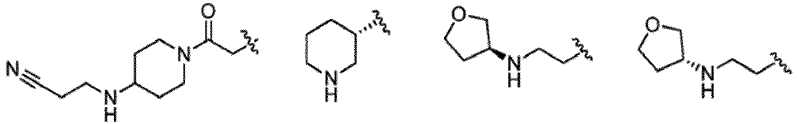

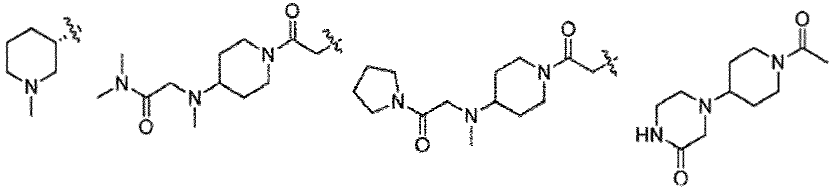

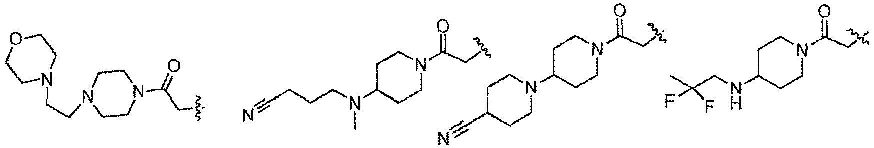

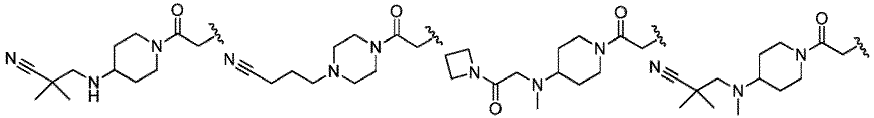

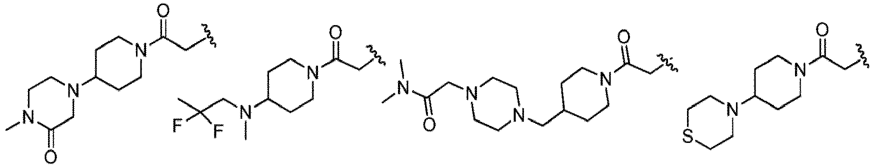

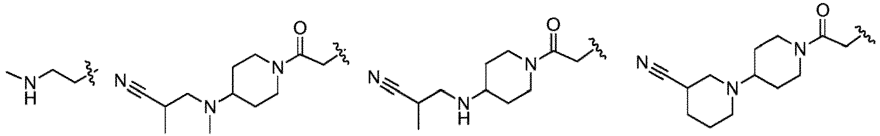

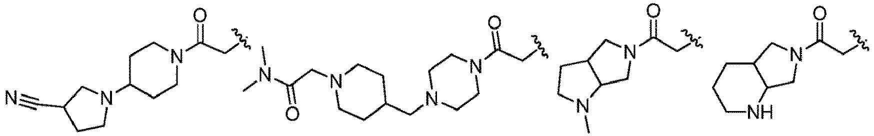

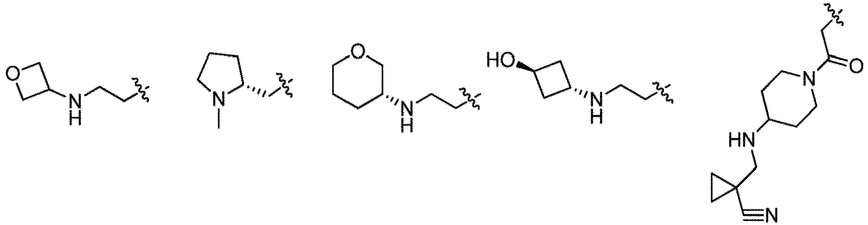

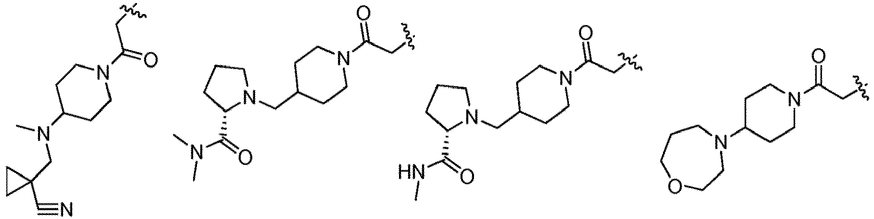

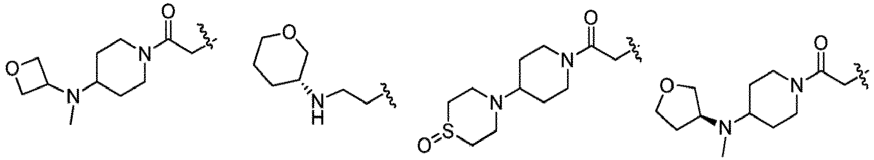

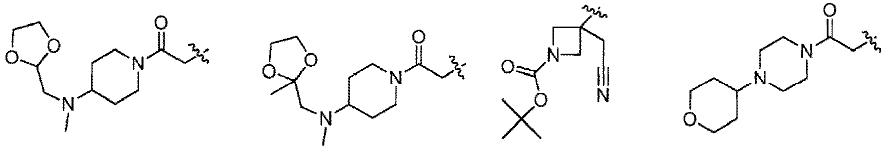

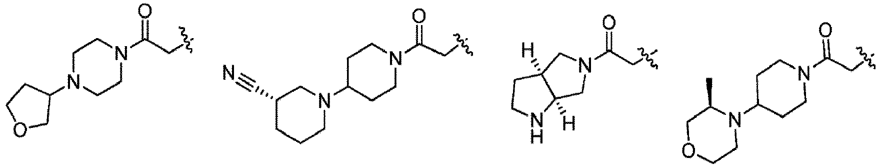

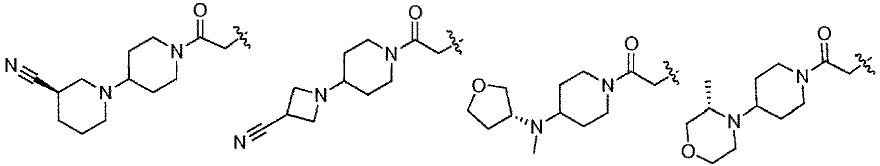

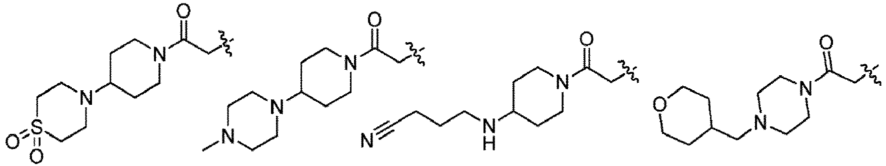

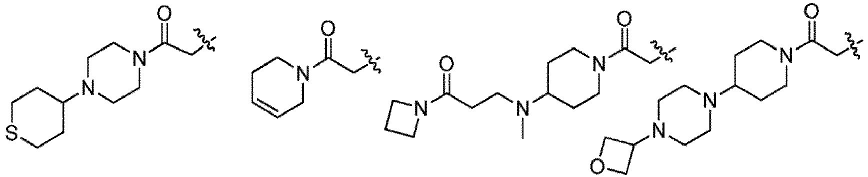

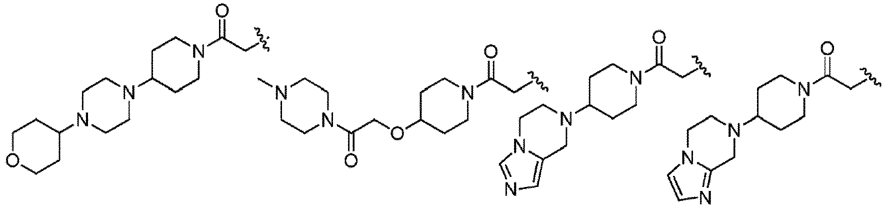

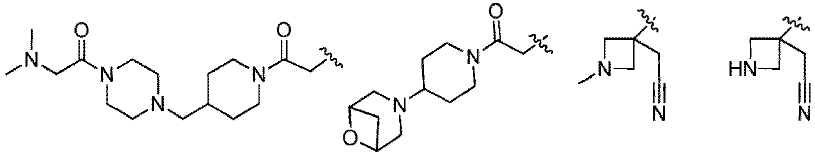

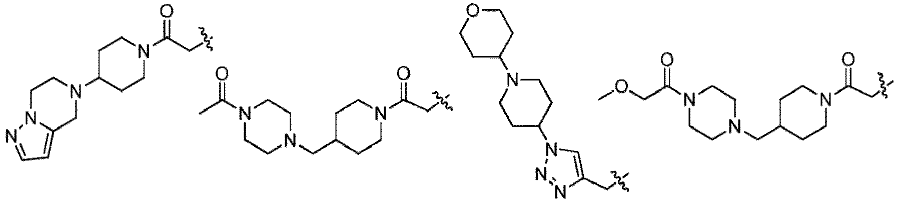

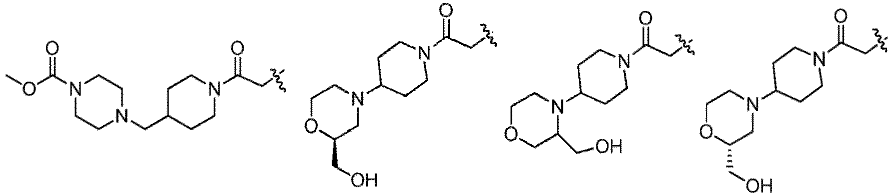

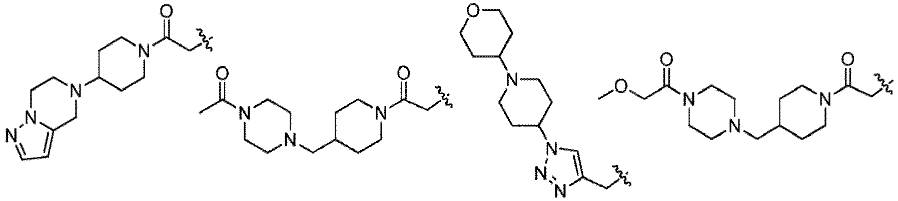

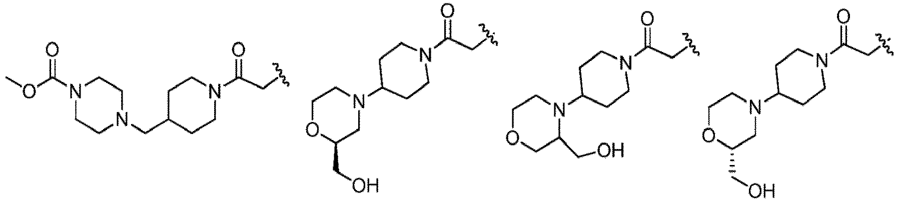

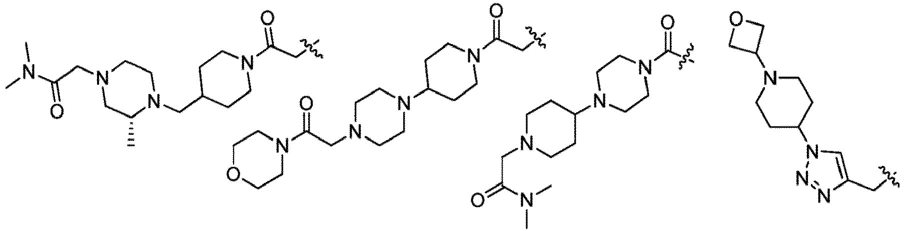

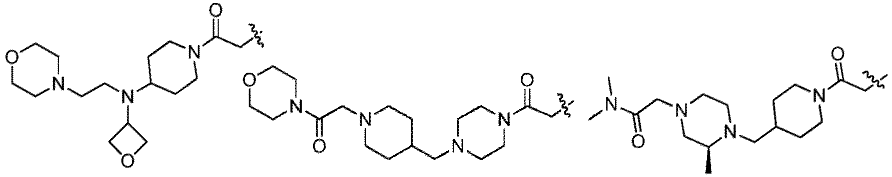

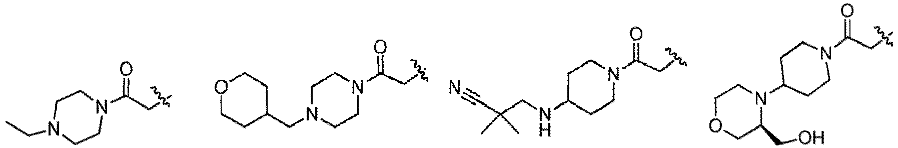

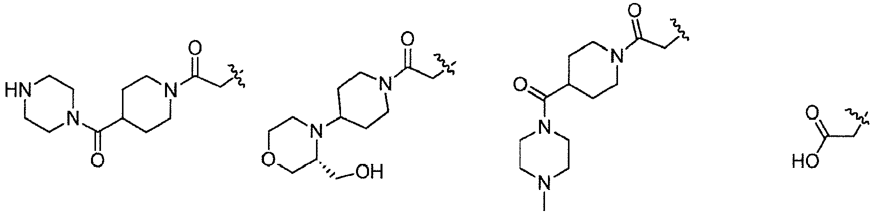

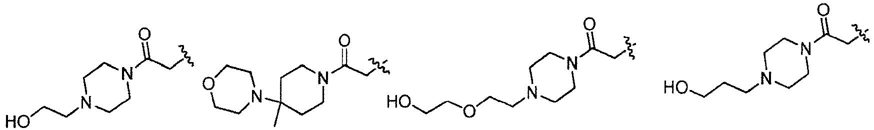

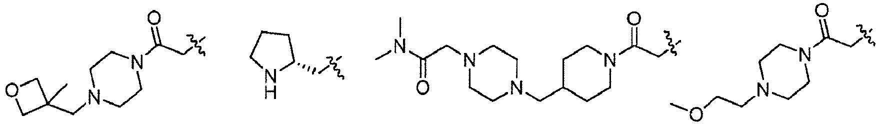

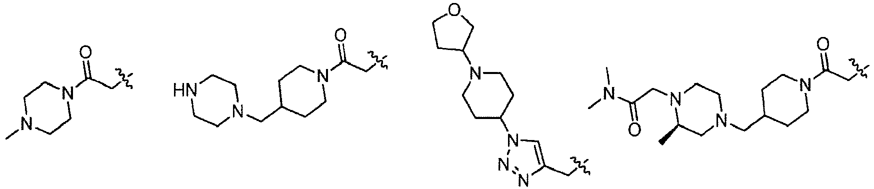

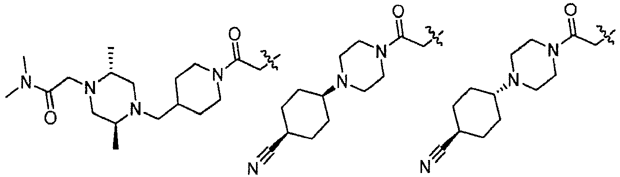

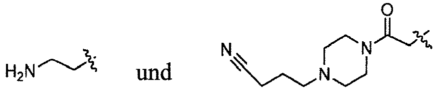

6. Verbindung nach einem der Ansprüche 1-5, wobei R² C₁-C₆-Alkyl, C₃-C₆-Cycloalkyl, oder 3-6-gliedriges Heterocyclyl ist, oder ein Salz davon.

7. Verbindung nach einem der Ansprüche 1-5, wobei R² Methyl, Ethyl, Isopropyl, Cyclopropyl oder Oxetanyl ist, oder ein Salz davon.

8. Verbindung nach einem der Ansprüche 1-7, wobei n 1 ist, oder ein Salz davon.

9. Verbindung nach einem der Ansprüche 1-7, wobei n 2 ist, oder ein Salz davon.

10. Verbindung nach Anspruch 1, die
a) eine Verbindung der Formel (00B): 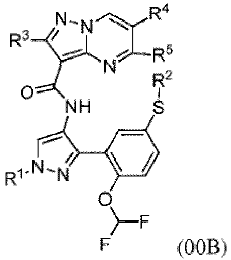 oder ein Salz davon;
b) eine Verbindung der Formel (00C): 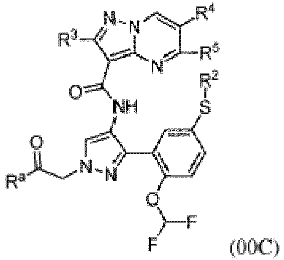 oder ein Salz davon;
c) eine Verbindung der Formel (00D): 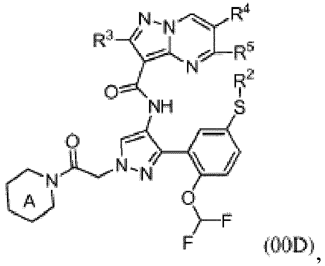 wobei der Ring A gegebenenfalls mit einer oder mehreren Gruppen R^{e} substituiert ist; oder ein Salz davon;
d) eine Verbindung der Formel (00E): 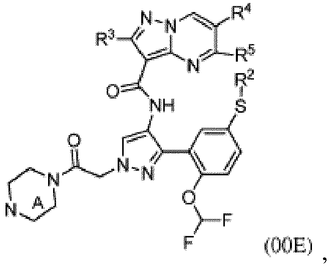 wobei der Ring A gegebenenfalls mit einer oder mehreren Gruppen R^{e} substituiert ist; oder ein Salz davon;
e) eine Verbindung der Formel (00F): 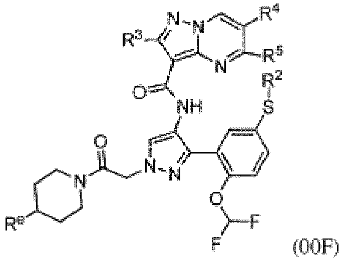 oder ein Salz davon; oder
f) eine Verbindung der Formel (00G): 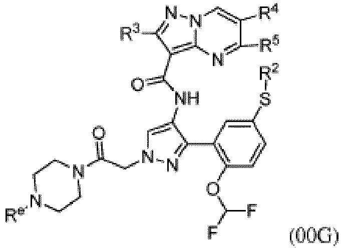 oder ein Salz davon ist.

11. Verbindung nach Anspruch 10, wobei R² C₁-C₆-Alkyl, C₃-C₆-Cycloalkyl oder 3-6-gliedriges Heterocyclyl ist, oder ein Salz davon.

12. Verbindung nach Anspruch 10, wobei R² Methyl, Ethyl, Isopropyl, Cyclopropyl oder Oxetanyl ist, oder ein Salz davon.

13. Verbindung nach Anspruch 10-12, wobei R^{e} ausgewählt ist aus der Gruppe, bestehend aus Methyl, Ethyl,

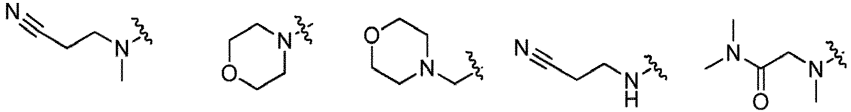

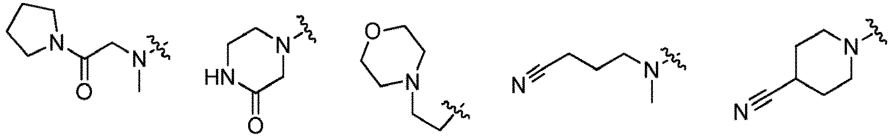

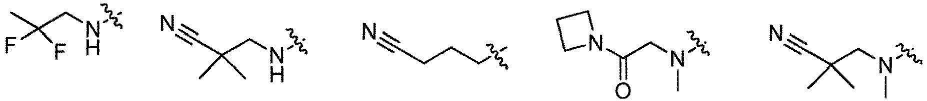

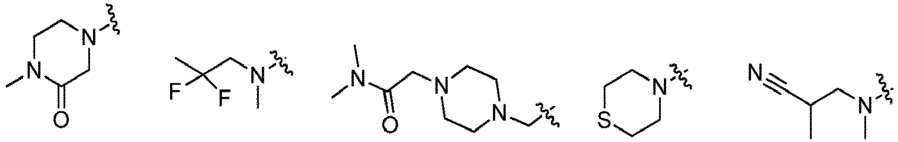

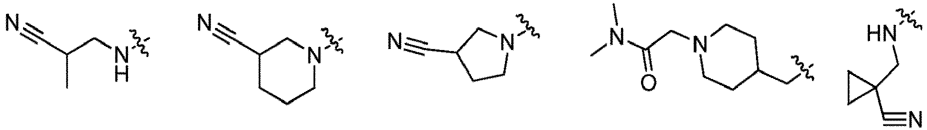

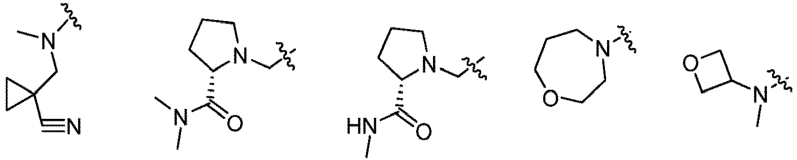

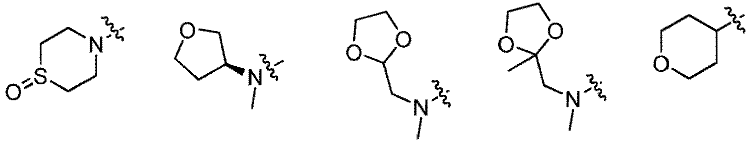

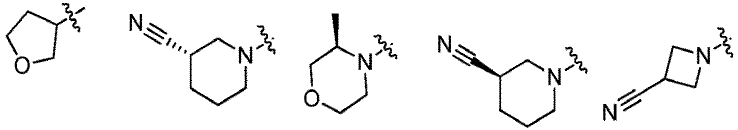

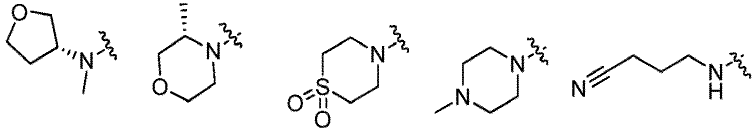

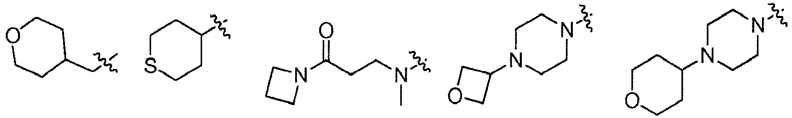

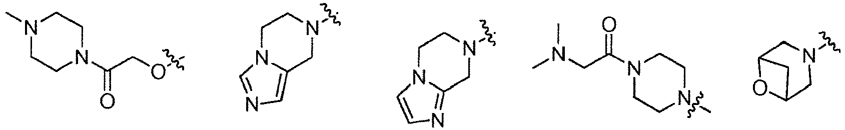

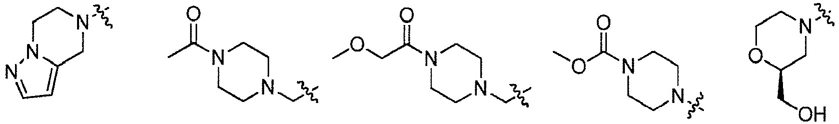

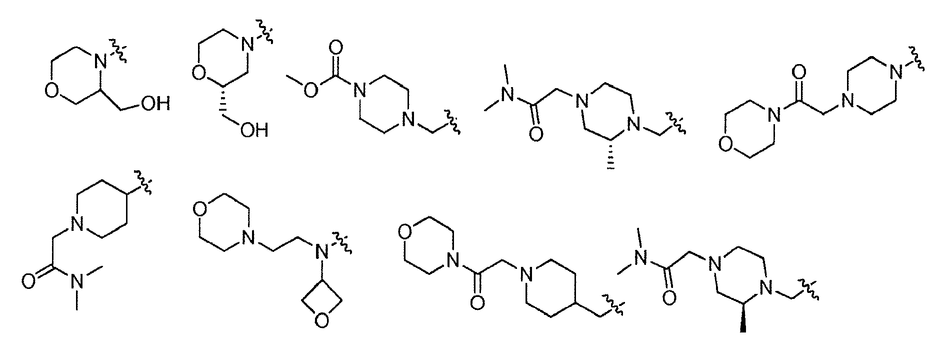

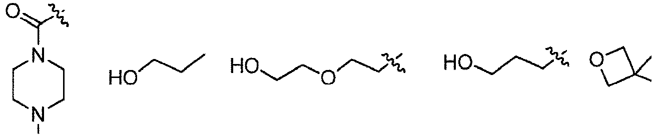

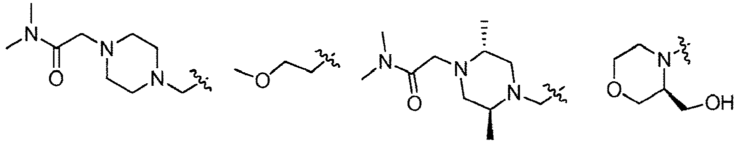

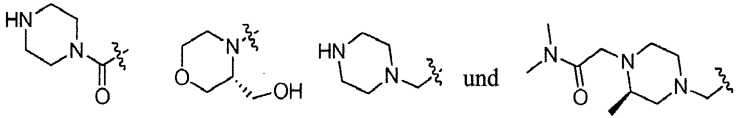

14. Verbindung nach Anspruch 1, ausgewählt aus: 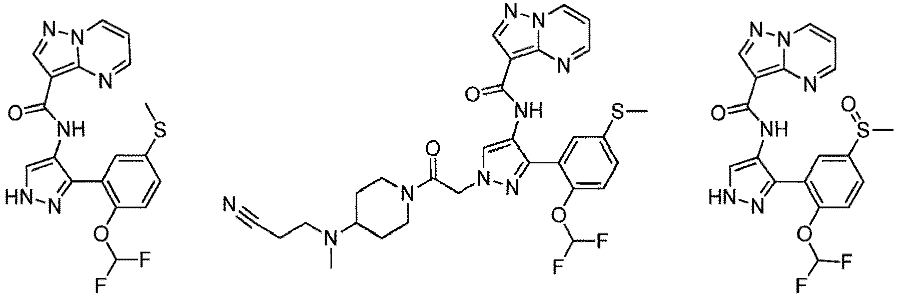 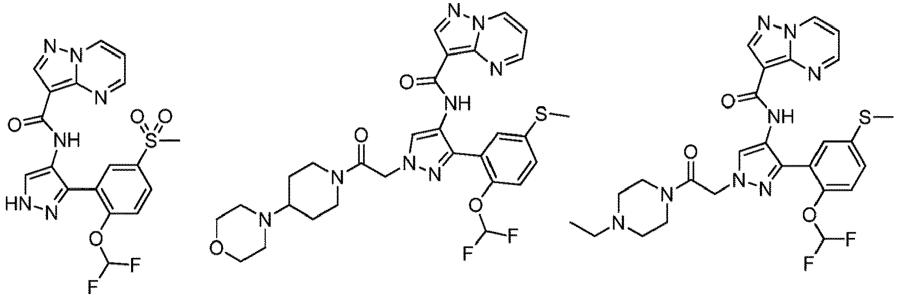 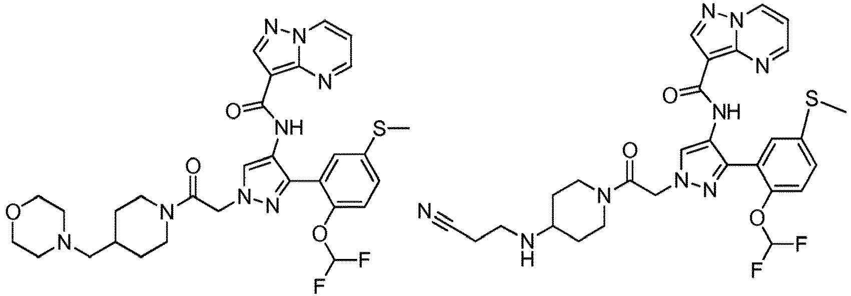 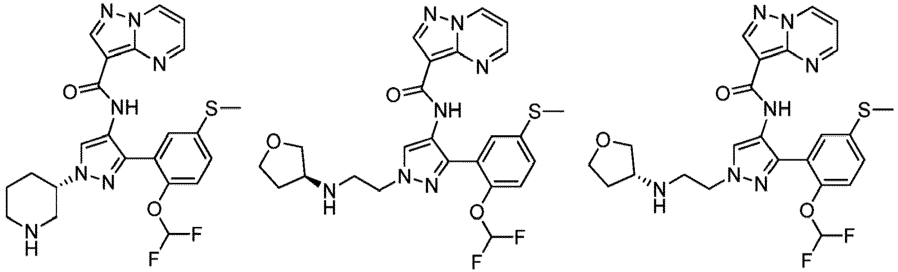 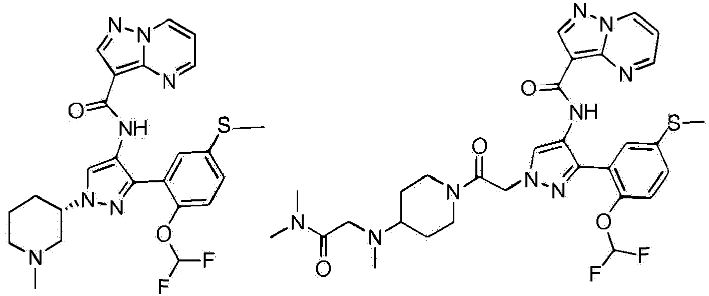 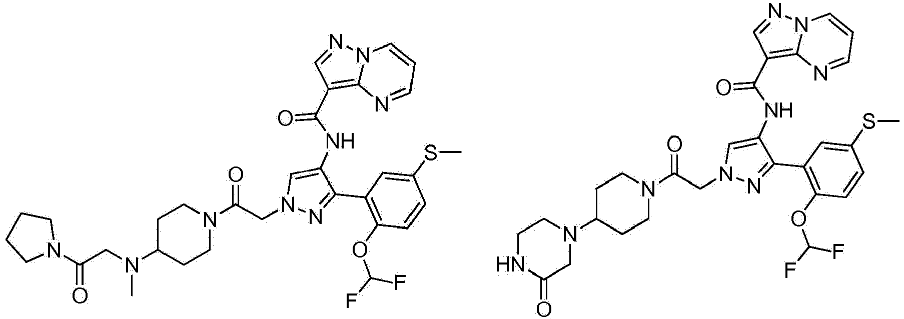 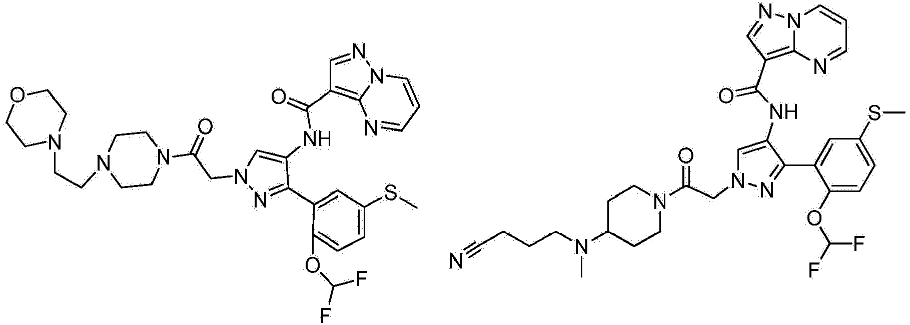 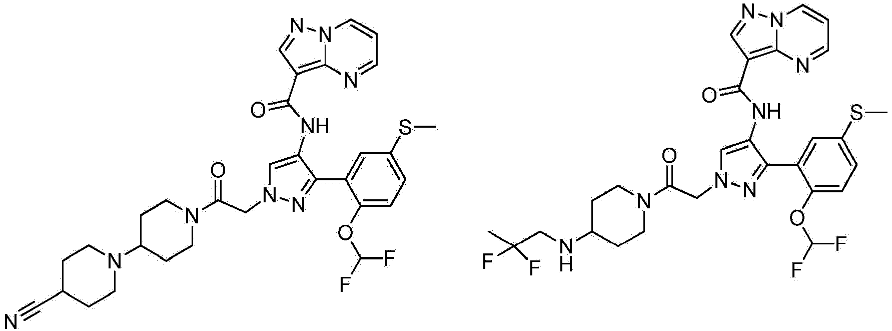 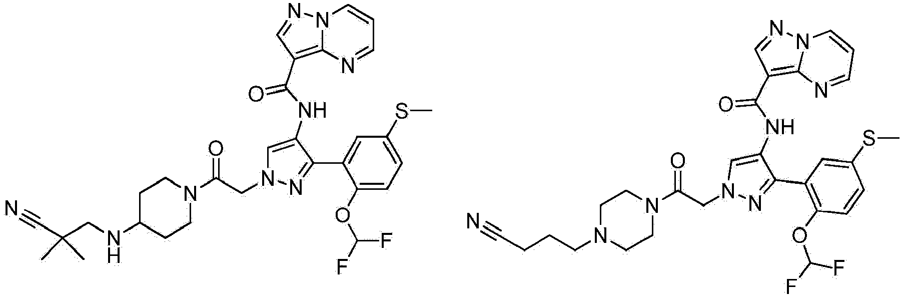 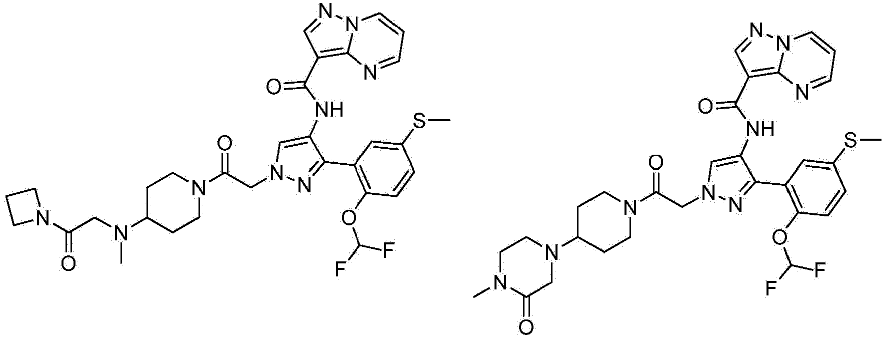 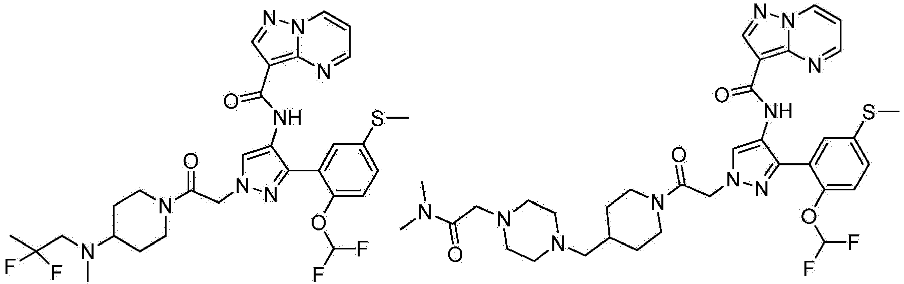 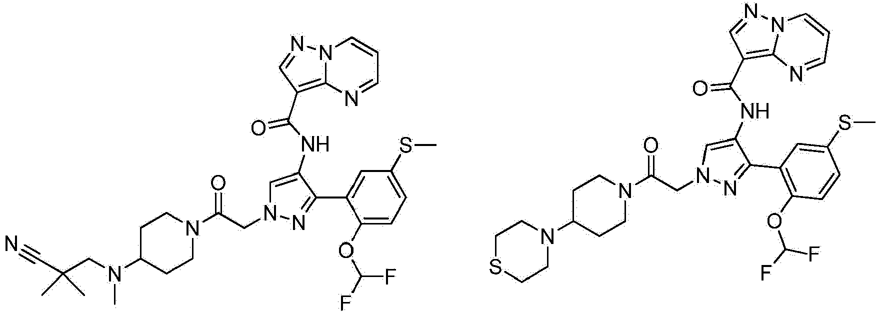 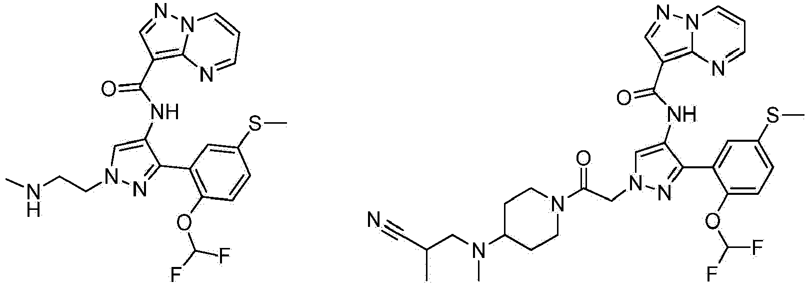 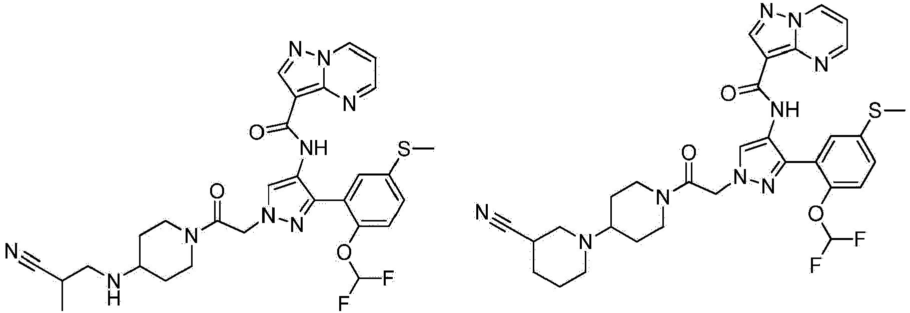 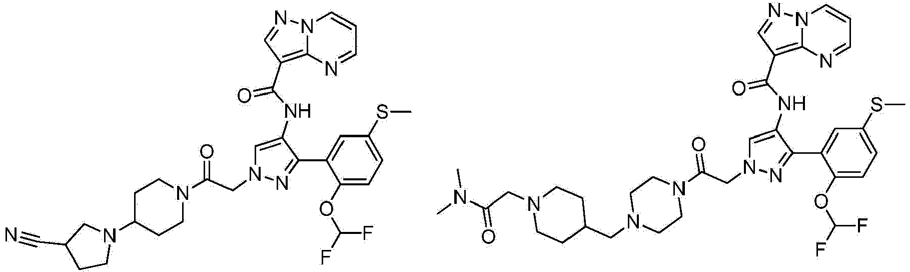 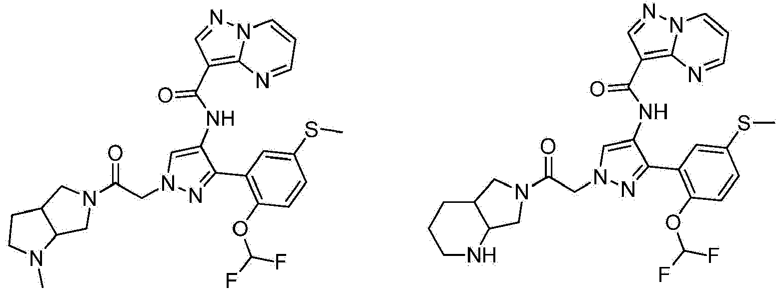 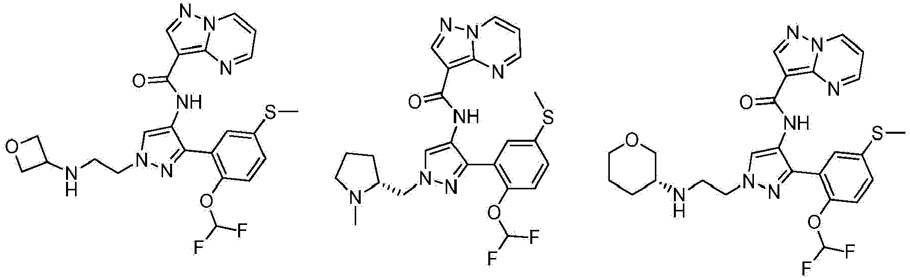 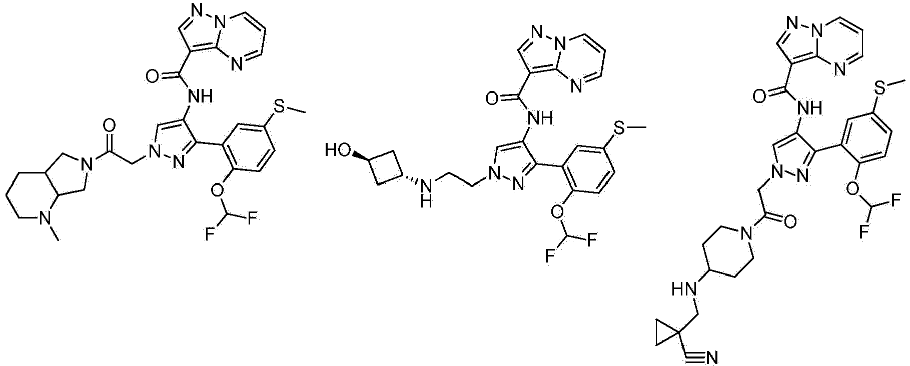 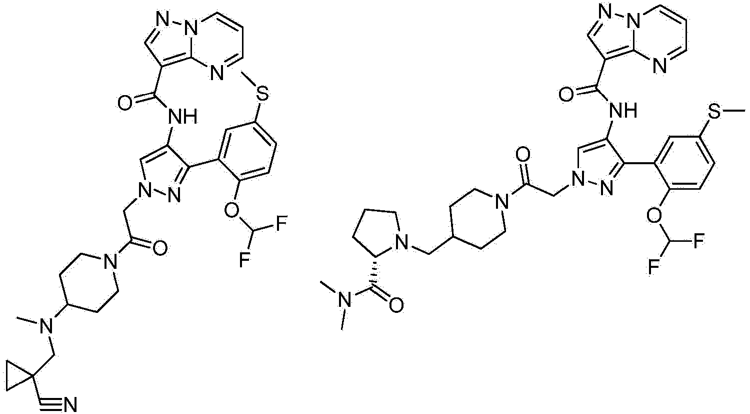 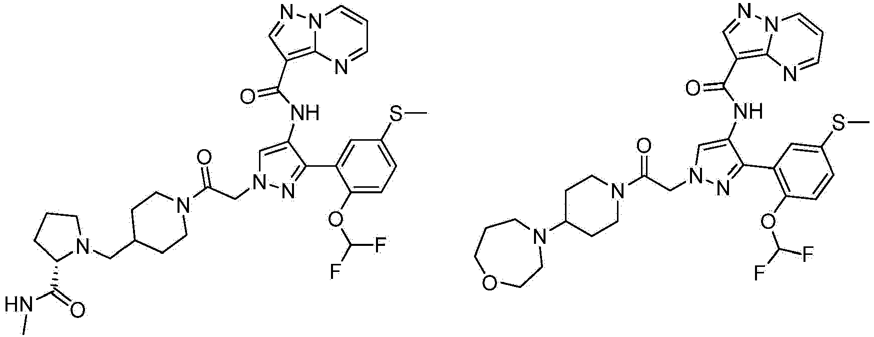 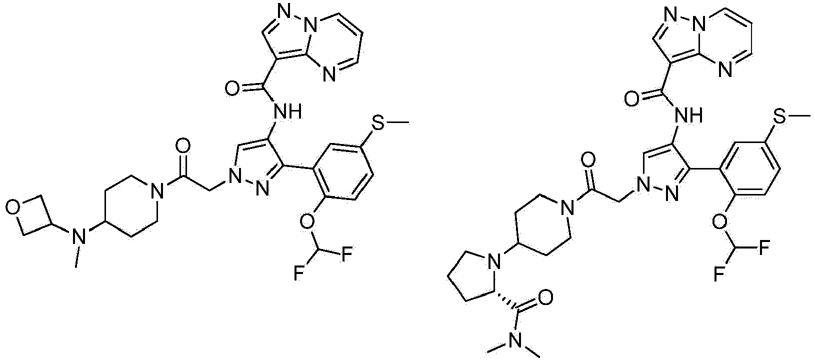 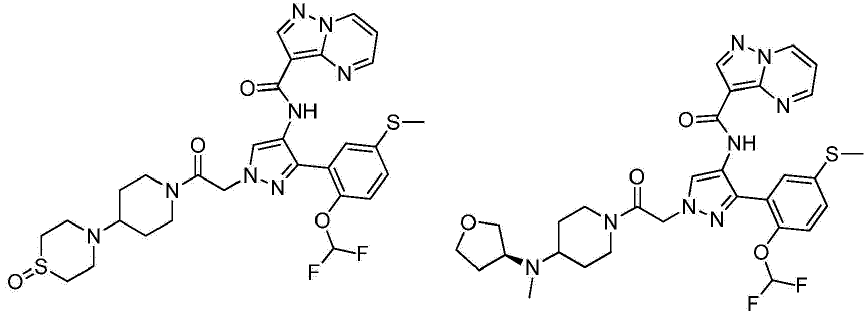 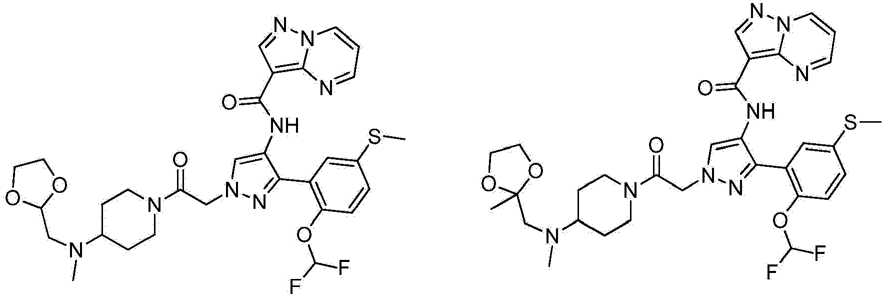 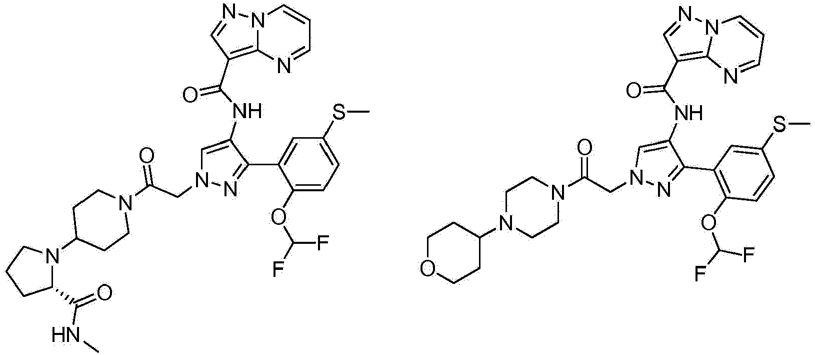 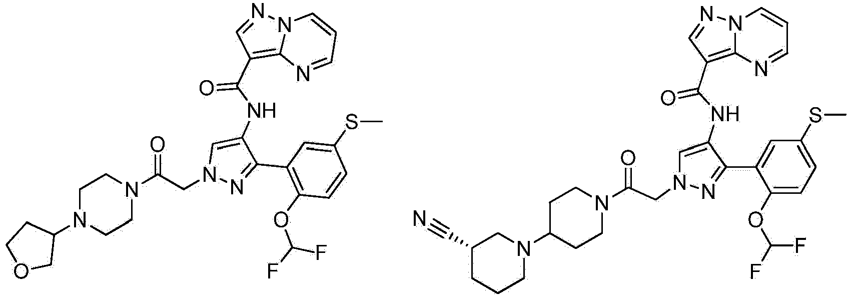 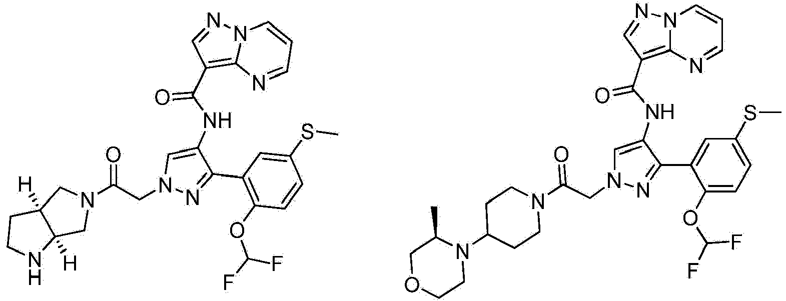 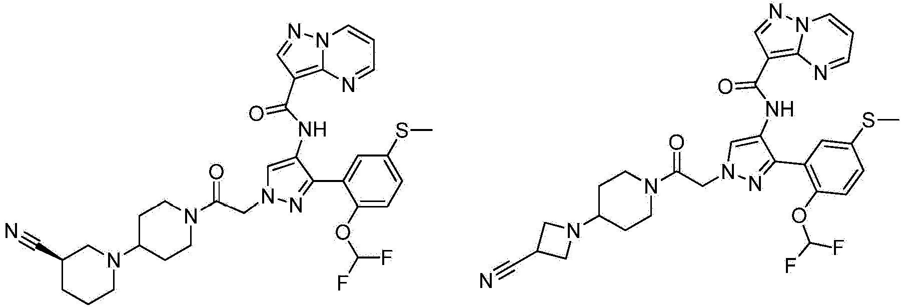 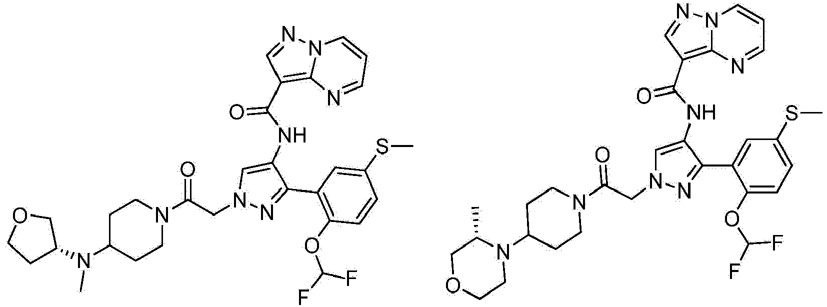 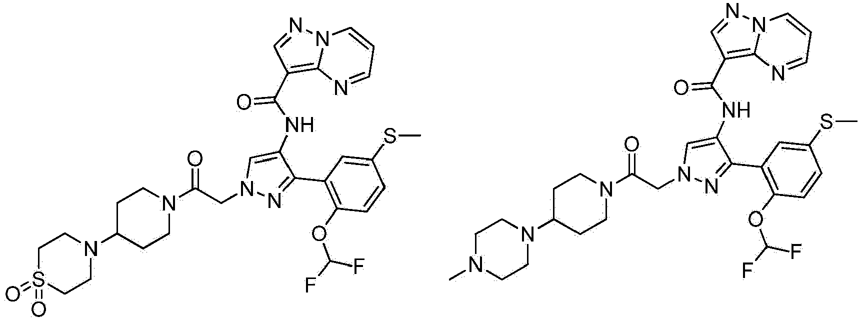 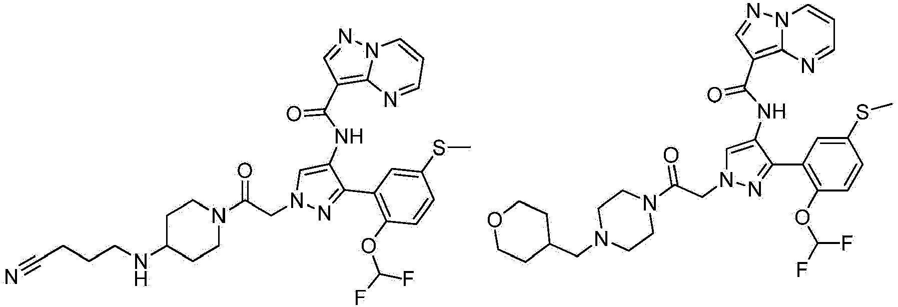 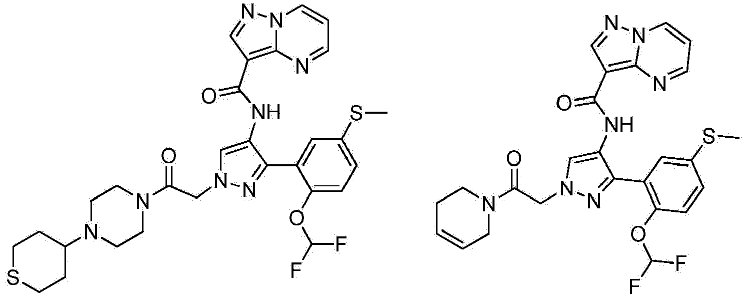 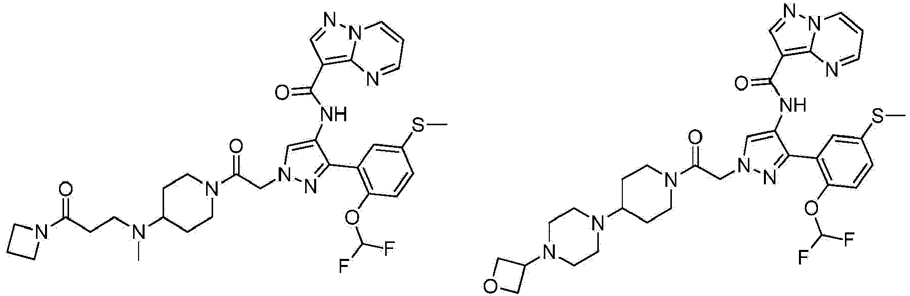 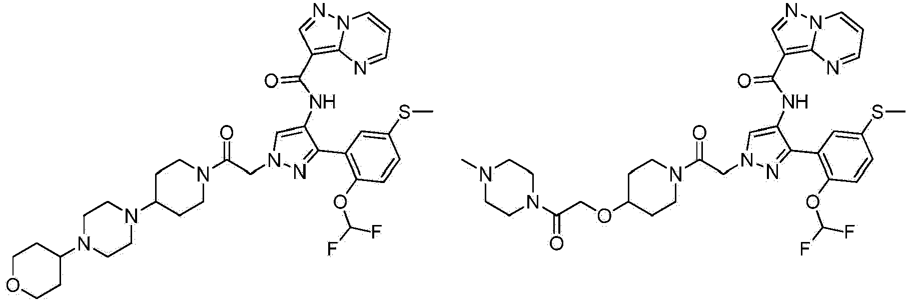 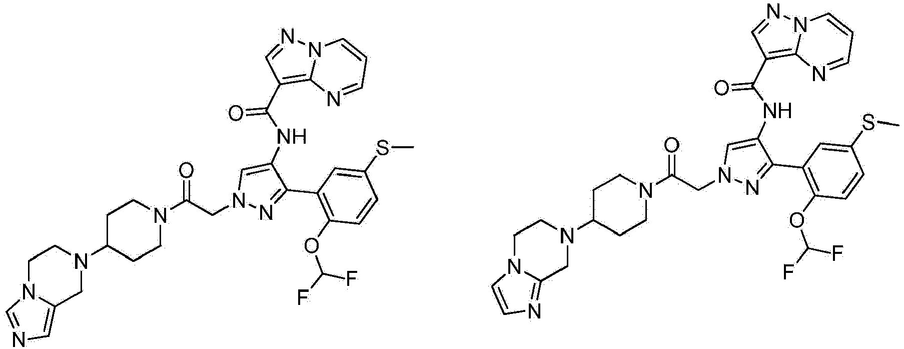 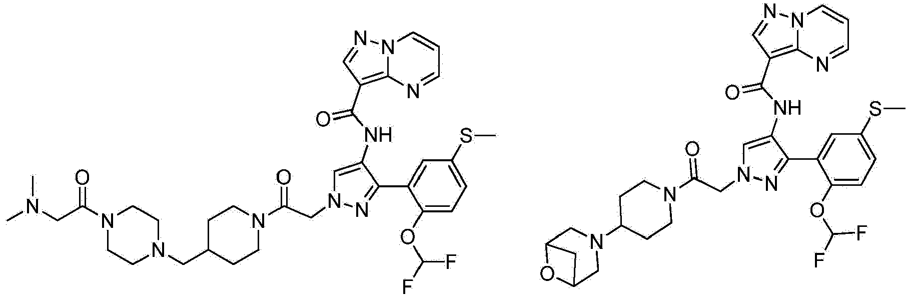 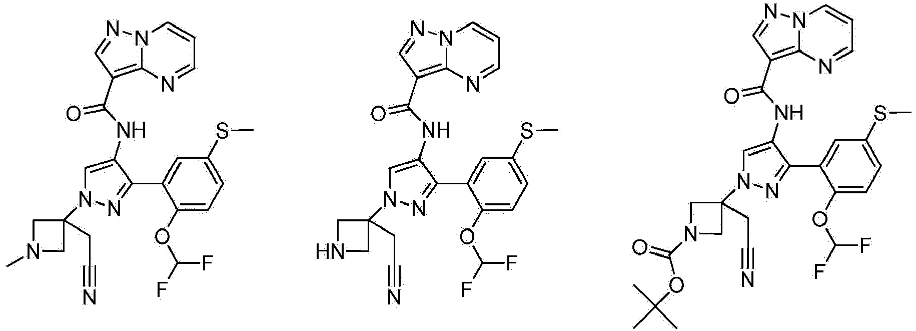 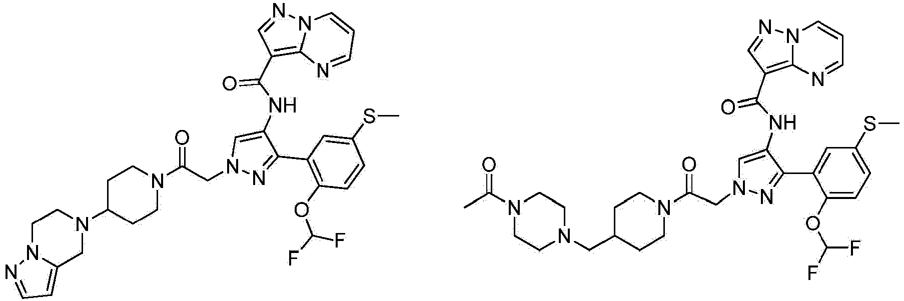 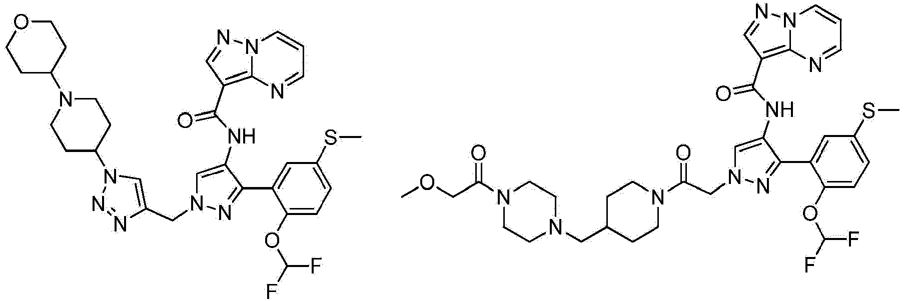 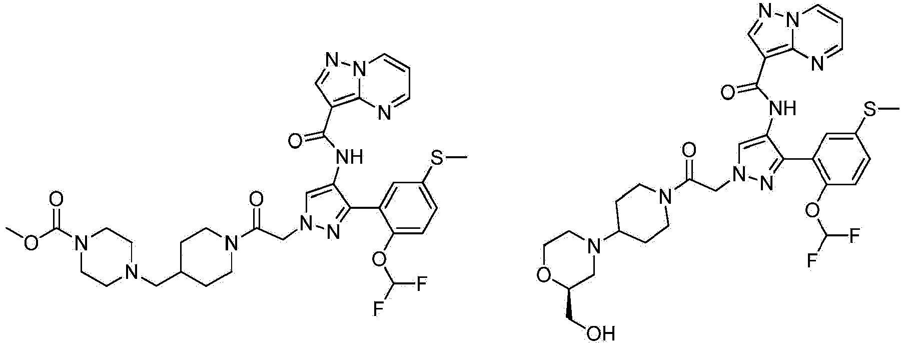 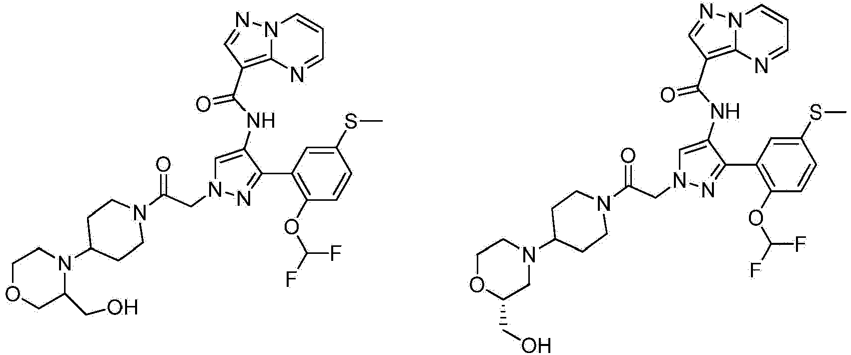 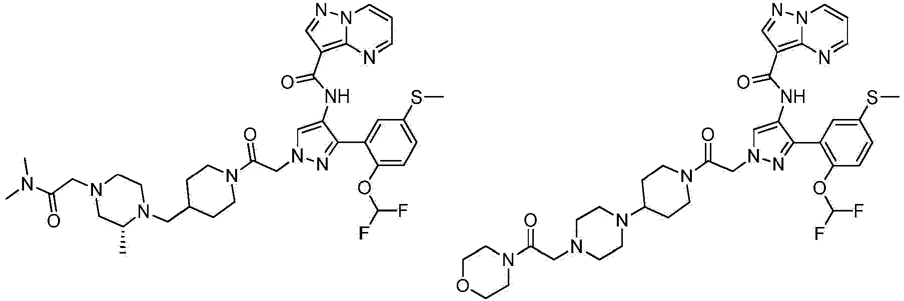 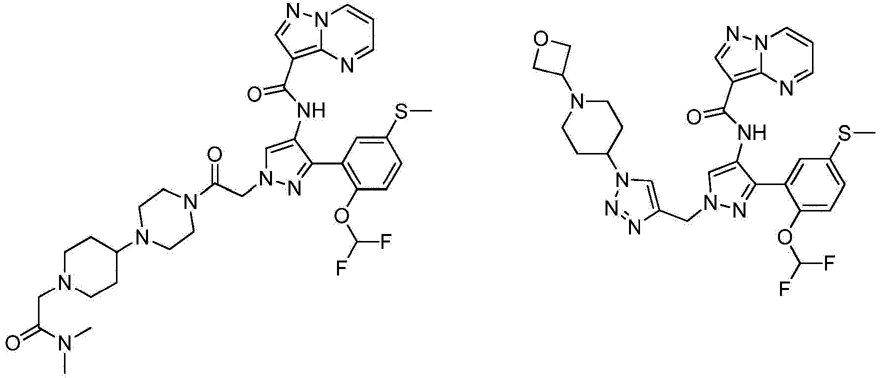 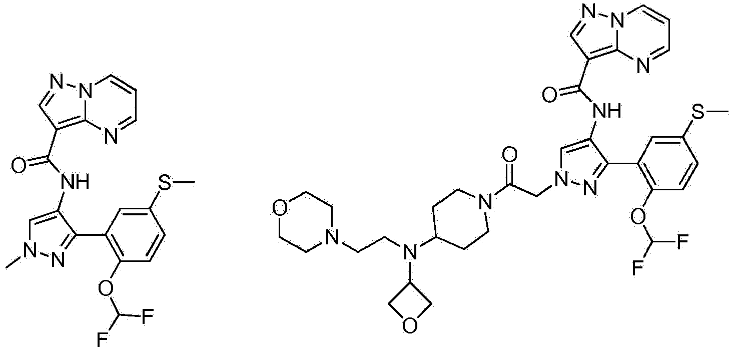 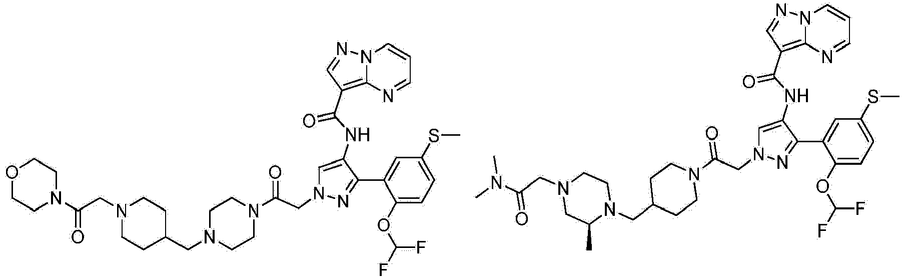 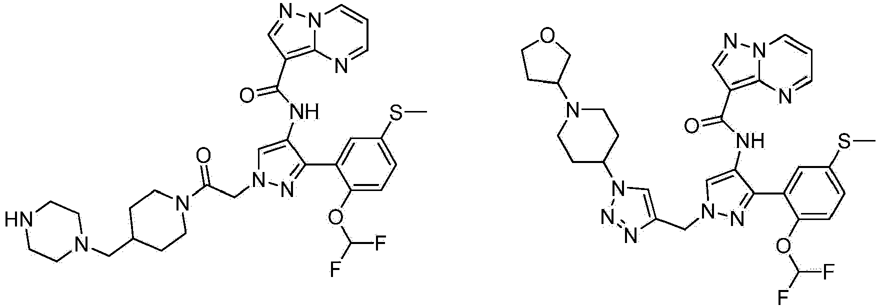 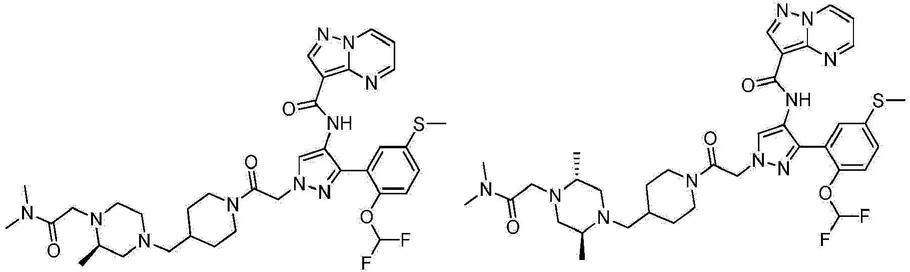 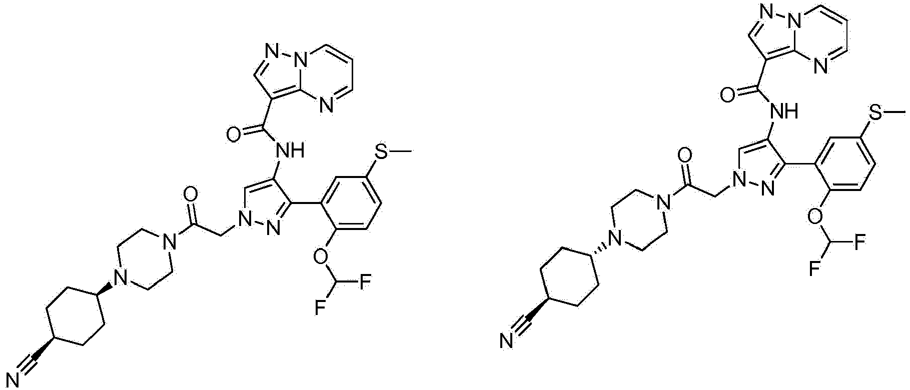 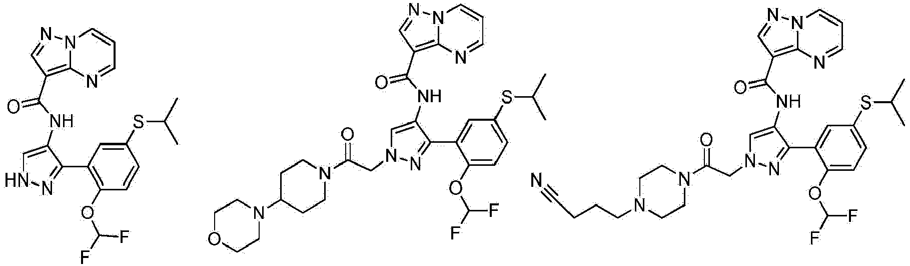 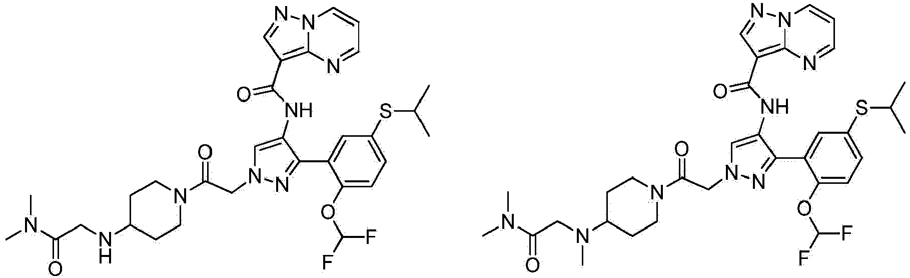 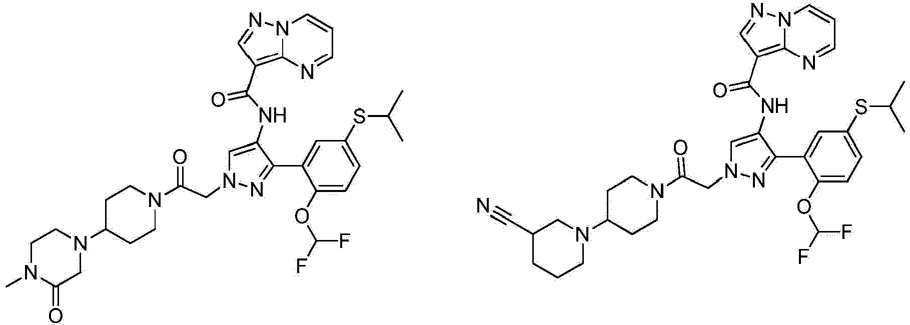 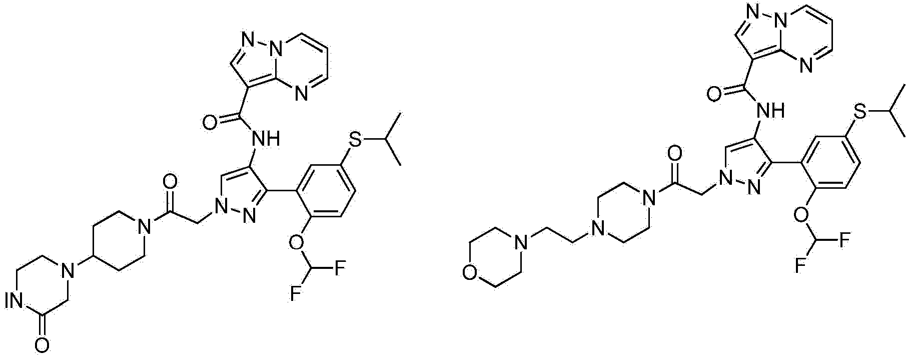 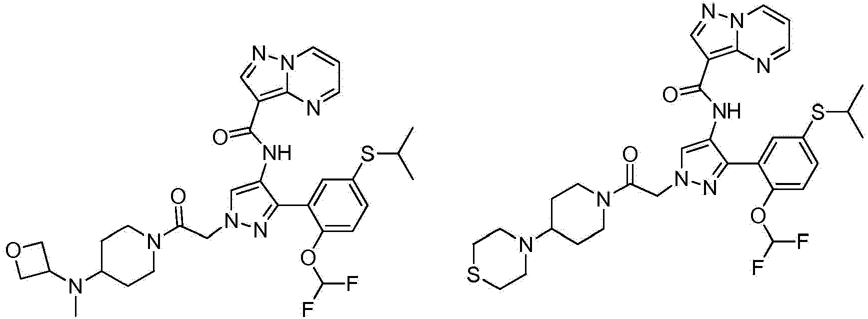 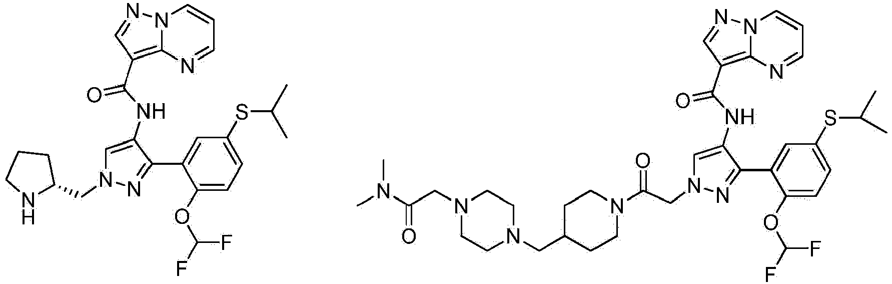 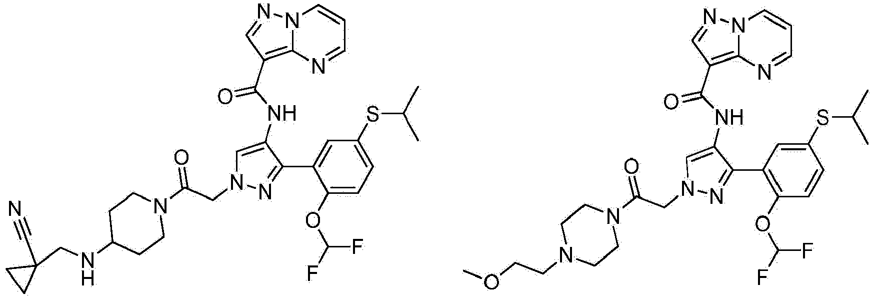 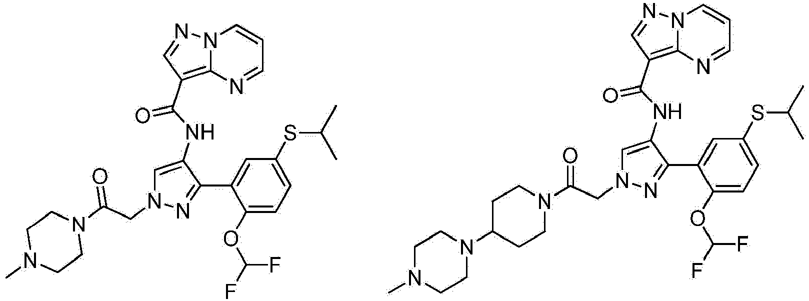 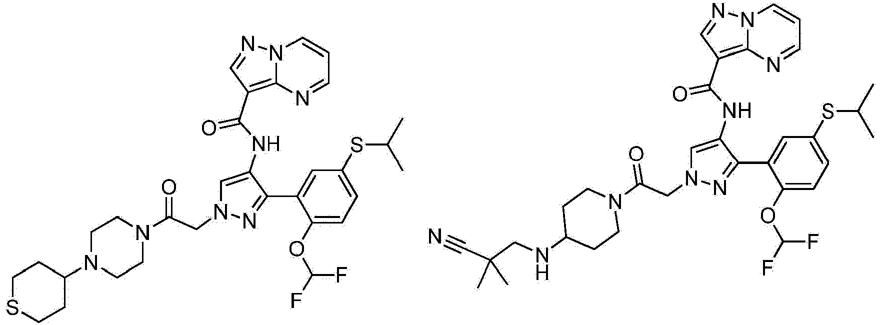 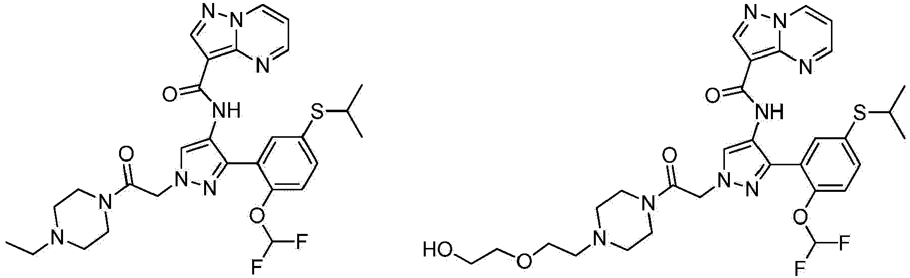 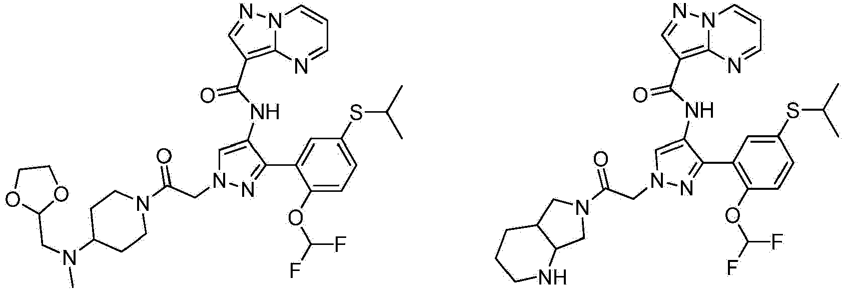 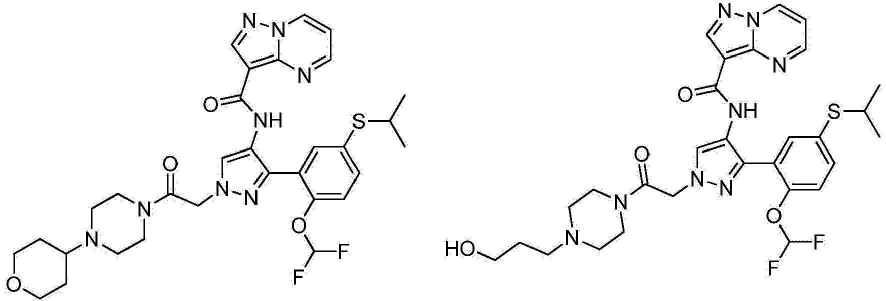 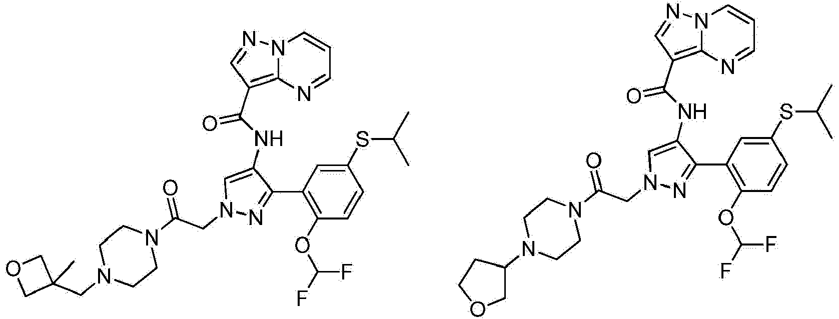 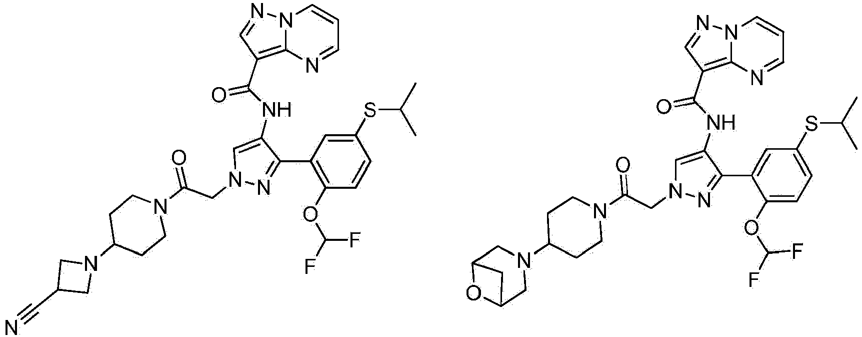 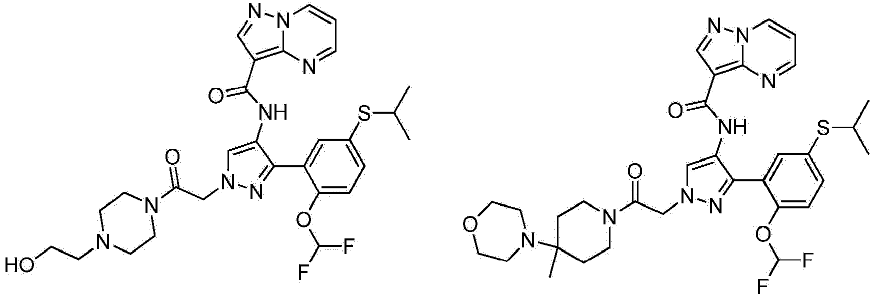 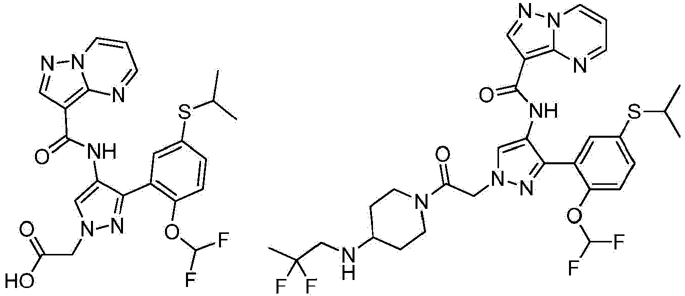 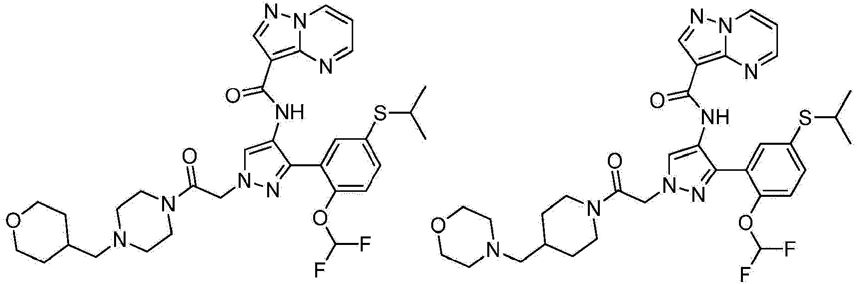 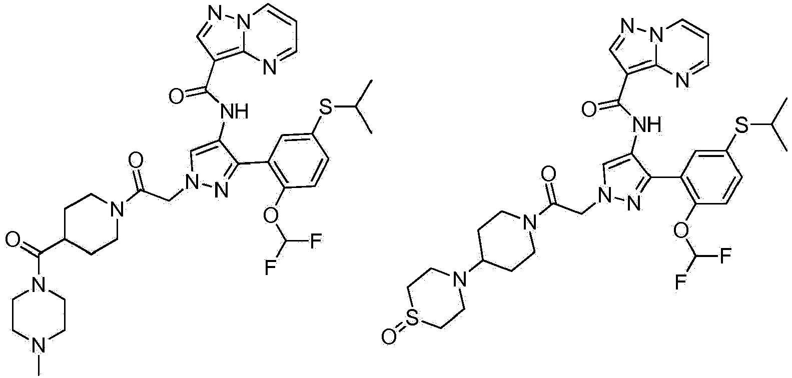 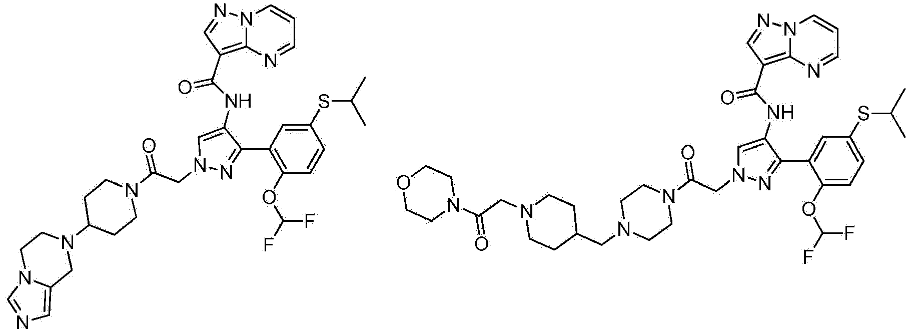 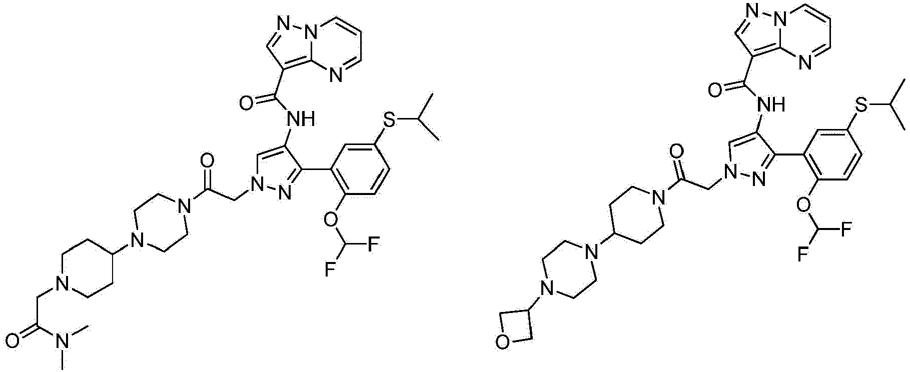 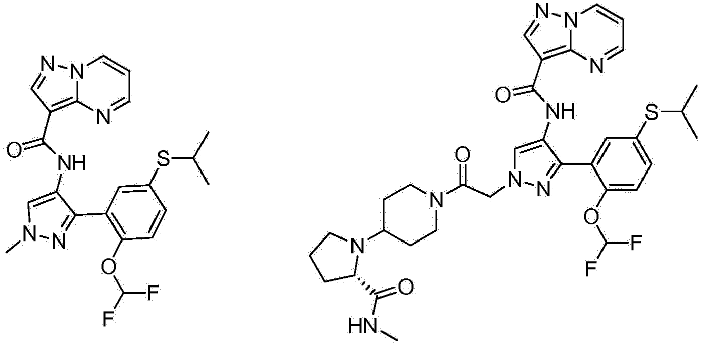 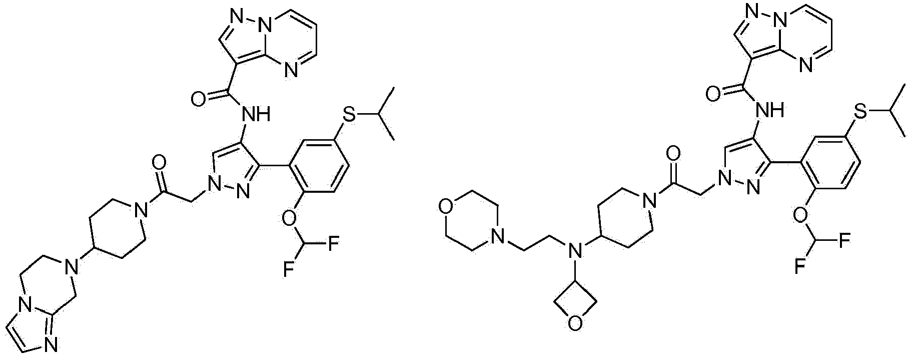 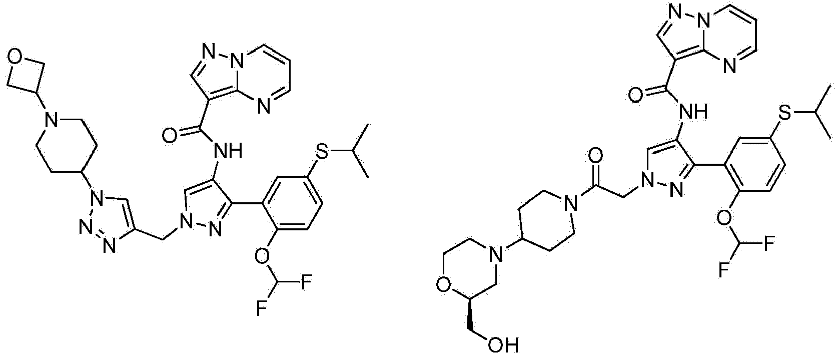 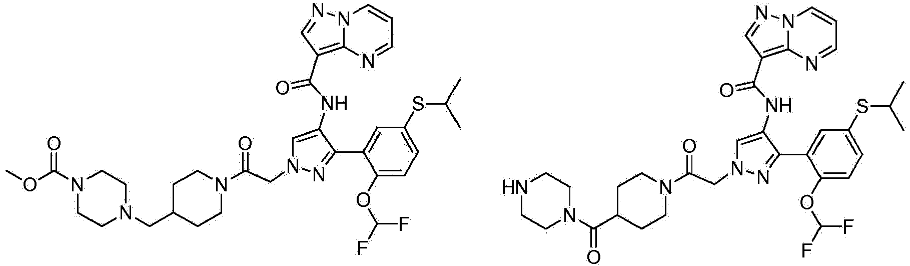 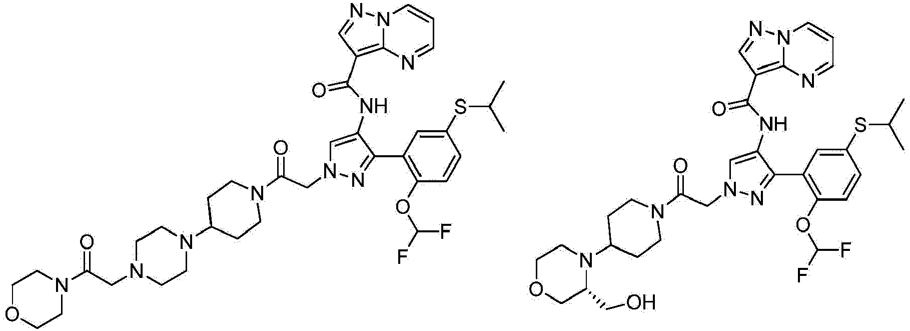 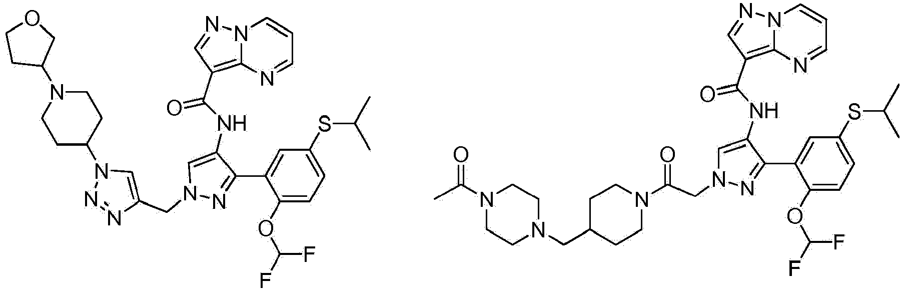 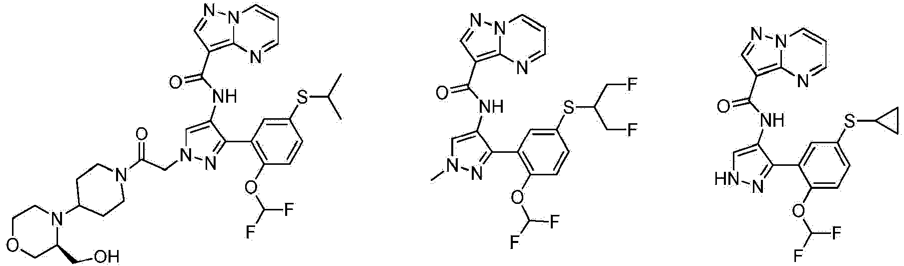 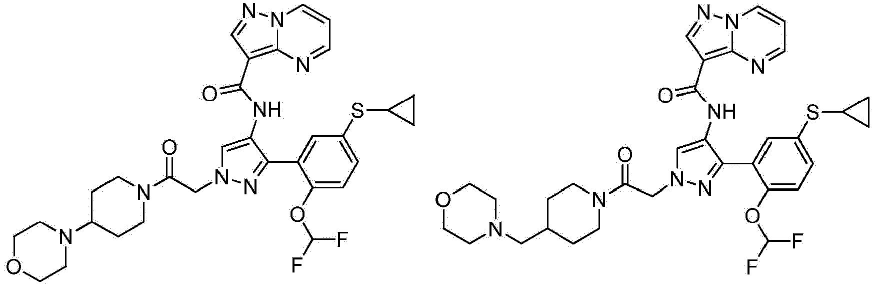 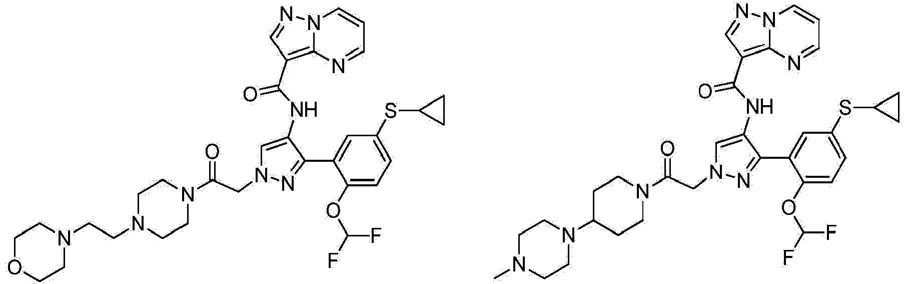 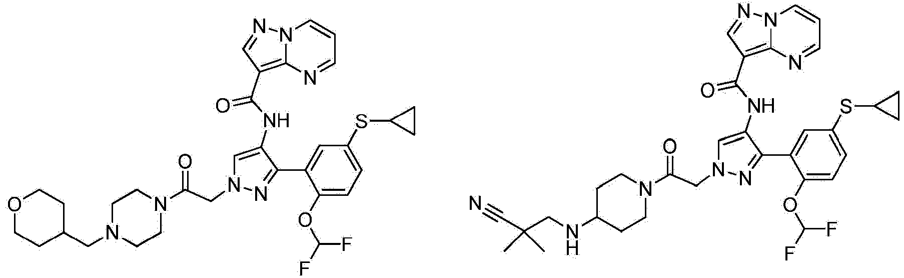 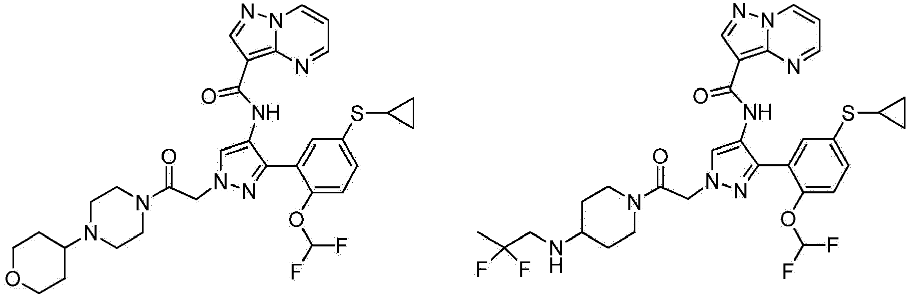 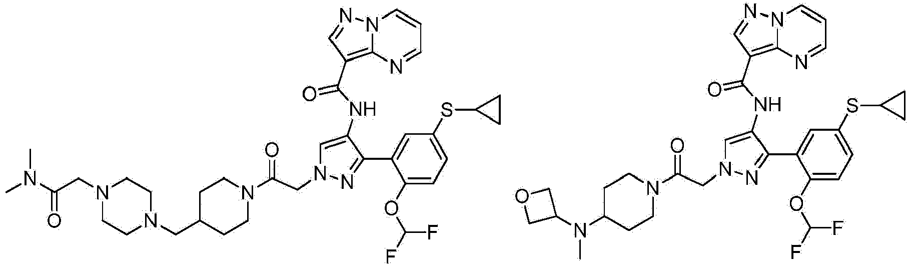 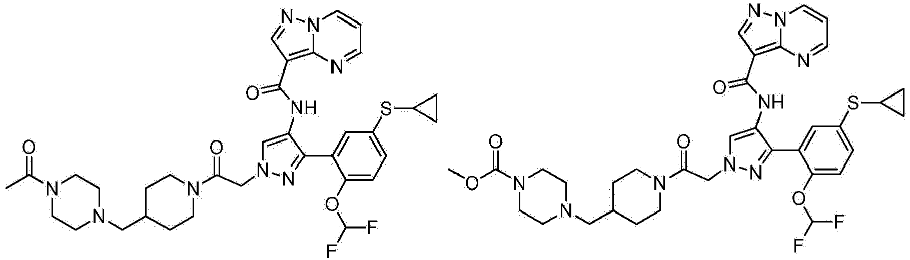 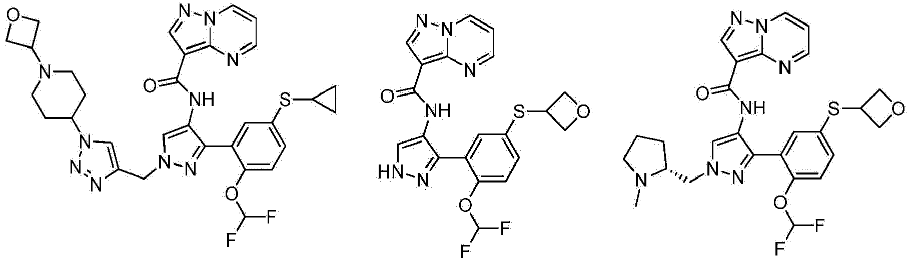 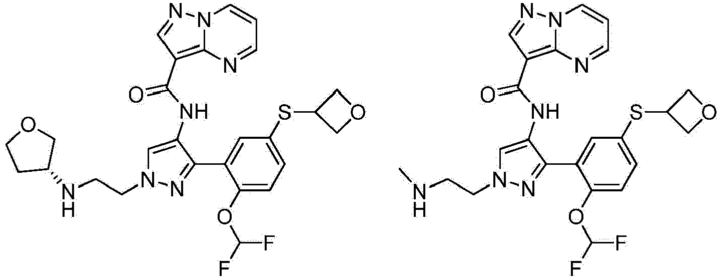 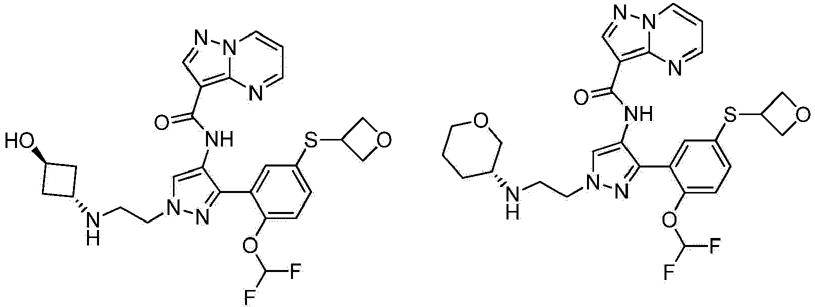 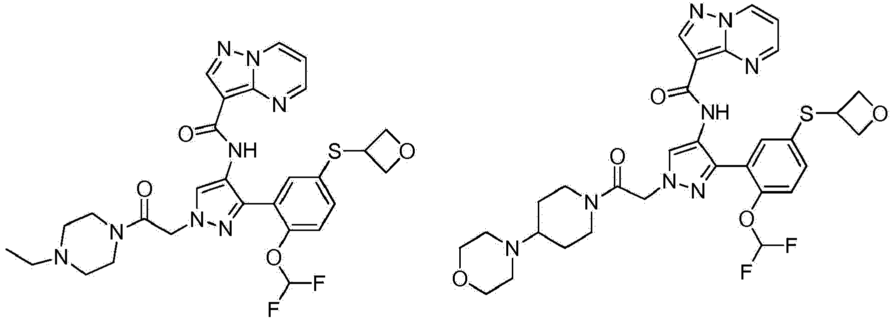 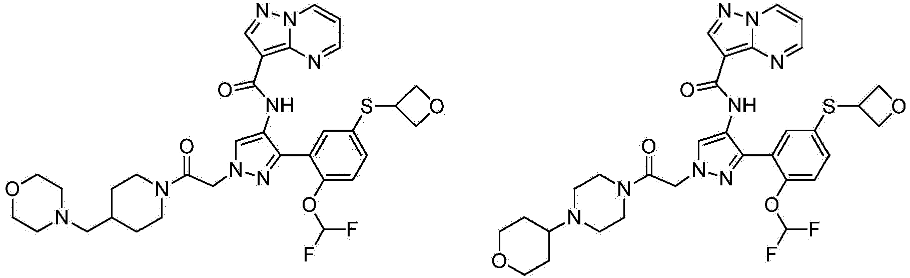 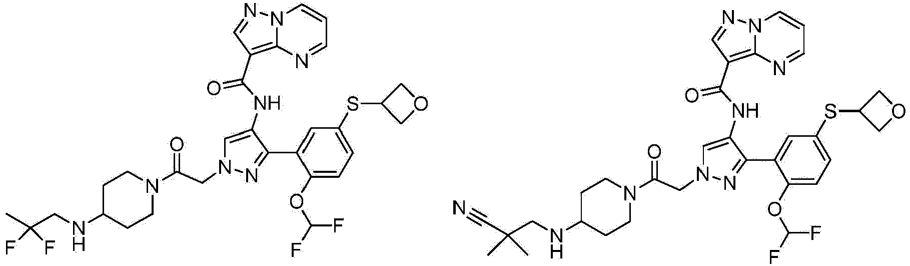 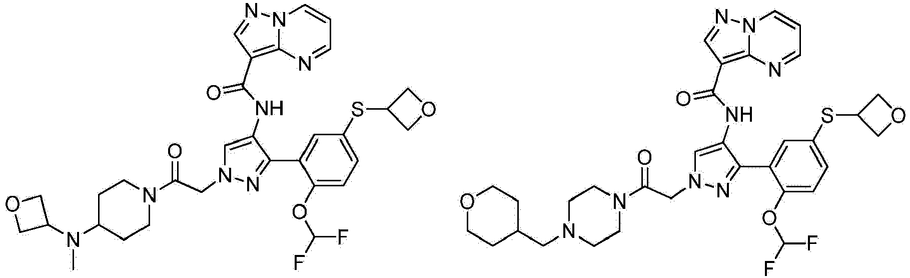 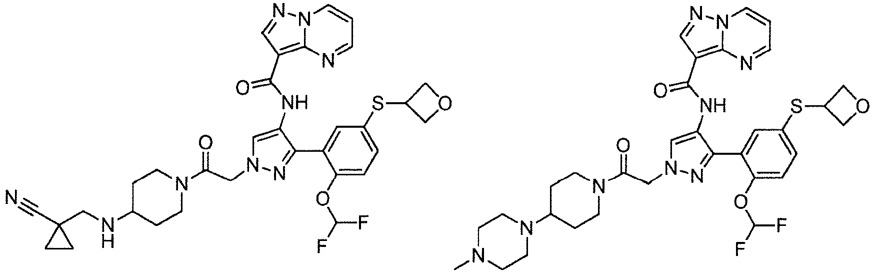 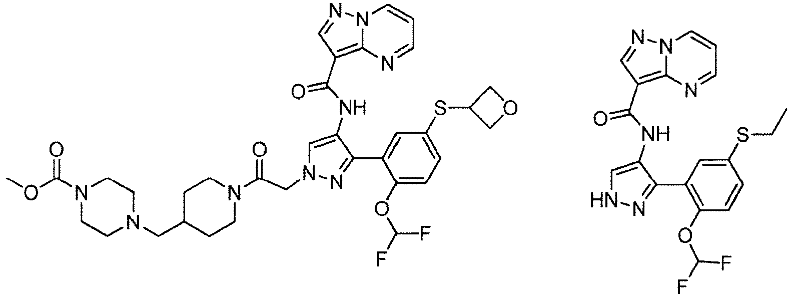 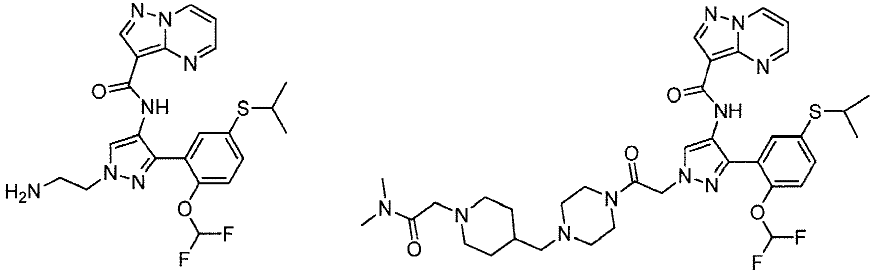 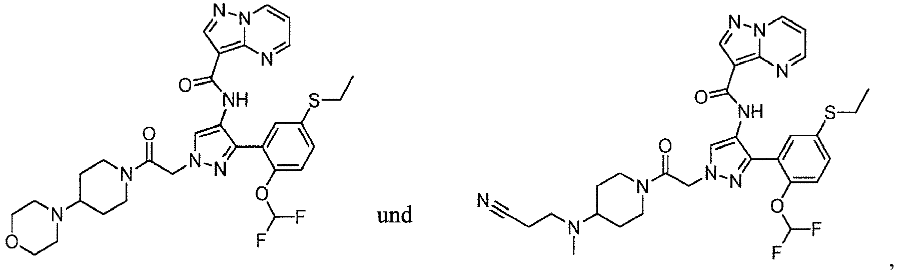 oder ein Salz davon.

15. Verbindung nach Anspruch 1, ausgewählt aus: 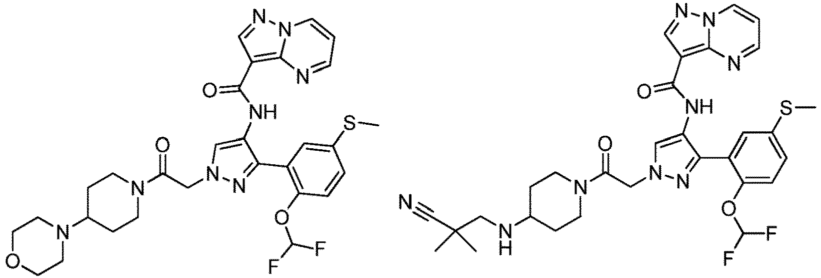 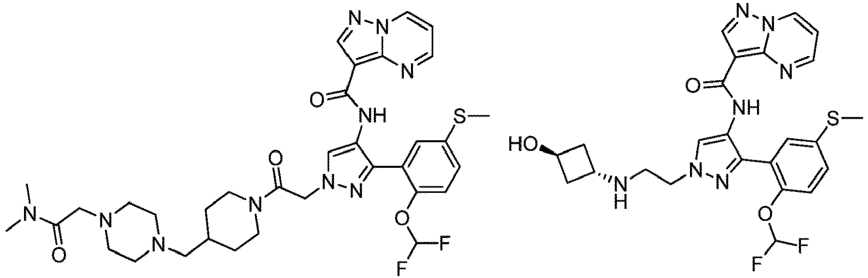 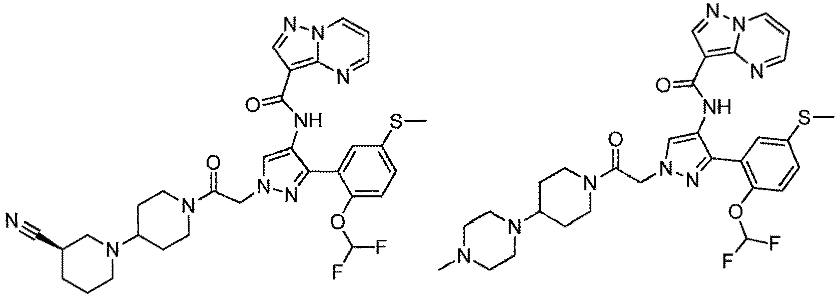 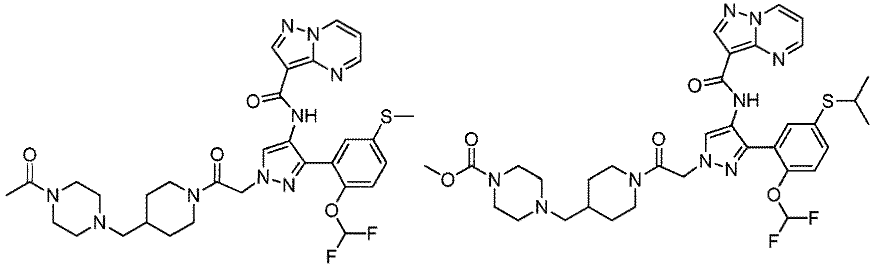 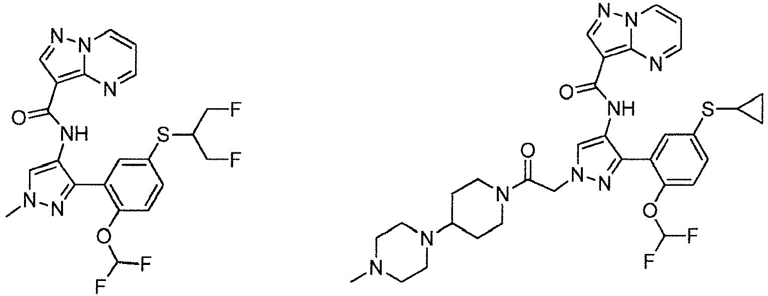 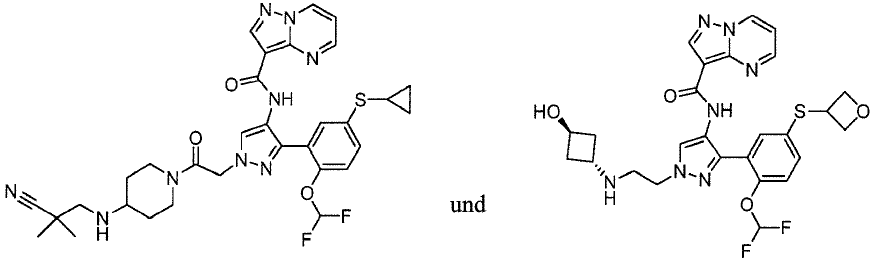 oder ein Salz davon.

16. Pharmazeutische Zusammensetzung, umfassend eine Verbindung nach einem der Ansprüche 1-15 oder ein Salz davon und einen pharmazeutisch unbedenklichen Träger, ein pharmazeutisch unbedenkliches Verdünnungsmittel oder einen pharmazeutisch unbedenklichen Hilfsstoff.

17. Verbindung nach einem der Ansprüche 1-15 oder ein pharmazeutisch unbedenkliches Salz davon zur Verwendung bei der Behandlung einer Entzündungskrankheit.

## Revendications

1. Composé de formule (00A) : 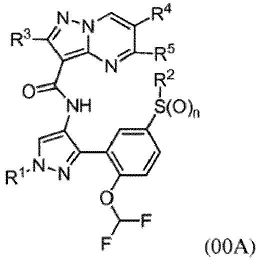 ou un de ses sels, dans laquelle :
R¹ représente H, un alkyle en C₁-C₆, un alcényle en C₂-C₆, un alcynyle en C₂-C₆, un -(alkyle en C₀-C₃)CN, un -(alkyle en C₀-C₃)OR^{a}, un -(alkyle en C₀-C₃)R^{a}, un -(alkyle en C₀-C₃)SR^{a}, un -(alkyle en C₀-C₃)NR^{a}R^{b}, un -(alkyle en C₀-C₃)OCF₃, un -(alkyle en C₀-C₃)CF₃, un -(alkyle en C₀-C₃)NO₂, un -(alkyle en C₀-C₃)C(O)R^{a}, un -(alkyle en C₀-C₃)C(O)OR^{a}, un -(alkyle en C₀-C₃)C(O)NR^{a}R^{b}, un -(alkyle en C₀-C₃)NR^{a}C(O)R^{b}, un -(alkyle en C₀-C₃)S(O)₁₋₂R^{a}, un -(alkyle en C₀-C₃)NR^{a}S(O)₁₋₂R^{b}, un -(alkyle en C₀-C₃)S(O)₁₋₂NR^{a}R^{b}, un -(alkyle en C₀-C₃)(hétéroaryle à 5 à 6 chaînons) ou un -(alkyle en C₀-C₃)phényle, R¹ étant éventuellement substitué par un ou plusieurs groupes choisis indépendamment dans le groupe constitué par un halogène, un alkyle en C₁-C₃, un oxo, un -CF₃, un -(alkyle en C₀-C₃)OR^{c} et un -(alkyle en C₀-C₃)NR^{c}R^{d} ;
R^{a} représente indépendamment un hydrogène, un alkyle en C₁-C₆, un cycloalkyle en C₃-C₆, un hétérocyclyle à 3 à 10 chaînons, un hétéroaryle à 5 à 6 chaînons, -C(O)R^{c}, -C(O)OR^{c}, -C(O)NR^{c}R^{d}, -NR^{c}C(O)R^{d}, -S(O)₁₋₂R^{c}, -NR^{c}S(O)₁₋₂R^{d} ou -S(O)₁₋₂NR^{c}R^{d}, tout cycloalkyle en C₃-C₆, hétérocyclyle à 3 à 10 chaînons et hétéroaryle à 5 à 6 chaînons de R^{a} étant éventuellement substitué par un ou plusieurs groupes R^{e} ;
R^{b} représente indépendamment un hydrogène ou un alkyle en C₁-C₃, ledit alkyle étant éventuellement substitué par un ou plusieurs groupes choisis indépendamment dans le groupe constitué par un halogène et un oxo ; ou
R^{c} et R^{d} sont choisis indépendamment dans le groupe constitué par un hydrogène, un hétérocyclyle à 3 à 6 chaînons, un cycloalkyle en C₃-C₆ et un alkyle en C₁-C₃, tout hétérocyclyle à 3 à 6 chaînons, cycloalkyle en C₃-C₆ et alkyle en C₁-C₃ de R^{c} et R^{d} étant éventuellement substitué par un ou plusieurs groupes choisis indépendamment dans le groupe constitué par un halogène et un oxo ; ou R^{c} et R^{d} sont pris conjointement avec l'atome auquel ils sont attachés pour former un hétérocyclyle à 3 à 6 chaînons, éventuellement substitué par un ou plusieurs groupes choisis indépendamment dans le groupe constitué par un halogène, un oxo, un -CF₃ et un alkyle en C₁-C₃ ;
chaque R^{e} est choisi indépendamment dans le groupe constitué par un oxo, OR^{f}, NR^{f}R^{g}, un halogène, un hétérocyclyle à 3 à 10 chaînons, un cycloalkyle en C₃-C₆ et un alkyle en C₁-C₆, tout cycloalkyle en C₃-C₆ et alkyle en C₁-C₆ de R^{e} étant éventuellement substitué par un ou plusieurs groupes choisis indépendamment dans le groupe constitué par OR^{f}, NR^{f}R^{g}, un halogène, un hétérocyclyle à 3 à 10 chaînons, un oxo et un cyano, et tout hétérocyclyle à 3 à 10 chaînons de R^{e} étant éventuellement substitué par un ou plusieurs groupes choisis indépendamment dans le groupe constitué par un halogène, un oxo, un cyano, un -CF₃, NR^{h}R^{k}, un hétérocyclyle à 3 à 6 chaînons et un alkyle en C₁-C₃ qui est éventuellement substitué par un ou plusieurs groupes choisis indépendamment dans le groupe constitué par un halogène, un oxo, OR^{f} et NR^{h}R^{k} ;
R^{f} et R^{g} sont chacun choisis indépendamment dans le groupe constitué par un hydrogène, un alkyle en C₁-C₆, un hétérocyclyle à 3 à 6 chaînons et un cycloalkyle en C₃-C₆, tout alkyle en C₁-C₆, hétérocyclyle à 3 à 6 chaînons et cycloalkyle en C₃-C₆ de R^{f} et R^{g} étant éventuellement substitué par un ou plusieurs R^{m} ;
chaque R^{m} est choisi indépendamment dans le groupe constitué par un halogène, un cyano, un oxo, un cycloalkyle en C₃-C₆, un hétérocyclyle à 3 à 6 chaînons, un hydroxy et NR^{h}R^{k}, tout cycloalkyle en C₃-C₆ et hétérocyclyle à 3 à 6 chaînons de R^{m} étant éventuellement substitué par un ou plusieurs groupes choisis indépendamment dans le groupe constitué par un halogène, un oxo, un cyano et un alkyle en C₁-C₃ ;
R^{h} et R^{k} sont choisis chacun indépendamment dans le groupe constitué par un hydrogène et un alkyle en C₁-C₆ qui est éventuellement substitué par un ou plusieurs groupes choisis indépendamment dans le groupe constitué par un halogène, un cyano, un hétérocyclyle à 3 à 6 chaînons et un oxo ; ou R^{h} et R^{k} sont pris conjointement avec l'atome auquel ils sont attachés pour former un hétérocyclyle à 3 à 6 chaînons qui est éventuellement substitué par un ou plusieurs groupes choisis indépendamment dans le groupe constitué par un halogène, un cyano, un oxo, un -CF₃ et un alkyle en C₁-C₃ qui est éventuellement substitué par un ou plusieurs groupes choisis indépendamment dans le groupe constitué par un halogène et un oxo ;
R² représente un alkyle en C₁-C₆, un alcényle en C₂-C₆, un alcynyle en C₂-C₆, un cycloalkyle en C₃-C₆, un hétérocyclyle à 3 à 6 chaînons, un (cycloalkyle en C₃-C₆)alkyle en C₁-C₆, un (hétérocyclyle à 3 à 6 chaînons)alkyle en C₁-C₆, un -C(O)(cycloalkyle en C₃-C₆) ou un -C(O)(hétérocyclyle à 3 à 6 chaînons), R² étant éventuellement substitué par un ou plusieurs halogènes ;
n représente 0, 1, ou 2 ;
R³ représente H ou NH₂ ;
R⁴ représente H ou CH₃ ; et
R⁵ représente H ou NH₂.

2. Composé selon la revendication 1 dans lequel R¹ représente H, un alkyle en C₁-C₆, un -(alkyle en C₀-C₃)R^{a}, un -(alkyle en C₀-C₃)NR^{a}R^{b}, un -(alkyle en C₀-C₃)C(O)R^{a} ou un -(alkyle en C₀-C₃)C(O)OR^{a}, ou un de ses sels.

3. Composé selon la revendication 1 dans lequel R¹ représente H, ou un de ses sels.

4. Composé de la revendication 1 dans lequel R¹ représente un alkyle en C₁-C₆, un alcényle en C₂-C₆, un alcynyle en C₂-C₆, un -(alkyle en C₀-C₃)CN, un -(alkyle en C₀-C₃)OR^{a}, un -(alkyle en C₀-C₃)R^{a}, un -(alkyle en C₀-C₃)SR^{a}, un -(alkyle en C₀-C₃)NR^{a}R^{b}, un -(alkyle en C₀-C₃)OCF₃, un -(alkyle en C₀-C₃)CF₃, un -(alkyle en C₀-C₃)NO₂, un -(alkyle en C₀-C₃)C(O)R^{a}, un -(alkyle en C₀-C₃)C(O)OR^{a}, un -(alkyle en C₀-C₃)C(O)NR^{a}R^{b}, un -(alkyle en C₀-C₃)NR^{a}C(O)R^{b}, un -(alkyle en C₀-C₃)S(O)₁₋₂R^{a}, un -(alkyle en C₀-C₃)NR^{a}S(O)₁₋₂R^{b}, un -(alkyle en C₀-C₃)S(O)₁₋₂NR^{a}R^{b}, un -(alkyle en C₀-C₃)(hétéroaryle à 5 à 6 chaînons) ou un -(alkyle en C₀-C₃)phényle, R¹ étant éventuellement substitué par un ou plusieurs groupes choisis indépendamment dans le groupe constitué par un halogène, un alkyle en C₁-C₃, un oxo, un -CF₃, un -(alkyle en C₀-C₃)OR^{c} et un -(alkyle en C₀-C₃)NR^{c}R^{d}.

5. Composé selon la revendication 1 ou un de ses sels, dans lequel R¹ est choisi dans le groupe constitué par H, un méthyle,

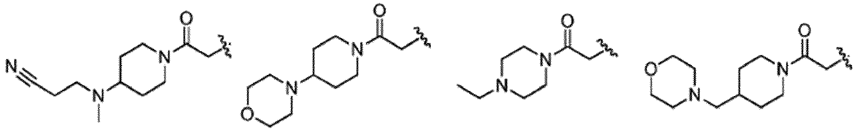

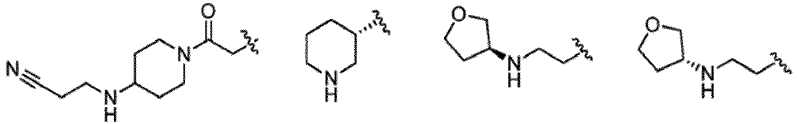

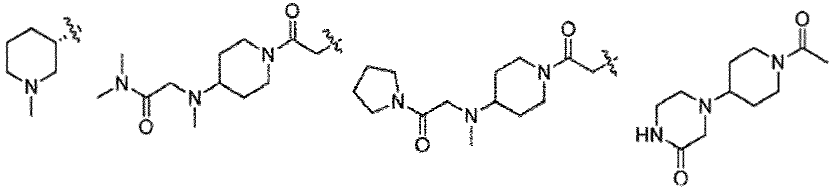

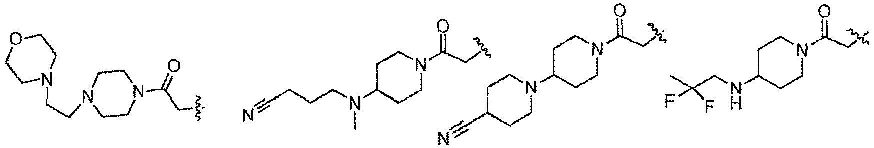

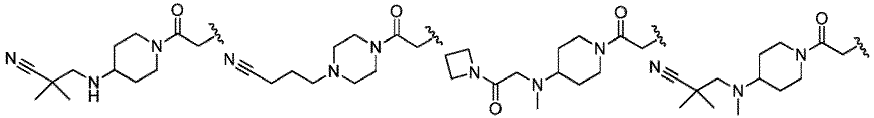

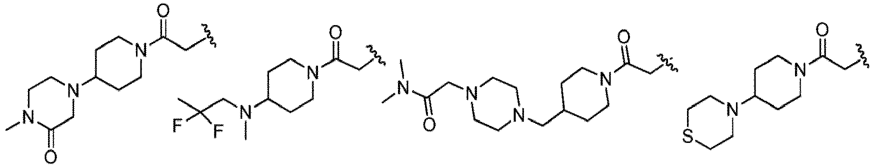

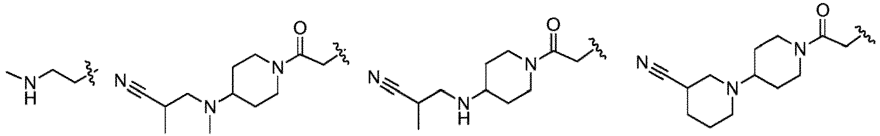

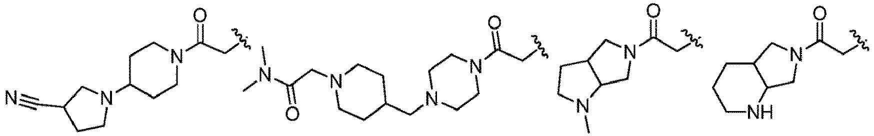

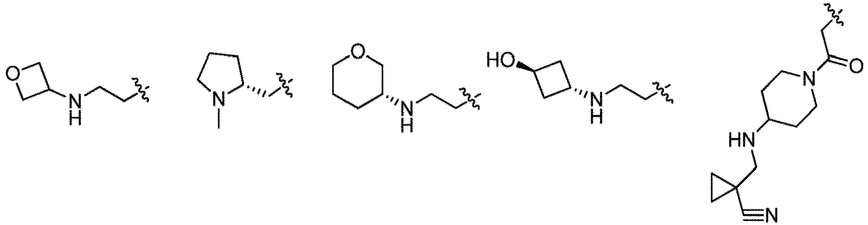

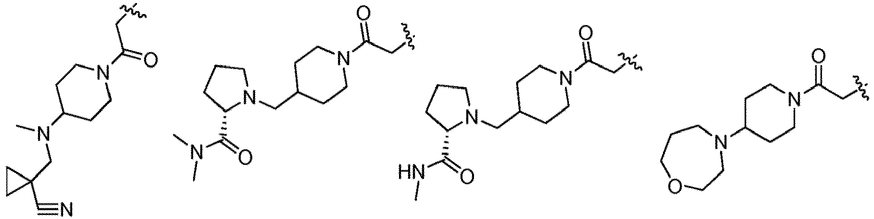

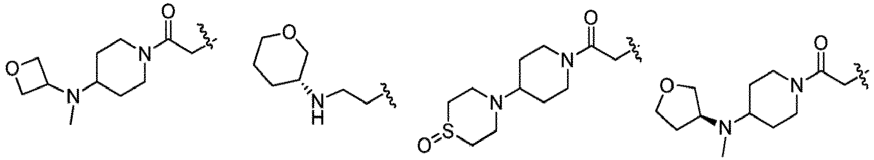

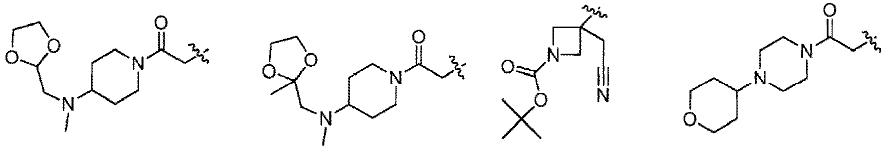

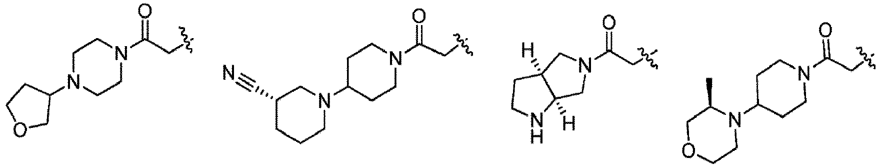

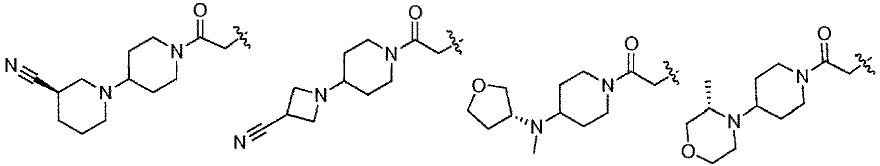

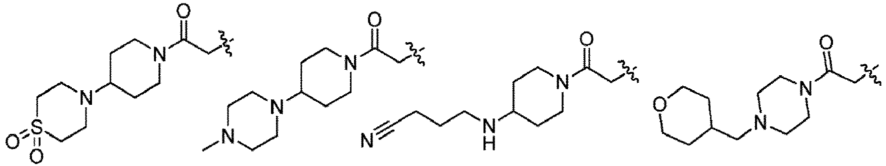

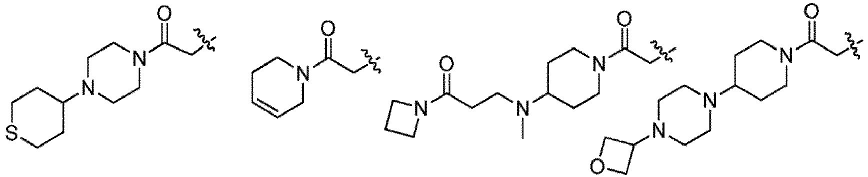

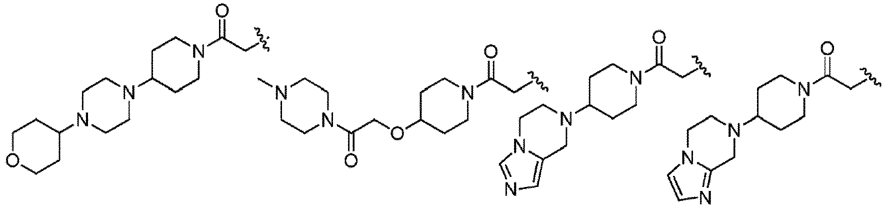

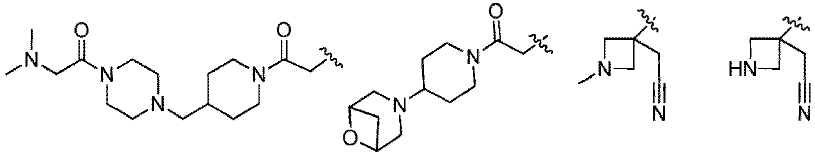

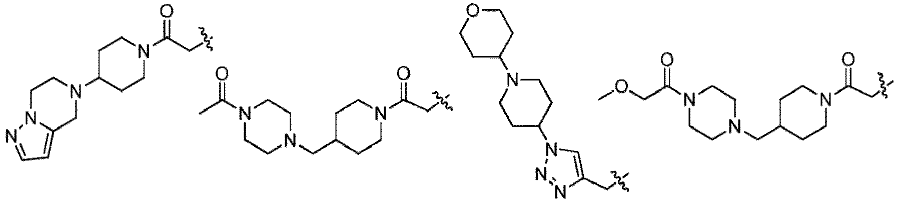

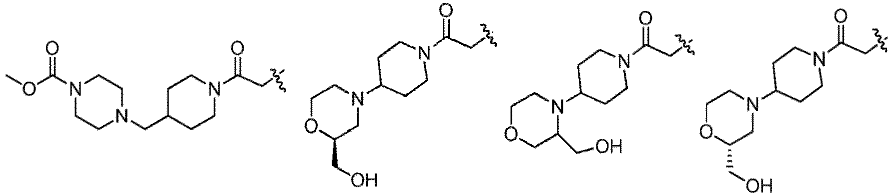

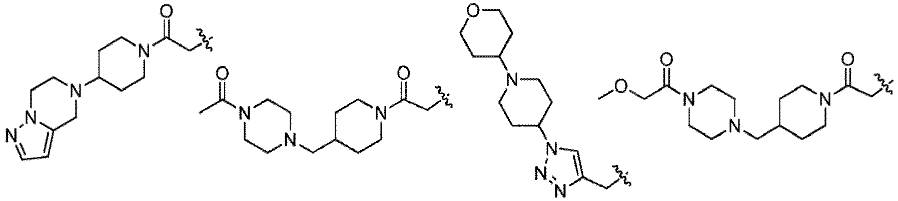

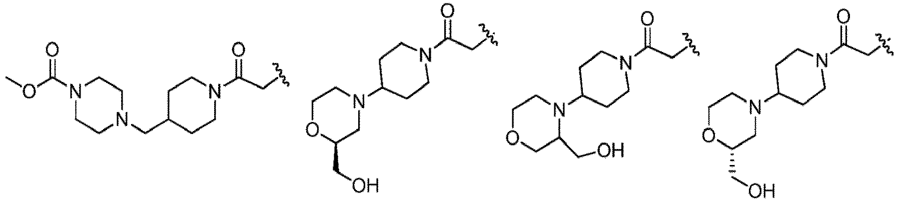

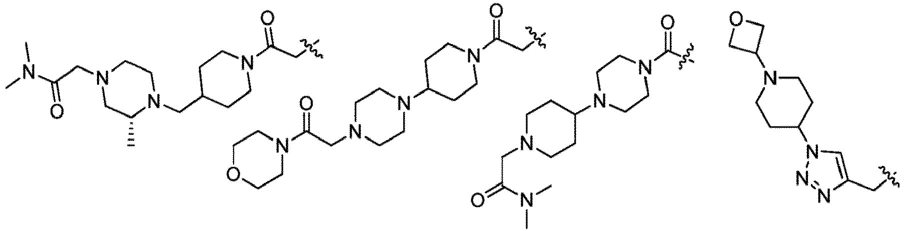

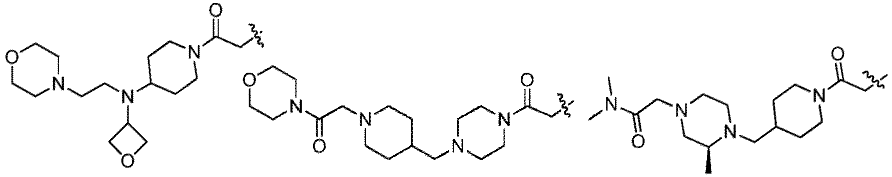

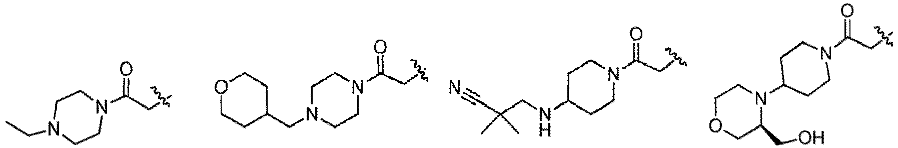

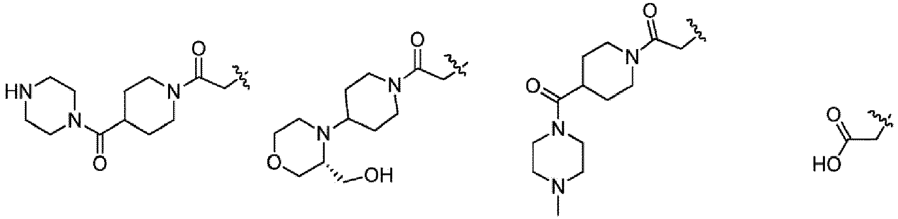

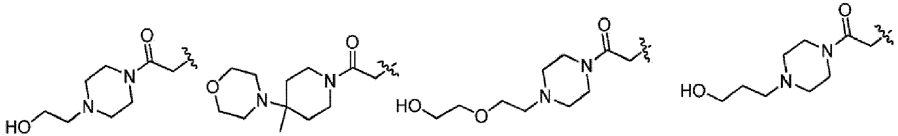

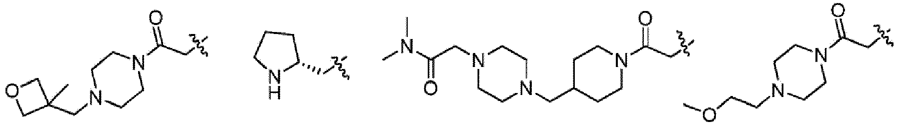

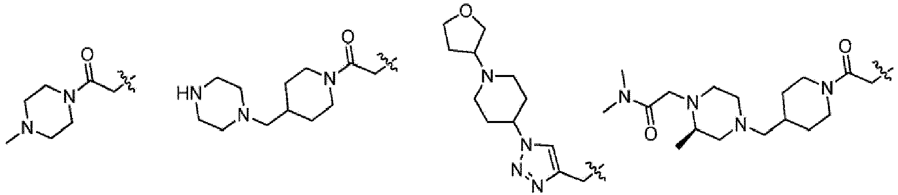

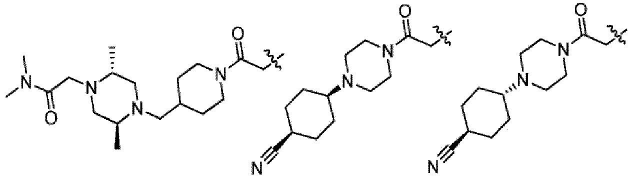

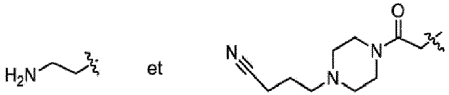

6. Composé de l'une quelconque des revendications 1 à 5 dans lequel R² représente un alkyle en C₁-C₆, un cycloalkyle en C₃-C₆ ou un hétérocyclyle à 3 à 6 chaînons, ou un de ses sels.

7. Composé selon l'une quelconque des revendications 1 à 5 dans lequel R² représente un méthyle, un éthyle, un isopropyle, un cyclopropyle ou un oxétanyle, ou un de ses sels.

8. Composé de l'une quelconque des revendications 1 à 7 dans lequel n représente 1, ou un de ses sels.

9. Composé selon l'une quelconque des revendications 1 à 7 dans lequel n représente 2, ou un de ses sels.

10. Composé selon la revendication 1 qui est
a) un composé de formule (00B) : 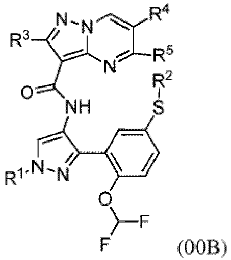 ou un de ses sels ;
b) un composé de formule (00C) : 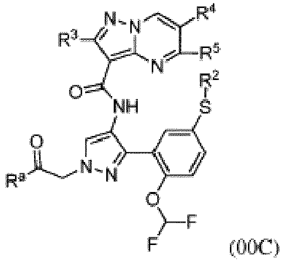 ou un de ses sels ;
c) un composé de formule (00D) : 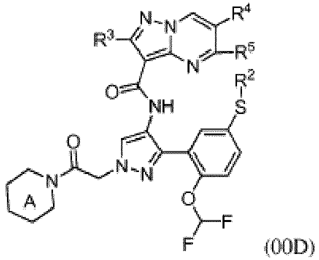 dans laquelle le cycle A est éventuellement substitué par un ou plusieurs groupes R^{e} ; ou un de ses sels ;
d) un composé de formule (00E) : 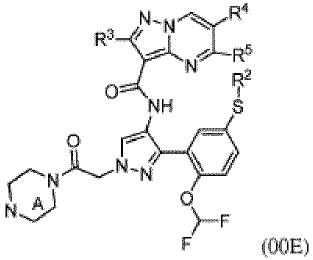 dans laquelle le cycle A est éventuellement substitué par un ou plusieurs groupes R^{e} ; ou un de ses sels ;
e) un composé de formule (00F) : 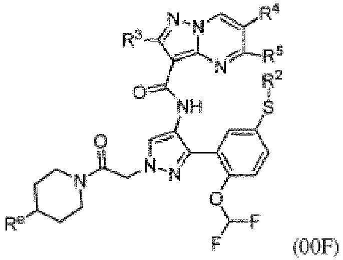 ou un de ses sels ; ou
f) un composé de formule (00G) : 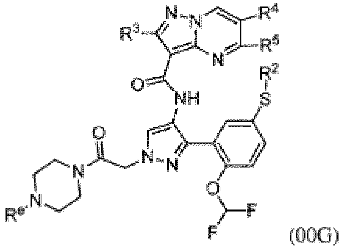 ou un de ses sels.

11. Composé selon la revendication 10 dans lequel R² représente un alkyle en C₁-C₆, un cycloalkyle en C₃-C₆ ou un hétérocyclyle à 3 à 6 chaînons, ou un de ses sels.

12. Composé selon la revendication 10 dans lequel R² représente un méthyle, un éthyle, un isopropyle, un cyclopropyle ou un oxétanyle, ou un de ses sels.

13. Composé selon les revendications 10 à 12 dans lequel R^{e} est choisi dans le groupe constitué par un méthyle, un éthyle,

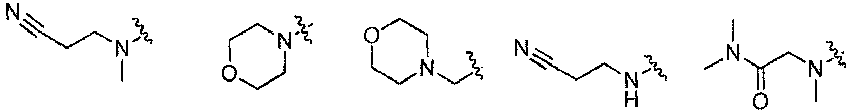

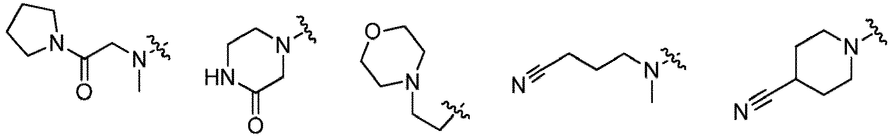

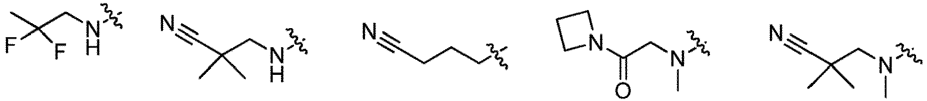

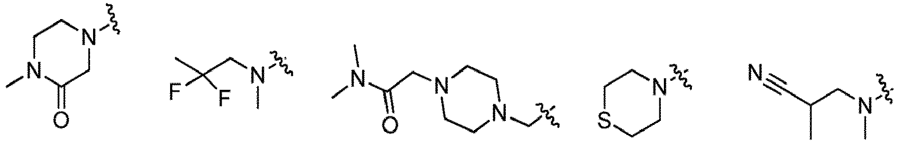

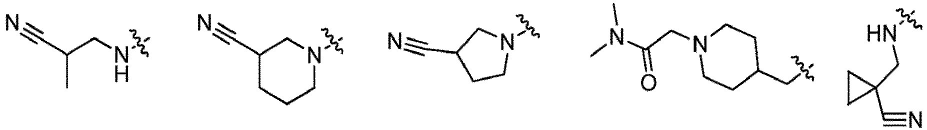

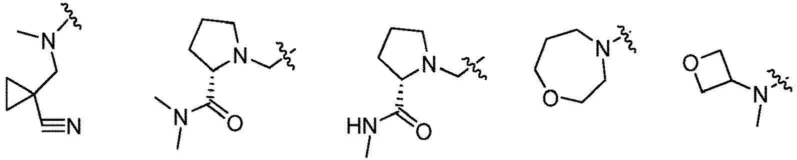

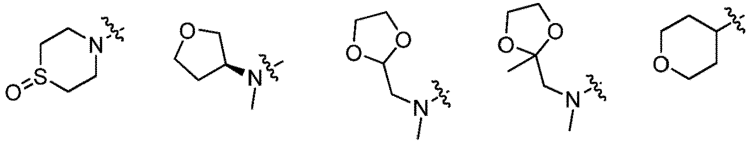

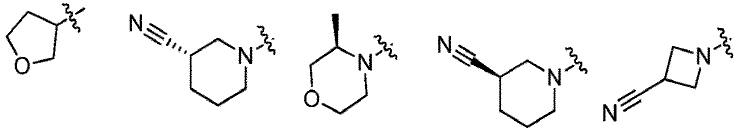

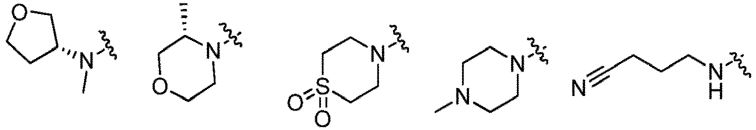

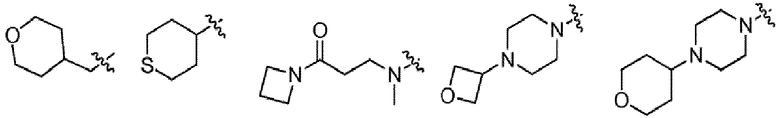

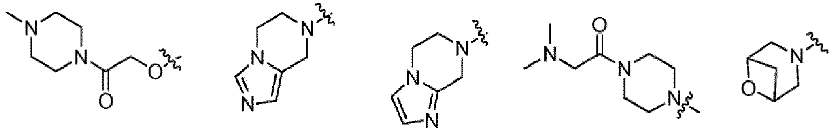

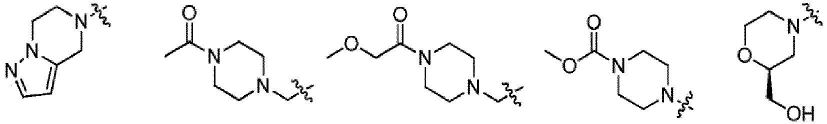

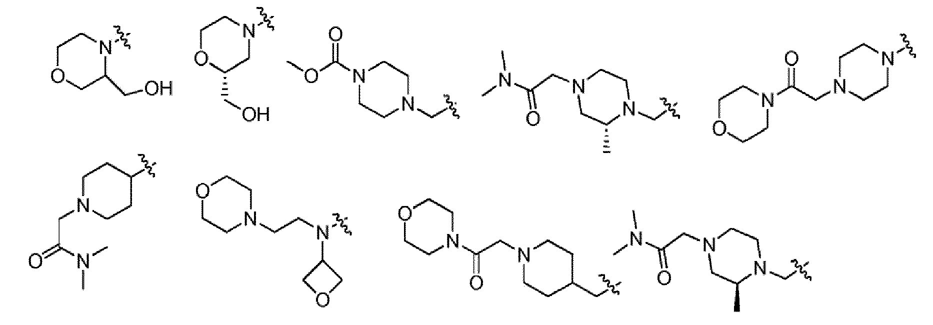

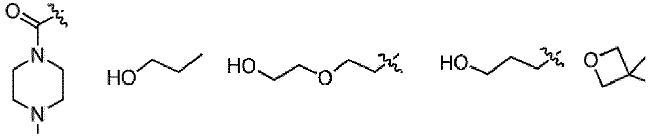

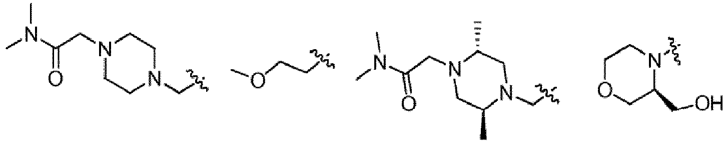

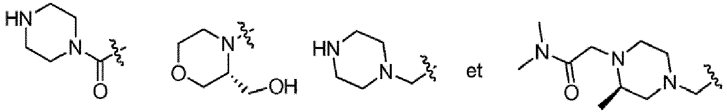

14. Composé selon la revendication 1 choisi parmi : 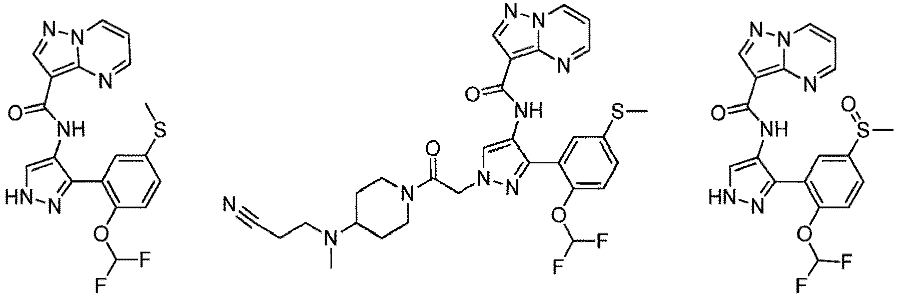 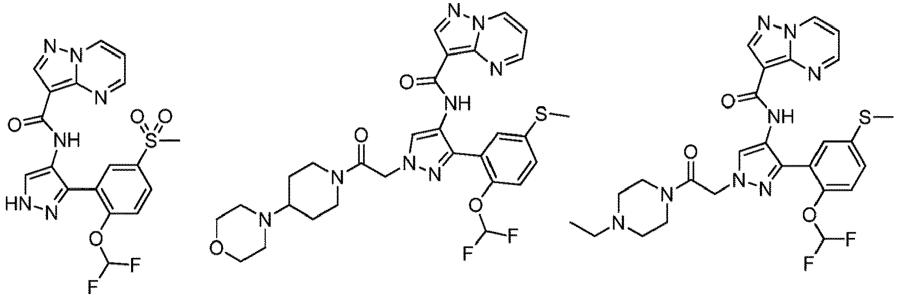 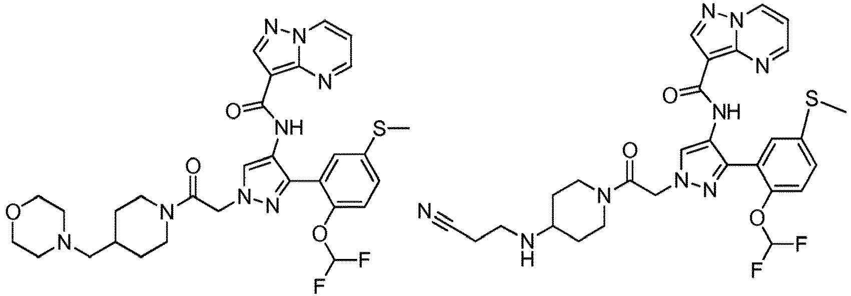 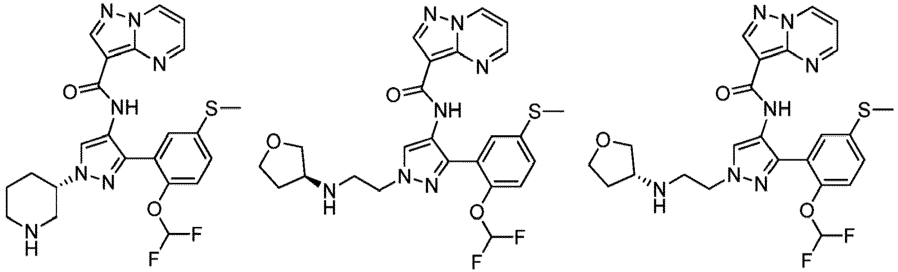 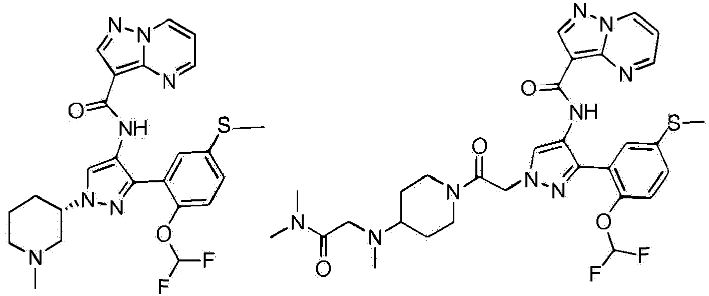 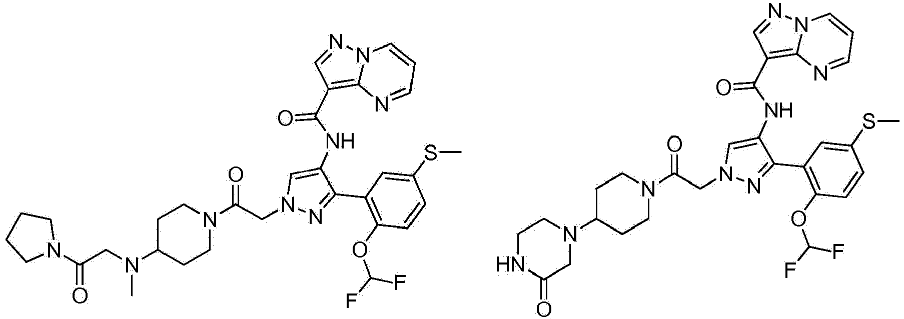 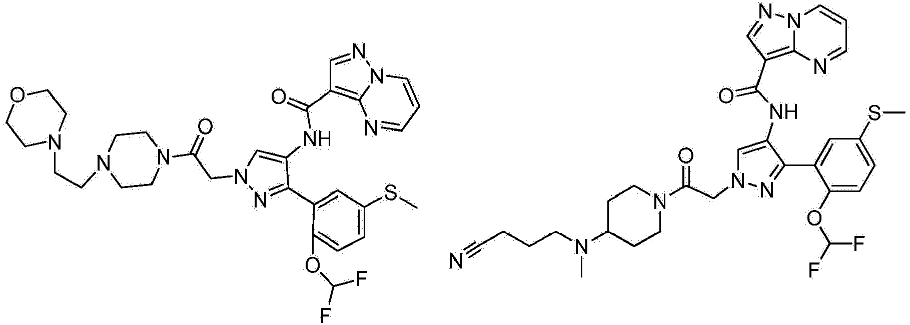 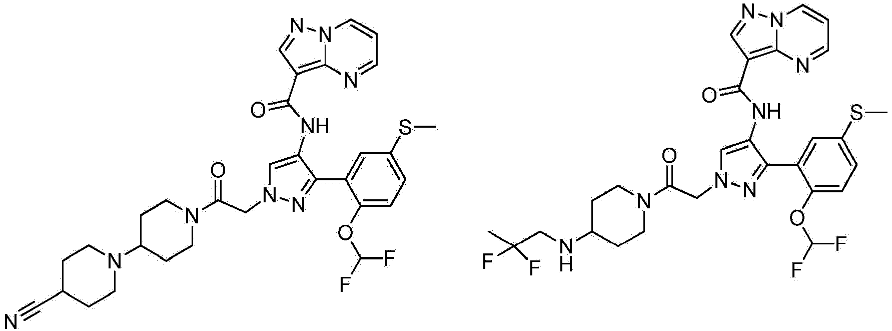 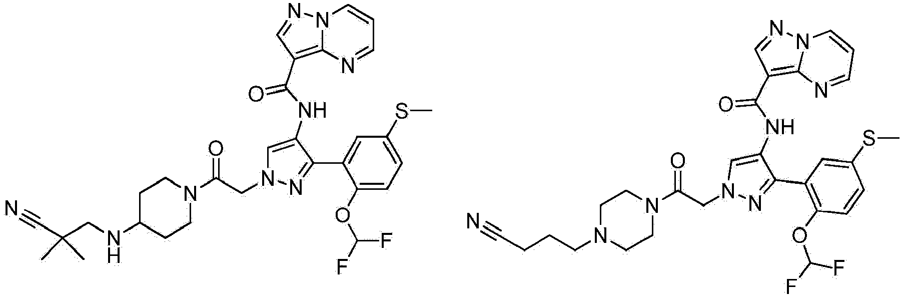 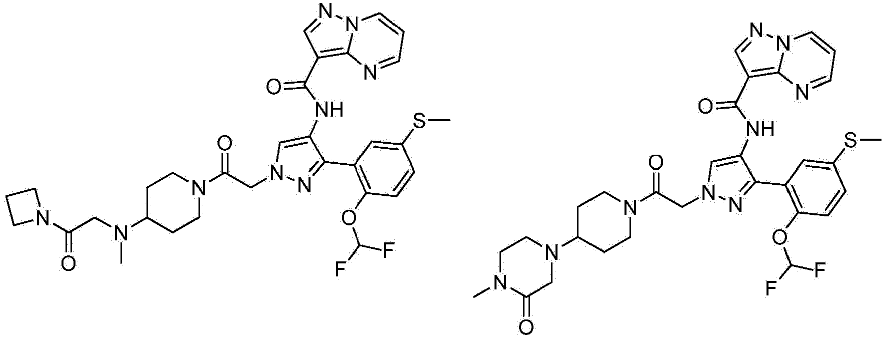 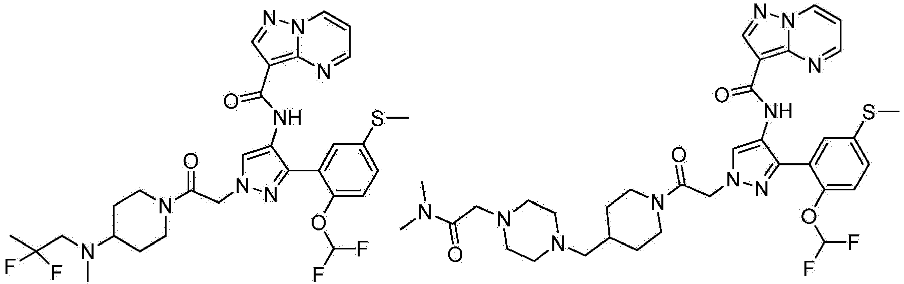 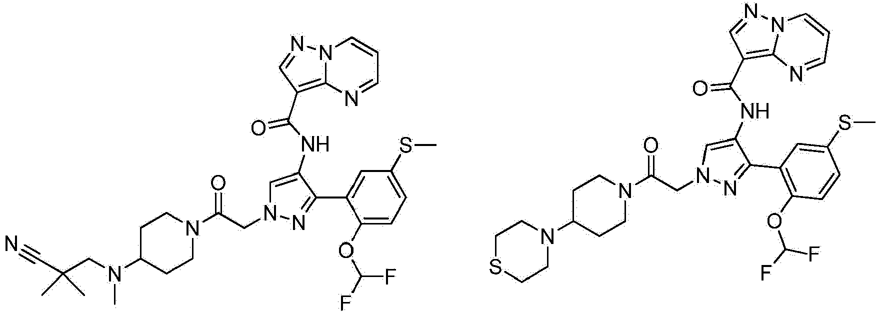 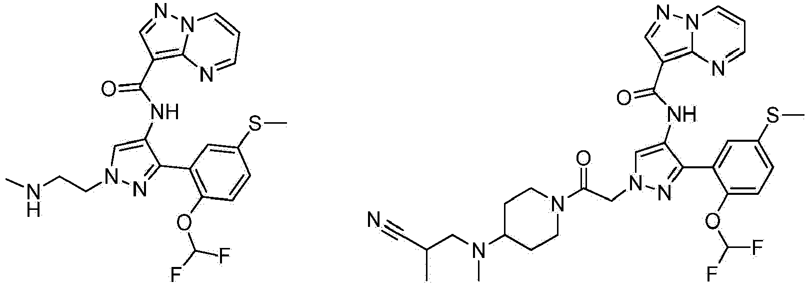 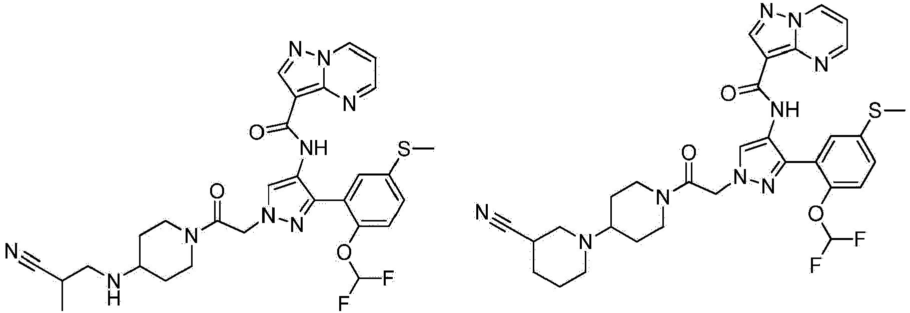 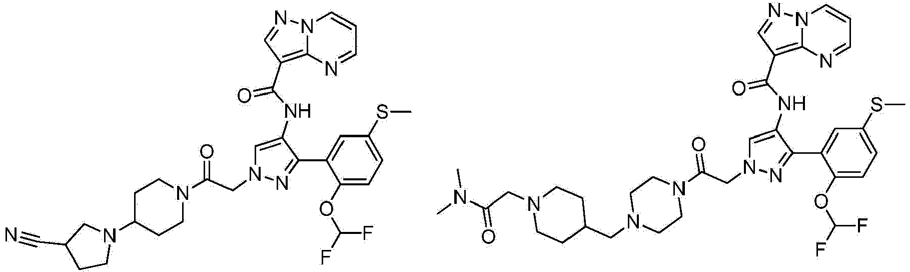 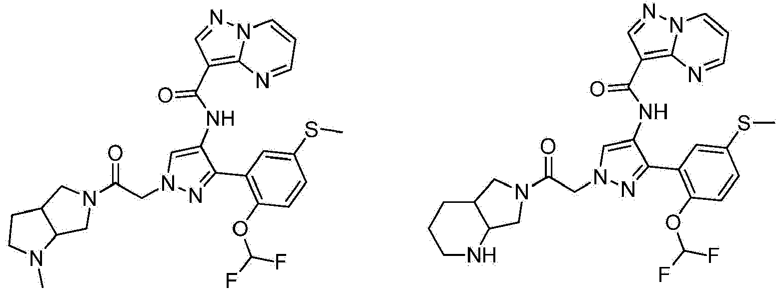 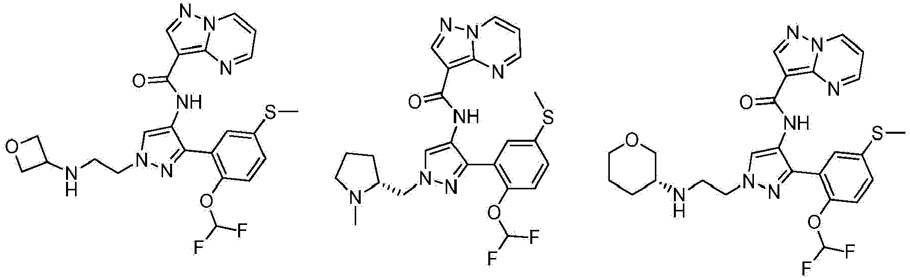 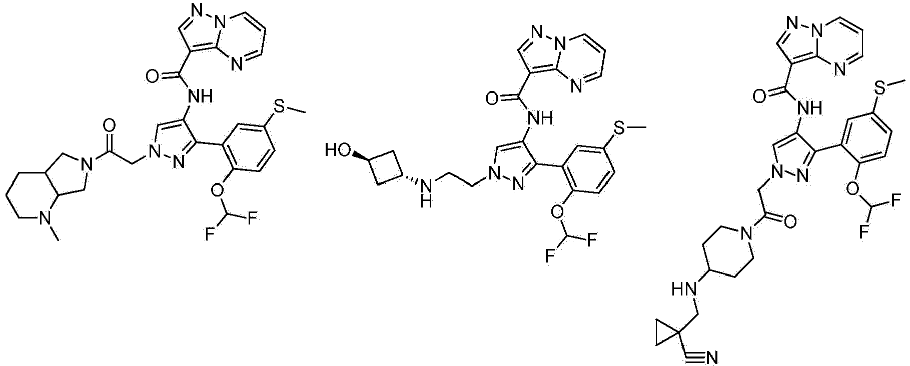 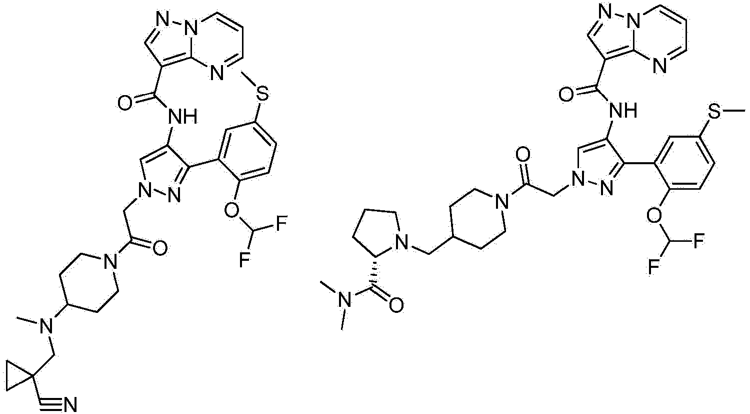 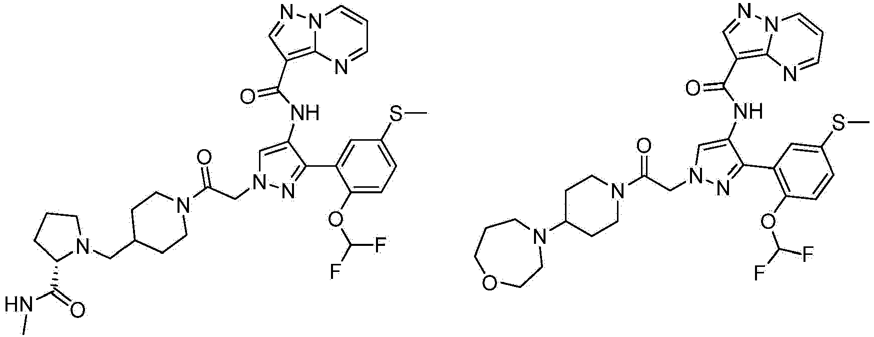 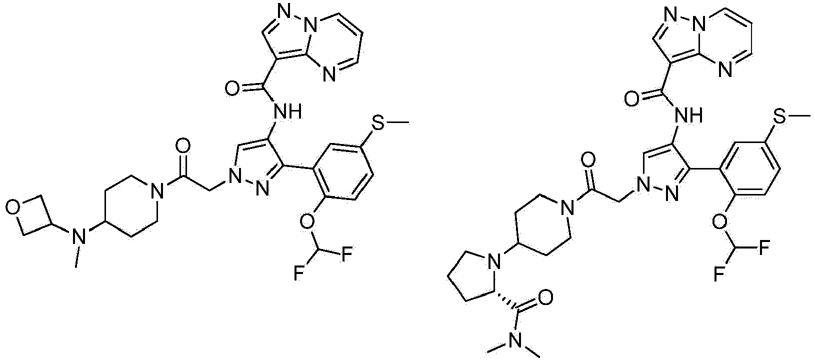 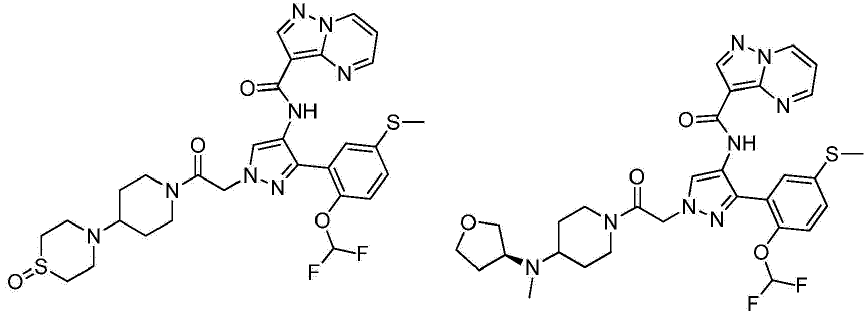 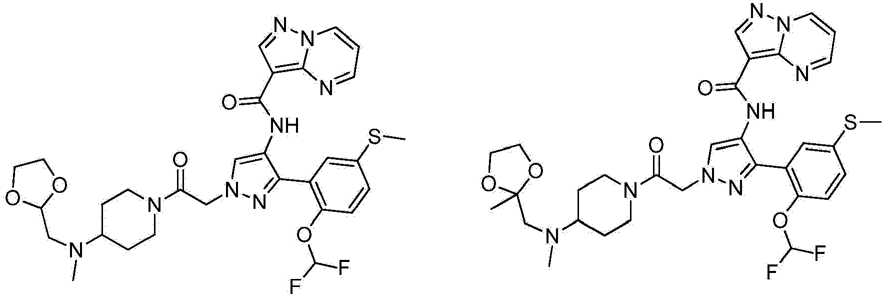 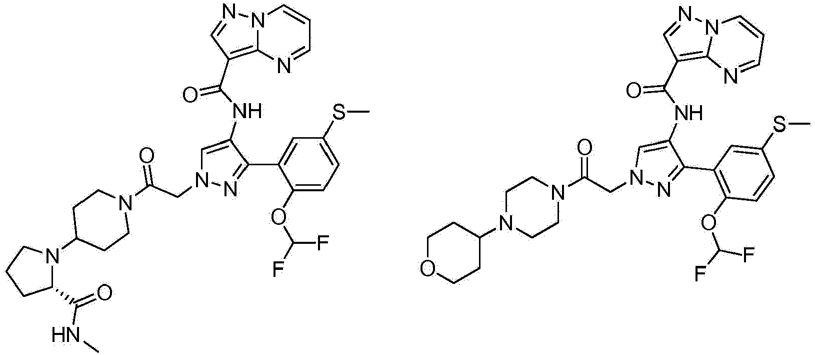 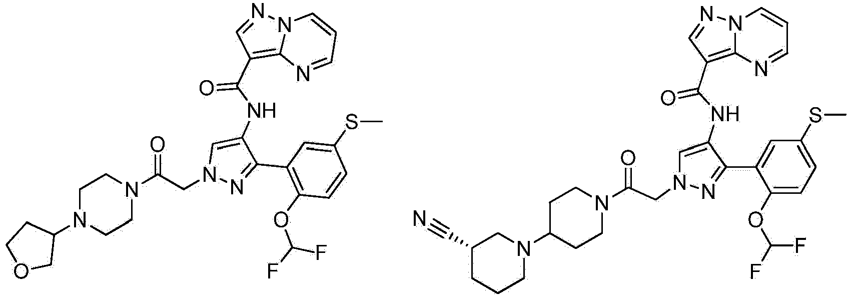 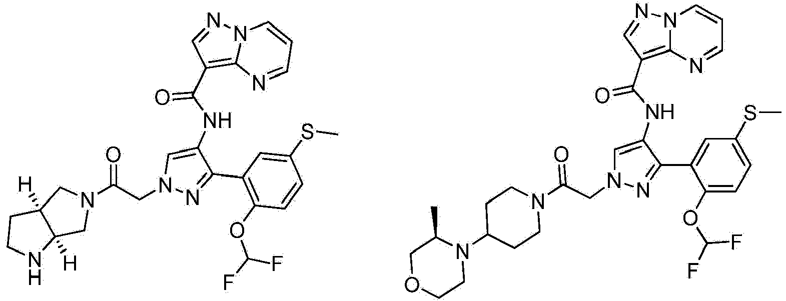 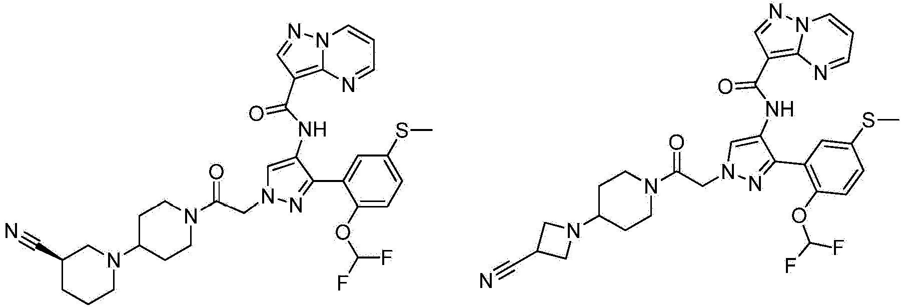 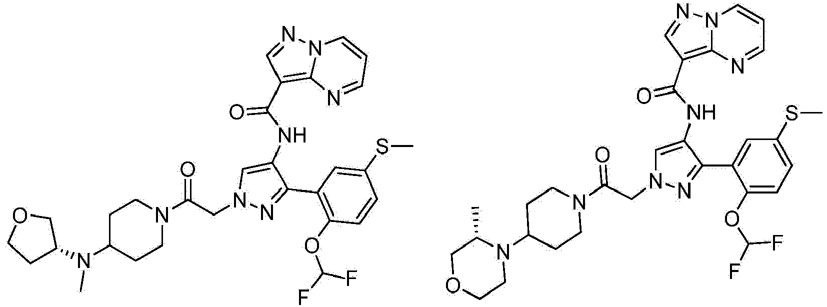 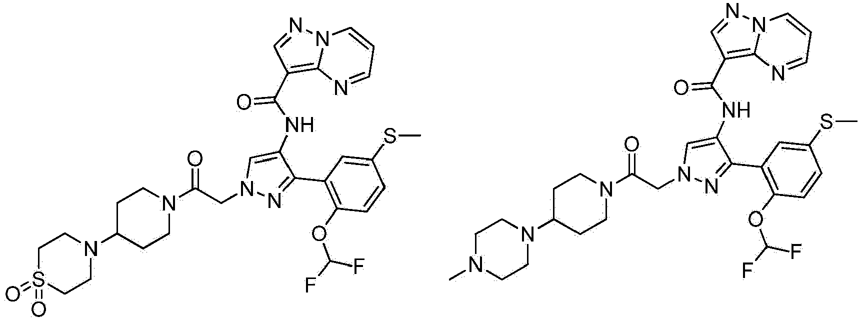 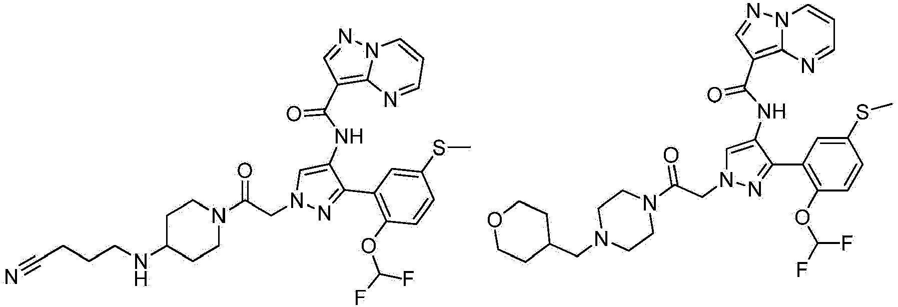 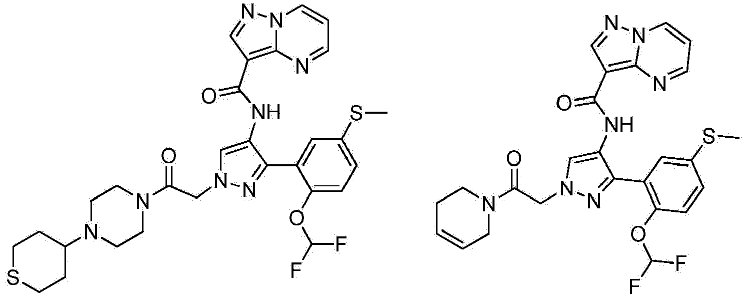 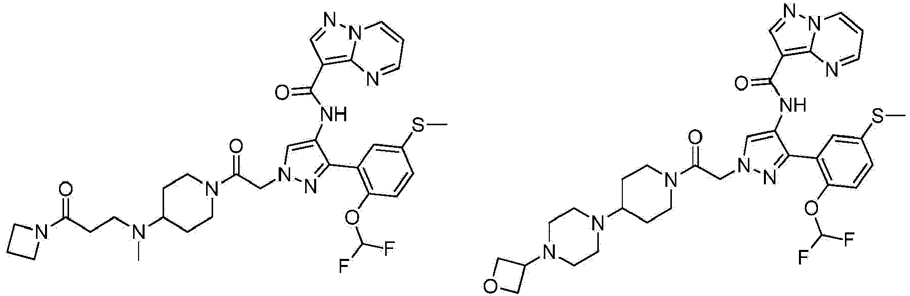 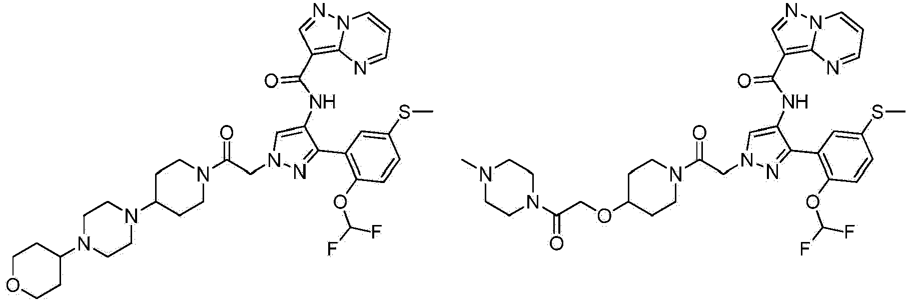 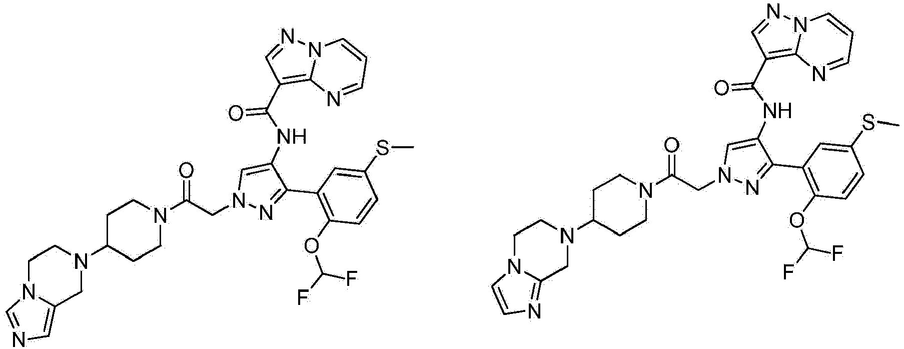 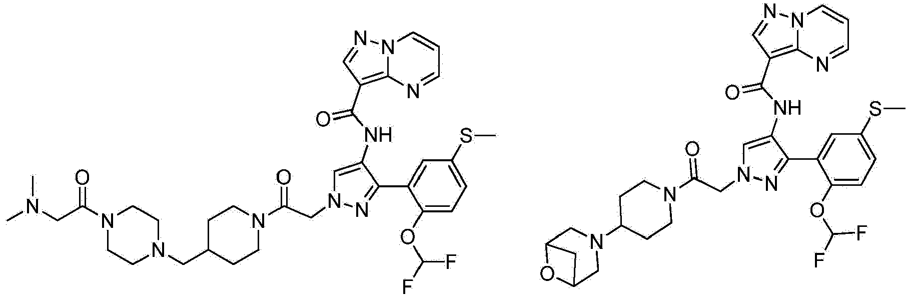 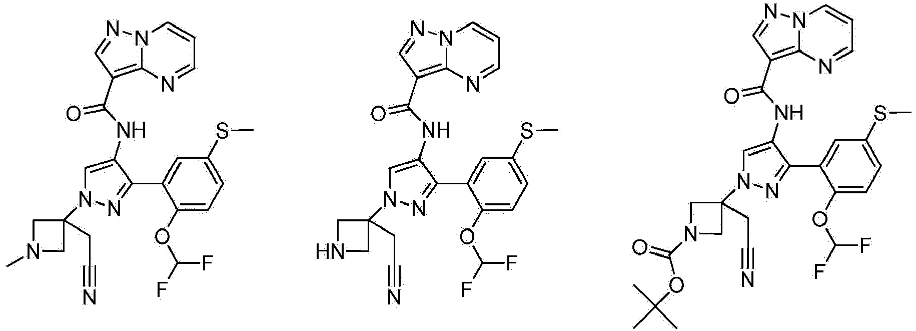 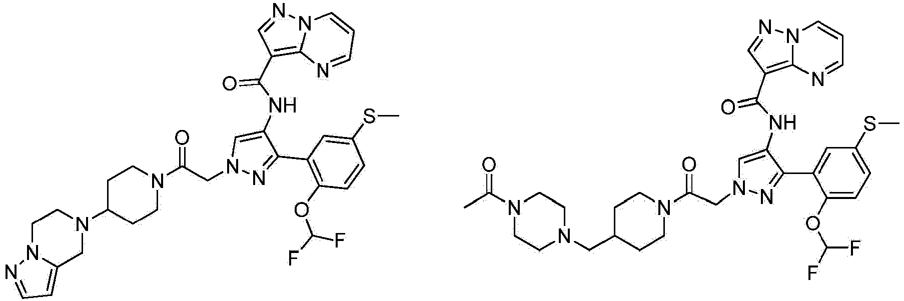 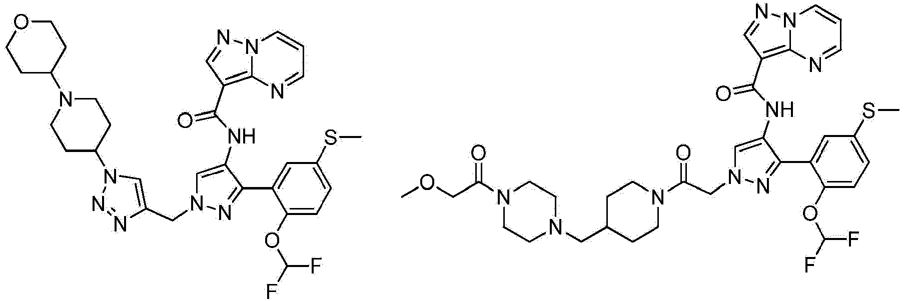 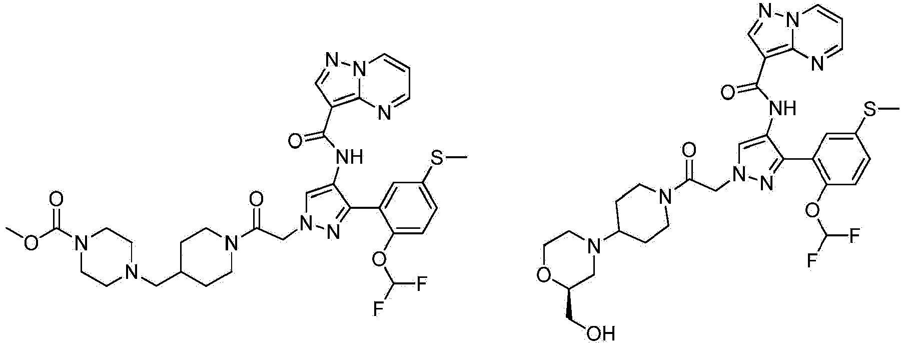 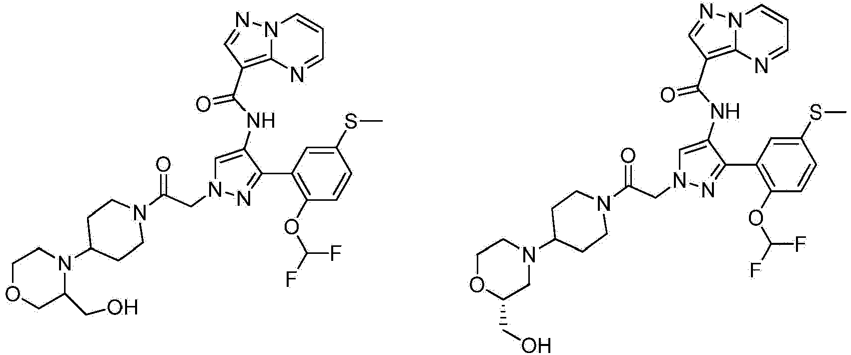 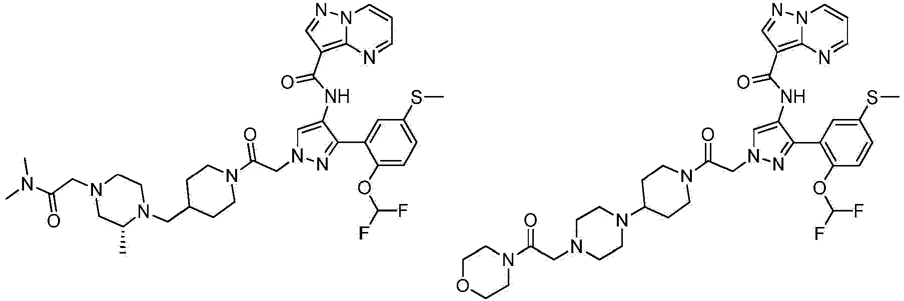 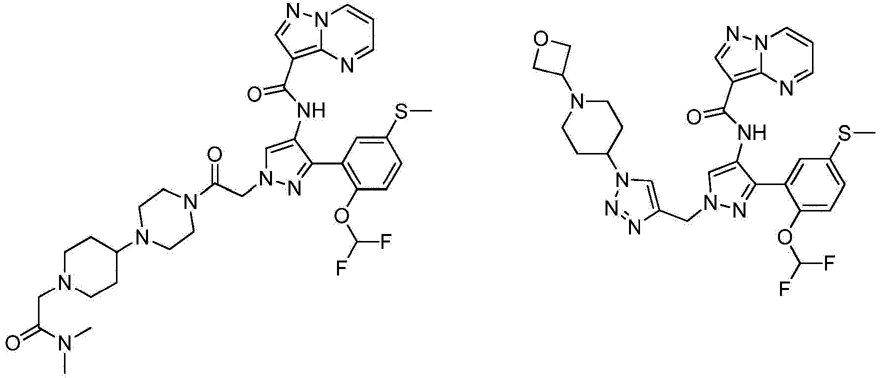 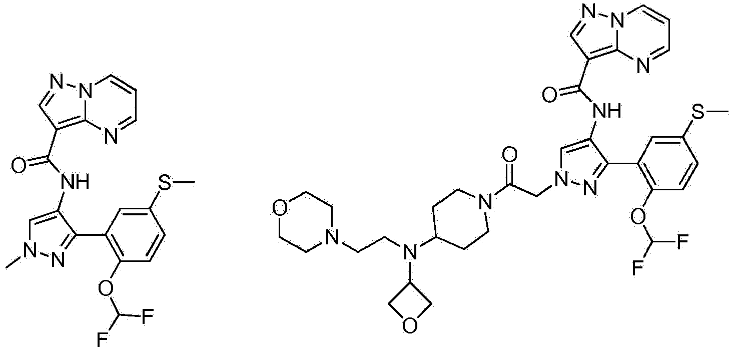 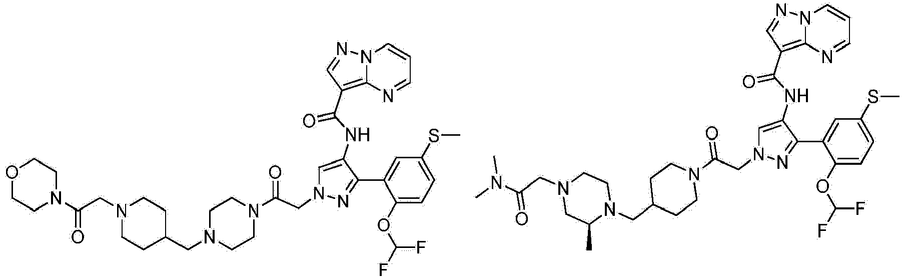 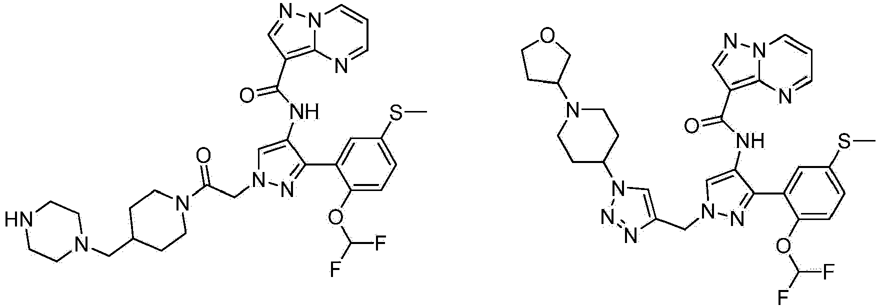 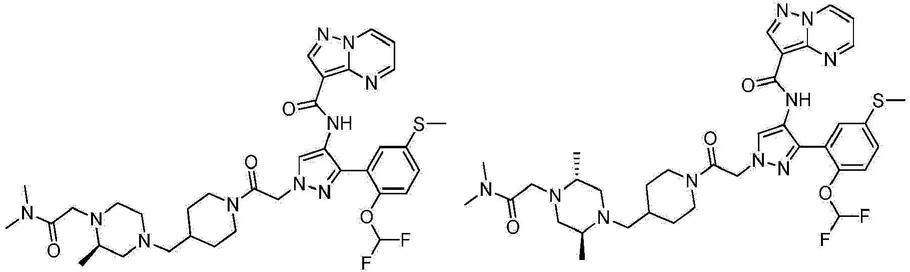 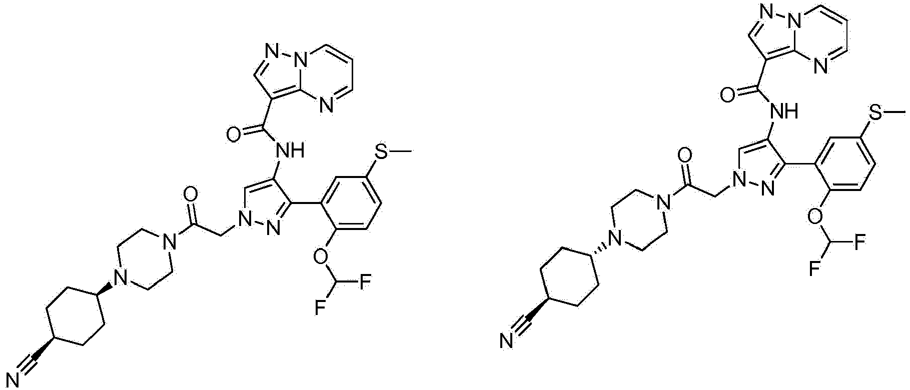 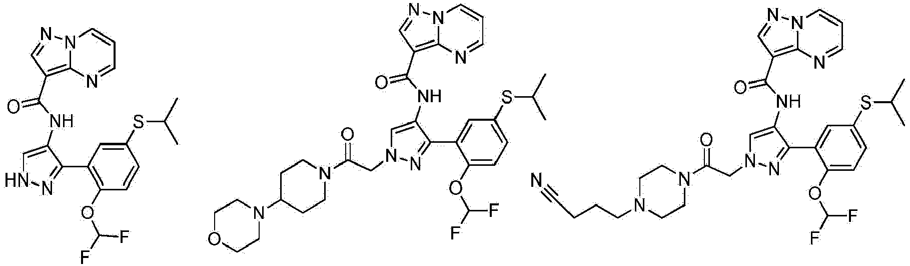 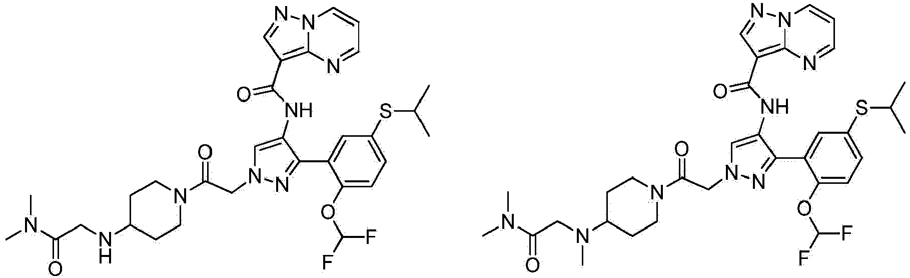 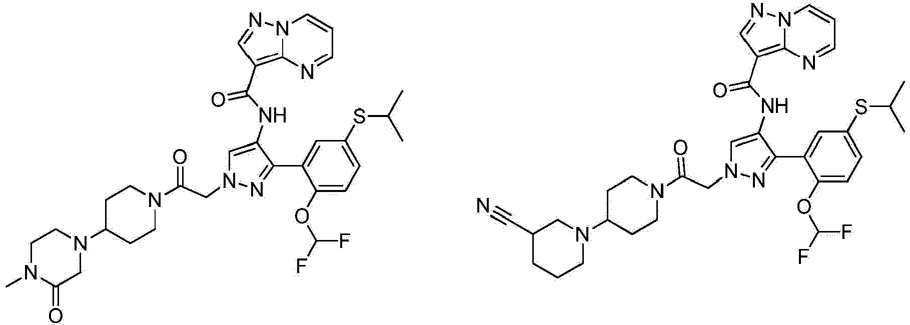 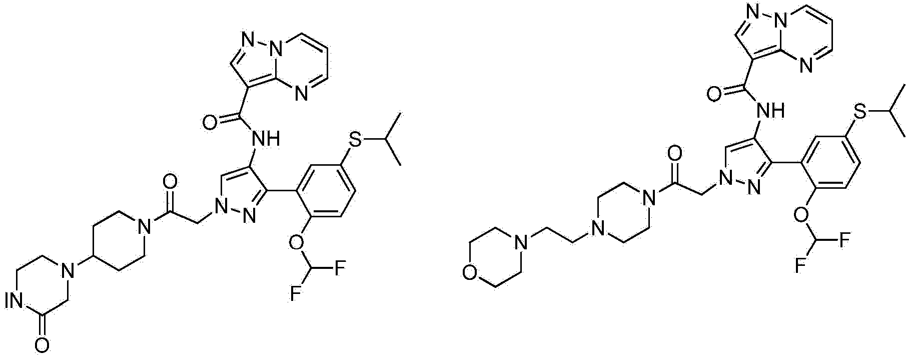 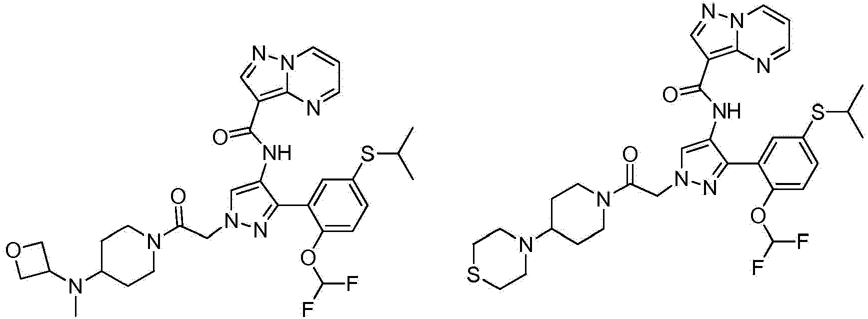 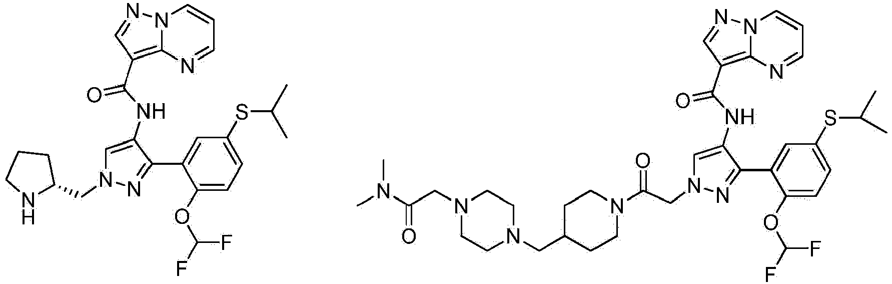 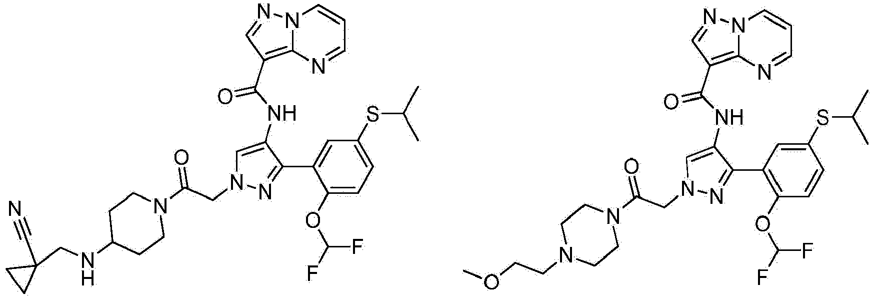 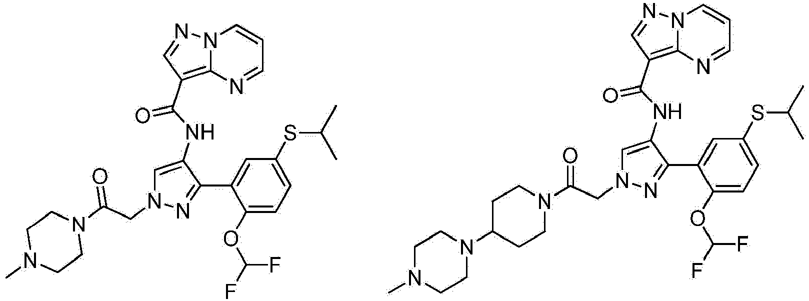 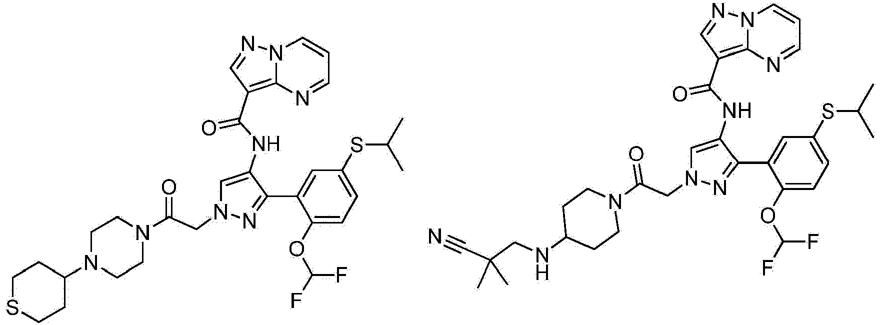 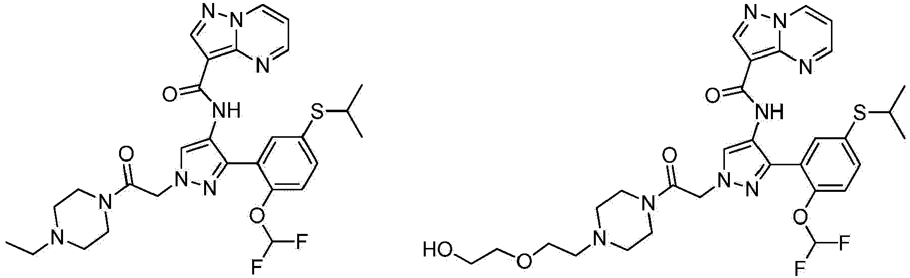 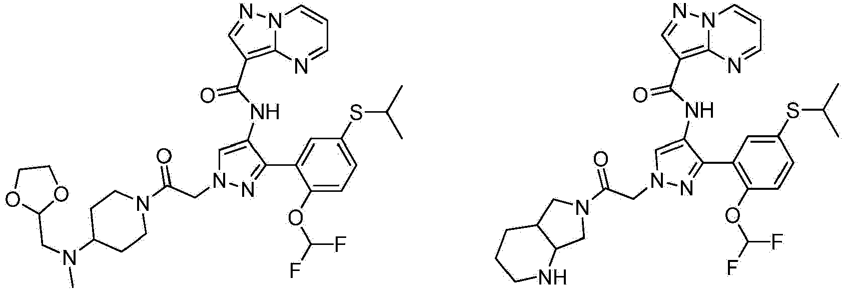 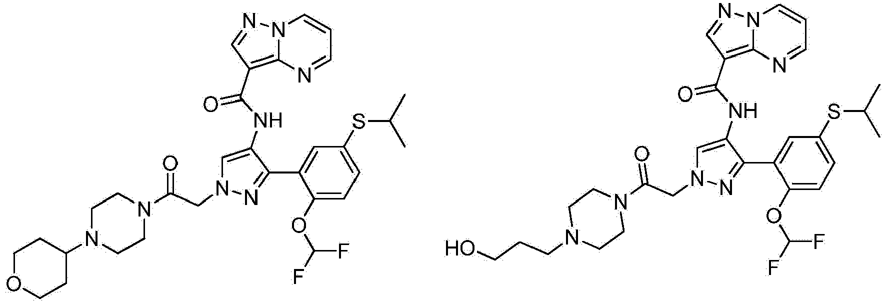 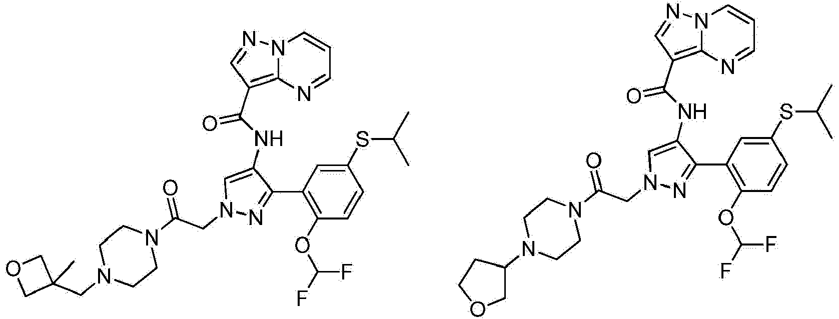 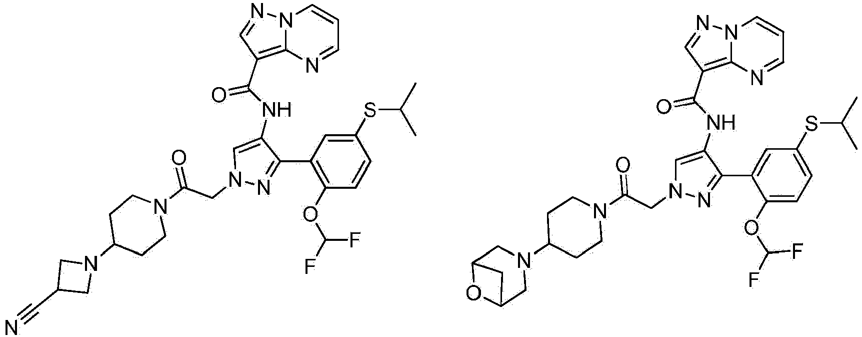 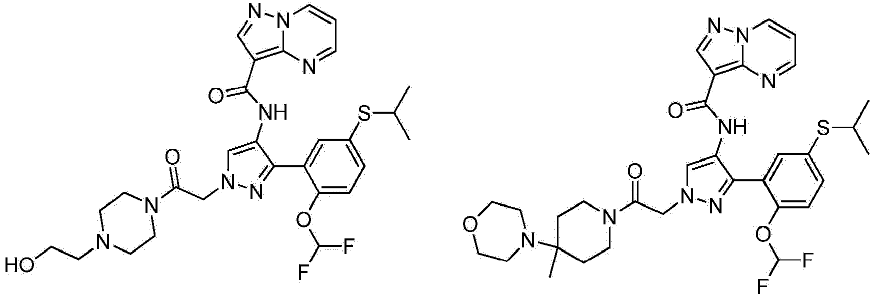 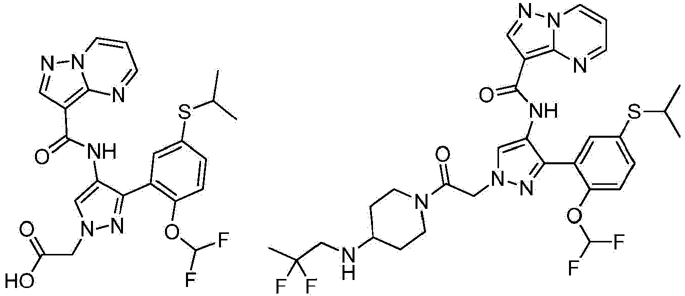 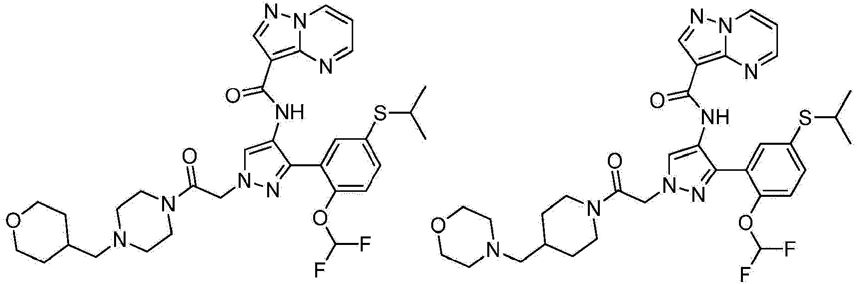 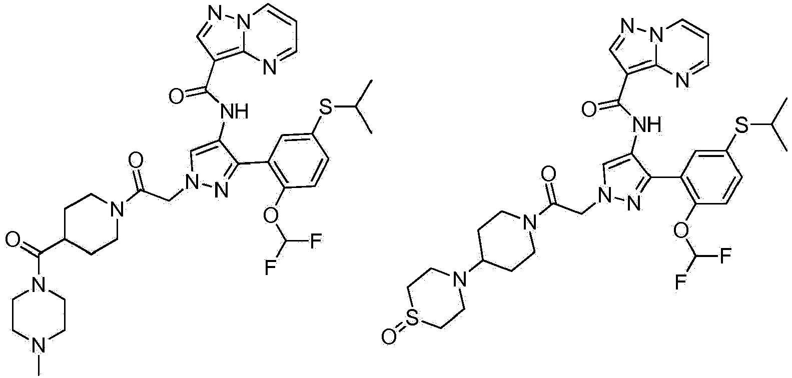 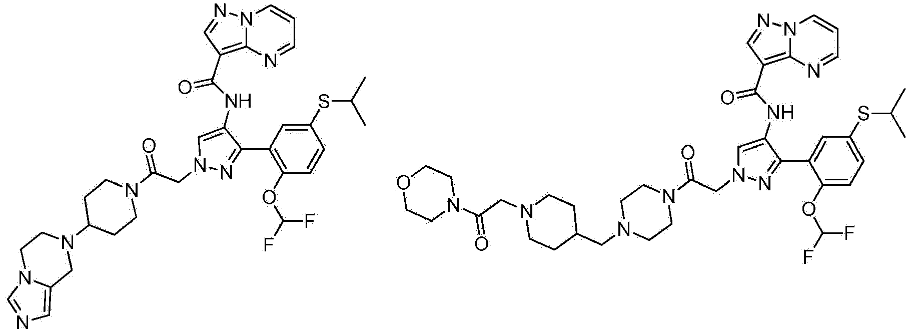 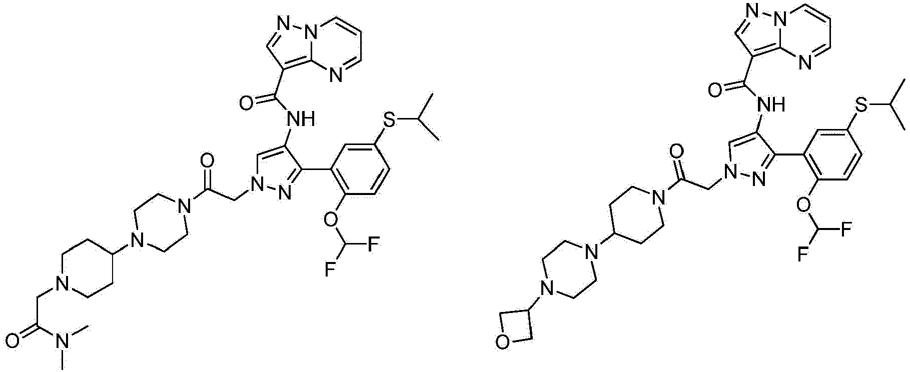 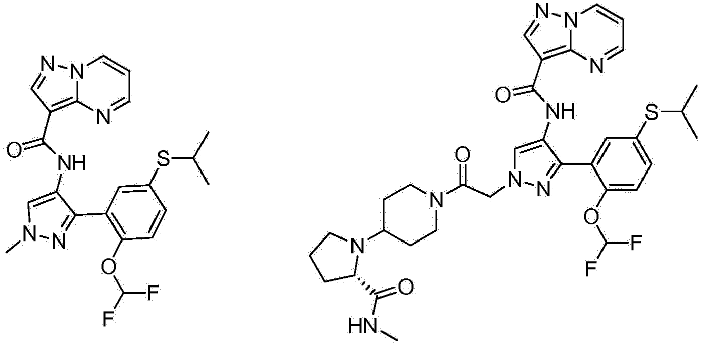 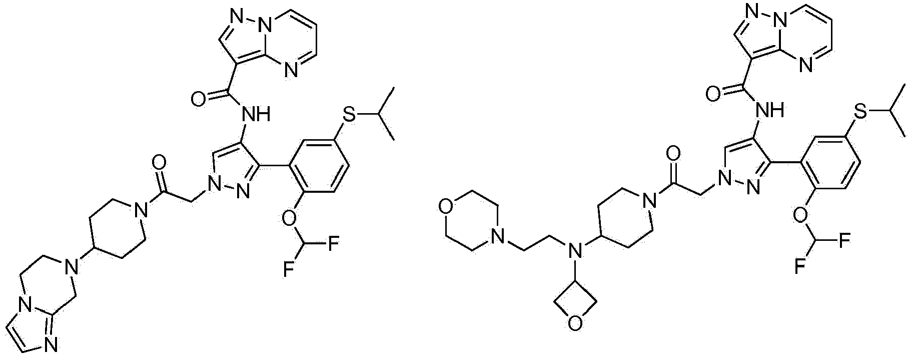 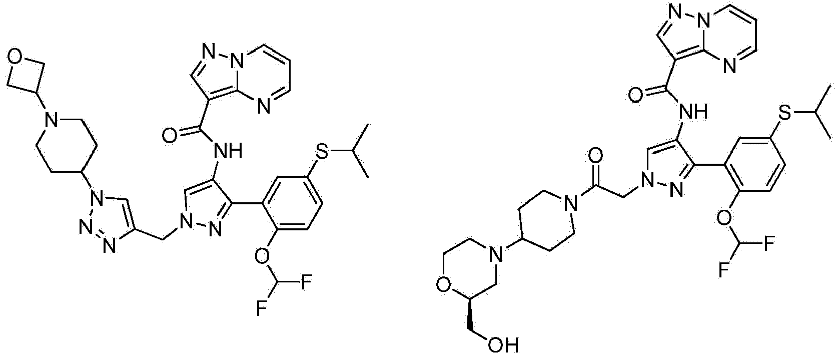 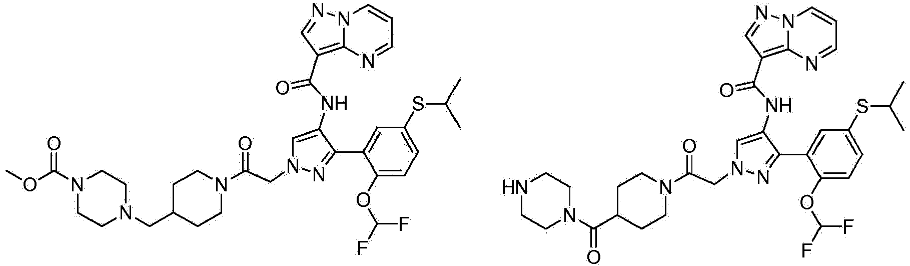 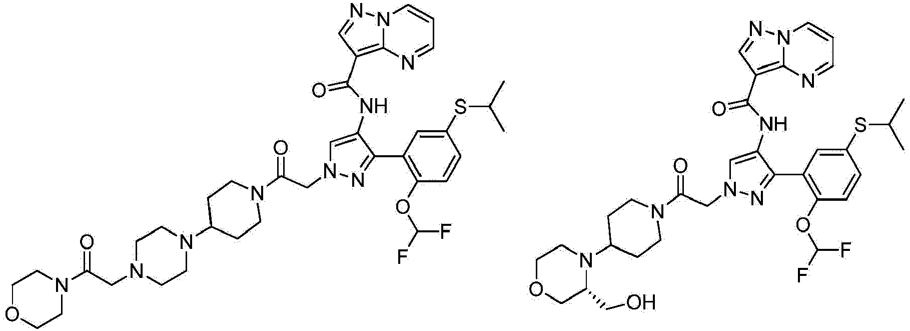 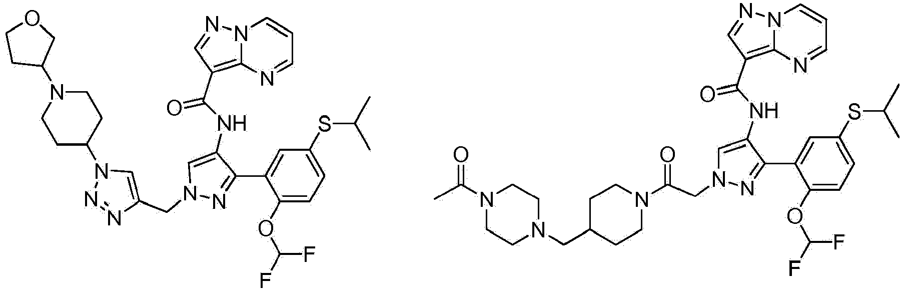 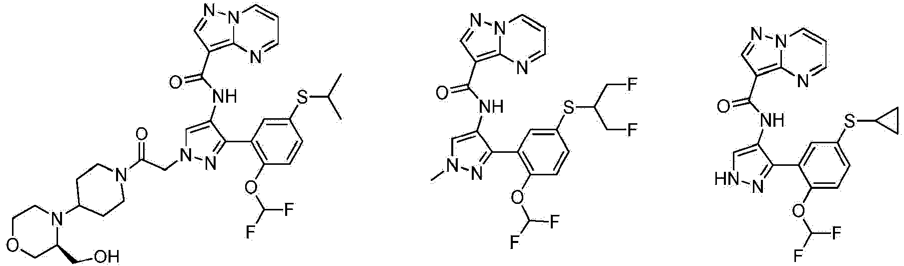 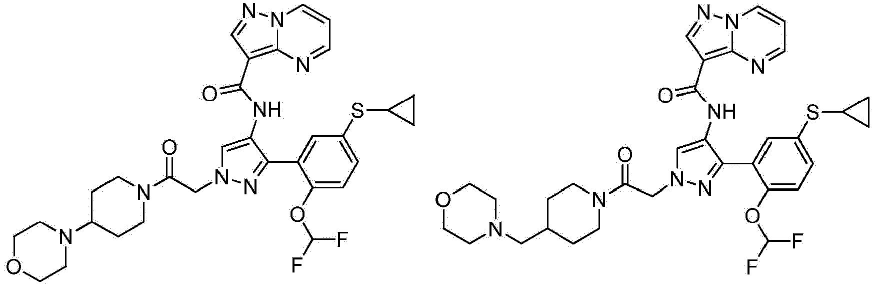 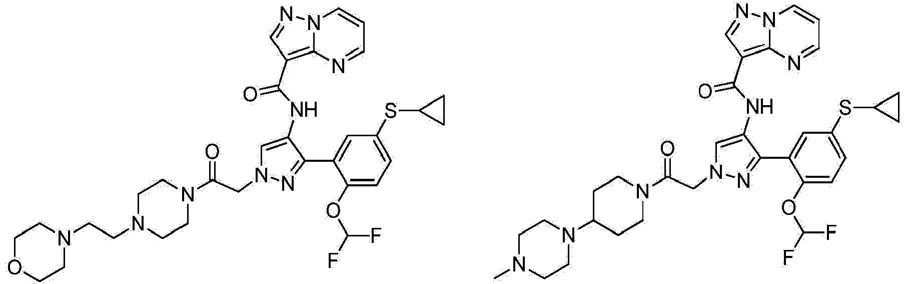 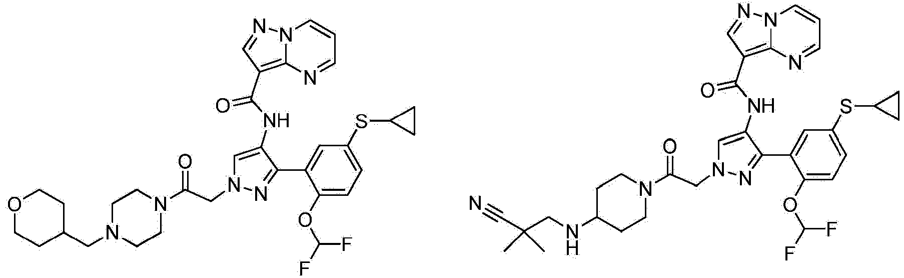 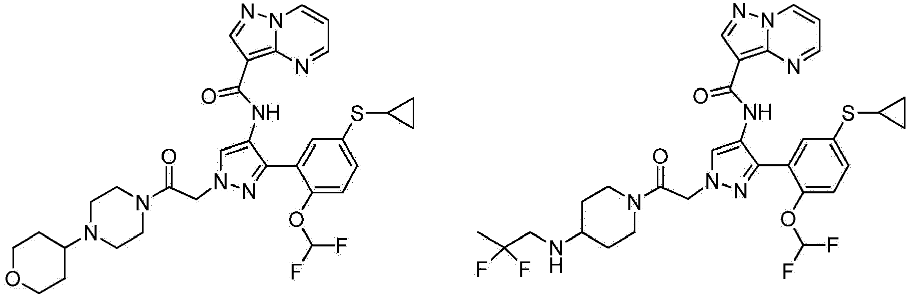 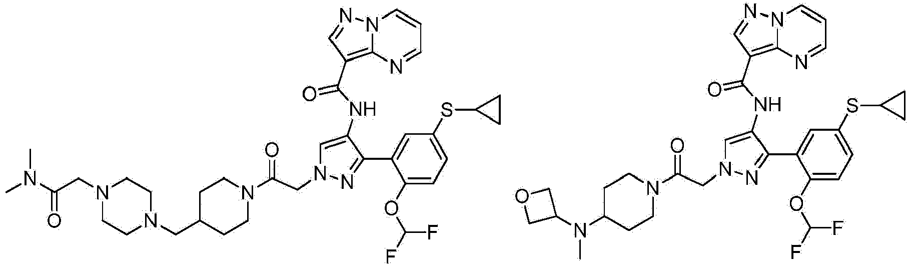 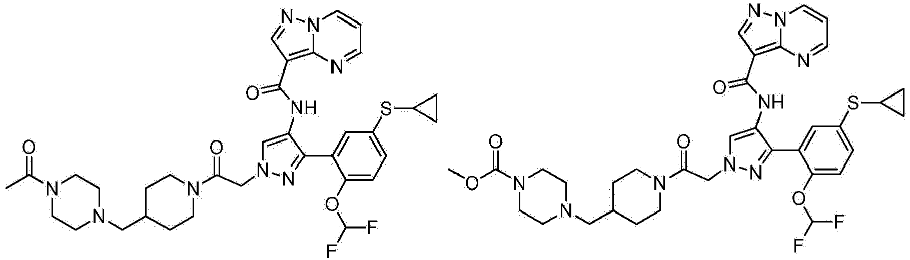 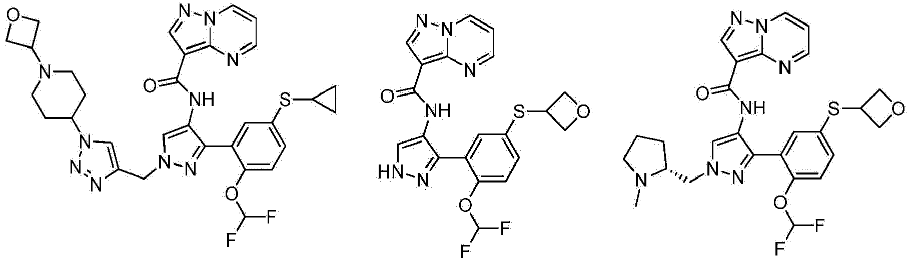 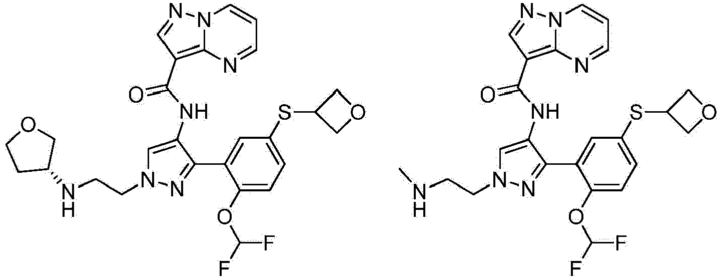 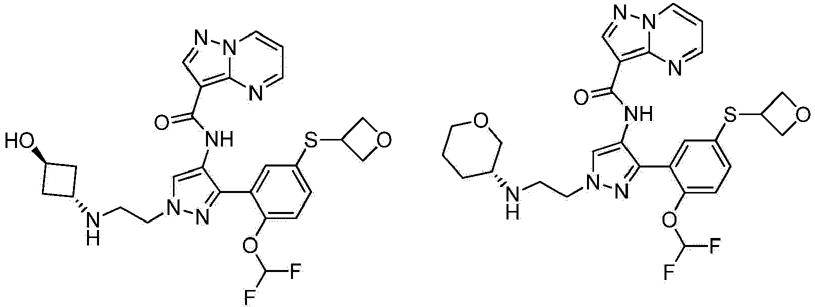 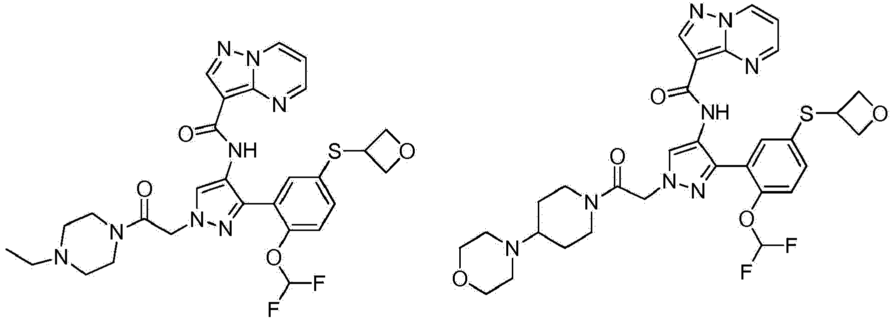 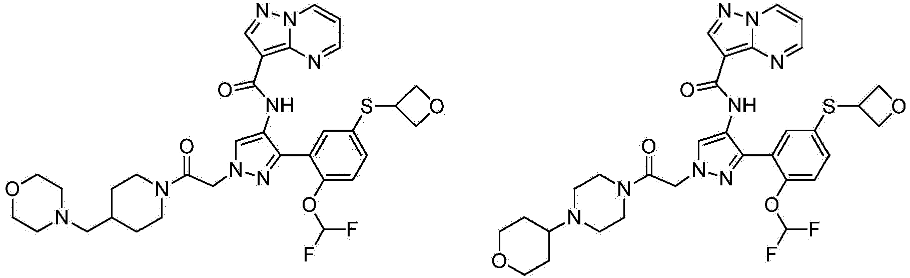 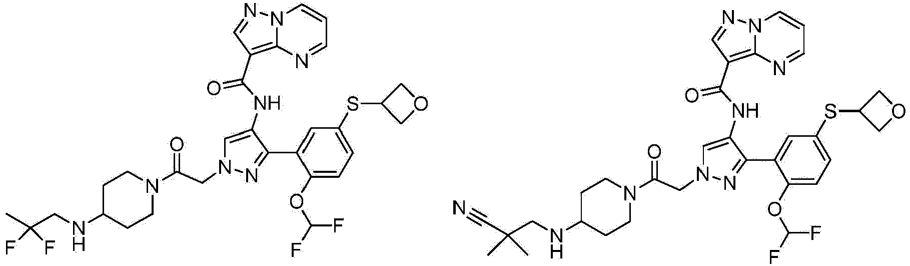 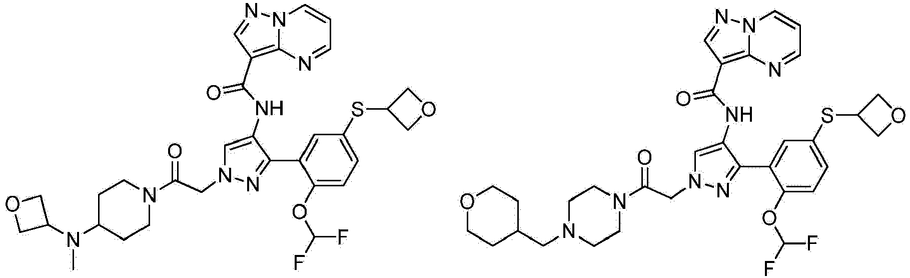 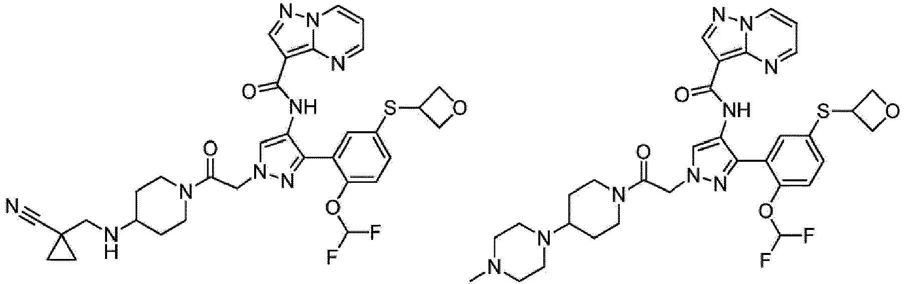 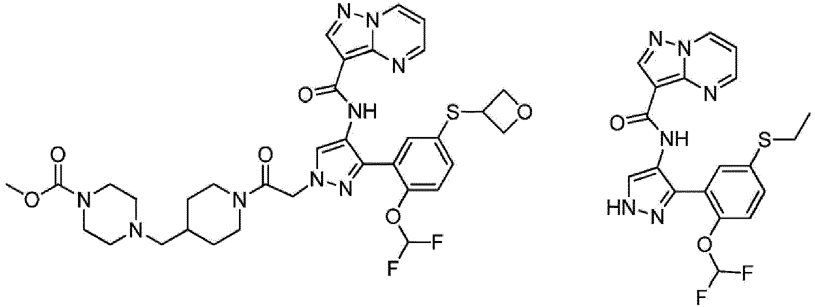 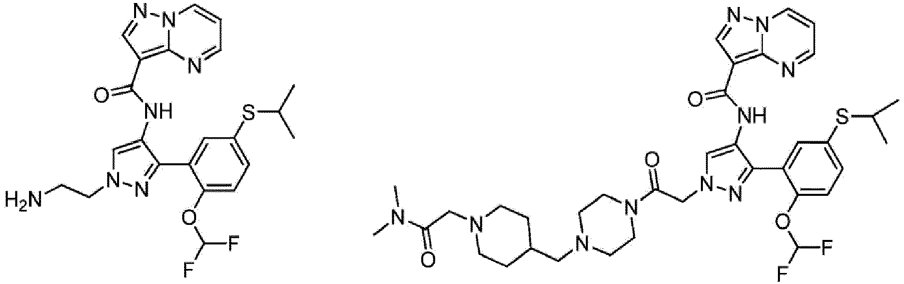 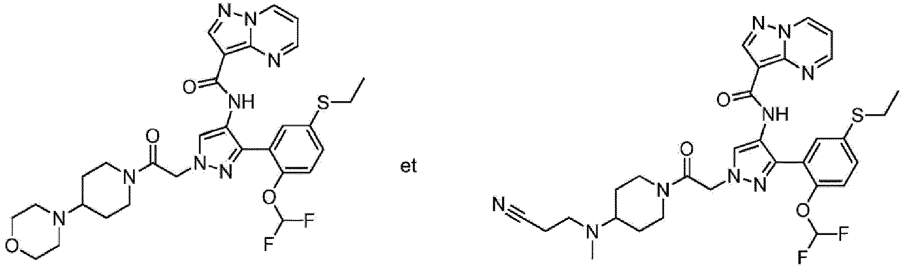 ou un de leurs sels.

15. Composé selon la revendication 1 choisi parmi : 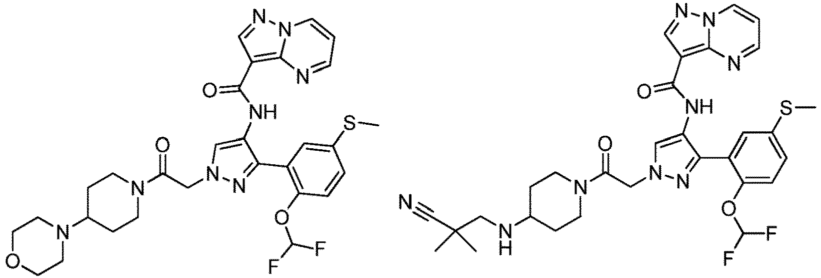 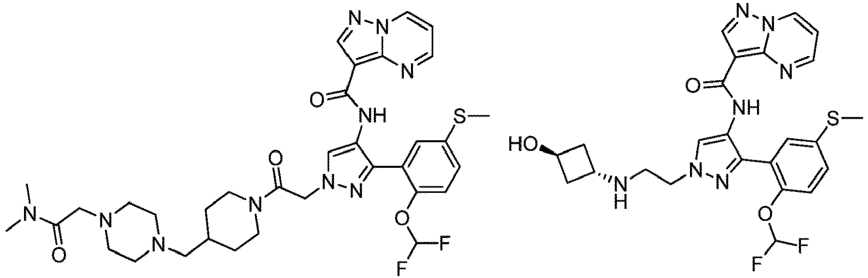 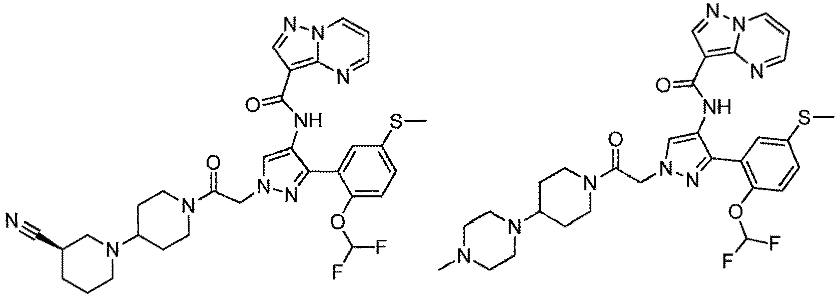 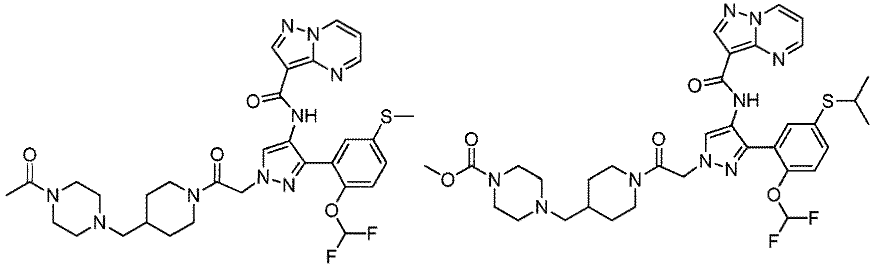 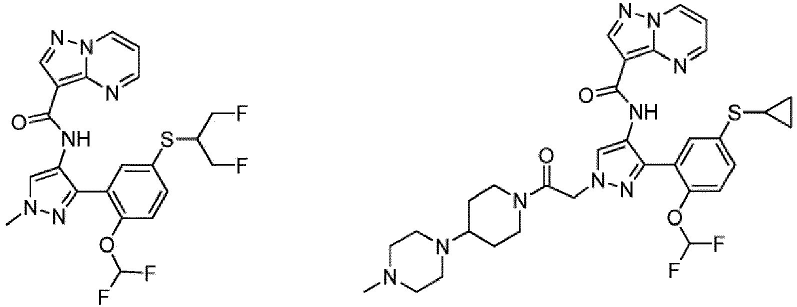 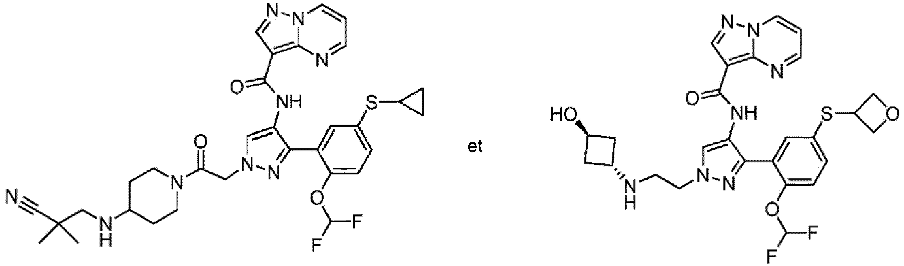 ou un de leurs sels.

16. Composition pharmaceutique comprenant un composé selon l'une quelconque des revendications 1 à 15 ou un de ses sels, et un véhicule, un diluant ou un excipient pharmaceutiquement acceptable.

17. Composé selon l'une quelconque des revendications 1 à 15 ou un de ses sels pharmaceutiquement acceptables, pour une utilisation dans le traitement d'une maladie inflammatoire.
